# EUROPEAN PATENT APPLICATION

(11) **EP 4 549 439 A1**
(43) Date of publication of application: **07.05.2025**
(21) Application number: 23830535.3
(22) Date of filing: 30.06.2023
(51) Int. Cl.: C07D 471/04, C07D 471/14, C07D 487/04, C07D 498/04, C07D 519/00, A61K 31/505, A61K 31/519, A61K 31/4745, A61P 35/00

(54) **SUBSTITUTED PYRIMIDINE-FUSED RING INHIBITOR, METHOD FOR PREPARING SAME, AND USE THEREOF**

(30) Priority: 01.07.2022 CN 202210774864; 24.08.2022 CN 202211020839; 27.03.2023 CN 202310306783
(71) Applicant: Suzhou Zelgen Biopharmaceutical Co., Ltd., Jiangsu 215300 (CN); Shanghai Zelgen Pharma.Tech Co., Ltd., Shanghai 201203 (CN)
(72) Inventor: LV, Binhua, Shanghai 201203 (CN); CUI, Dawei, Shanghai 201203 (CN)
(74) Representative: dompatent von Kreisler Selting Werner - Partnerschaft von Patent- und Rechtsanwälten mbB
(86) International application number: PCT/CN2023/105261
(87) International publication number: WO 2024/002373

(57) **Abstract**

The present invention relates to a substituted pyrimidine-fused ring inhibitor, a method for preparing same, and use thereof. Specifically, the compound of the present invention has a structure represented by formula (I). Further disclosed are a method for preparing the compound, and use of the compound as a KRAS mutation inhibitor. The compound has a good selective inhibition effect on KRAS mutation and has better pharmacodynamic and pharmacokinetic performance and lower toxic and side effects.

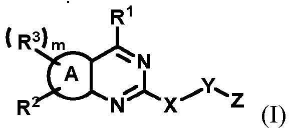

## Description

### TECHNICAL FIELD

The present disclosure relates to the field of pharmaceuticals, and particularly to a substituted pyrimidine-fused ring inhibitor, a method for preparing same, and use thereof.

### BACKGROUND

Approximately one-quarter of all human tumors are caused by RAS mutations, which result in nearly one million deaths each year. In the RAS family, KRAS mutations account for 85% of all RAS mutations. KRAS mutations have been found in nearly 90% of pancreatic cancers, 30-40% of colon cancers, and 15-20% of lung cancers (mainly non-small cell lung cancer).

KRAS protein is a small GTPase encoded by the KRAS gene and serves as an important regulatory protein for cell growth. Once KRAS is activated, it can activate multiple signaling pathways to promote cell proliferation. The most common mutation sites in the KRAS protein are codons 12, 13 and 61, with mutations at codon 12 being the most frequent. The most predominant mutations in KRAS G12 mutations are G12A, G12C, G12D, G12V, G12S, etc., among which mutations such as G12C, G12D, and G12V primarily occur in NSCLC, CRC, and pancreatic cancer. To date, no drug targeting multiple KRAS G12 mutations simultaneously has been approved for marketing.

Since KRAS mutation target proteins are pathologically associated with a variety of diseases, particularly lung cancer, pancreatic cancer, colorectal cancer, etc., there is currently a need for novel KRAS mutation inhibitors for clinical treatment. Highly selective and highly active KRAS mutation inhibitors have the potential to more effectively treat diseases such as cancers caused by KRAS mutations, while reducing off-target effects. Therefore, there is a more urgent clinical need for such inhibitors.

### SUMMARY

The present disclosure aims to provide a class of novel compounds with selective inhibitory effects on KRAS mutations and/or better pharmacodynamic performance, and use thereof.

In a first aspect of the present disclosure, provided is a compound of formula (I), or a stereoisomer, a tautomer, a crystalline form, a pharmaceutically acceptable salt, a hydrate, a solvate or a prodrug thereof: 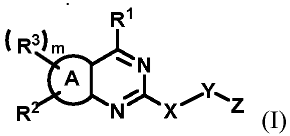 wherein in the formula, 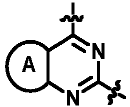 is selected from the group consisting of the following groups: 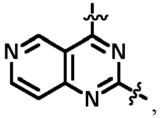 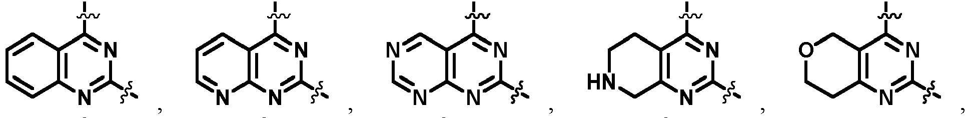 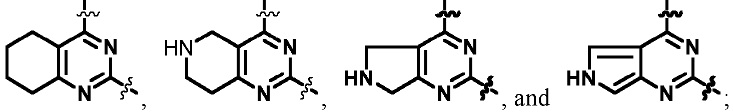 R¹ is selected from the group consisting of the following substituted or unsubstituted groups: C₁-C₁₀ alkyl, C₃-C₂₀ cycloalkyl, saturated or unsaturated 4- to 20-membered heterocyclyl, -NR^{a}R^{b}, and -OR^{a}, wherein the substitution refers to substitution with one or more R; with the proviso that R¹ is not substituted or unsubstituted 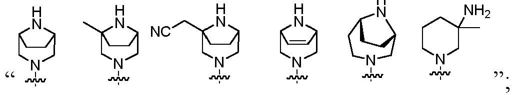 R^{a} and R^{b} are each independently selected from the group consisting of the following substituted or unsubstituted groups: hydrogen, deuterium, C₁-C₁₀ alkyl, C₃-C₂₀ cycloalkyl, and saturated or unsaturated 4- to 20-membered heterocyclyl, wherein the substitution refers to substitution with one or more R; preferably, R¹ is selected from the group consisting of the following substituted or unsubstituted groups: 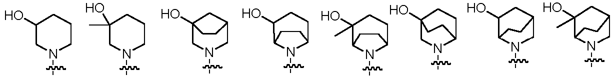 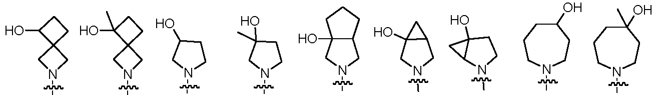 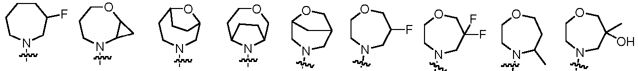 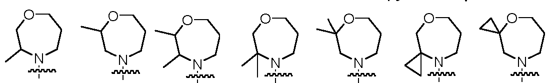 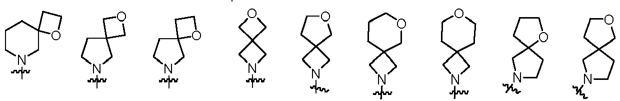 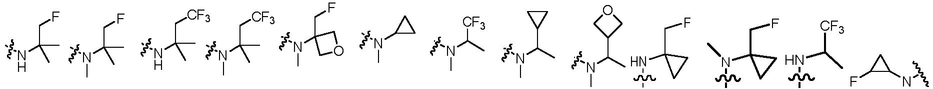 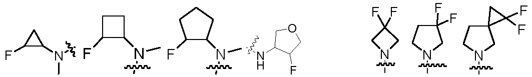 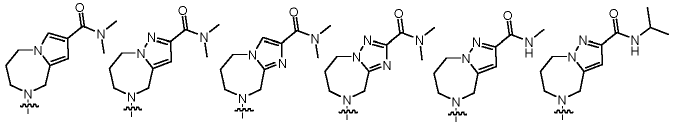 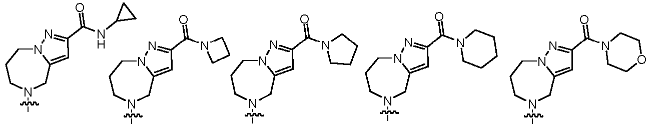 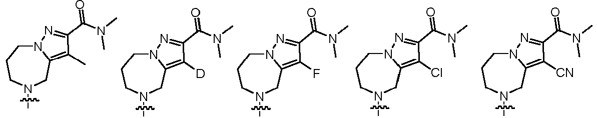 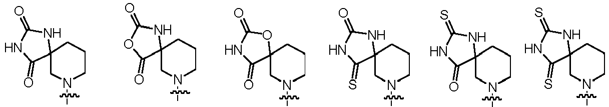 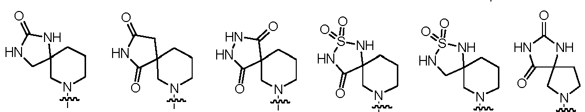 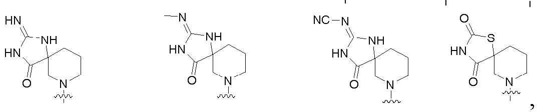
wherein the substitution refers to substitution with one or more R;
R² is selected from the group consisting of the following substituted or unsubstituted groups: C₆-C₁₄ aryl and 5- to 14-membered heteroaryl, wherein the substitution refers to substitution with one or more R;
each R³ is independently selected from the group consisting of the following substituted or unsubstituted groups: H, deuterium, halogen, cyano, ester group, amino, amido, sulfonyl, ureido, C₁-C₆ alkyl, C₁-C₆ deuterated alkyl, C₁-C₆ haloalkyl, C₃-C₆ cycloalkyl, 4- to 6-membered heterocyclyl, C₁-C₆ alkyloxy, C₃-C₆ cycloalkyloxy, and 4- to 6-membered heterocyclyloxy;
m is an integer of 0, 1, 2, 3, 4, 5, or 6 (m is preferably 0, 1, or 2);
X is selected from: a bond, O, NH, and N(C₁-C₃ alkyl) (X is preferably O);
Y is selected from: a bond and substituted or unsubstituted C₁-C₆ alkylene, wherein the substitution refers to substitution with one or more R (Y is preferably C₁-C₆ alkylene, more preferably methylene);
   1) Z is selected from: 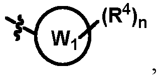
      wherein ring W₁ is selected from the group consisting of the following substituted or unsubstituted groups: C₃-C₂₀ cycloalkylene and saturated or unsaturated 4- to 20-membered heterocyclylene, wherein the substitution refers to substitution with one or more R;
      R⁴ is selected from: H and -L₁-Q₁-L₂-L₃, with the proviso that when W₁ is 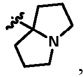 R⁴ is not H,
      wherein
         i) Q₁ is selected from: O, S, SO₂, NH, NR⁵, CONH, CONR⁵, SO₂NH, and SO₂NR⁵, wherein R⁵ is selected from the group consisting of the following substituted or unsubstituted groups: -C₁-C₆ alkyl, -C₃-C₆ cycloalkyl, -4- to 6-membered heterocyclyl, -C₆-C₁₀ aryl, and -5- to 10-membered heteroaryl, wherein the substitution refers to substitution with one or more R; and
            L₁ is selected from: none and substituted or unsubstituted C₁-C₆ alkylene, wherein the substitution refers to substitution with one or more R; and
            L₂ is selected from: none and substituted or unsubstituted C₁-C₆ alkylene, wherein the substitution refers to substitution with one or more R; and
            L₃ is selected from the group consisting of the following substituted or unsubstituted groups: - C₁-C₆ alkyl, C₁-C₆ alkoxy, -C₃-C₆ cycloalkyl, -4- to 10-membered heterocyclyl, -O-C₃-C₆ cycloalkyl, -O-4- to 6-membered heterocyclyl, -C₁-C₆ alkylene C₃-C₆ cycloalkyl, -C₁-C₆ alkylene 4- to 6-membered heterocyclyl, -C₁-C₆ alkylene-O-C₁-C₆ alkyl, -C₁-C₆ alkylene-O-C₃-C₆ cycloalkyl, -C₁-C₆ alkylene-O-4- to 6-membered heterocyclyl, NHR^{9'}, and NR^{9'}R^{10'}; R^{9'} and R^{10'} are each independently selected from the group consisting of the following substituted or unsubstituted groups: C₁-C₆ alkyl, C₃-C₁₀ cycloalkyl, 4- to 10-membered heterocyclyl, (C₃-C₁₀ cycloalkyl)C₁-C₆ alkyl, and (4- to 10-membered heterocyclyl)C₁-C₆ alkyl, or R^{9'} and R^{10'}, together with the N atom to which they are attached, form substituted or unsubstituted 4- to 10-membered heterocyclyl, wherein the substitution refers to substitution with one or more R;
               or
         ii) -L₁-Q₁ is selected from the group consisting of the following substituted or unsubstituted groups: -C₄-C₈ alkyl, -C₁-C₆ haloalkyl, -C₃-C₁₀ cycloalkyl, -4- to 10-membered heterocyclyl, - C₆-C₁₀ aryl, -5- to 10-membered heteroaryl, -C₁-C₃ alkylene-(C₃-C₁₀ cycloalkyl), -C₁-C₃ alkylene-(4- to 10-membered heterocyclyl), -C₁-C₃ alkylene-(C₆-C₁₀ aryl), and -C₁-C₃ alkylene-(5- to 10-membered heteroaryl), wherein the substitution refers to substitution with one or more R; and
            L₂ and L₃ are both absent;
            when X is a bond, n is selected from: integers of 0, 1, 2, 3, 4, 5, and 6; when X is not a bond, n is selected from: integers of 1, 2, 3, 4, 5, and 6;
               or
   2) Z is selected from: 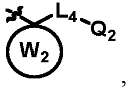 with the proviso that when 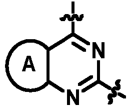 is 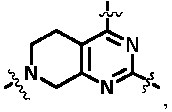 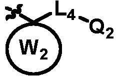 is not 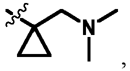
      wherein ring W₂ is selected from the group consisting of the following substituted or unsubstituted groups: C₃-C₆ cycloalkylene and saturated or unsaturated 4- to 6-membered heterocyclylene, wherein the substitution refers to substitution with one or more R;
      L₄ is selected from the group consisting of the following substituted or unsubstituted groups: a bond, C₁-C₆ alkylene, and deuterated C₁-C₆ alkylene, wherein the substitution refers to substitution with one or more R;
      Q₂ is selected from the group consisting of the following substituted or unsubstituted groups: C₁-C₆ alkoxy, C₃-C₁₀ cycloalkyloxy, 4- to 10-membered heterocyclyloxy, C₃-C₁₀ cycloalkyl C₁-C₆ alkyleneoxy, 4- to 10-membered heterocyclyl C₁-C₆ alkyleneoxy, C₃-C₁₀ cycloalkyl, 4- to 10-membered heterocyclyl, NHR⁹, and NR⁹R¹⁰; R⁹ and R¹⁰ are each independently selected from the group consisting of the following substituted or unsubstituted groups: C₁-C₆ alkyl, C₃-C₁₀ cycloalkyl, 4- to 10-membered heterocyclyl, (C₃-C₁₀ cycloalkyl)C₁-C₆ alkyl, and (4- to 10-membered heterocyclyl)C₁-C₆ alkyl, or R⁹ and R¹⁰, together with the N atom to which they are attached, form substituted or unsubstituted 4- to 10-membered heterocyclyl, wherein the substitution refers to substitution with one or more R;
      each R is identical or different and is independently selected from: H, deuterium, vinyl, ethynyl, C₁-C₆ alkyl, deuterated C₁-C₆ alkyl, halogenated C₁-C₆ alkyl, (C₃-C₆ cycloalkyl)C₁-C₆ alkyl, (4-to 6-membered heterocyclyl)C₁-C₆ alkyl, (C₁-C₆ alkoxy)C₁-C₆ alkyl, (C₃-C₆ cycloalkyloxy)C₁-C₆ alkyl, (4- to 6-membered heterocyclyloxy)C₁-C₆ alkyl, (C₁-C₆ alkyl)vinyl, deuterated (C₁-C₆ alkyl)vinyl, halogenated (C₁-C₆ alkyl)vinyl, (C₁-C₆ alkyl)ethynyl, deuterated (C₁-C₆ alkyl)ethynyl, halogenated (C₁-C₆ alkyl)ethynyl, (C₃-C₆ cycloalkyl)ethynyl, (4- to 6-membered heterocyclyl)ethynyl, C₁-C₆ alkoxy, deuterated C₁-C₆ alkoxy, halogenated C₁-C₆ alkoxy, C₃-C₆ cycloalkyloxy, 4- to 6-membered heterocyclyloxy, C₃-C₆ cycloalkyl, 4- to 6-membered heterocyclyl, C₆-C₁₀ aryl, 5- to 10-membered heteroaryl, halogen, nitro, hydroxy, oxo (=O), cyano, ester group, amino, amido, sulfonyl, and ureido.

In another preferred embodiment, R¹ is selected from the group consisting of the following substituted or unsubstituted groups: C₃-C₂₀ cycloalkyl and saturated or unsaturated 4- to 20-membered heterocyclyl, wherein the substitution refers to substitution with one or more R; with the proviso that R¹ is not substituted or unsubstituted "".

In another preferred embodiment, each R is identical or different and is independently selected from: H, deuterium, vinyl, ethynyl, C₁-C₆ alkyl, deuterated C₁-C₆ alkyl, halogenated C₁-C₆ alkyl, (C₃-C₆ cycloalkyl)C₁-C₆ alkyl, (4- to 6-membered heterocyclyl)C₁-C₆ alkyl, (C₁-C₆ alkoxy)C₁-C₆ alkyl, (C₃-C₆ cycloalkyloxy)C₁-C₆ alkyl, (4- to 6-membered heterocyclyloxy)C₁-C₆ alkyl, (C₁-C₆ alkyl)vinyl, deuterated (C₁-C₆ alkyl)vinyl, halogenated (C₁-C₆ alkyl)vinyl, (C₁-C₆ alkyl)ethynyl, deuterated (C₁-C₆ alkyl)ethynyl, halogenated (C₁-C₆ alkyl)ethynyl, (C₃-C₆ cycloalkyl)ethynyl, (4- to 6-membered heterocyclyl)ethynyl, C₁-C₆ alkoxy, deuterated C₁-C₆ alkoxy, halogenated C₁-C₆ alkoxy, C₃-C₆ cycloalkyloxy, 4- to 6-membered heterocyclyloxy, C₃-C₆ cycloalkyl, 4- to 6-membered heterocyclyl, C₆-C₁₀ aryl, 5- to 10-membered heteroaryl, halogen, nitro, hydroxy, oxo (=O), cyano, ester group, amino, amido, sulfonyl, ureido, sulfonamido (e.g., SO₂NH₂), C₃-C₆ cycloalkyl O-, 4- to 6-membered heterocyclyl O-, C₃-C₆ cycloalkyl OH, and 4- to 6-membered heterocyclyl OH.

In another preferred embodiment, 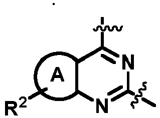 is selected from the group consisting of the following groups: 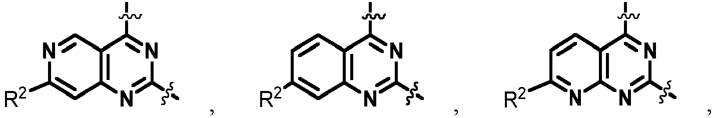 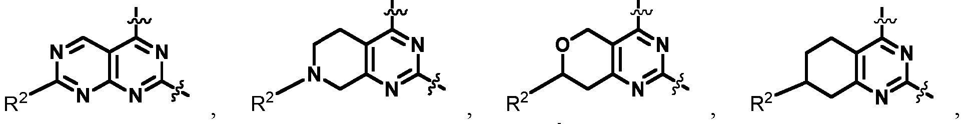 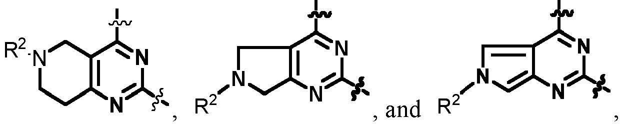 wherein R² is as defined above.

In another preferred embodiment, the compound has a structure represented by formula (II-A) or formula (II-B): 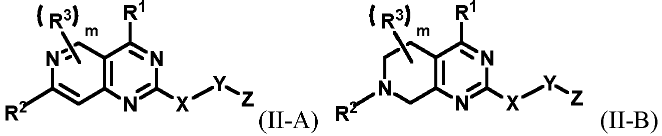 wherein in the formula,
R¹, R², R³, X, Y, Z, and m are as defined above.

In another preferred embodiment, 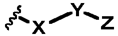 is 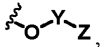 wherein Z is as defined above, and preferably, 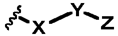 is selected from:

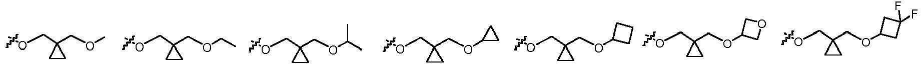

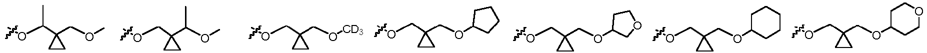

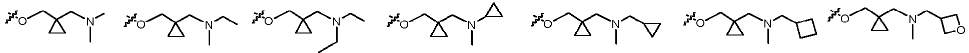

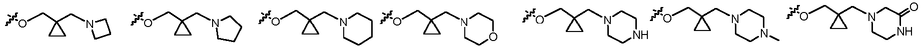

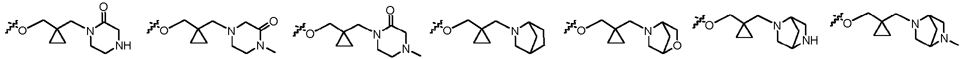

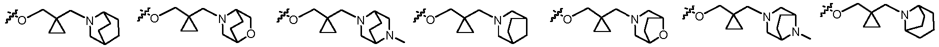

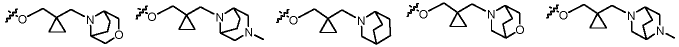

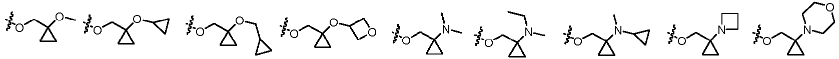

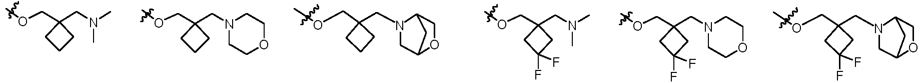

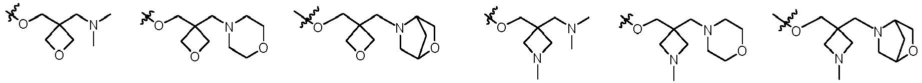

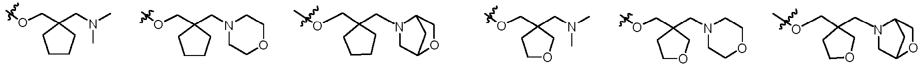

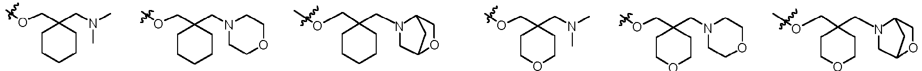

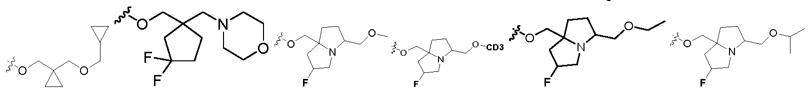

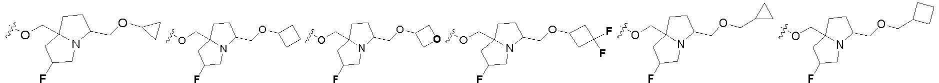

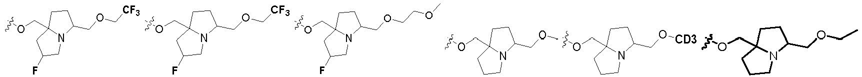

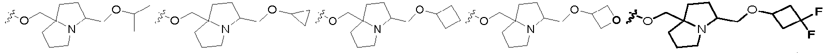

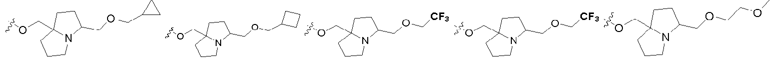

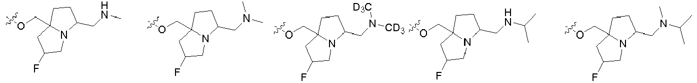

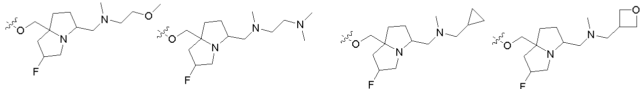

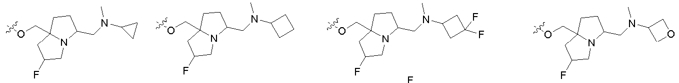

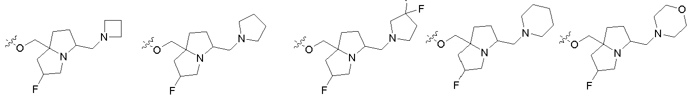

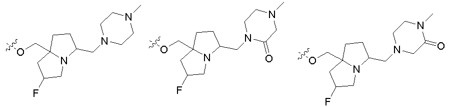

In another preferred embodiment, the compound has a structure represented by formula (III-A1), formula (III-A2), formula (III-B1), or formula (III-B2): 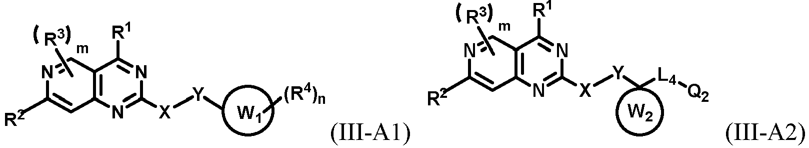 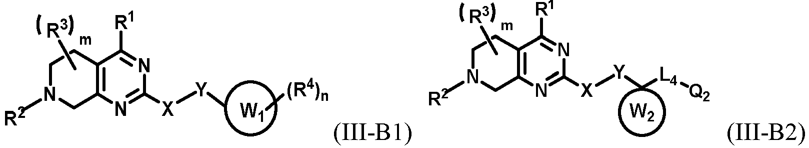 wherein in the formula,
R¹, R², R³, R⁴, X, Y, W₁, W₂, L₄, Q₂, m, and n are as defined above.

In another preferred embodiment, the compound has a structure represented by formula (IV-A1), formula (IV-A2), formula (IV-B1), or formula (IV-B2): 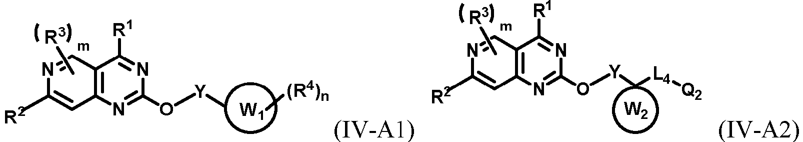 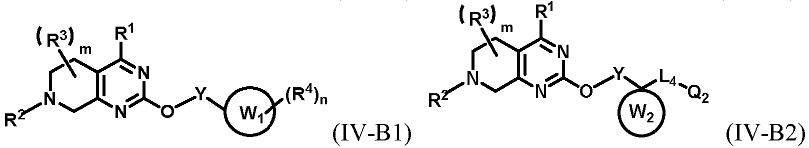 wherein in the formula,
R¹, R², R³, Y, W₁, W₂, L₄, Q₂, m, and n are as defined above.

In another preferred embodiment, W₁ is substituted or unsubstituted 7- to 10-membered bicyclic heterocyclyl or substituted or unsubstituted C₇-C₁₀ bicyclic cycloalkyl, and preferably, W₁ is substituted or unsubstituted 8-membered N-containing heterocyclyl, wherein the substitution refers to substitution with one or more R, and R is as defined above.

In another preferred embodiment, W₁ is substituted or unsubstituted 7- to 10-membered bicyclic heterocyclyl, substituted or unsubstituted C₇-C₁₀ bicyclic cycloalkyl, substituted or unsubstituted 7- to 12-membered tricyclic heterocyclyl, or substituted or unsubstituted C₇-C₁₂ tricyclic cycloalkyl, and preferably, W₁ is substituted or unsubstituted 8- to 12-membered (e.g., 8-, 9-, 10- or 11-membered) N-containing heterocyclyl, wherein the substitution refers to substitution with one or more R, and R is as defined above.

In another preferred embodiment, 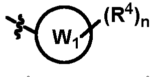 is selected from: 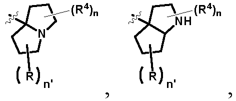 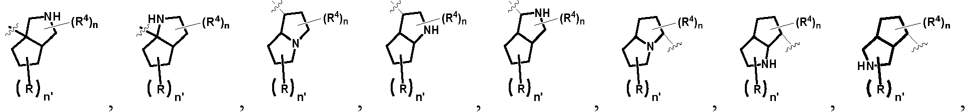 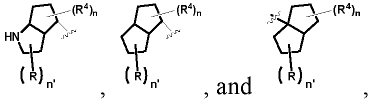 or is selected from: 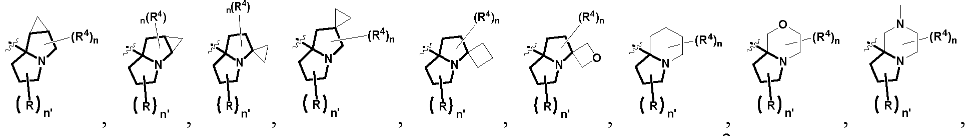 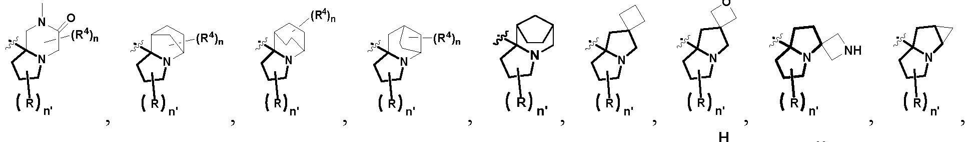 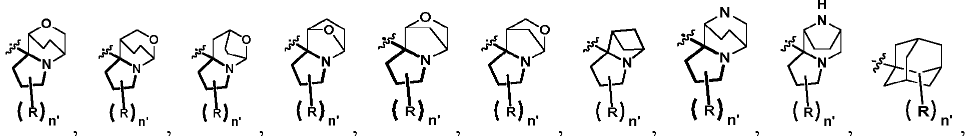 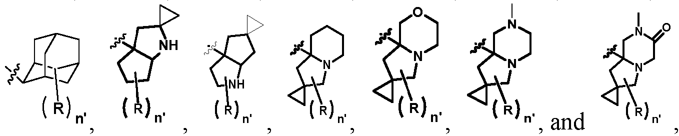 wherein n' is an integer of 0, 1, 2, 3, 4, 5, or 6; R, R⁴, and n are as defined above, and substitution with R or R⁴ may occur on any one of the rings of the polycyclic group (e.g., bridged or spiro cyclic group); more preferably, 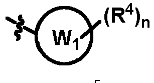 is selected from: 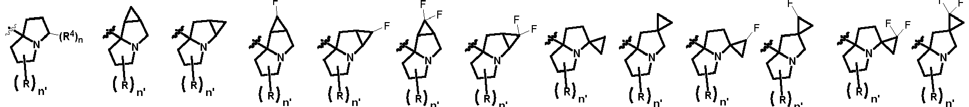 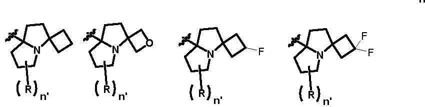 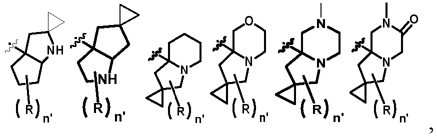 wherein n' and R are as defined above, and substitution with R may occur on any one of the rings of the polycyclic group (e.g., bridged or spiro cyclic group).

In another preferred embodiment, 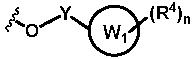 is

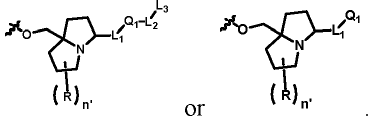

In another preferred embodiment, the compound has a structure represented by formula V:

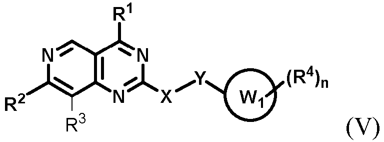

Preferably, the compound has a structure represented by formula VI: 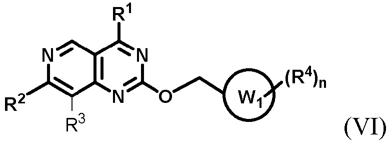 wherein R¹, R², R³, R⁴, X, Y, W₁, and n are as defined above.

In another preferred embodiment, R³ is selected from: H, halogen, CN, nitro, OH, amino, C1-C6 alkyl, C1-C6 alkoxy, halogenated C1-C6 alkyl, and halogenated C1-C6 alkoxy; preferably, R³ is selected from: H, F, methoxy, methyl, trifluoromethyl, and trifluoromethoxy.

In another preferred embodiment, 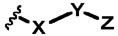 is 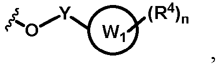 preferably

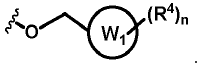

In another preferred embodiment, 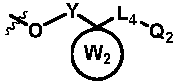 is selected from:

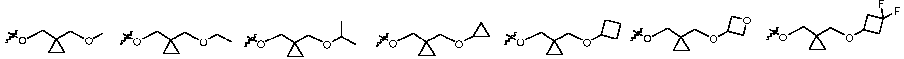

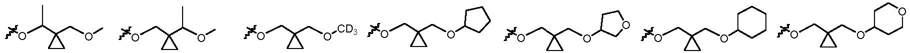

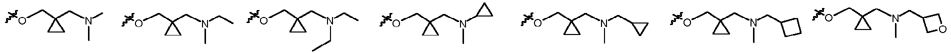

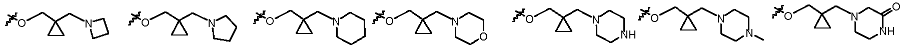

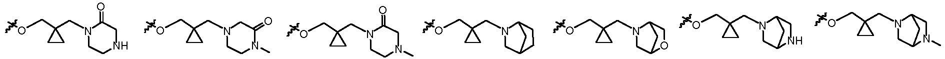

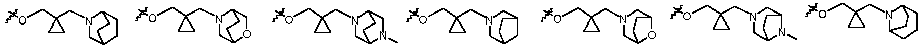

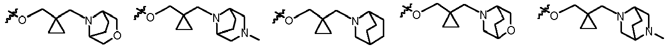

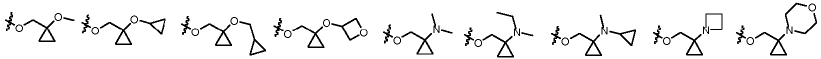

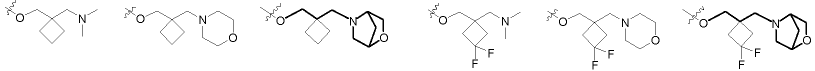

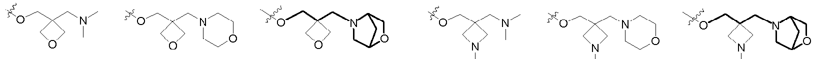

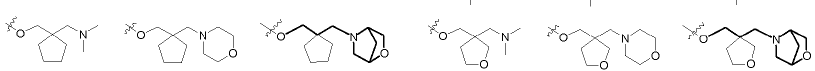

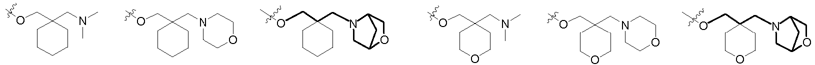

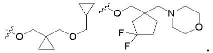

In another preferred embodiment, the compound has a structure represented by formula (V-A1) or formula (V-B1): 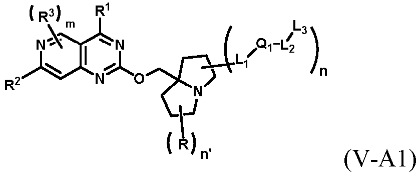 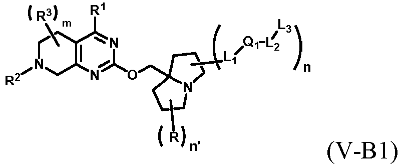 wherein in the formula,
n' is an integer of 0, 1, 2, 3, 4, 5, or 6;
R¹, R², R³, R, L₁, L₂, L₃, Q₁, m, n, and n' are as defined above.

In another preferred embodiment, 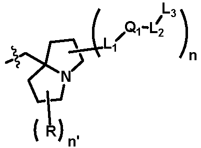 is

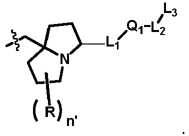

In another preferred embodiment, -L₁-Q₁-L₂-L₃ is selected from the group consisting of the following substituted or unsubstituted groups: -C₁-C₆ alkoxy, -C₃-C₁₀ cycloalkyloxy, -4- to 10-membered heterocyclyloxy, (C₃-C₁₀ cycloalkyl)C₁-C₆ alkyleneoxy, (4- to 10-membered heterocyclyl)C₁-C₆ alkyleneoxy, -O-C₁-C₆ alkylene-O-(C₁-C₆ alkyl), -C₁-C₆ alkylene-O-(C₁-C₆ alkylene(C₃-C₁₀ cycloalkyl)), -C₁-C₆ alkylene-O-(C₁-C₆ alkylene(4- to 10-membered heterocyclyl)), -O-C₁-C₆ alkylene-NHR^{9"}, -O-C₁-C₆ alkylene-NR^{9"}R^{10"}, -C₁-C₆ alkylene-O-(C₁-C₆ alkylene NHR^{9"}), -C₁-C₆ alkylene-O-(C₁-C₆ alkylene NR^{9"}R^{10"}), -NRn-(C₁-C₆ alkylene NHR^{9"}), -NRn-(C₁-C₆ alkylene NR^{9"}R^{10"}), -C₁-C₆ alkylene NHR^{9"}, -C₁-C₆ alkylene NR^{9"}R^{10"}, NHR^{9"}, and NR^{9"}R^{10"}; Rn is selected from: H or substituted or unsubstituted C1-C6 alkyl; R^{9"} and R^{10"} are each independently selected from the group consisting of the following substituted or unsubstituted groups: C₁-C₆ alkyl, C₃-C₁₀ cycloalkyl, 4- to 10-membered heterocyclyl, (C₃-C₁₀ cycloalkyl)C₁-C₆ alkyl, and (4- to 10-membered heterocyclyl)C₁-C₆ alkyl, or R^{9"} and R^{10"}, together with the N atom to which they are attached, form substituted or unsubstituted 4- to 10-membered heterocyclyl, wherein the substitution refers to substitution with one or more R.

In another preferred embodiment, -L₁-Q₁-L₂-L₃ is selected from the group consisting of the following substituted or unsubstituted groups: -C₄-C₈ alkyl, -C₁-C₆ haloalkyl, -C₃-C₁₀ cycloalkyl, -4- to 10-membered heterocyclyl, -C₆-C₁₀ aryl, -5- to 10-membered heteroaryl, -C₁-C₃ alkylene-(C₃-C₁₀ cycloalkyl), -C₁-C₃ alkylene-(4- to 10-membered heterocyclyl), -C₁-C₃ alkylene-(C₆-C₁₀ aryl), and -C₁-C₃ alkylene-(5- to 10-membered heteroaryl), wherein the substitution refers to substitution with one or more R.

In another preferred embodiment, -L₁-Q₁-L₂-L₃ is selected from: 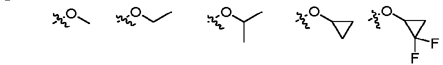 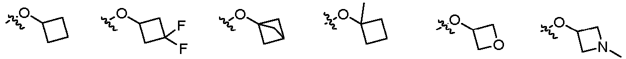 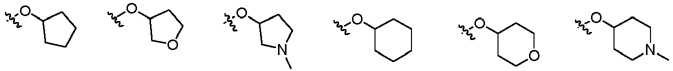 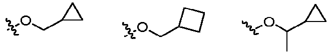 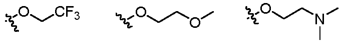 or is selected from: 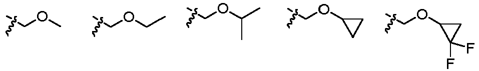 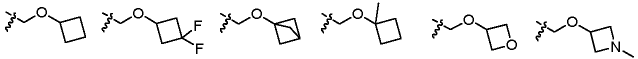 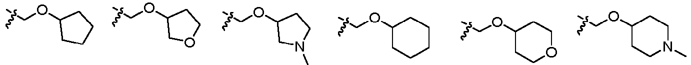 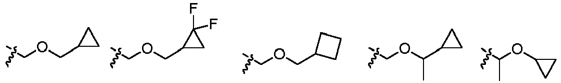 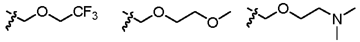 or is selected from: 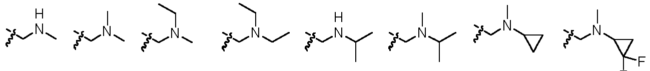 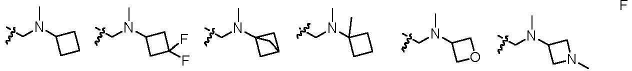 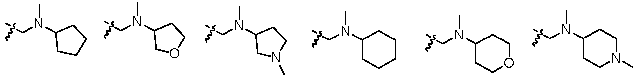 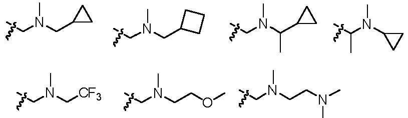 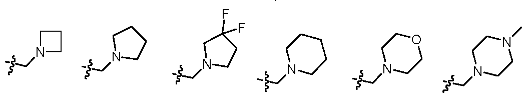 or is selected from: 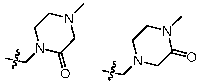 or is selected from:

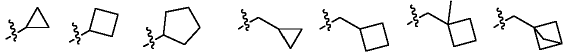

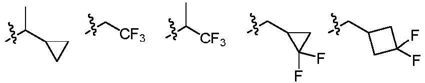

In another preferred embodiment, 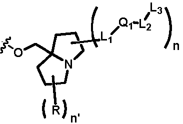 is selected from: 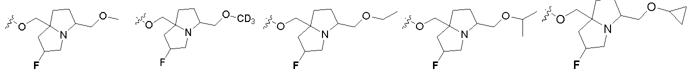 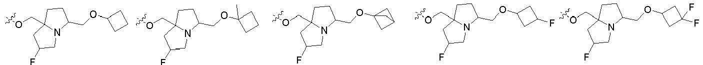 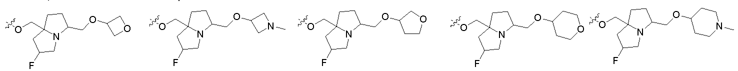 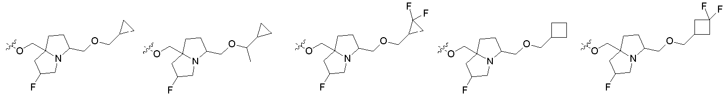 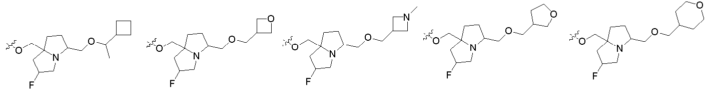 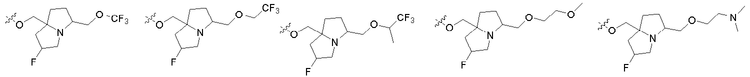 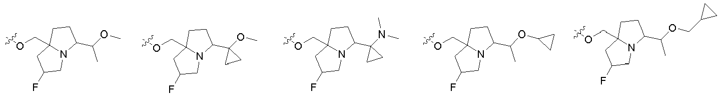 or is selected from: 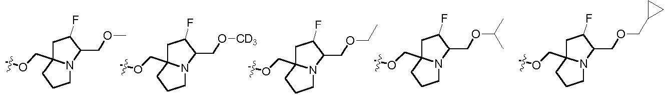 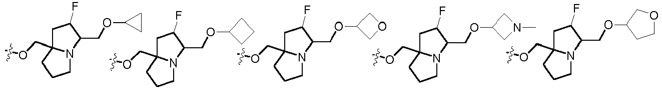 or is selected from: 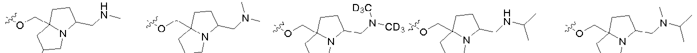 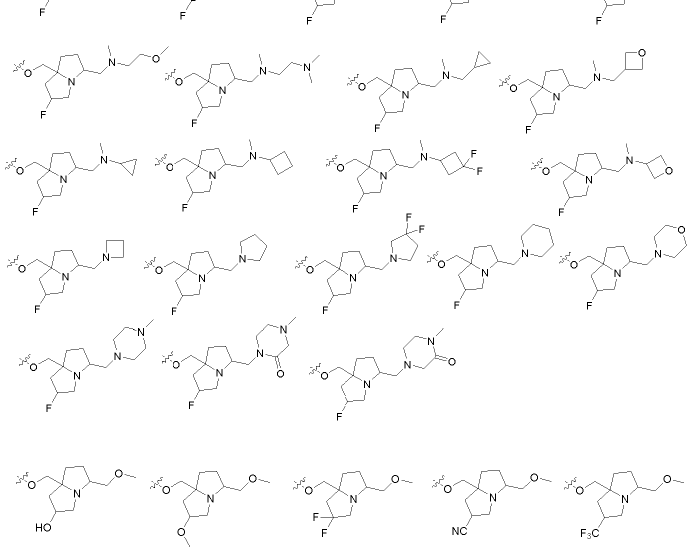 or is selected from: 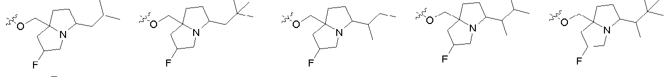 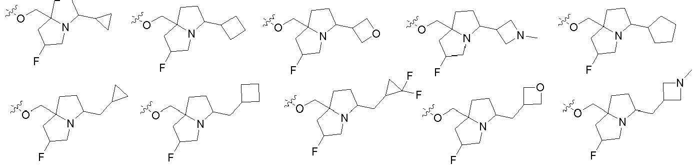 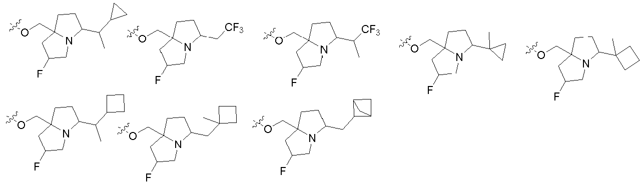 or is selected from:

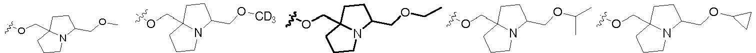

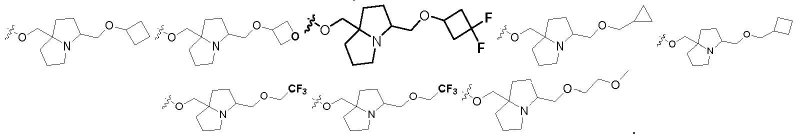

In another preferred embodiment, R² is selected from the group consisting of the following substituted or unsubstituted groups: C₆-C₁₀ aryl and 5- to 10-membered heteroaryl, wherein the substitution refers to substitution with one or more R; R is selected from: halogen, hydroxy, cyano, NH₂, C₁-C₆ alkyl, halogenated C₁-C₆ alkyl, C₁-C₆ alkoxy, halogenated C₁-C₆ alkoxy, C₃-C₆ cycloalkyl, 4- to 6-membered heterocyclyl (e.g., 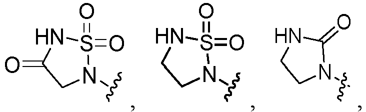 and 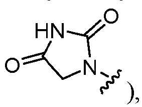 C₂-C₆ alkenyl, C₂-C₆ alkynyl, ester group (e.g., COC1-C6 alkyl), amino (e.g., NH₂ and C1-C6 alkyl NH₂), amido (e.g., CONH₂, CONHC1-C6 alkyl, CONHCH₃, and CONH-C3-C6 cycloalkyl), sulfonyl (e.g., SO₂C1-C6 alkyl), ureido, sulfonamido (e.g., SO₂NH₂), C₃-C₆ cycloalkyl O-, 4- to 6-membered heterocyclyl O-, C₃-C₆ cycloalkyl OH, and 4- to 6-membered heterocyclyl OH.

In another preferred embodiment, 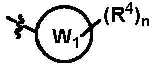 is selected from 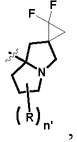 preferably 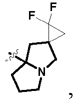 more preferably

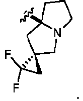

In another preferred embodiment, R² is selected from the group consisting of the following substituted or unsubstituted groups: phenyl, naphthyl, 5- to 6-membered monocyclic heteroaryl (e.g., pyridyl), 9- to 10-membered bicyclic heteroaryl (e.g., indazolyl, benzothiazole, benzothiophene, and benzofuran), wherein the substitution is substitution with one or more groups selected from the group consisting of: halogen, hydroxy, cyano, NH₂, C₁-C₆ alkyl, halogenated C₁-C₆ alkyl, C₁-C₆ alkoxy, halogenated C₁-C₆ alkoxy, C₃-C₆ cycloalkyl, 4- to 6-membered heterocyclyl (e.g., 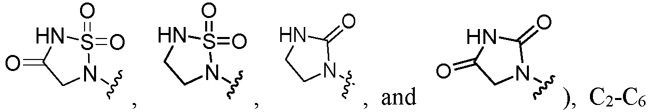 alkenyl, C₂-C₆ alkynyl, ester group (e.g., COC1-C6 alkyl), amino (e.g., NH₂ and C1-C6 alkyl NH₂), amido (e.g., CONH₂, CONHC1-C6 alkyl, CONHCH₃, and CONH-C3-C6 cycloalkyl), sulfonyl (e.g., SO₂C1-C6 alkyl), ureido, sulfonamido (e.g., SO₂NH₂), C₃-C₆ cycloalkyl O-, 4- to 6-membered heterocyclyl O-, C₃-C₆ cycloalkyl OH, and 4- to 6-membered heterocyclyl OH, and preferably, R² is selected from:

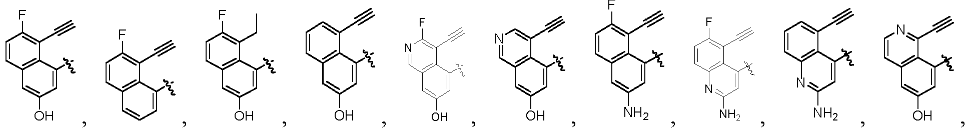

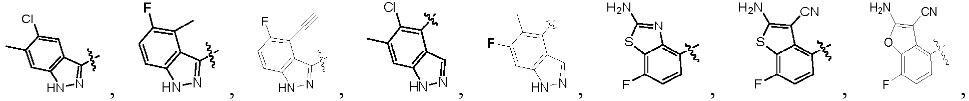

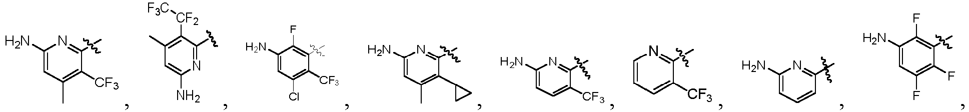

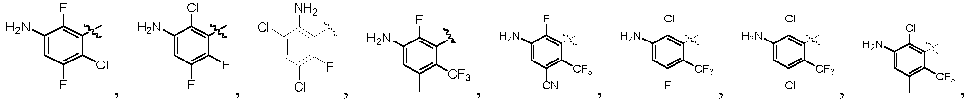

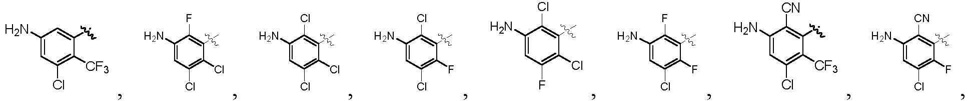

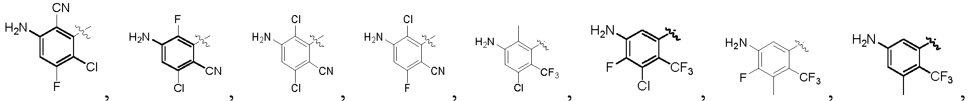

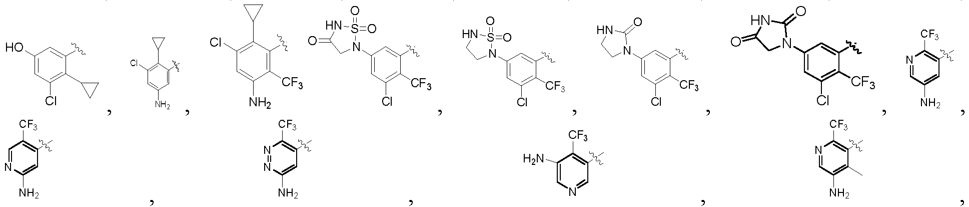

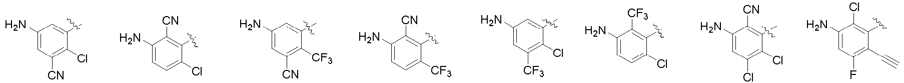

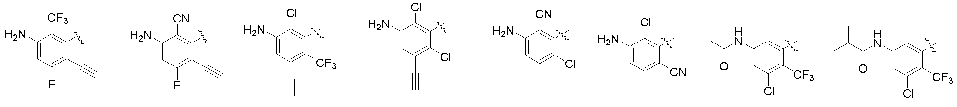

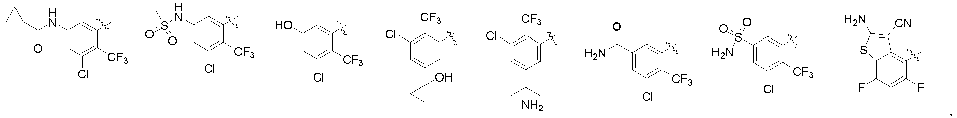

In another preferred embodiment, the compound is selected from the group consisting of: 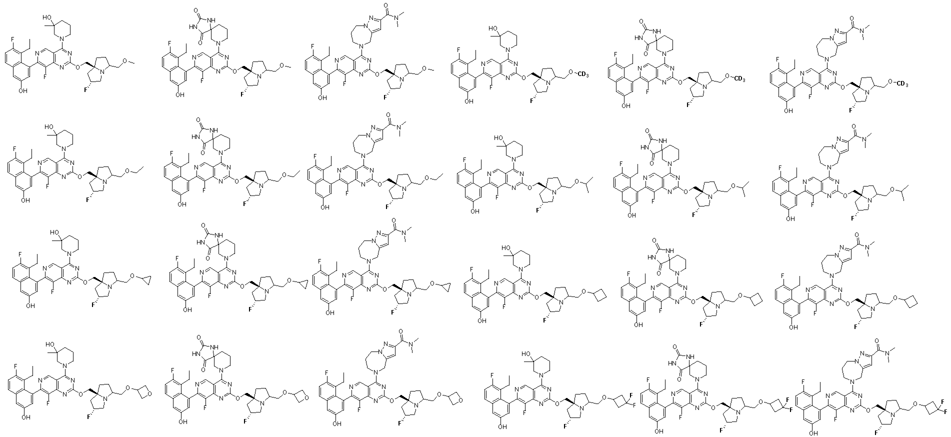 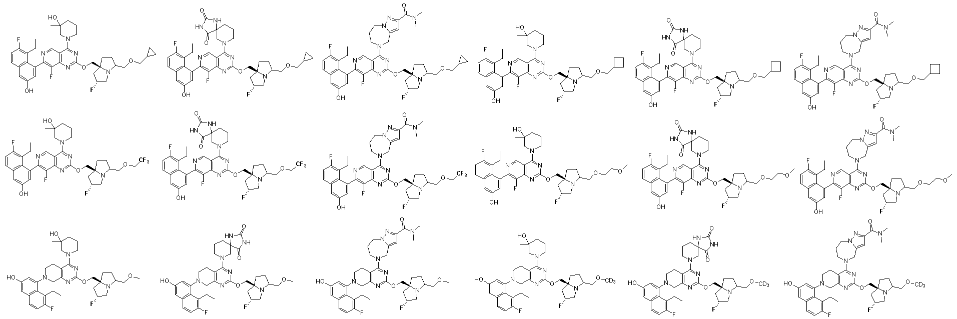 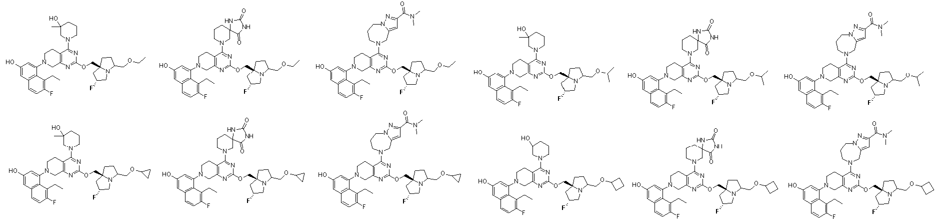 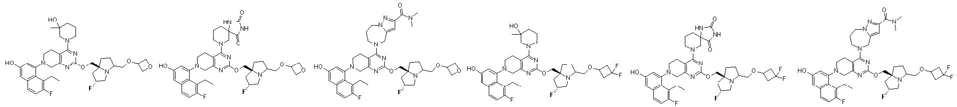 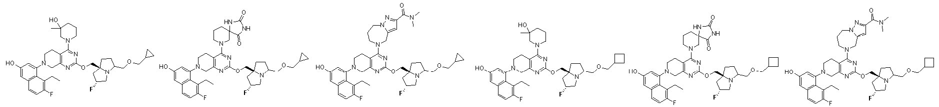 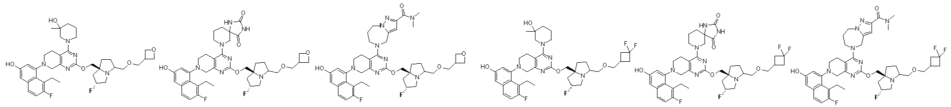 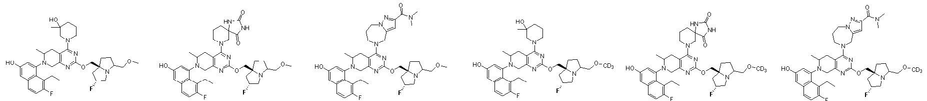 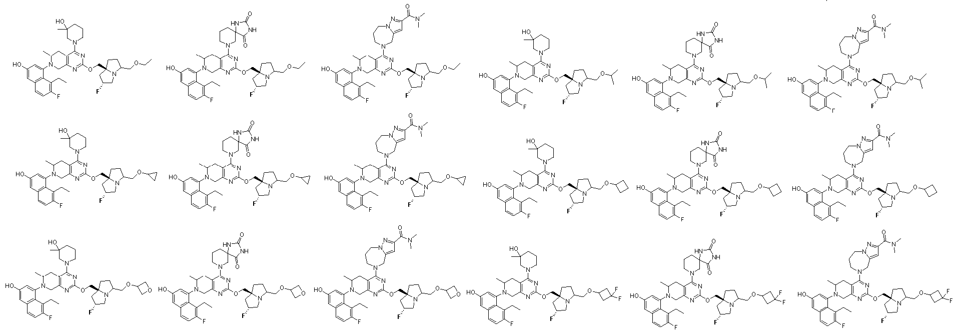 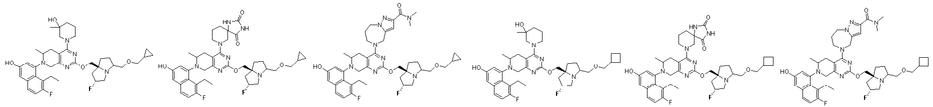 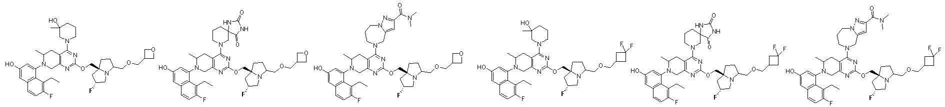 or is selected from: 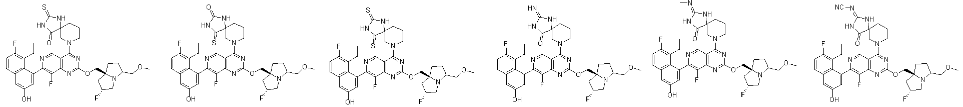 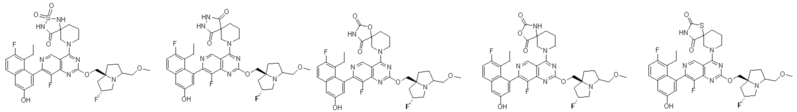 or is selected from: 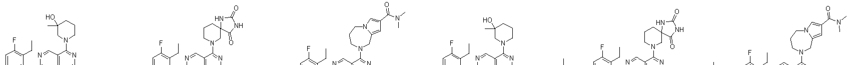 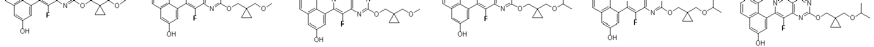 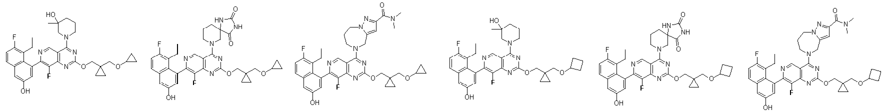 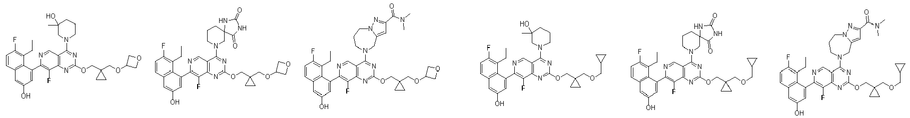 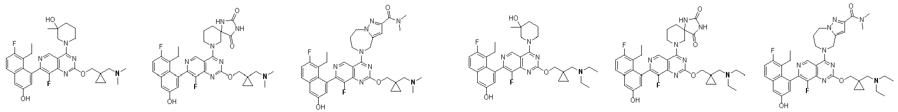 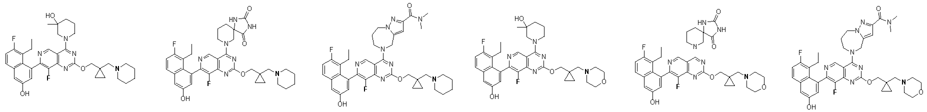 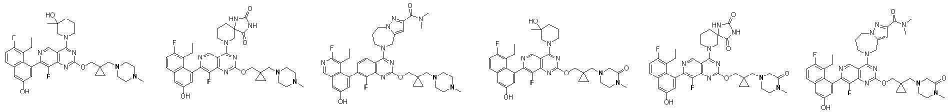 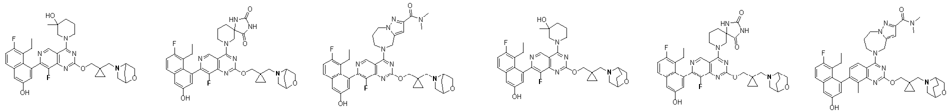 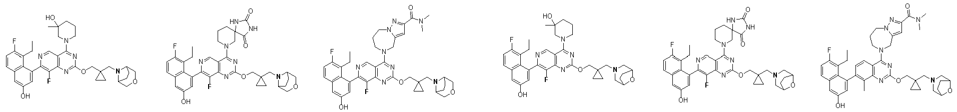 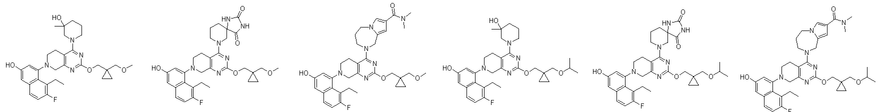 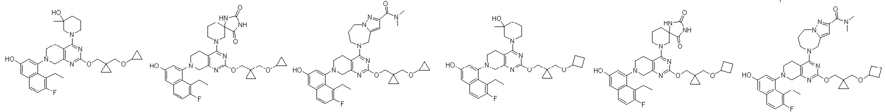 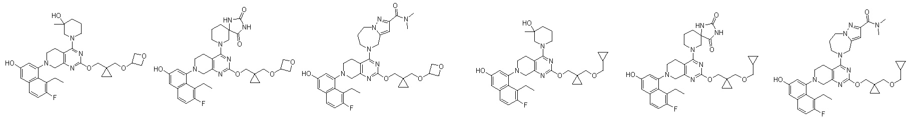 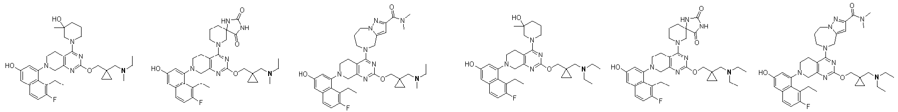 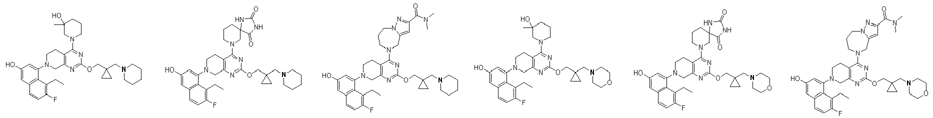 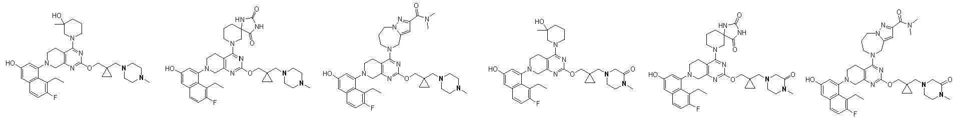 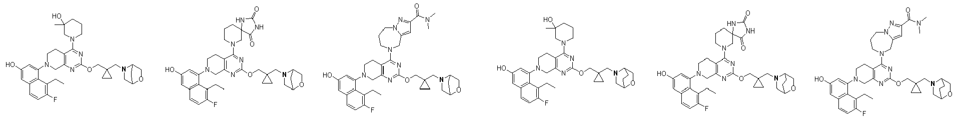 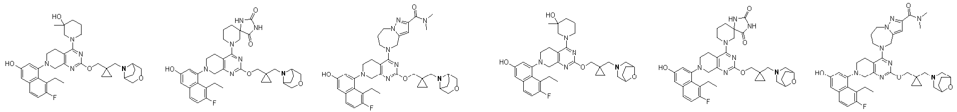 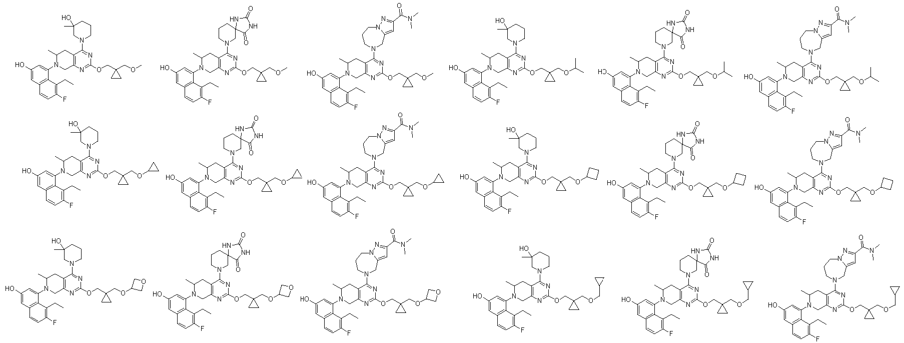 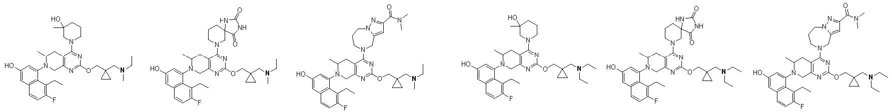 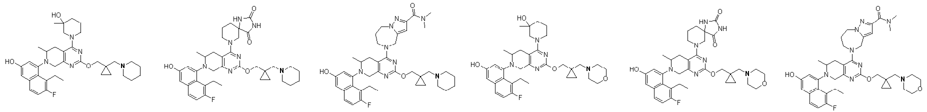 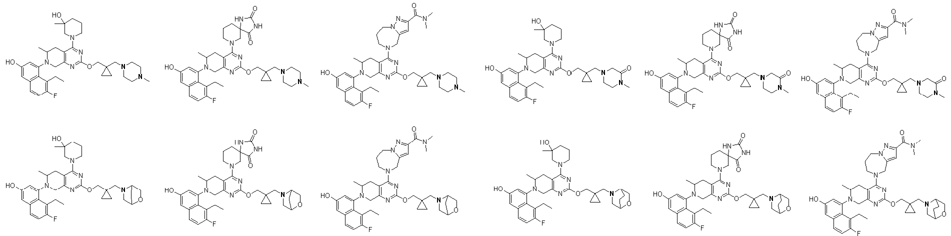 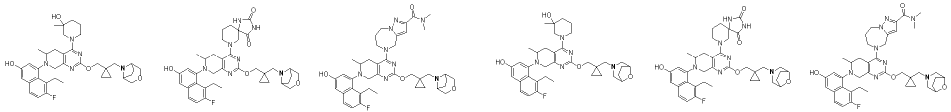 or is selected from: 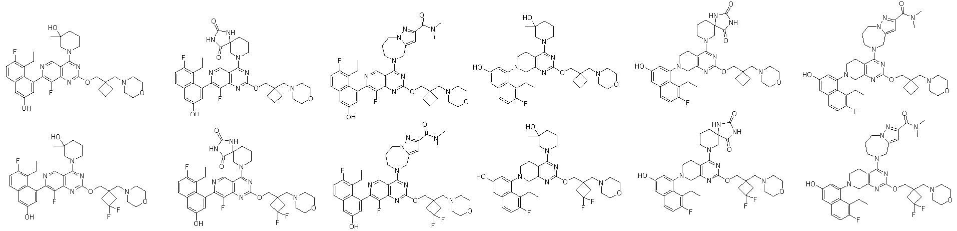 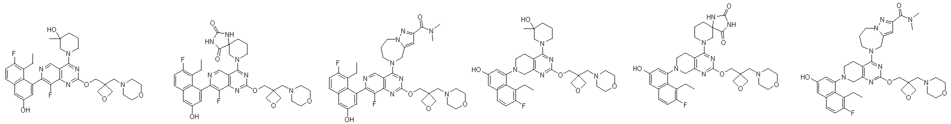 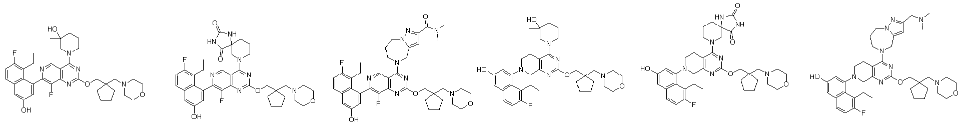 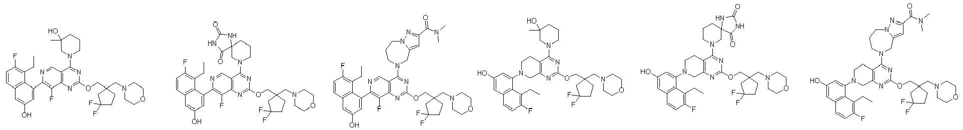 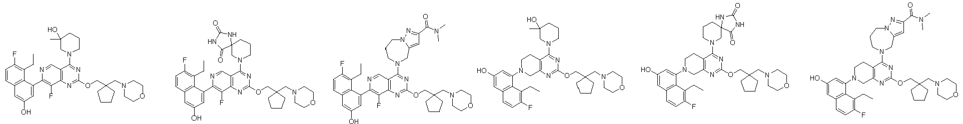 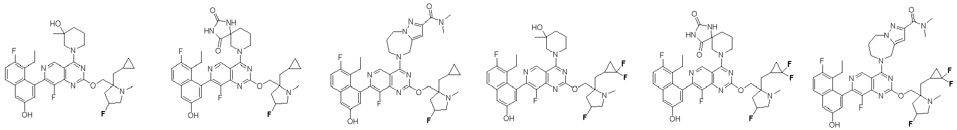 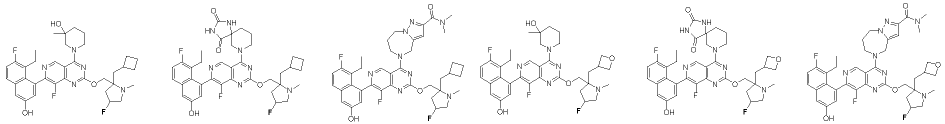 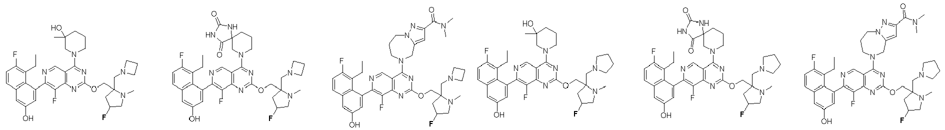 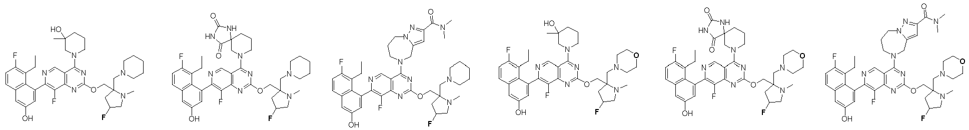 or is selected from: 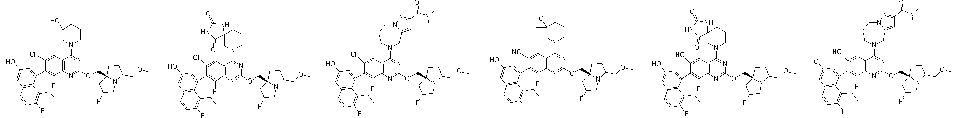 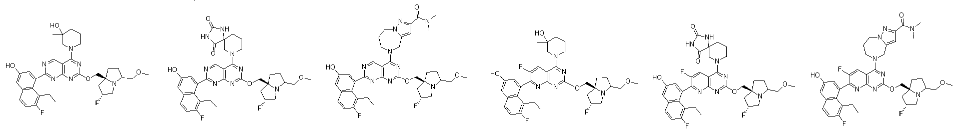 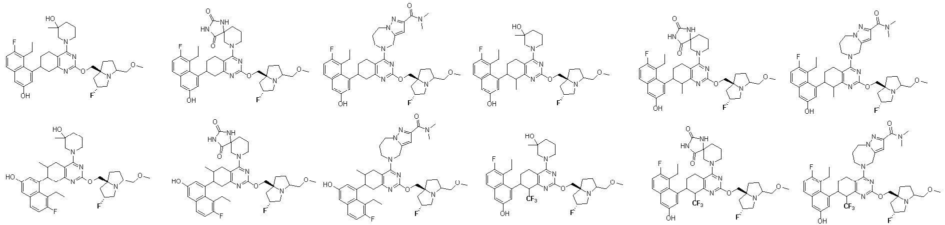 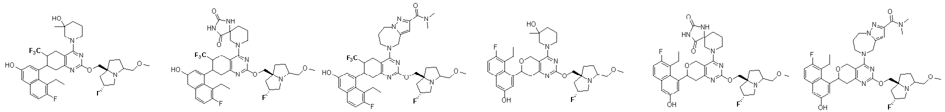 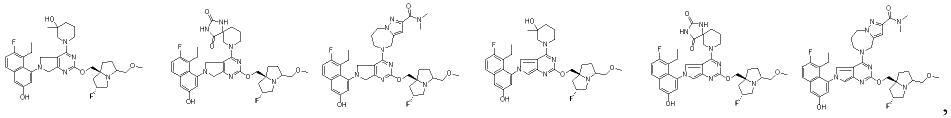 or is selected from: 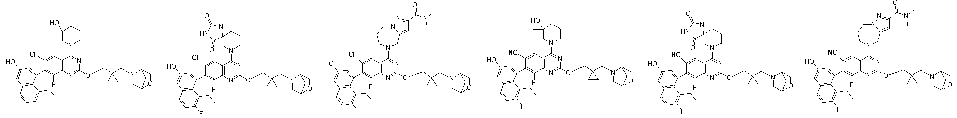 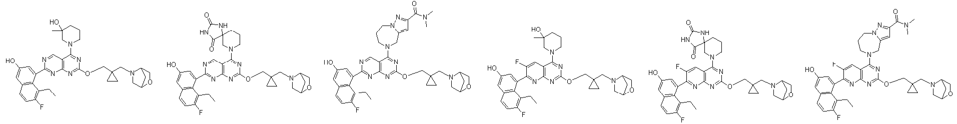 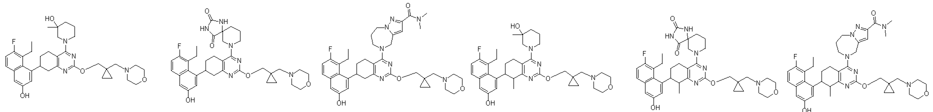 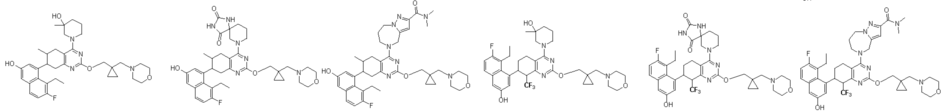 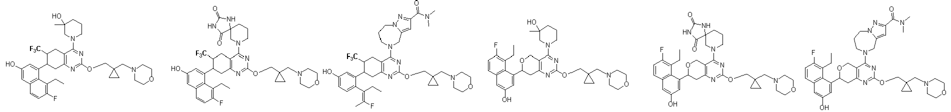 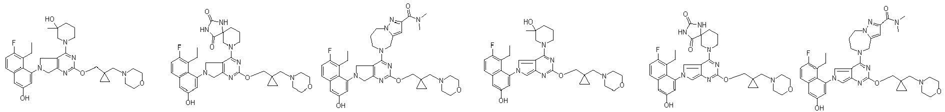 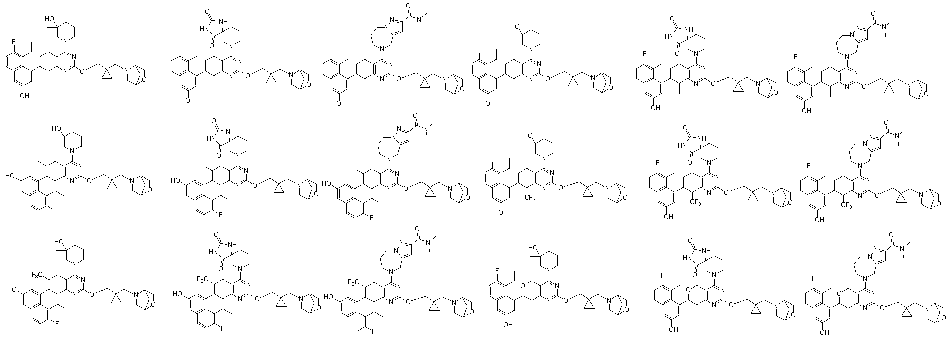 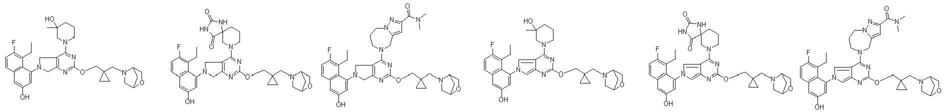 or is selected from: 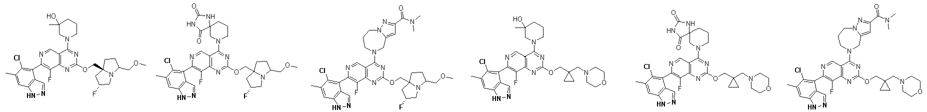 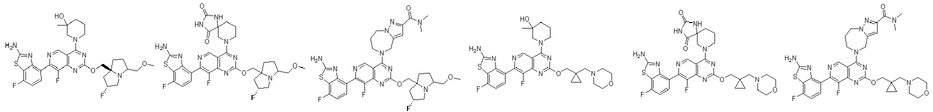 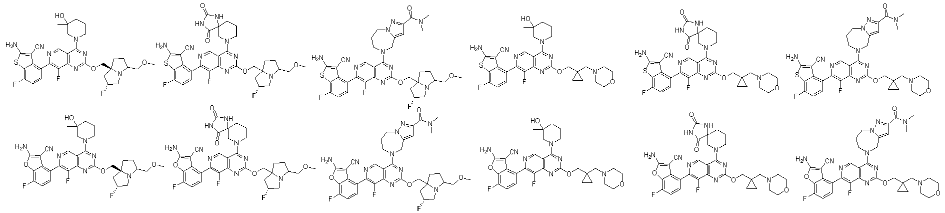 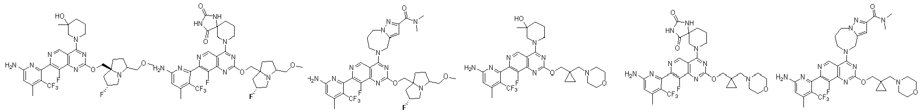 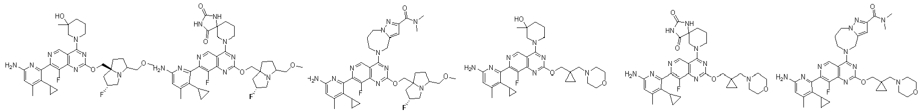 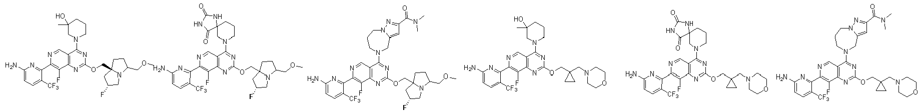 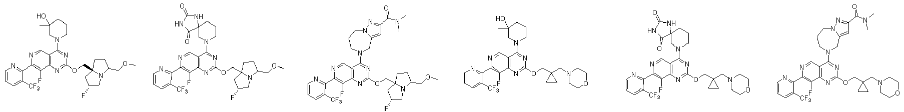 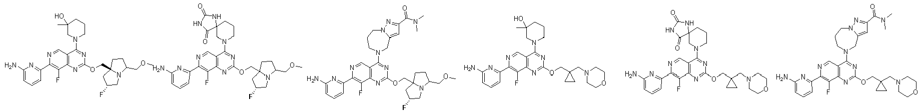 or is selected from: 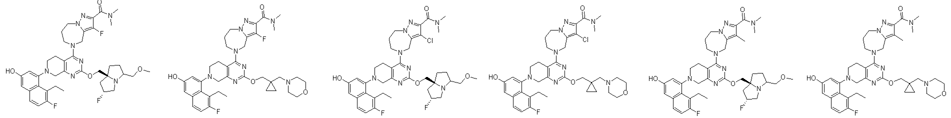 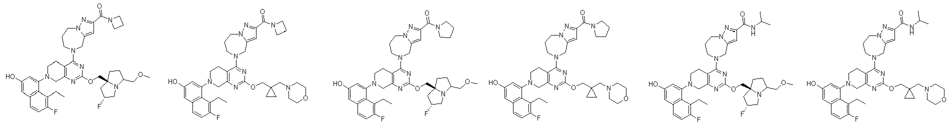 or is selected from: 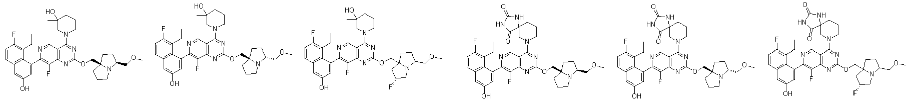 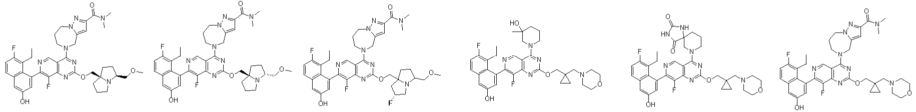 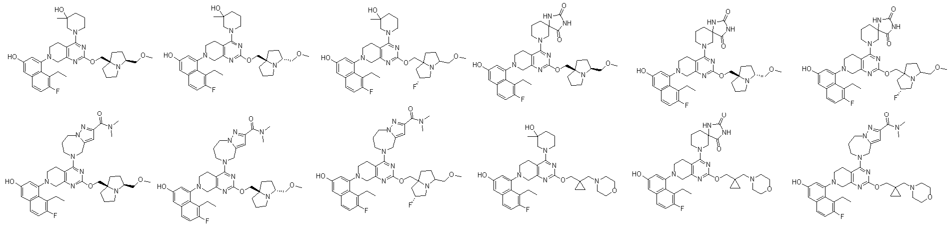 or is selected from:

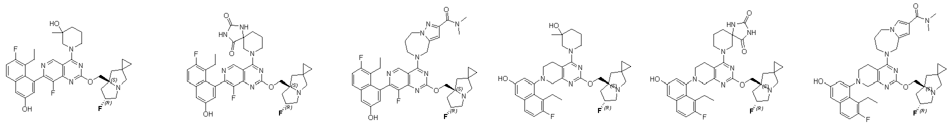

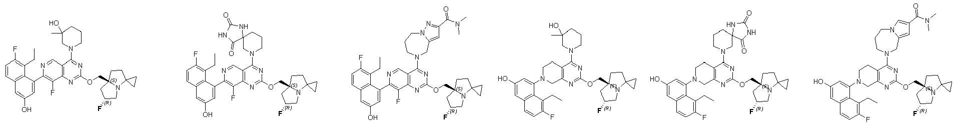

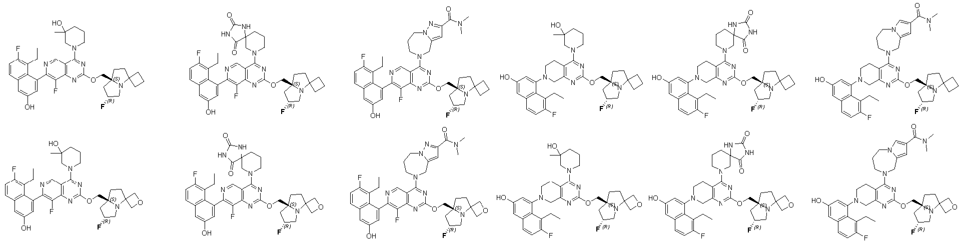

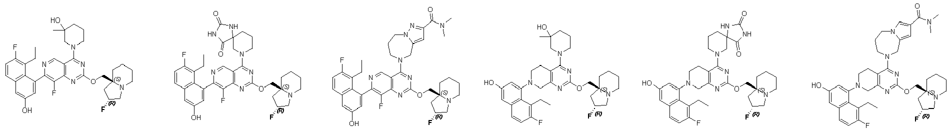

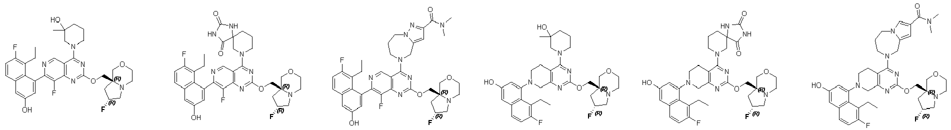

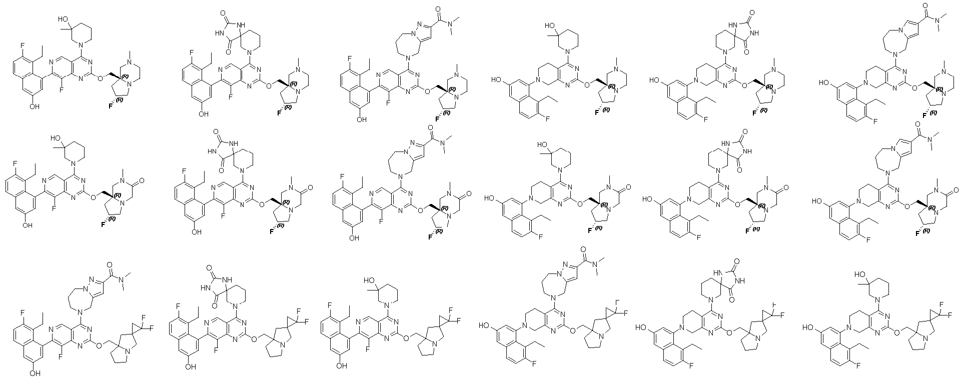

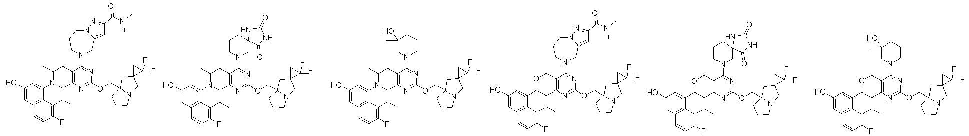

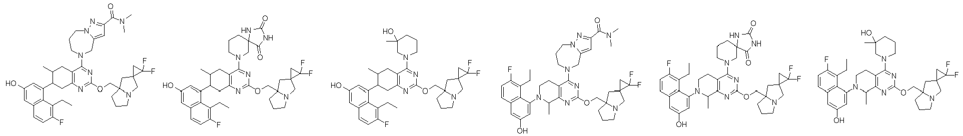

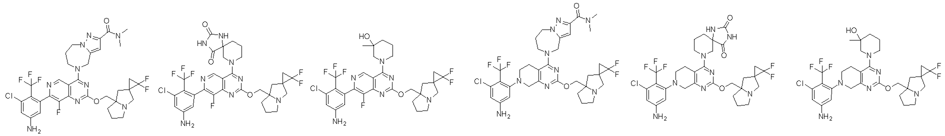

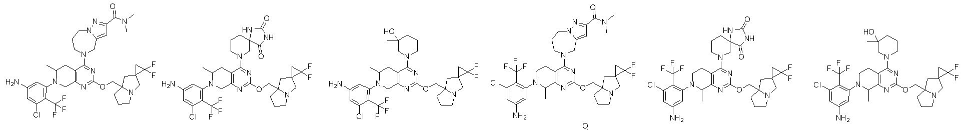

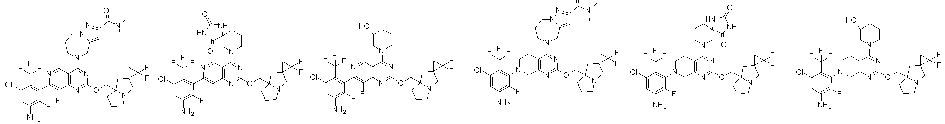

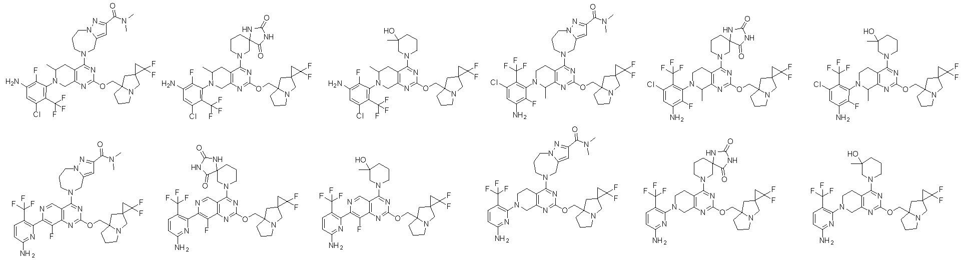

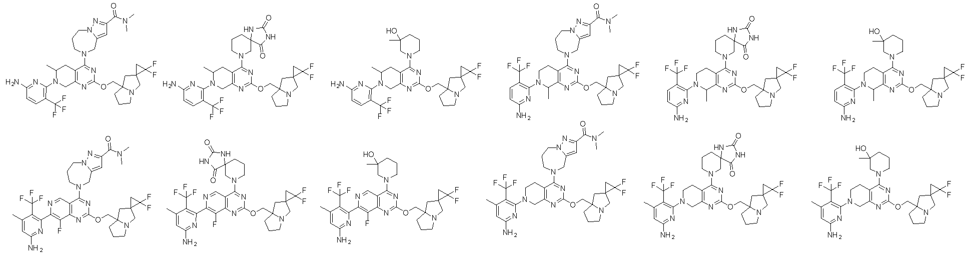

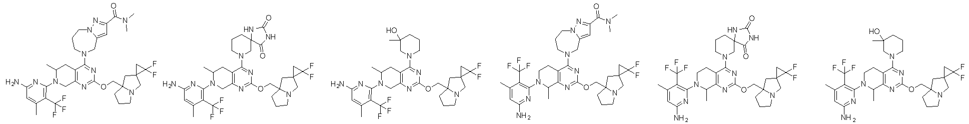

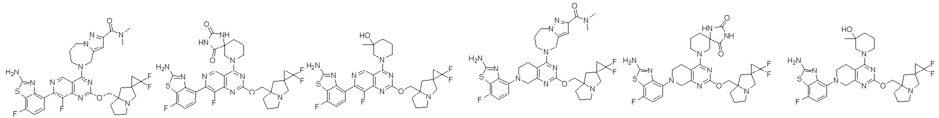

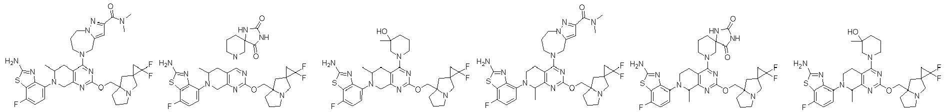

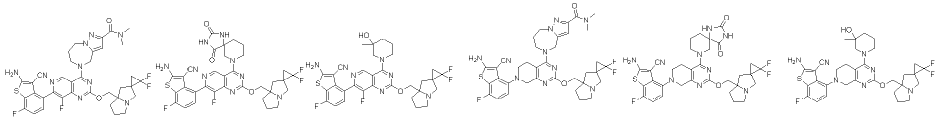

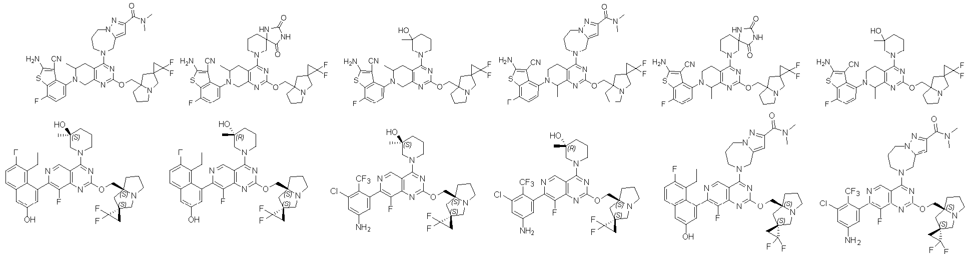

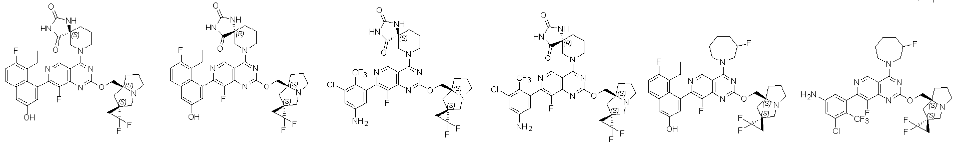

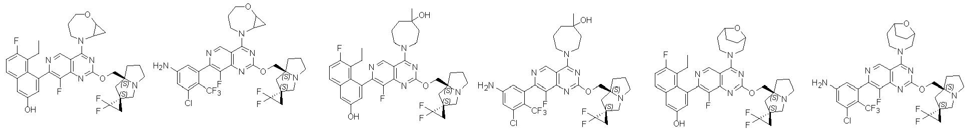

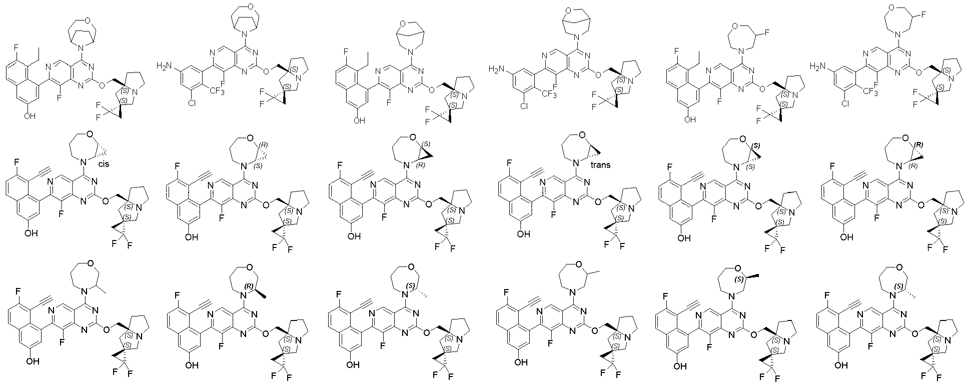

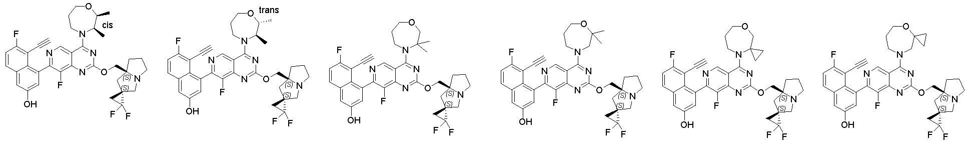

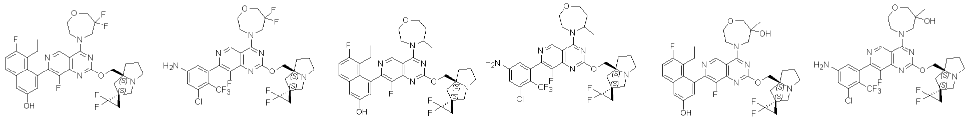

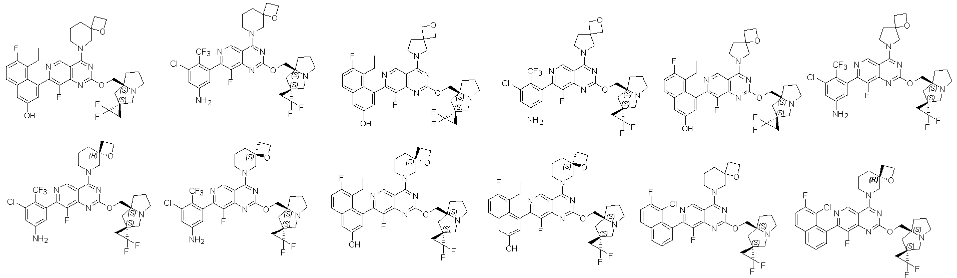

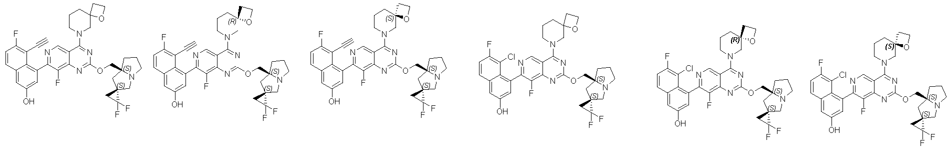

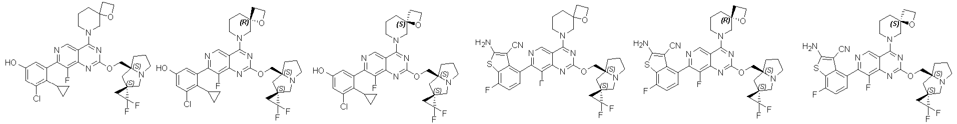

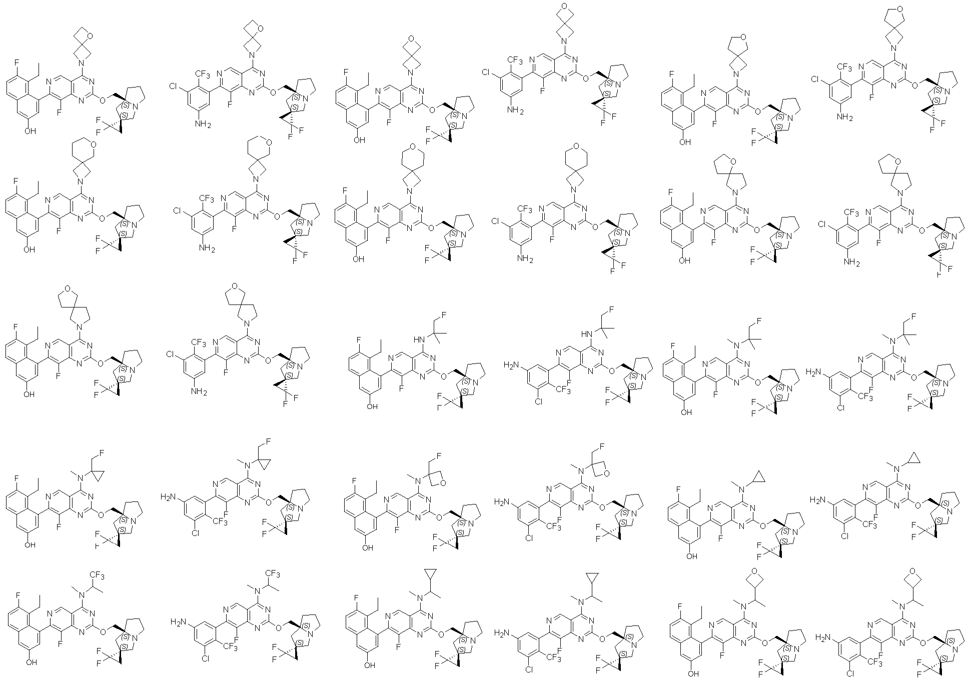

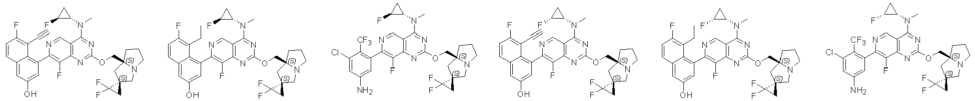

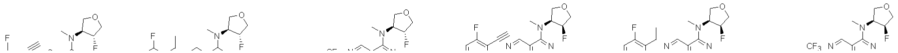

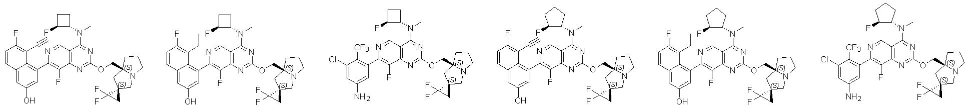

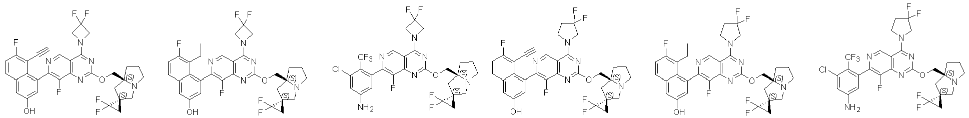

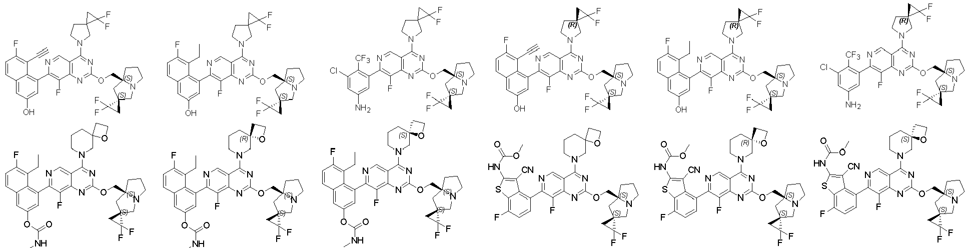

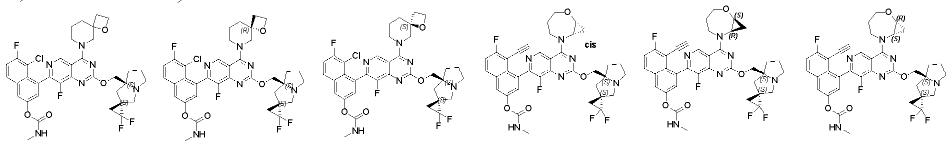

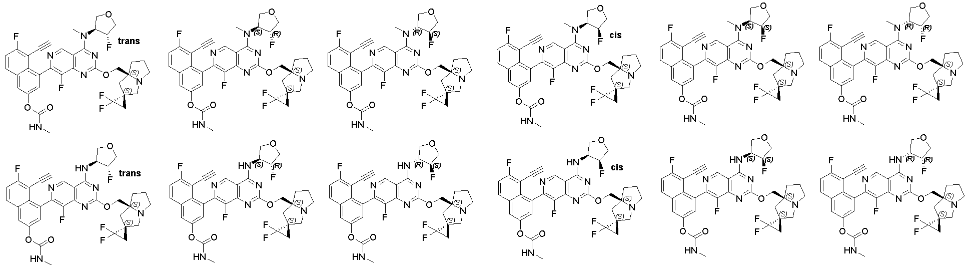

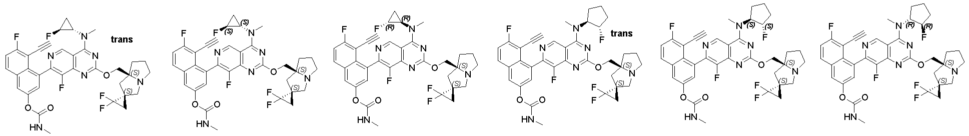

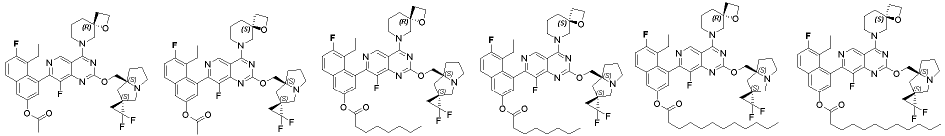

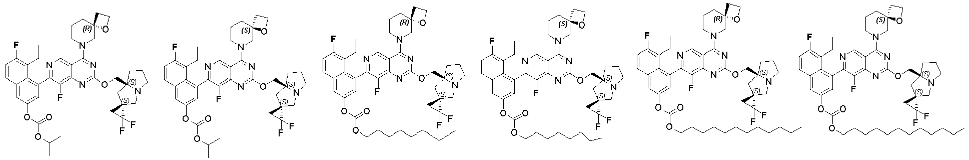

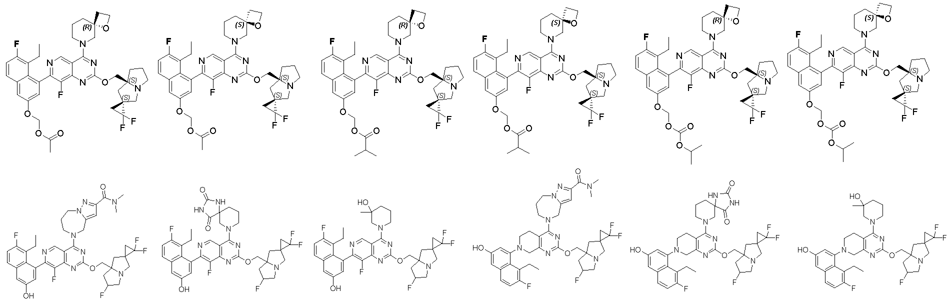

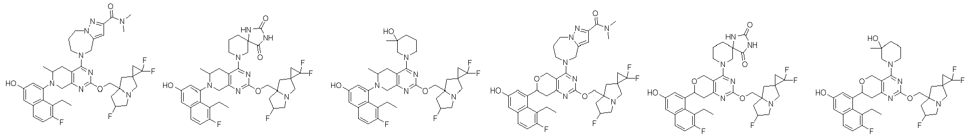

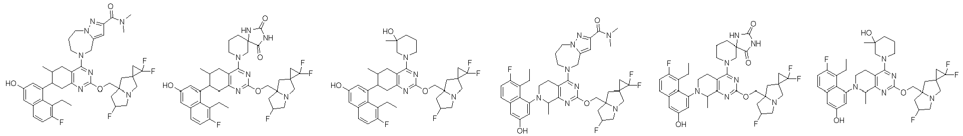

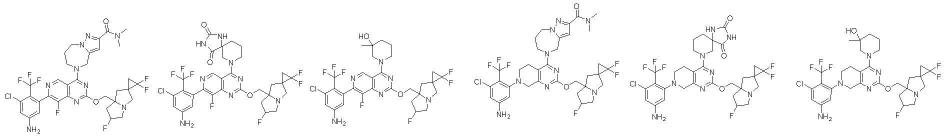

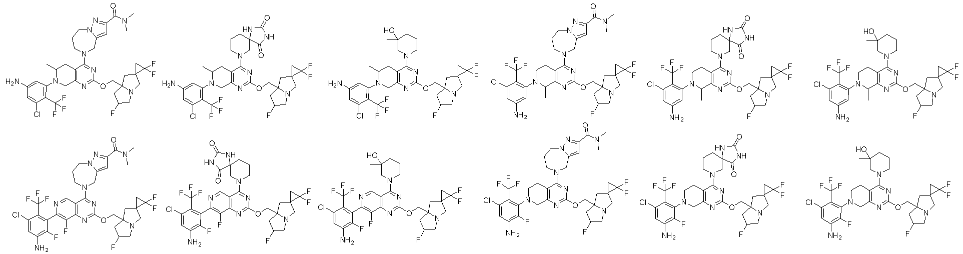

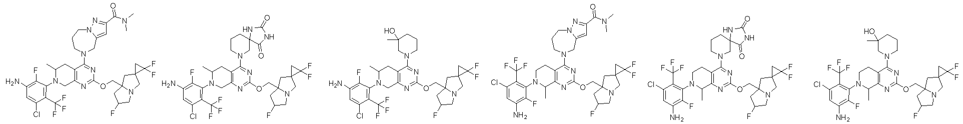

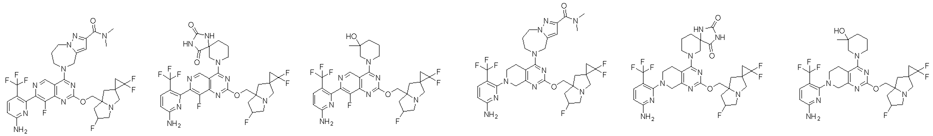

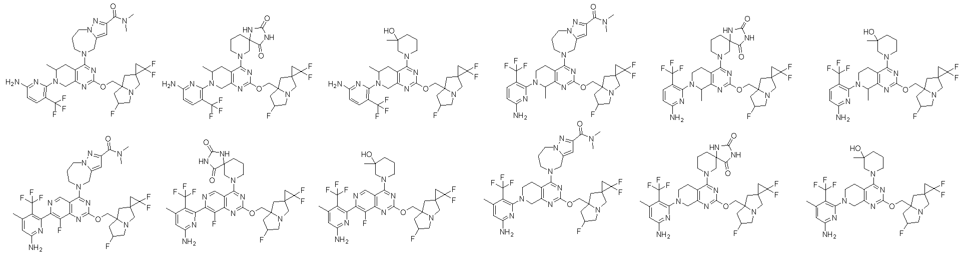

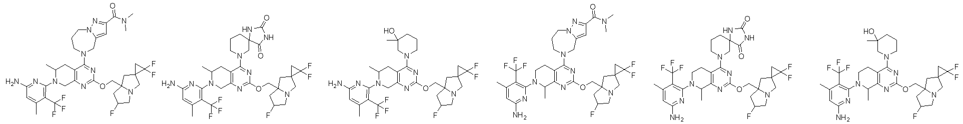

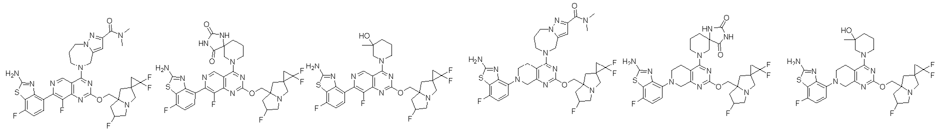

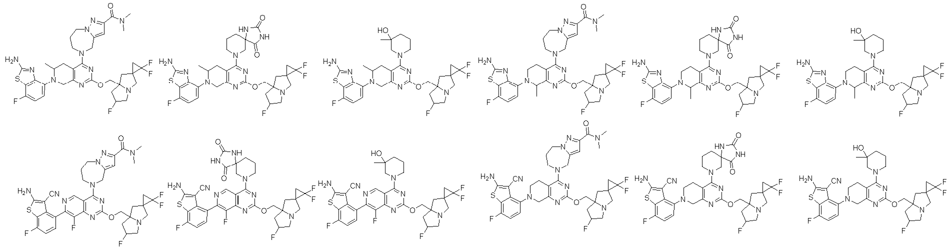

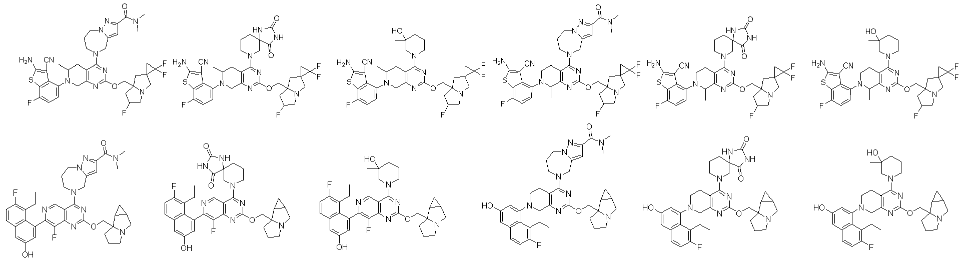

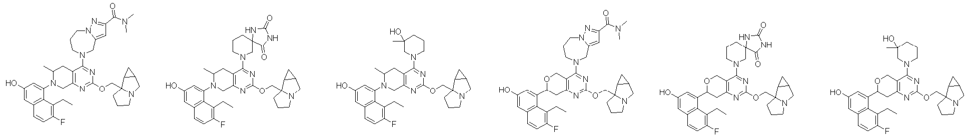

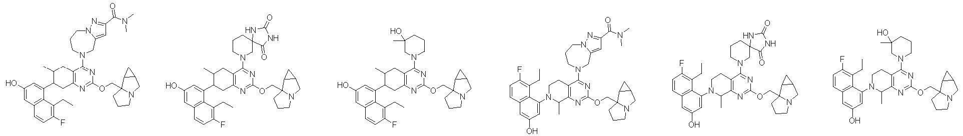

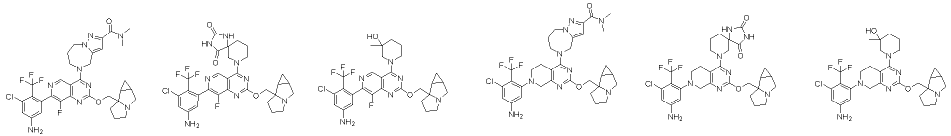

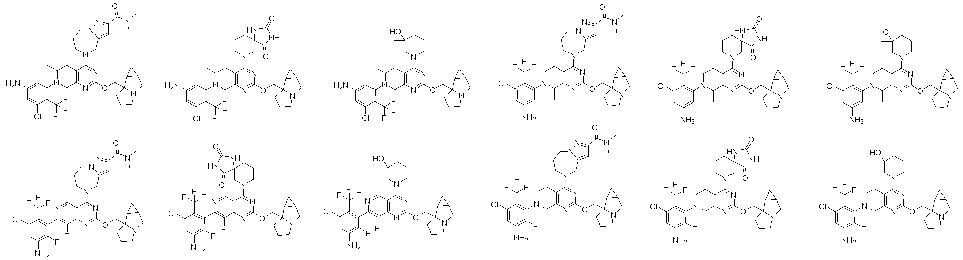

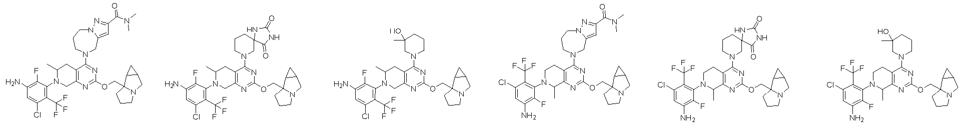

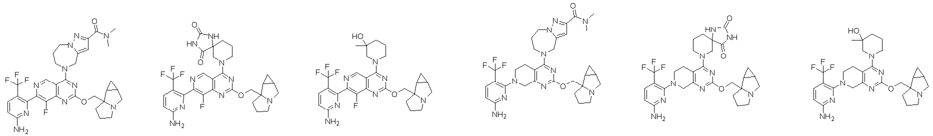

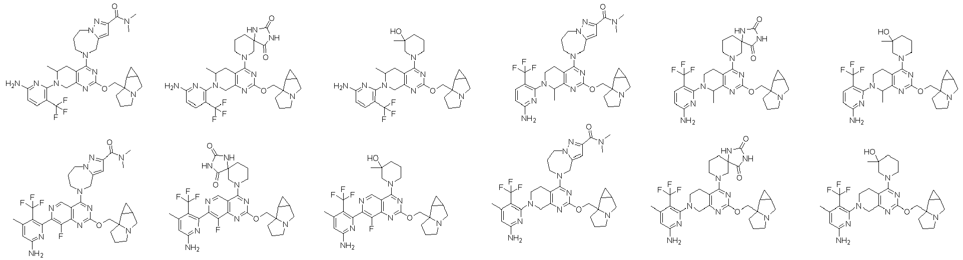

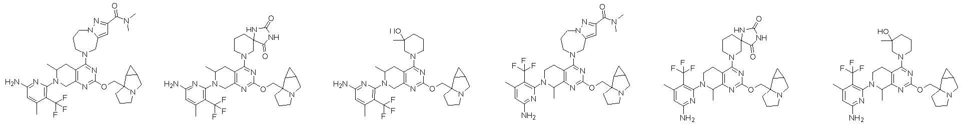

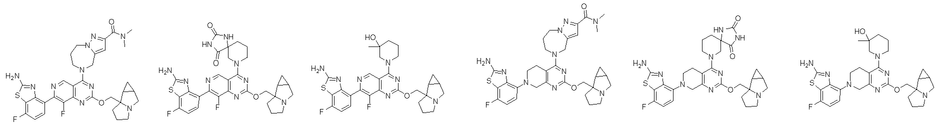

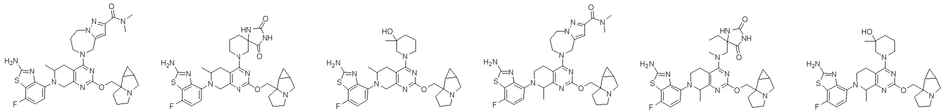

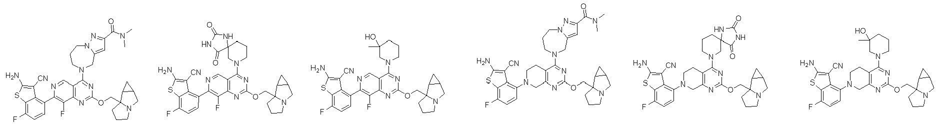

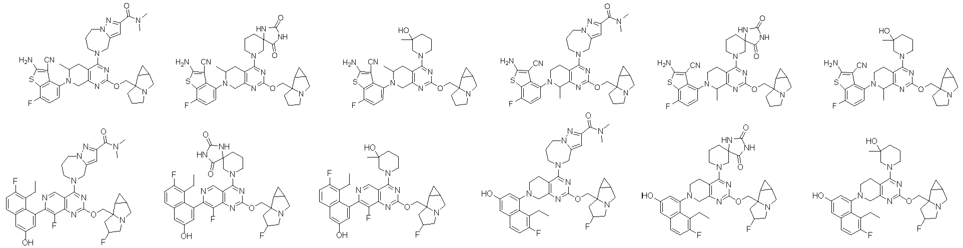

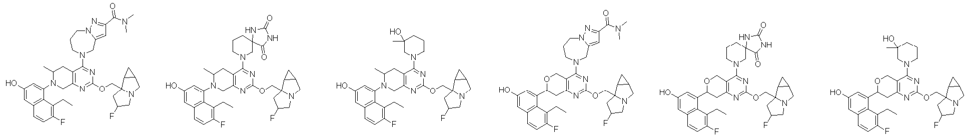

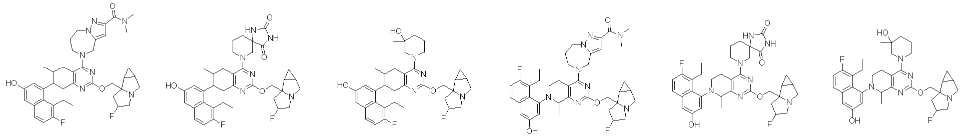

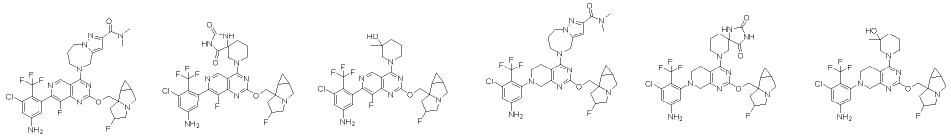

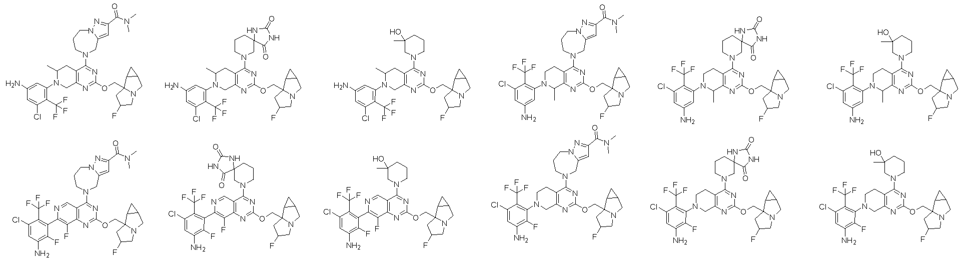

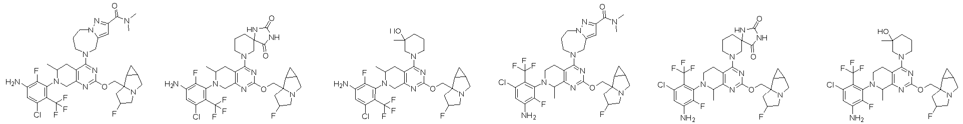

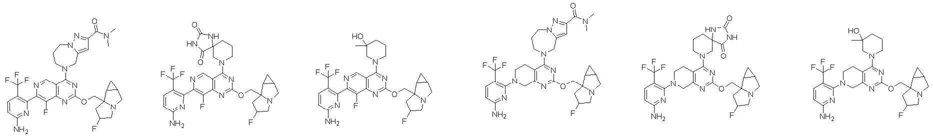

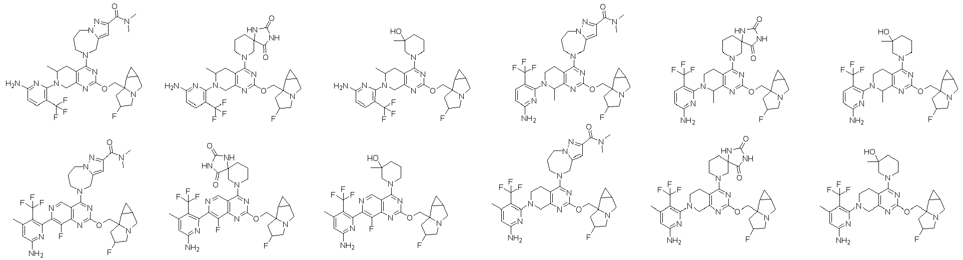

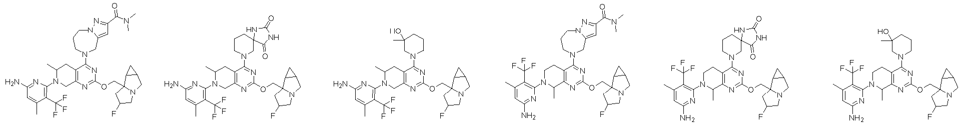

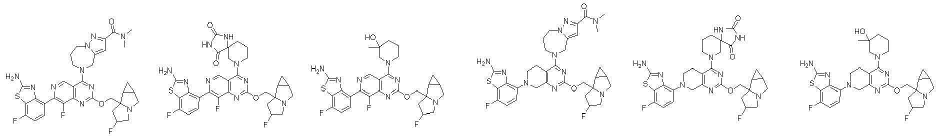

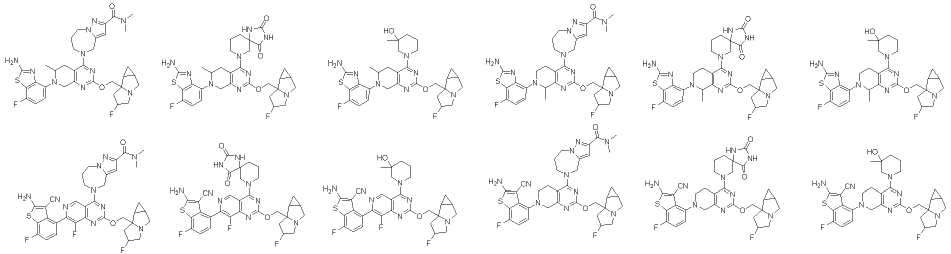

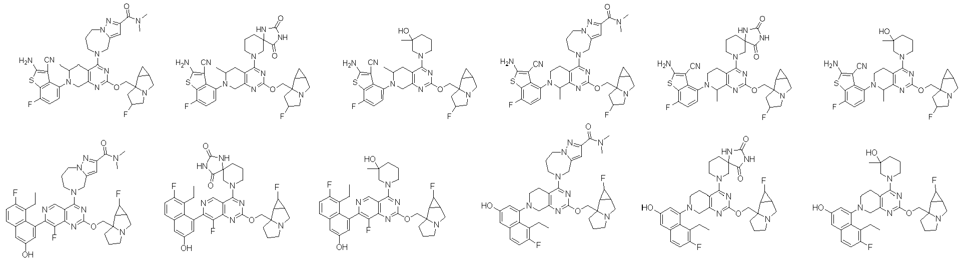

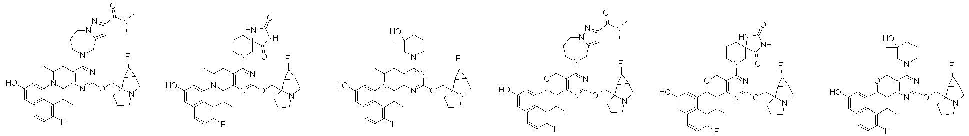

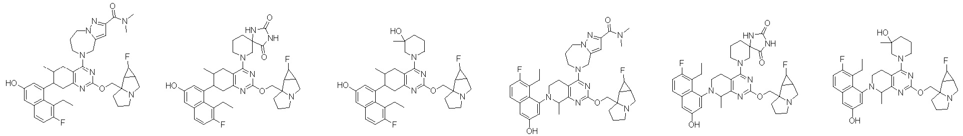

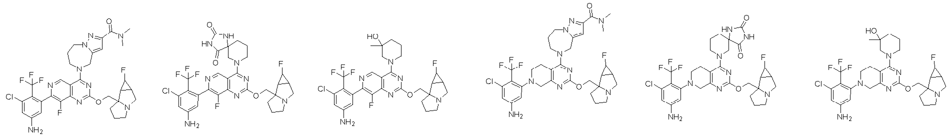

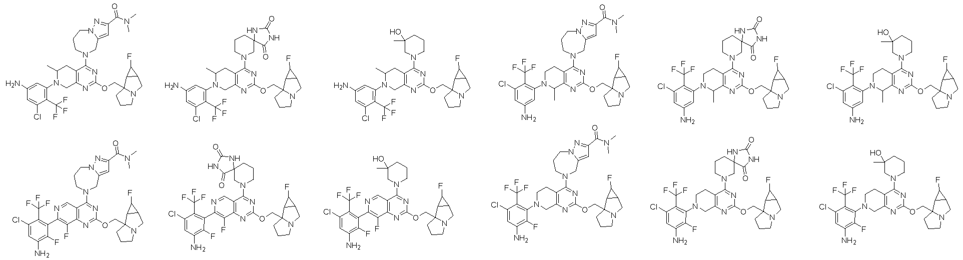

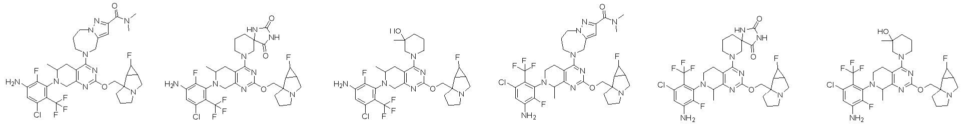

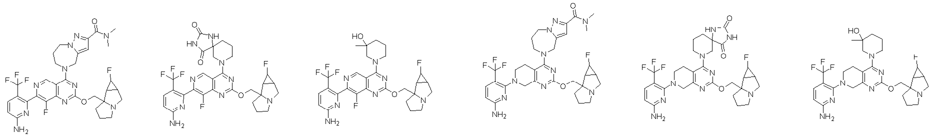

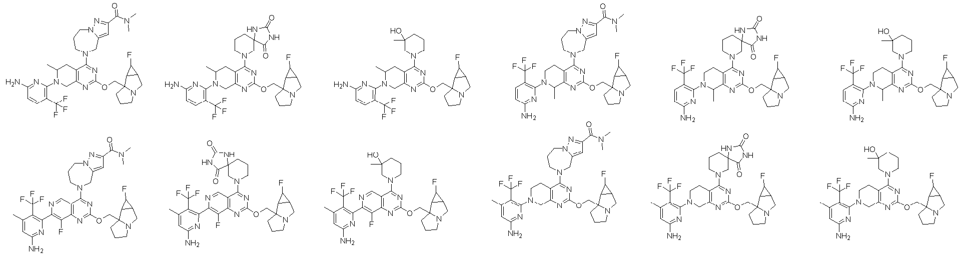

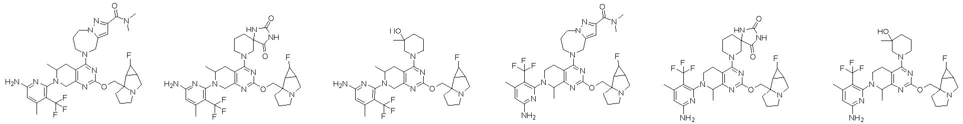

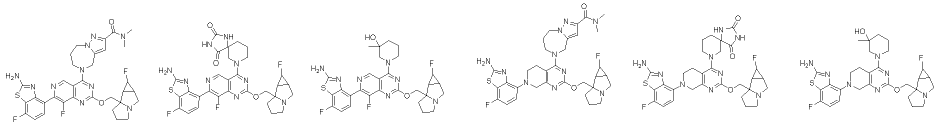

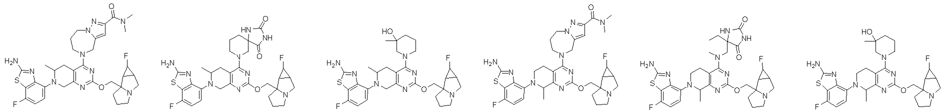

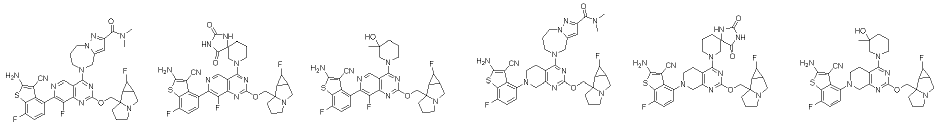

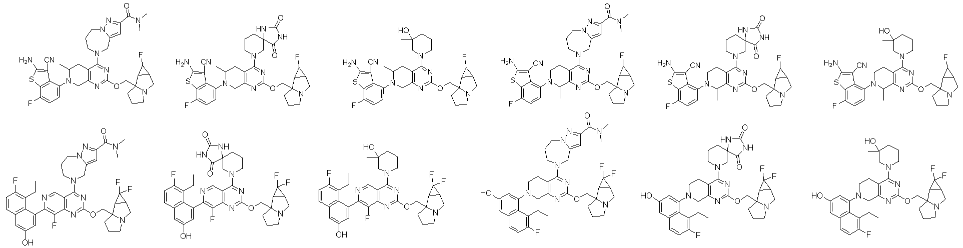

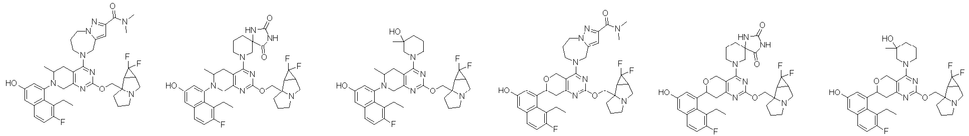

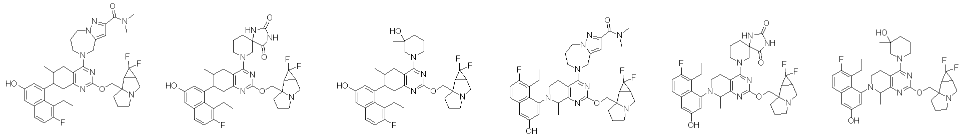

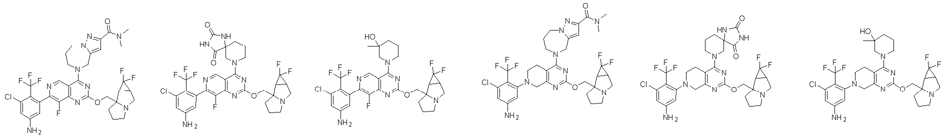

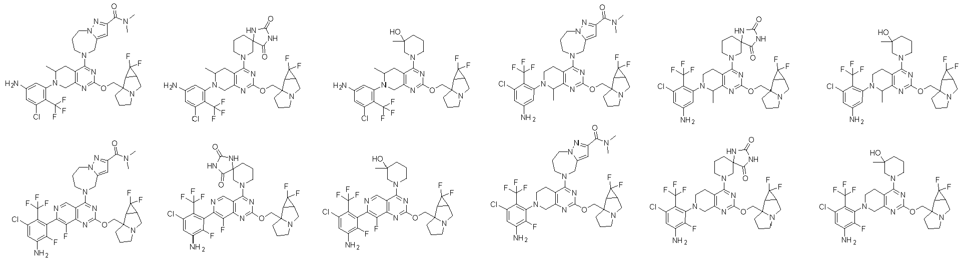

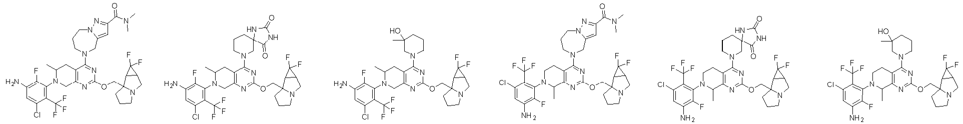

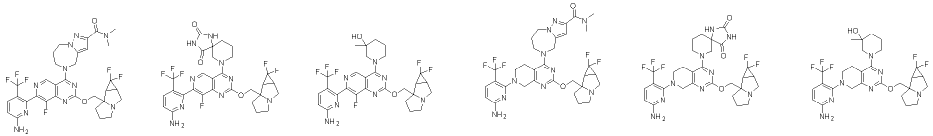

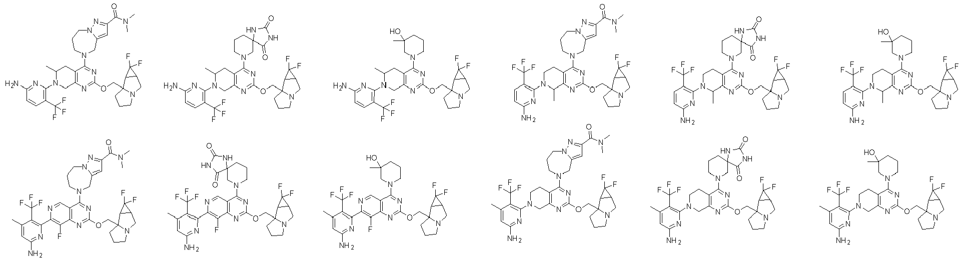

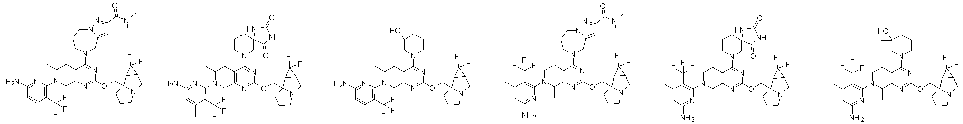

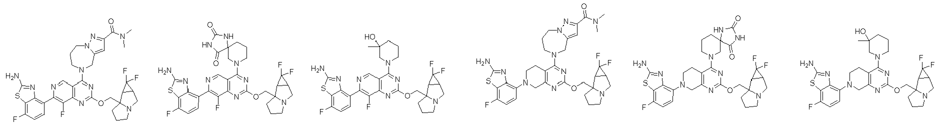

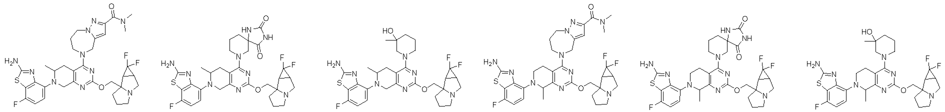

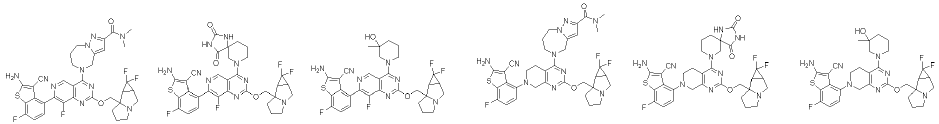

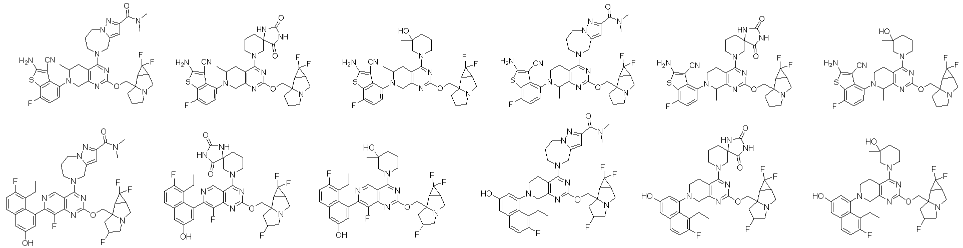

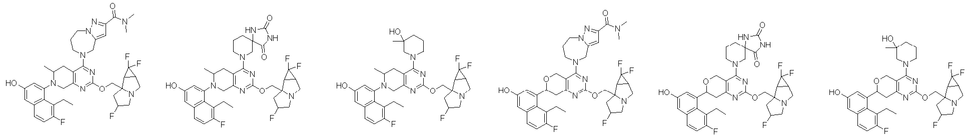

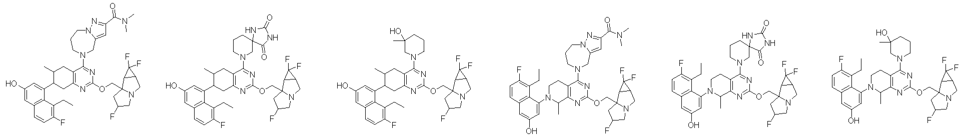

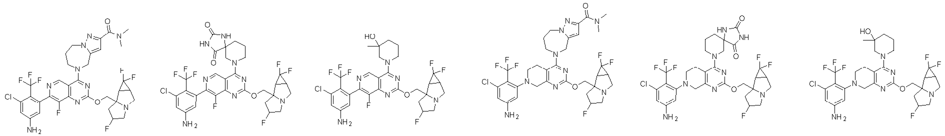

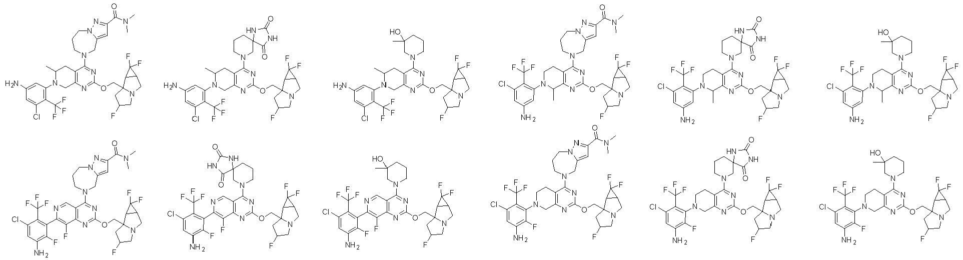

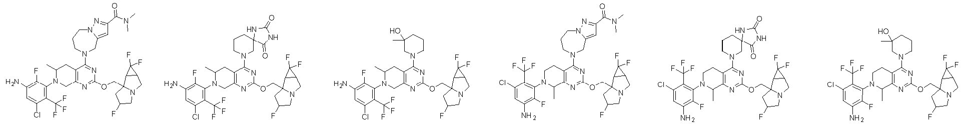

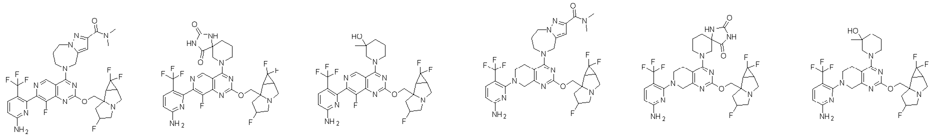

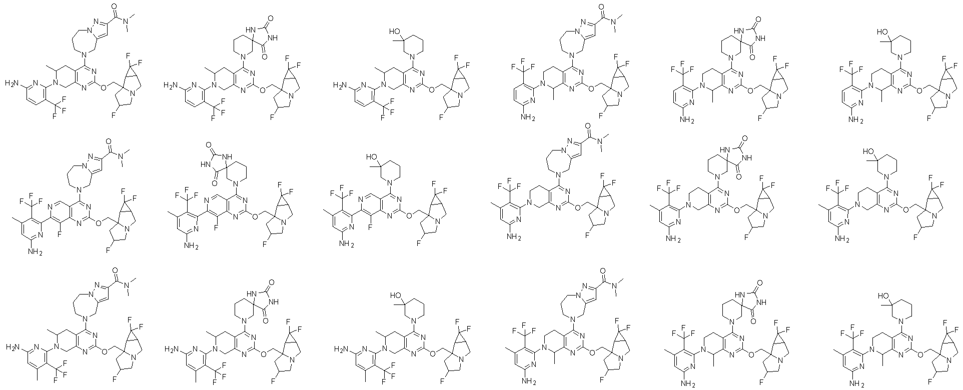

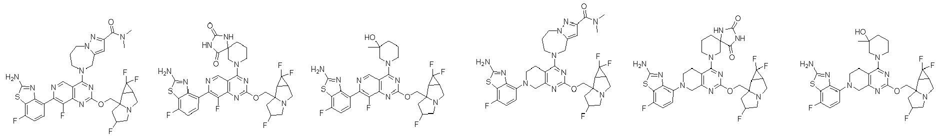

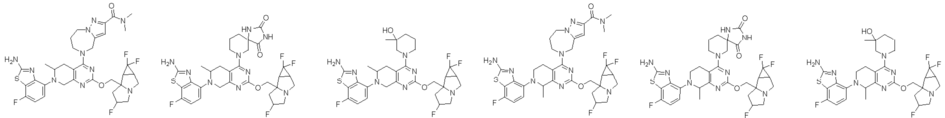

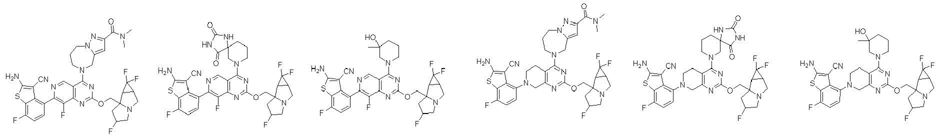

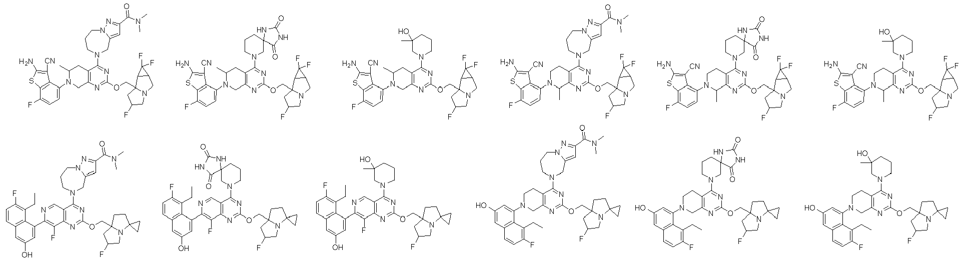

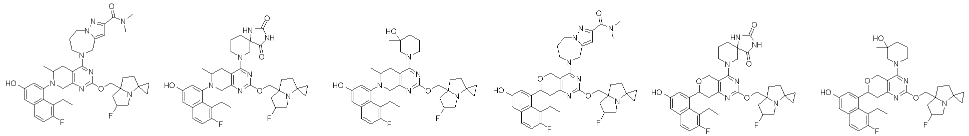

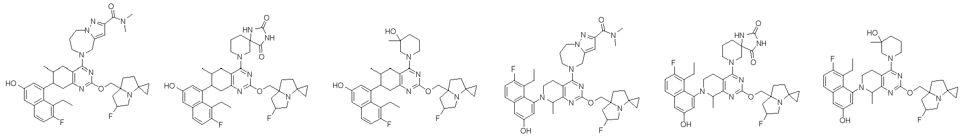

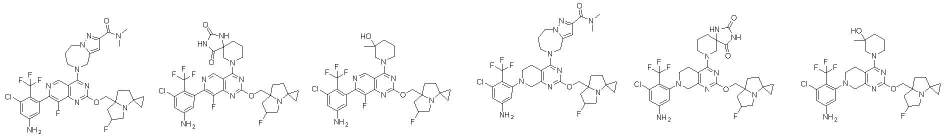

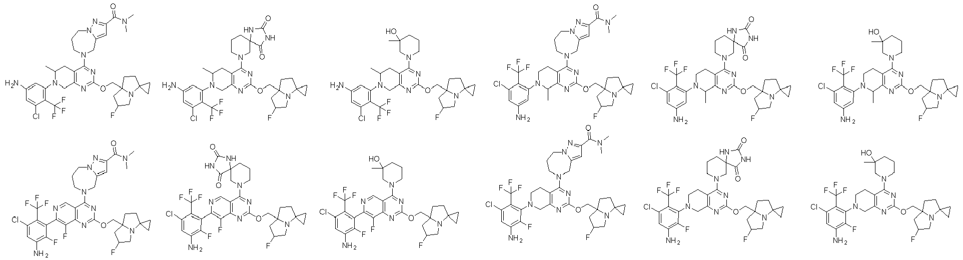

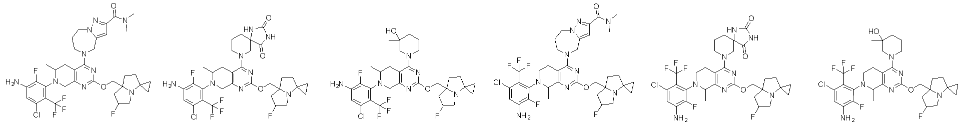

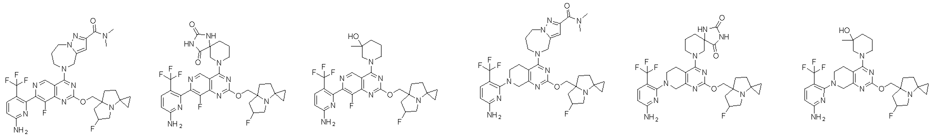

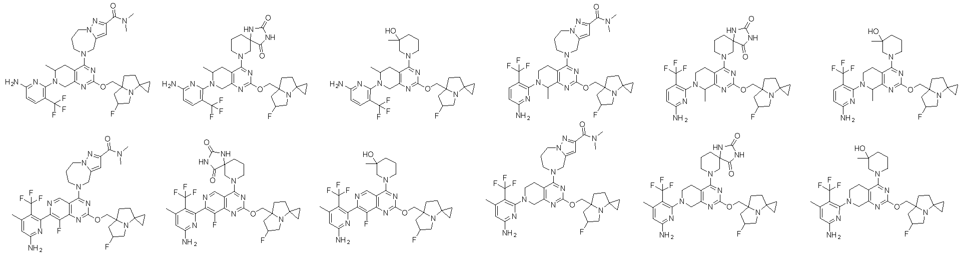

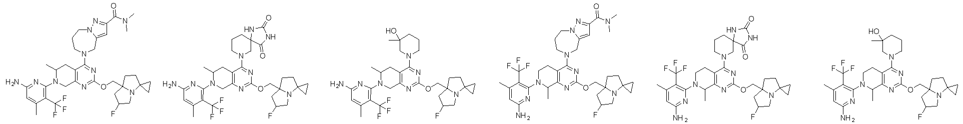

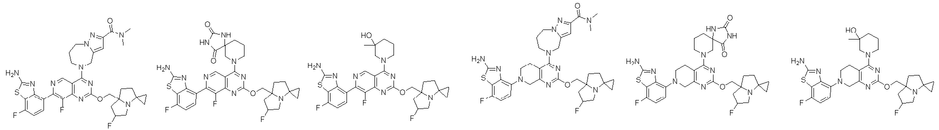

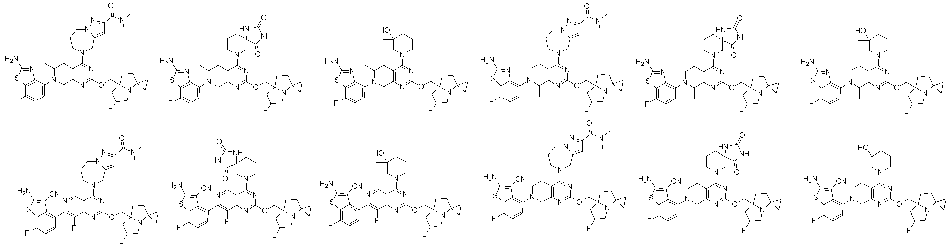

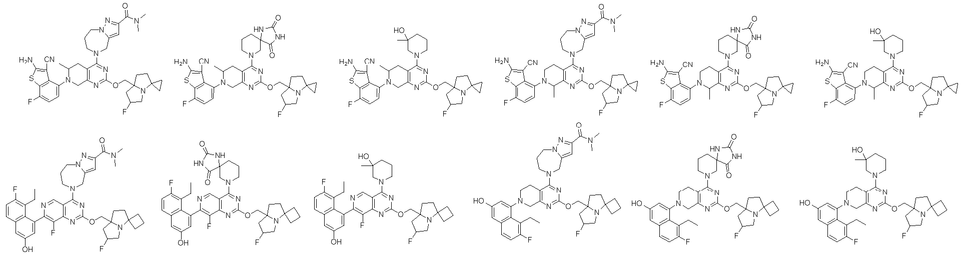

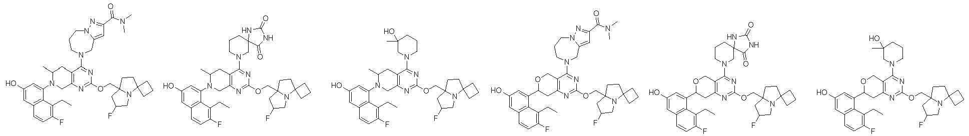

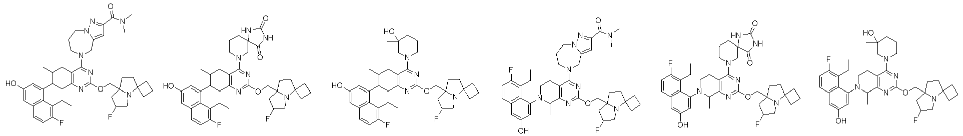

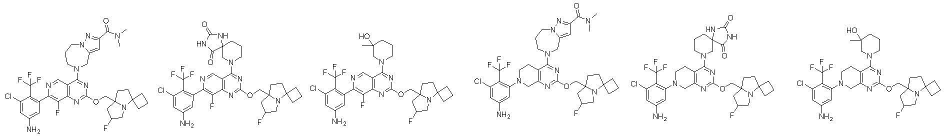

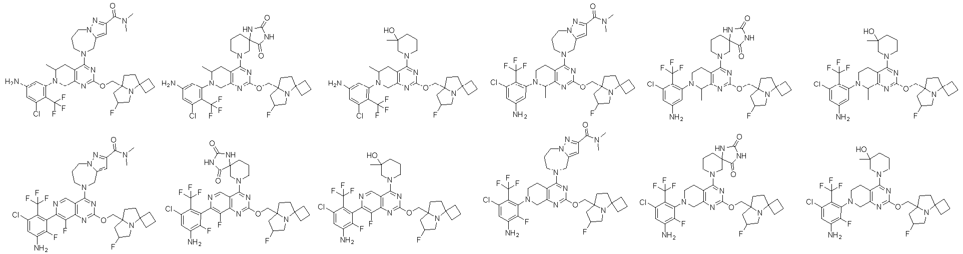

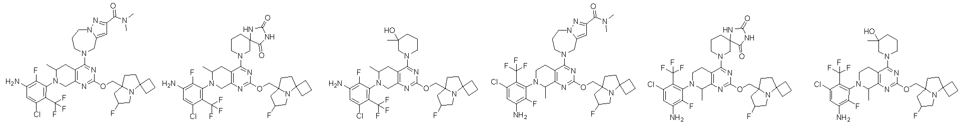

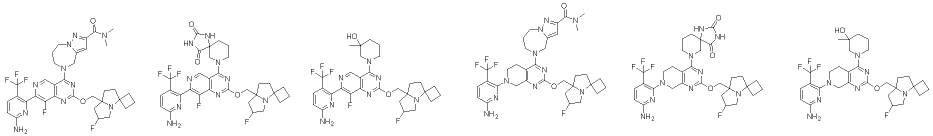

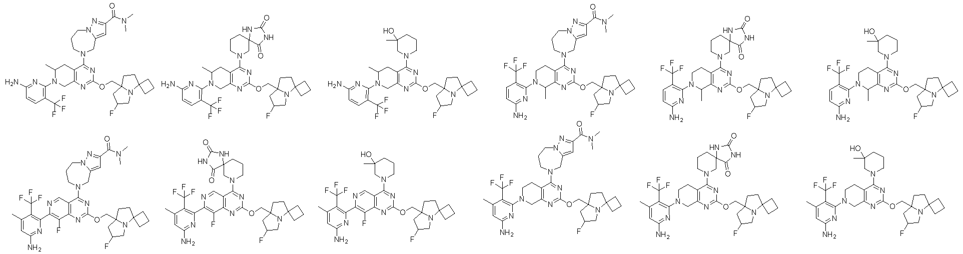

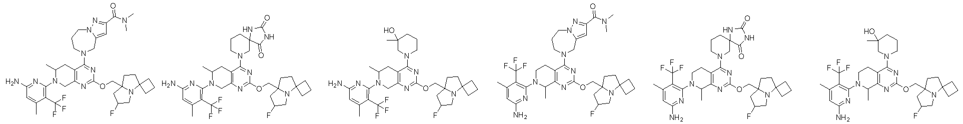

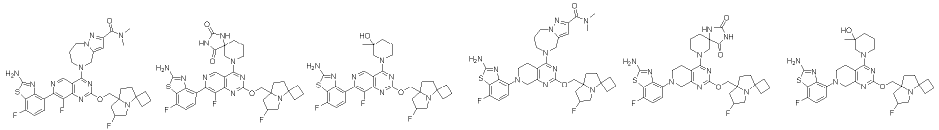

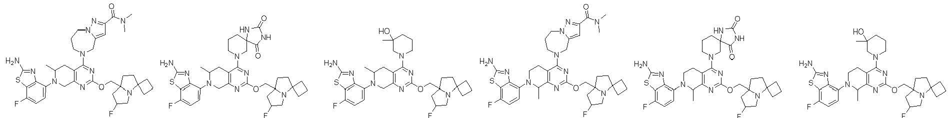

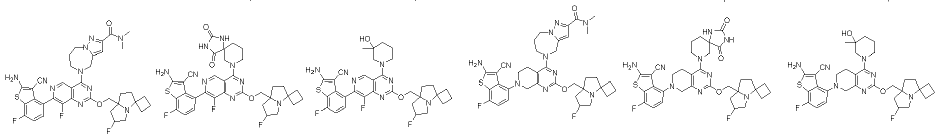

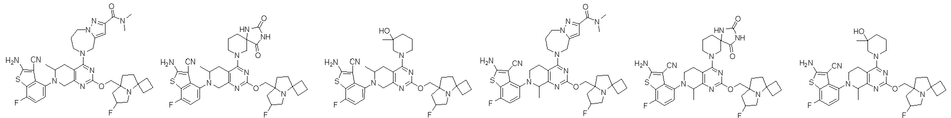

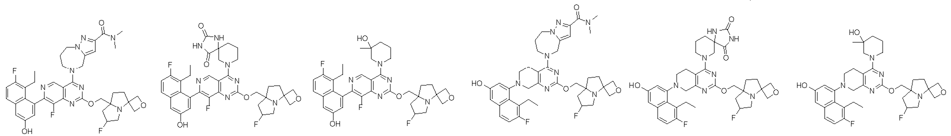

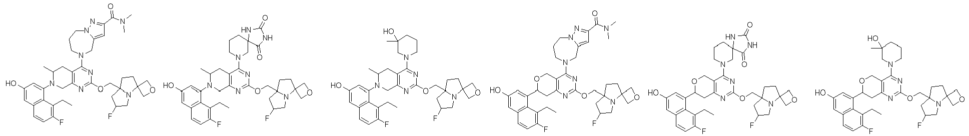

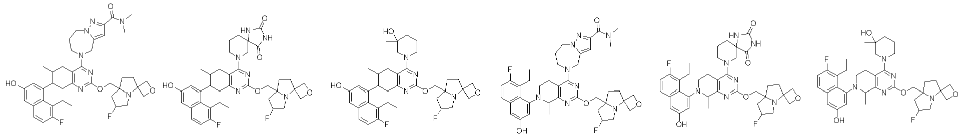

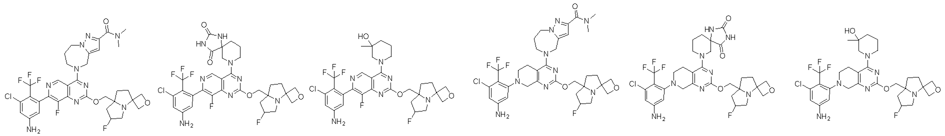

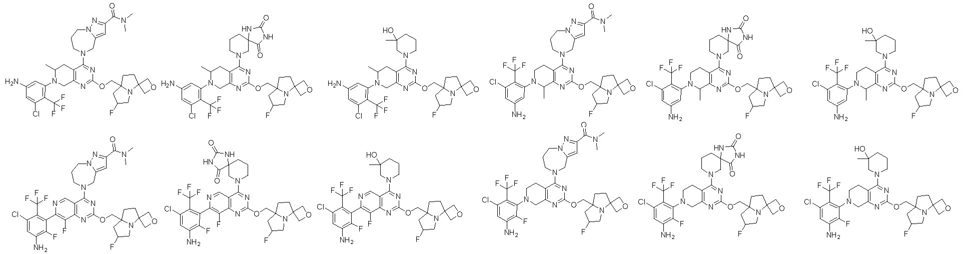

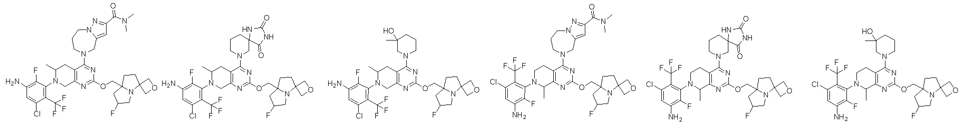

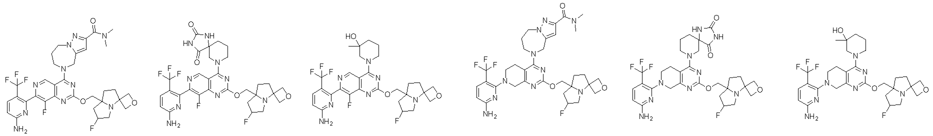

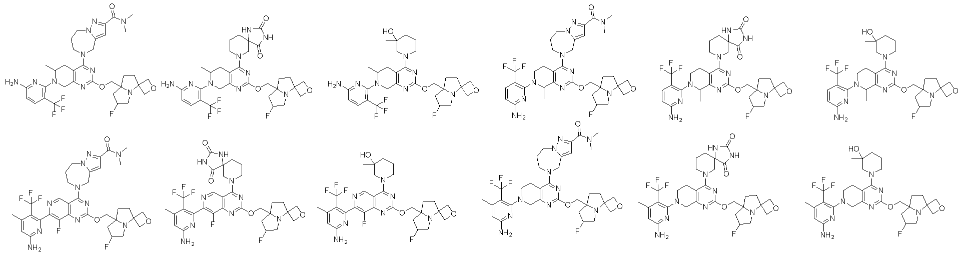

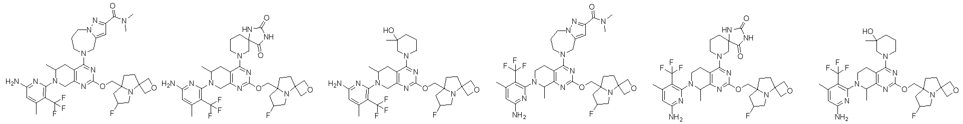

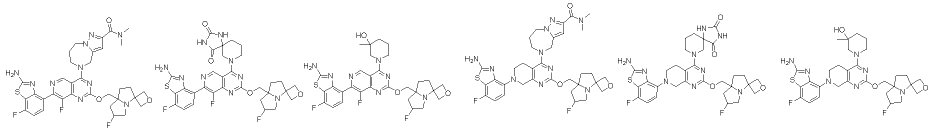

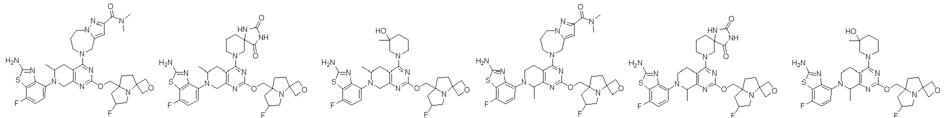

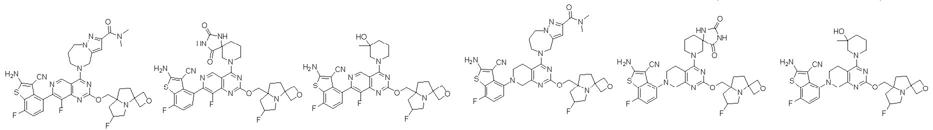

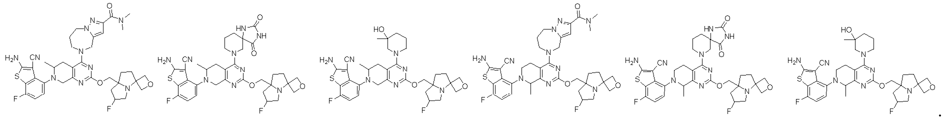

In another preferred embodiment, the compound is preferably a compound prepared in the examples.

In a second aspect of the present disclosure, provided is a method for preparing the compound of formula (I), or the stereoisomer, the tautomer, the crystalline form, the pharmaceutically acceptable salt, the hydrate, the solvate or the prodrug thereof, which comprises the steps of: 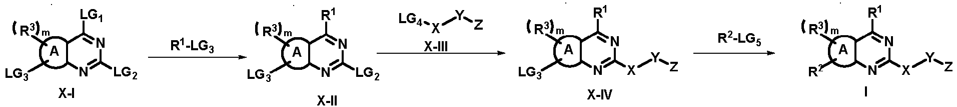
(i) reacting a compound of formula X-I with a compound R¹-LG₃ in an inert solvent, in the presence of a base, with or without a Pd catalyst, and with or without a condensing agent, to give a compound of formula X-II;
(ii) reacting the compound of formula X-II with a compound of formula X-III in an inert solvent, in the presence of a base and with or without a Pd catalyst, to give a compound of formula X-IV; and
(iii) reacting the compound of formula X-IV with a compound of formula V-6 (R²-LG₅) in an inert solvent, in the presence of a base and with or without a Pd catalyst, to give the compound of formula I; LG₁, LG₂, LG₃, LG₄, and LG₅ are leaving groups, each independently selected from: H, OH, halogen, OTf, OTs, OMs, -B(OH)₂, -B(KBF₃), -Sn(ⁿBu)₃, 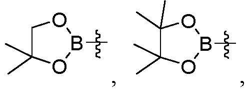 and the like; the Pd catalyst is selected from: Pd(OAc)2, Pd(dba)2, Pd2(dba)3, XPhos PdG2, RuPhos PdG2, XantPhos-Pd-G2, cataCXium A-Pd-G2, XPhos PdG3, RuPhos PdG3, XantPhos-Pd-G3, cataCXium A-Pd-G3, BrettPhos PdG3, SPhos PdG3, tBuXPhos-Pd-G3, XantPhos-Pd-G4, BrettPhos PG4, SPhos Pd G4, cataCXium A-Pd-G4, Rockphos PdG4, and the like; R¹, R², R³, ring A, X, Y, Z, and m are as defined above.

In a third aspect of the present disclosure, provided is a pharmaceutical composition comprising one or more compounds, or stereoisomers, tautomers, crystalline forms, pharmaceutically acceptable salts, hydrates, solvates or prodrugs thereof according to the first aspect; and a pharmaceutically acceptable carrier.

In another preferred embodiment, the pharmaceutical composition further comprises a drug selected from the group consisting of: PD-1 inhibitors (e.g., nivolumab, pembrolizumab, pidilizumab, cemiplimab, JS-001, SHR-120, BGB-A317, IBI-308, GLS-010, GB-226, STW204, HX008, HLX10, BAT 1306, AK105, LZM 009, or biosimilars thereof), PD-L1 inhibitors (e.g., durvalumab, atezolizumab, avelumab, CS1001, KN035, HLX20, SHR-1316, BGB-A333, JS003, CS1003, KL-A167, F520, GR1405, MSB2311, or biosimilars thereof), CD20 antibodies (e.g., rituximab, obinutuzumab, ofatumumab, veltuzumab, tositumomab, 131I-tositumomab, ibritumomab, 90Y-ibritumomab, 90In-ibritumomab, and ibritumomab tiuxetan), CD47 antibodies (e.g., Hu5F9-G4, CC-90002, TTI-621, TTI-622, OSE-172, SRF-231, ALX-148, NI-1701, SHR-1603, IBI188, and IMM01), ALK inhibitors (e.g., ceritinib, alectinib, brigatinib, lorlatinib, and ocatinib), PI3K inhibitors (e.g., idelalisib, duvelisib, dactolisib, taselisib, bimiralisib, omipalisib, and buparlisib), BTK inhibitors (e.g., ibrutinib, tirabrutinib, acalabrutinib, zanubrutinib, and vecabrutinib), EGFR inhibitors (e.g., afatinib, gefitinib, erlotinib, lapatinib, dacomitinib, icotinib, canertinib, sapitinib, naquotinib, pyrotinib, rociletinib, and osimertinib), VEGFR inhibitors (e.g., sorafenib, pazopanib tablet, regorafenib, sitravatinib, ningetinib, cabozantinib, sunitinib, and donafenib), HDAC inhibitors (e.g., givinostat, tucidinostat, vorinostat, fimepinostat, droxinostat, entinostat, dacinostat, quisinostat, and tacedinaline), CDK inhibitors (e.g., palbociclib, ribociclib, abemaciclib, milciclib, trilaciclib, and lerociclib), MEK inhibitors (e.g., selumetinib (AZD6244), trametinib (GSK1120212), PD0325901, U0126, pimasertib (AS-703026), and PD184352 (CI-1040)), mTOR inhibitors (e.g., vistusertib), SHP2 inhibitors (e.g., RMC-4630, JAB-3068, and TNO155), SOS1 inhibitors (e.g., BI 1701963), and combinations thereof.

In a fourth aspect of the present disclosure, provided is use of the compound, or the stereoisomer, the tautomer, the crystalline form, the pharmaceutically acceptable salt, the hydrate, the solvate or the prodrug thereof according to the first aspect, or the pharmaceutical composition according to the third aspect in the preparation of a medicament for preventing and/or treating a disease associated with the activity or expression level of a KRAS mutation (e.g., a KRAS G12 mutation), wherein preferably, the disease is a tumor or a disorder.

In another preferred embodiment, the disease is selected from the group consisting of: lung cancer, breast cancer, prostate cancer, esophageal cancer, colorectal cancer, bone cancer, kidney cancer, gastric cancer, liver cancer, colon cancer, melanoma, lymphoma, blood cancer, brain tumor, myeloma, soft tissue sarcoma, pancreatic cancer, and skin cancer.

In a fifth aspect of the present disclosure, provided is a non-diagnostic and non-therapeutic method for inhibiting a KRAS mutation (e.g., a KRAS G12 mutation), which comprises the step of: administering to a subject in need an effective amount of the compound, or the stereoisomer, the tautomer, the crystalline form, the pharmaceutically acceptable salt, the hydrate, the solvate or the prodrug thereof according to the first aspect, or the pharmaceutical composition according to the third aspect.

In another preferred embodiment, the subject is a mammal, preferably a human.

In a sixth aspect of the present disclosure, provided is a method for inhibiting the activity of a KRAS mutation (e.g., a KRAS G12 mutation) *in vitro,* which comprises the step of: contacting the compound, or the stereoisomer, the tautomer, the crystalline form, the pharmaceutically acceptable salt, the hydrate, the solvate or the prodrug thereof according to the first aspect, or the pharmaceutical composition according to the third aspect with a protein or a cell, thereby inhibiting the activity of the KRAS mutation (e.g., KRAS ^{G12D}).

In another preferred embodiment, the cell is selected from the group consisting of: macrophages, intestinal cells (including intestinal stem cells and intestinal epithelial cells), and a combination thereof.

In another preferred embodiment, the cell is derived from a rodent (e.g., mouse or rat) or a primate (e.g., human).

It will be appreciated that within the scope of the present disclosure, the above various technical features of the present disclosure and the technical features specifically described below (as in the examples) can be combined with each other to constitute a new or preferred technical solution. Due to limited space, such combinations will not be recited herein.

### DETAILED DESCRIPTION

The inventors, through long-term and in-depth studies, have unexpectedly prepared a novel class of compounds having selective inhibitory effects on KRAS mutations (e.g., KRAS G12 mutations) and/or better pharmacodynamic performance. On this basis, the inventors completed the present disclosure.

### Terms

In the present disclosure, unless otherwise specified, the terms used have the general meanings known to those skilled in the art.

The term "alkyl" refers to a linear or branched alkyl group containing 1 to 20 carbon atoms, such as 1 to 18 carbon atoms, especially 1 to 18 carbon atoms, preferably 1 to 10 carbon atoms (C1-C10), more preferably 1 to 6 carbon atoms (C1-C6). Typical "alkyl" groups include methyl, ethyl, propyl, isopropyl, *n*-butyl, *tert*-butyl, isobutyl, 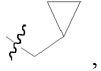 pentyl, isopentyl, heptyl, 4,4-dimethylpentyl, octyl, 2,2,4-trimethylpentyl, nonyl, decyl, undecyl, dodecyl, and the like. In the present disclosure, the alkyl also includes substituted alkyl. "Substituted alkyl" refers to an alkyl group in which one or more positions are substituted, particularly with 1 to 4 substituents, and the substitution may occur at any position. Typical substituents include, but are not limited to, one or more of the following groups: such as hydrogen, deuterium, halogen (e.g., a monohalogen substituent or polyhalogen substituent such as trifluoromethyl or alkyl containing Cl₃), cyano, nitro, oxygen (e.g., =O), trifluoromethyl, trifluoromethoxy, cycloalkyl, alkenyl, cycloalkenyl, alkynyl, heterocyclic ring, aromatic ring, ORₐ, SRₐ, S(=O)Rₑ, S(=O)₂Rₑ, P(=O)₂Rₑ, S(=O)₂ORₑ, P(=O)₂ORₑ, NR_{d}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein Rₐ may independently represent hydrogen, deuterium, C1-C6 alkyl, C3-C8 cycloalkyl, C2-C6 alkenyl, C3-C6 cycloalkenyl, C2-C6 alkynyl, 5- to 14-membered heterocyclic ring, or C6-C14 aromatic ring; R_{b}, R_{c}, and R_{d} may independently represent hydrogen, deuterium, C1-C6 alkyl, C3-C8 cycloalkyl, 5- to 14-membered heterocyclic ring, or C6-C14 aromatic ring, or R_{b} and R_{c}, together with the N atom, may form a heterocyclic ring; Rₑ may independently represent hydrogen, C1-C6 alkyl, C3-C8 cycloalkyl, C2-C6 alkenyl, C3-C6 cycloalkenyl, C2-C6 alkynyl, 5- to 14-membered heterocyclic ring, or C6-C14 aromatic ring. The above typical substituents, such as alkyl, cycloalkyl, alkenyl, cycloalkenyl, alkynyl, heterocyclic ring, or aromatic ring, may be optional for substitution.

The term "alkylene" refers to a group formed by further removal of one hydrogen atom from an "alkyl or substituted alkyl" group, such as methylene, ethylene, propylene, isopropylene (e.g., 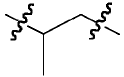 ), butylene (e.g., 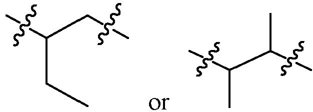 ), pentylene (e.g., 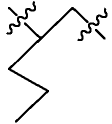 ), hexylene (e.g., 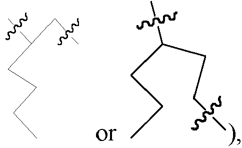 heptylene (e.g., 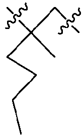 ), and 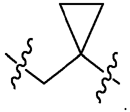 In addition, the term also includes an alkylene group (e.g., C1-C18 alkylene) in which one methylene group is replaced by a cycloalkylene group (e.g., C3-C20 cycloalkylene), such as "C1-C18 alkylene C3-C20 cycloalkylene" or "C3-C20 cycloalkylene C1-C18 alkylene".

The terms "C1-C18 alkylene C3-C20 cycloalkylene" and "C3-C20 cycloalkylene C1-C18 alkylene" have the same meaning and refer to a group formed by removal of two hydrogen atoms from a cycloalkylalkyl or alkylcycloalkyl group, such as 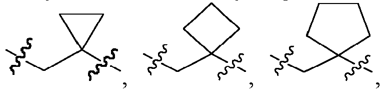 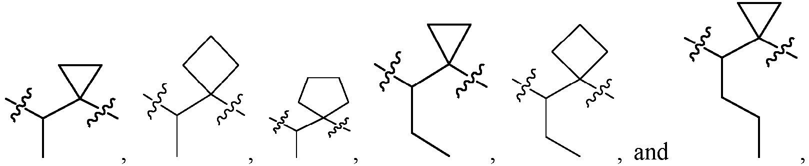 preferably C1-C6 alkylene C3-C6 cycloalkylene.

In the present disclosure, the term "alkenyl" represents a linear or branched hydrocarbon group containing one or more double bonds and generally having a length of 2 to 20 carbon atoms. The alkenyl is preferably C2-C6 alkenyl, more preferably C2-C4 alkenyl. The alkenyl includes, but is not limited to, vinyl, propenyl, butenyl, 1-methyl-2-buten-1-yl, and the like. In the present disclosure, the alkenyl includes substituted alkenyl.

The term "alkynyl" represents a linear or branched hydrocarbon group containing one or more triple bonds and generally having a length of 2 to 20 carbon atoms. The alkynyl is preferably C2-C6 alkynyl, more preferably C2-C4 alkynyl. The alkynyl includes, but is not limited to, ethynyl, propynyl, or similar groups thereof. In the present disclosure, the alkynyl also includes substituted alkynyl, where the substituent may be halo, hydroxy, cyano, nitro, or the like.

In the present disclosure, the term "cycloalkyl" refers to a fully saturated cyclic hydrocarbon group including 1 to 4 rings each containing 3 to 8 carbon atoms. The term "C₃-C₂₀ cycloalkyl" refers to a cycloalkyl group containing 3, 4, 5, 6, 7, 8, 9, 10, 11, 12, 13, 14, 15, 16, 17, 18, 19, or 20 carbon atoms. The cycloalkyl is preferably C₃-C₁₄ cycloalkyl, more preferably C₃-C₁₀ cycloalkyl, more preferably C₃-C₆ monocyclic cycloalkyl or C₇-C₁₀ bicyclic or tricyclic cycloalkyl. "Substituted cycloalkyl" refers to a cycloalkyl group in which one or more positions are substituted, particularly with 1 to 4 substituents, and the substitution may occur at any position. In the present disclosure, the "cycloalkyl" includes substituted cycloalkyl. Typical substituents include, but are not limited to, one or more of the following groups: such as hydrogen, deuterium, halogen (e.g., a monohalogen substituent or polyhalogen substituent such as trifluoromethyl or alkyl containing Cl₃), cyano, nitro, oxygen (e.g., =O), trifluoromethyl, trifluoromethoxy, cycloalkyl, alkenyl, cycloalkenyl, alkynyl, heterocyclic ring, aromatic ring, ORₐ, SRₐ, S(=O)Rₑ, S(=O)₂Rₑ, P(=O)₂Rₑ, S(=O)₂ORₑ, P(=O)₂ORₑ, NR_{d}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{d}R_{c}, NR_{d}P(=O)₂NR_{b}R_{c}, NR_{b}C(=O)Rₐ, or NR_{b}P(=O)₂Rₑ, wherein Rₐ may independently represent hydrogen, deuterium, alkyl, cycloalkyl, alkenyl, cycloalkenyl, alkynyl, heterocyclic ring, or aromatic ring; R_{b}, R_{c}, and R_{d} may independently represent hydrogen, deuterium, alkyl, cycloalkyl, heterocyclic ring, or aromatic ring, or R_{b} and R_{c}, together with the N atom, may form a heterocyclic ring; Rₑ may independently represent hydrogen, deuterium, alkyl, cycloalkyl, alkenyl, cycloalkenyl, alkynyl, heterocyclic ring, or aromatic ring. The above typical substituents may be optional for substitution. Typical substituents also include spiro, pyrimidine-fused or fused ring substituents, particularly spirocycloalkyl, spirocycloalkenyl, spiroheterocyclic ring (excluding heteroaromatic ring), pyrimidine-fused cycloalkyl, pyrimidine-fused cycloalkenyl, pyrimidine-fused heterocyclic ring (excluding heteroaromatic ring), fused cycloalkyl, fused cycloalkenyl, fused heterocyclyl, or fused aromatic ring groups, and the above cycloalkyl, cycloalkenyl, heterocyclyl, and heteroaryl groups may be optional for substitution. Examples of cycloalkyl include, but are not limited to, cyclopropyl, cyclobutyl, cyclopentyl, cyclohexyl, adamantyl, and the like.

The term "C3-C20 cycloalkylene" refers to a group formed by removal of two hydrogen atoms from cycloalkyl, such as:

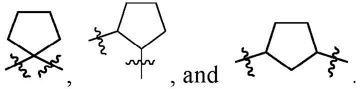

In the present disclosure, the term "heterocyclyl" refers to a fully saturated or partially unsaturated cyclic group (including but not limited to, for example, 3- to 7-membered monocyclic, 4- to 7-membered monocyclic, 6- to 11-membered bicyclic, or 8- to 16-membered tricyclic or polycyclic ring systems), wherein at least one heteroatom is present in a ring containing at least one carbon atom. The term "4- to 20-membered heterocyclyl" refers to a heterocyclyl group containing 4, 5, 6, 7, 8, 9, 10, 11, 12, 13, 14, 15, 16, 17, 18, 19, or 20 ring atoms. "Heterocyclyl" and "saturated or unsaturated heterocyclyl" have the same meaning. "Heterocyclyl" is preferably 4- to 14-membered heterocyclyl (including but not limited to, for example, 4- to 6-membered monocyclic, 7- to 10-membered bicyclic, or 8- to 14-membered tricyclic or polycyclic ring systems), more preferably 4- to 12-membered heterocyclyl, more preferably 4- to 10-membered heterocyclyl, such as 4- to 6-membered monocyclic heterocyclyl, 7- to 11-membered bicyclic or tricyclic heterocyclyl, more preferably 4- to 8-membered heterocyclyl, more preferably 4- to 6-membered heterocyclyl. Each heteroatom-containing heterocyclic ring may have 1, 2, 3, or 4 heteroatoms selected from nitrogen atoms, oxygen atoms, and sulfur atoms, among which the nitrogen atoms or the sulfur atoms may be oxidized and the nitrogen atoms may also be quaternized. The heterocyclyl group may be attached to any heteroatom or carbon atom residue of the ring or ring system molecule, preferably to an N or C atom on the ring or ring system molecule. Typical monocyclic heterocyclic rings include, but are not limited to, azetidinyl, pyrrolidinyl, oxetanyl, pyrazolinyl, imidazolinyl, imidazolidinyl, oxazolidinyl, isoxazolidinyl, thiazolidinyl, isothiazolidinyl, tetrahydrofuranyl, piperidinyl, piperazinyl, 2-oxopiperazinyl, 2-oxopiperidinyl, 2-oxopyrrolidinyl, hexahydroazepinyl, 4-piperidone, tetrahydropyranyl, morpholinyl, thiomorpholinyl, thiomorpholinosulfoxyl, thiomorpholinosulfonyl, 1,3-dioxanyl, tetrahydro-1,1-dioxythiophene, and the like. Polycyclic heterocyclyl groups include spiro, fused and pyrimidine-fused heterocyclyl groups; the spiro, fused and pyrimidine-fused heterocyclyl groups involved are optionally attached to other groups by single bonds or further attached to other cycloalkyl, heterocyclyl, aryl and heteroaryl groups by any two or more atoms on the rings; the heterocyclyl group may be substituted or unsubstituted, and when it is substituted, the substituent is preferably one or more groups independently selected from the group consisting of alkyl, deuterated alkyl, haloalkyl, alkoxy, haloalkoxy, alkenyl, alkynyl, alkylthio, alkylamino, halogen, amino, nitro, hydroxy, sulfhydryl, cyano, cycloalkyl, heterocyclyl, aryl, heteroaryl, cycloalkylthio, oxo, carboxyl, and a carboxylate group.

The term "4- to 20-membered heterocyclylene" refers to a group formed by removal of two hydrogen atoms from heterocyclyl, such as:

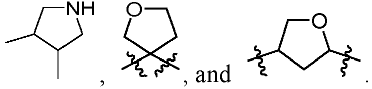

In the present disclosure, the term "aryl" refers to an aromatic cyclic hydrocarbon group having 1 to 5 rings, and particularly refers to monocyclic and bicyclic groups. "C₆-C₁₄ aryl" refers to an aromatic cyclic hydrocarbon group containing 6, 7, 8, 9, 10, 11, 12, 13, or 14 ring carbon atoms.

The aryl includes phenyl, biphenyl, or naphthyl. Any aryl group containing two or more aromatic rings (e.g., bicyclic rings, etc.) may have its aromatic rings attached by single bonds (e.g., biphenyl) or fused (e.g., naphthalene, anthracene, etc.). "Substituted aryl" refers to an aryl group in which one or more positions are substituted, particularly with 1 to 3 substituents, and the substitution may occur at any position. Typical substituents include, but are not limited to, one or more of the following groups: such as hydrogen, deuterium, halogen (e.g., a monohalogen substituent or polyhalogen substituent such as trifluoromethyl or alkyl containing Cl₃), cyano, nitro, oxo (e.g., =O), trifluoromethyl, trifluoromethoxy, cycloalkyl, alkenyl, cycloalkenyl, alkynyl, heterocyclic ring, aromatic ring, ORₐ, SRₐ, S(=O)Rₑ, S(=O)₂Rₑ, P(=O)₂Rₑ, S(=O)₂ORₑ, P(=O)₂ORₑ, NR_{d}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein Rₐ may independently represent hydrogen, deuterium, alkyl, cycloalkyl, alkenyl, cycloalkenyl, alkynyl, heterocyclyl, or aryl; R_{b}, R_{c}, and R_{d} may independently represent hydrogen, deuterium, alkyl, cycloalkyl, heterocyclic ring, or aromatic ring, or R_{b} and R_{c}, together with the N atom, may form a heterocyclic ring; Rₑ may independently represent hydrogen, deuterium, alkyl, cycloalkyl, alkenyl, cycloalkenyl, alkynyl, heterocyclyl, or aryl. The above typical substituents may be optional for substitution. Typical substituents also include fused ring substituents, particularly fused cycloalkyl, fused cycloalkenyl, fused heterocyclyl, or fused aromatic ring groups, and the above cycloalkyl, cycloalkenyl, heterocyclyl, and heteroaryl groups may be optional for substitution.

The term "heteroaryl" refers to an aromatic cyclic hydrocarbon group containing 1 to 4 heteroatoms selected from oxygen, nitrogen, and sulfur. "5- to 14-membered heteroaryl" refers to a heteroaromatic system containing 1 to 4 heteroatoms and 5 to 14 ring atoms. The heteroaryl is preferably a 5- to 10-membered ring, more preferably a 5- or 6-membered ring, such as pyrrolyl, pyrazolyl, imidazolyl, oxazolyl, isoxazolyl, thiazolyl, thiadiazolyl, isothiazolyl, furanyl, pyridyl, pyrazinyl, pyrimidinyl, pyridazinyl, triazinyl, triazolyl, and tetrazolyl. "Heteroaryl" may be substituted or unsubstituted, and when it is substituted, the substituent is preferably one or more groups independently selected from the group consisting of alkyl, deuterated alkyl, haloalkyl, alkoxy, haloalkoxy, alkenyl, alkynyl, alkylthio, alkylamino, halogen, amino, nitro, hydroxy, sulfhydryl, cyano, cycloalkyl, heterocyclyl, aryl, heteroaryl, cycloalkylthio, oxo, carboxyl, and a carboxylate group.

In the present disclosure, the term "alkoxy" refers to a linear or branched alkoxy group, including alkyl-O- and alkyl-O-alkyl; "C₁-C₁₈ alkoxy" refers to a linear or branched alkoxy group having 1 to 18 carbon atoms, including C₁-C₁₈ alkyl-O- and -C₁-C₆ alkyl-O-C₁-C₆ alkyl; non-limiting examples include methoxy, ethoxy, propoxy, isopropoxy, butoxy, and the like. C₁-C₈ alkoxy is preferred, and C1-C₆ alkoxy is more preferred.

In the present disclosure, the term "cycloalkyloxy" refers to cycloalkyl-O-; "C₃-C₂₀ cycloalkyloxy" refers to C₃-C₂₀ cycloalkyl-O-, wherein the C₃-C₂₀ cycloalkyl is as defined above.

In the present disclosure, the term "heterocyclyloxy" refers to heterocyclyl-O-; "4- to 20-membered heterocyclyloxy" refers to 4- to 20-membered heterocyclyl-O-, wherein the 4- to 20-membered heterocyclyl is as defined above.

In the present disclosure, the term "C₁-C₁₈ alkyleneoxy" refers to a group formed by removal of one hydrogen atom from "C₁-C₁₈ alkoxy".

In the present disclosure, the term "halogen" or "halo-" refers to chlorine, bromine, fluorine, or iodine.

In the present disclosure, the term "halo" or "halogenated" refers to substitution with halogen. In the present disclosure, the term "deuterated" refers to substitution with deuterium.

In the present disclosure, the term "hydroxy" refers to a group with the structure OH.

In the present disclosure, the term "nitro" refers to a group with the structure NO₂.

In the present disclosure, the term "cyano" refers to a group with the structure CN.

In the present disclosure, the term "ester group" refers to a group with the structure -COOR, wherein R represents hydrogen, alkyl or substituted alkyl, cycloalkyl or substituted cycloalkyl, cycloalkenyl or substituted cycloalkenyl, aryl or substituted aryl, or a heterocyclic ring or a substituted heterocyclic ring. The ester group is preferably -COOC₁-C₆ alkyl.

The term "amino" refers to a group with a structure -NRR', wherein R and R' may independently represent hydrogen, alkyl or substituted alkyl, cycloalkyl or substituted cycloalkyl, cycloalkenyl or substituted cycloalkenyl, aryl or substituted aryl, or a heterocyclic ring or a substituted heterocyclic ring, as defined above. R and R' may be identical or different in a dialkylamine moiety. The amino is preferably NH₂, NHC₁-C₆ alkyl, or N(C₁-C₆ alkyl)₂.

The term "amido" refers to a group with a structure -CONRR', wherein R and R' may independently represent hydrogen, alkyl or substituted alkyl, cycloalkyl or substituted cycloalkyl, cycloalkenyl or substituted cycloalkenyl, aryl or substituted aryl, or a heterocyclic ring or a substituted heterocyclic ring, as defined above. R and R' may be identical or different in a dialkylamine moiety. The amido is preferably CONH₂, NHCO(C₁-C₆ alkyl), or NHCO(C₃-C₆ cycloalkyl).

The term "sulfonyl" refers to a group with a structure -SO₂R, wherein R may independently represent hydrogen, alkyl or substituted alkyl, cycloalkyl or substituted cycloalkyl, cycloalkenyl or substituted cycloalkenyl, aryl or substituted aryl, or a heterocyclic ring or a substituted heterocyclic ring, as defined above.

The term "sulfonamido" refers to a group with a structure -SO₂NRR', wherein R and R' may independently represent hydrogen, alkyl or substituted alkyl, cycloalkyl or substituted cycloalkyl, cycloalkenyl or substituted cycloalkenyl, aryl or substituted aryl, or a heterocyclic ring or a substituted heterocyclic ring, as defined above. The sulfonamido is preferably-SO₂NH₂, - SO₂NH(C₁-C₆ alkyl), or -SO₂NH(C₃-C₆ cycloalkyl).

The term "ureido" refers to a group with a structure -NRCONR'R", wherein R, R', and R" may independently represent hydrogen, alkyl or substituted alkyl, cycloalkyl or substituted cycloalkyl, cycloalkenyl or substituted cycloalkenyl, aryl or substituted aryl, or a heterocyclic ring or a substituted heterocyclic ring, as defined above. R, R', and R" may be identical or different in a dialkylamine moiety.

The term "alkylaminoalkyl" refers to a group with a structure -RNHR', wherein R and R' may independently represent hydrogen, alkyl or substituted alkyl, cycloalkyl or substituted cycloalkyl, cycloalkenyl or substituted cycloalkenyl, aryl or substituted aryl, or a heterocyclic ring or a substituted heterocyclic ring, as defined above. R and R' may be identical or different.

The term "dialkylaminoalkyl" refers to a group with a structure -RNHR'R", wherein R, R', and R" may independently represent alkyl or substituted alkyl, cycloalkyl or substituted cycloalkyl, cycloalkenyl or substituted cycloalkenyl, aryl or substituted aryl, or a heterocyclic ring or a substituted heterocyclic ring, as defined above. R, R', and R" may be identical or different in a dialkylamine moiety.

The term "heterocyclylalkyl" refers to a group with a structure -RR', wherein R may independently represent alkyl or substituted alkyl, cycloalkyl or substituted cycloalkyl, cycloalkenyl or substituted cycloalkenyl, or aryl or substituted aryl, and R' represents a heterocyclic ring or a substituted heterocyclic ring.

In the present disclosure, the term "substitute" means that one or more hydrogen atoms on a specific group are replaced by specific substituents. The specific substituents are those described correspondingly in the preceding text or those appearing in the various examples. Unless otherwise specified, a substituted group may have a substituent selected from a specific group at any substitutable position of that group, and the substituents at the various positions may be identical or different. It should be understood by those skilled in the art that combinations of substituents contemplated by the present disclosure are those that are stable or chemically available. The substituents are, for example (but not limited to): halogen, hydroxy, cyano, carboxyl (-COOH), C1-C6 alkyl, C2-C6 alkenyl, C2-C6 alkynyl, C3-C8 cycloalkyl, 3- to 12-membered heterocyclyl, aryl, heteroaryl, C1-C8 formyl, C2-C10 acyl, C2-C10 ester group, amino, C1-C6 alkoxy, C1-C10 sulfonyl, C1-C6 ureido, and the like.

Unless otherwise specified, it is assumed that any heteroatom that is not in a sufficient valence state has sufficient hydrogen atoms to supplement its valence state.

When the substituents are non-terminal substituents, they are subunits of the corresponding groups. For example, alkyl corresponds to alkylene, cycloalkyl corresponds to cycloalkylene, heterocyclyl corresponds to heterocyclylene, and alkoxy corresponds to alkyleneoxy.

In the present disclosure, "more" refers to 2, 3, 4, or 5.

### Active Ingredient

As used herein, "compound of the present disclosure" refers to a compound represented by formula I, and also includes a stereoisomer or optical isomer, a pharmaceutically acceptable salt, a prodrug or a solvate of the compound of formula I.

The salts that may be formed by the compound of the present disclosure are also within the scope of the present disclosure. Unless otherwise specified, the compound of the present disclosure should be understood to include salts thereof. The term "salt" used herein refers to an acidic or basic salt formed from an inorganic or organic acid or a base. In addition, when the compound of the present disclosure contains a basic moiety, it includes, but is not limited to, pyridine or imidazole; when the compound of the present disclosure contains an acidic moiety, it includes, but is not limited to, carboxylic acid; and zwitter-ions ("inner salts") that may be formed are included within the scope of the term "salt". Pharmaceutically acceptable (i.e., non-toxic, physiologically acceptable) salts are preferred, although other salts are useful, for example, in separation or purification steps during the preparation process. The compound of the present disclosure may form salts. For example, the compound I reacts with an appropriate (e.g., an equivalent amount) of an acid or a base, and a salt is obtained by salting out from a medium or by lyophilization in an aqueous solution.

The compound of the present disclosure may contain basic moieties, including but not limited to amine or pyridine or imidazole rings, which may form salts with organic or inorganic acids. Typical acids that may form salts include acetate (e.g., those formed with acetic acid or trihaloacetic acid, such as trifluoroacetic acid), adipate, alginate, ascorbate, aspartate, benzoate, benzenesulfonate, bisulfate, borate, butyrate, citrate, camphorate, camphorsulfonate, cyclopentanepropionate, diglycolate, dodecyl sulfate, ethanesulfonate, fumarate, glucoheptonate, glycerophosphate, hemisulfate, heptanoate, hexanoate, hydrochloride, hydrobromide, hydriodate, hydroxyethanesulfonate (e.g., 2-hydroxyethanesulfonate), lactate, maleate, methanesulfonate, naphthalenesulfonate (e.g., 2-naphthalenesulfonate), nicotinate, nitrate, oxalate, pectate, persulfate, phenpropionate (e.g., 3-phenpropionate), phosphate, picrate, pivalate, propionate, salicylate, succinate, sulfate (e.g., those formed with sulfuric acid), sulfonate, tartrate, thiocyanate, toluenesulfonate (e.g., *p*-toluenesulfonate), dodecanoate, and the like.

Certain compounds of the present disclosure may contain acidic moieties, including but not limited to carboxylic acids, which may form salts with various organic or inorganic bases. Typical salts formed by bases include: ammonium salts; alkali metal salts, such as sodium, lithium, and potassium salts; alkaline earth metal salts, such as calcium and magnesium salts; salts formed with organic bases (e.g., organic amines), such as benzathine, dicyclohexylamine, hydrabamine (a salt formed with *N*,*N*-di(dehydroabietyl)ethylenediamine), *N*-methyl-*D*-glucamine, *N*-methyl-D-glucamide, and *tert*-butylamine; and salts formed with amino acids such as arginine and lysine. Basic nitrogen-containing groups can form quaternary ammonium salts with halides, such as small molecule alkyl halides (e.g., methyl, ethyl, propyl, and butyl chlorides, bromides and iodides), dialkyl sulfates (e.g., dimethyl, diethyl, dibutyl and dipentyl sulfates), long-chain halides (e.g., decyl, dodecyl, tetradecyl and hexadecyl chlorides, bromides and iodides), and aralkyl halides (e.g., benzyl and phenyl bromides).

Prodrugs and solvates of the compounds of the present disclosure also fall within the scope of the present disclosure. The term "prodrug" herein refers to a compound that undergoes chemical transformation through a metabolic or chemical process to give a compound, salt, or solvate of the present disclosure when used in the treatment of a related disease. The compounds of the present disclosure include solvates, such as hydrates.

The compounds, salts or solvates of the present disclosure may exist in tautomeric forms (e.g., amides and imine ethers). All these tautomers are part of the present disclosure.

Stereoisomers of all the compounds (e.g., those asymmetric carbon atoms that may exist due to various substitutions), including enantiomeric and diastereomeric forms thereof, shall fall within the scope of the present disclosure. The independent stereoisomers of the compounds of the present disclosure may not exist simultaneously with other isomers (e.g., as a pure or substantially pure optical isomer with special activity), or may be present as a mixture, such as a racemate, or as a mixture formed with all or part of the other stereoisomers. The chiral centers of the present disclosure have either the S or R configuration, as defined by the recommendations of the International Union of Pure and Applied Chemistry (IUPAC) in 1974. The racemic forms may be resolved by physical methods, such as fractional crystallization, derivatization into diastereoisomers followed by separation through crystallization, or separation using chiral column chromatography. Individual optical isomers may be obtained from racemates by suitable methods, including but not limited to conventional methods, such as salt formation with an optically active acid followed by crystallization.

The content, by weight, of the compound of the present disclosure, which is obtained by preparation, separation and then purification, is equal to or greater than 90%, e.g., is equal to or greater than 95%, or is equal to or greater than 99% ("very pure" compound), as listed in the text description. Herein, such "very pure" compounds of the present disclosure are also part of the present disclosure.

All configurational isomers of the compounds of the present disclosure fall within the scope of the present disclosure, whether in mixture, pure or very pure form. The definition of the compounds of the present disclosure includes *cis* (*Z*) and *trans* (*E*) isomers of alkenes, as well as cis and trans isomers of carbocyclic and heterocyclic rings.

Throughout the specification, groups and substituents can be selected to provide stable moieties and compounds.

Specific functional groups and definitions of chemical terms are described in detail below. For the present disclosure, the chemical elements are consistent with those defined in Periodic Table of the Elements, CAS version, Handbook of Chemistry and Physics, 75th Ed. The definitions of specific functional groups are also described herein. In addition, the basic principles of organic chemistry, as well as specific functional groups and the reactivity thereof are also described in "Organic Chemistry", Thomas Sorrell, University Science Books, Sausalito: 1999, which is incorporated by reference in its entirety.

Certain compounds of the present disclosure may exist in the form of specific geometric isomers or stereoisomers. The present disclosure encompasses all the compounds, including cis and trans isomers, R and S enantiomers, diastereoisomers, (D) isomers, (L) isomers, racemic mixtures, and other mixtures. In addition, the asymmetric carbon atoms may represent substituents, such as alkyl groups. All the isomers and mixtures thereof are encompassed within the present disclosure. According to the present disclosure, the ratio of isomers in a mixture of the isomers may be varied. For example, in a mixture of only two isomers, there may be the following combinations: 50:50, 60:40, 70:30, 80:20, 90:10, 95:5, 96:4, 97:3, 98:2, 99:1, or 100:0; all ratios of the isomers fall within the scope of the present disclosure. Ratios similar to those easily understood by those of ordinary skill in the art, as well as ratios for mixtures of more complex isomers, also fall within the scope of the present disclosure.

The present disclosure also includes isotopically labeled compounds, equivalent to the disclosure of the original compounds herein. In fact, however, it usually occurs that one or more atoms are substituted with atoms with different atomic weights or mass numbers. Examples of isotopes that may be listed as part of the compounds of the present disclosure include isotopes of hydrogen, carbon, nitrogen, oxygen, phosphorus, sulfur, fluorine, and chlorine, such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl. The compounds or the enantiomers, diastereoisomers, isomers or pharmaceutically acceptable salts or solvates thereof (containing the isotopes of the above compounds or other isotope atoms) in the present disclosure are all within the scope of the present disclosure. Certain isotopically labeled compounds of the present disclosure, e.g., including radioisotopes of ³H and ¹⁴C, are also useful in experiments on the tissue distribution of drugs and substrates. Tritium (i.e., ³H) and carbon-14 (i.e.,¹⁴C) are relatively easy to prepare and detect. They are the first choice among isotopes. In addition, heavier isotope substitutions, such as deuterium (i.e., ²H), have advantages (e.g., increased *in vivo* half-life or reduced dose) in certain therapies due to their good metabolic stability and hence may be preferred in some cases. The isotopically labeled compounds may be prepared using general methods by replacing non-isotopically labeled reagents with readily available isotopically labeled reagents according to the schemes disclosed in the examples.

If the synthesis of a specific enantiomer of a compound of the present disclosure is to be designed, it can be prepared through asymmetric synthesis or by derivatization using a chiral auxiliary reagent and separation of the resulting diastereomeric mixture, followed by removal of the chiral auxiliary reagent to give a pure enantiomer. In addition, if the molecule contains a basic functional group (e.g., amino acid) or an acidic functional group (e.g., carboxyl), it can form a diastereoisomer salt with an appropriate optically active acid or base, and separation can then be carried out by conventional means such as fractional crystallization or chromatography to give a pure enantiomer.

As described herein, the compounds of the present disclosure may be substituted with any number of substituents or functional groups to expand the scope of the present disclosure. Generally, the term "substitute", whether appearing before or after the term "optionally", refers to the replacement of a hydrogen free radical by a specified structural substituent in a general formula containing substituents in a formulation of the present disclosure. When a plurality of positions in a specific structure are substituted with a plurality of specific substituents, each substituent at each position may be identical or different. The term "substitute" used herein includes substitution by all allowed organic compounds. In a broad sense, the allowed substituents include acyclic, cyclic, branched, unbranched, carbocyclic, heterocyclic, aromatic, and nonaromatic organic compounds. In the present disclosure, for example, the heteroatom nitrogen may be substituted with hydrogen or any of the allowed organic compounds mentioned above to supplement its valence state. In addition, the present disclosure is not intended to limit the organic compounds allowed for substitution in any way. The present disclosure believes that the combination of substituents and variable groups is excellent in the treatment of diseases, such as infectious diseases or proliferative diseases, in the form of stable compounds. The term "stable" herein means that a compound is stable enough to maintain its structural integrity when detected for a sufficiently long duration, preferably remaining effective for a sufficiently long duration, as used herein for the purpose described above.

Metabolites of the compounds and the pharmaceutically acceptable salts thereof involved in the present application, as well as prodrugs that can be transformed into the structures of the compounds and the pharmaceutically acceptable salts thereof involved in the present application in vivo, are also included in the claims of the present application. In the present disclosure, the term "prodrug" refers to a compound obtained by chemically modifying a drug to introduce a leaving group, which is inactive or less active *in vitro* but undergoes transformation *in vivo* (either enzyme-mediated (e.g., hydrolase, oxidase, etc.) or non-enzyme-mediated (e.g., pH, light, radiation, etc.)) to release the active drug and exert its pharmacological effect. Such prodrugs include, but are not limited to: carboxylates, phosphates, carbamates, carbonates, and the like. The structures of the leaving groups include (but are not limited to):

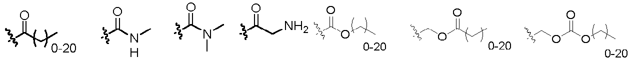

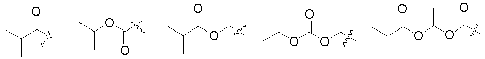

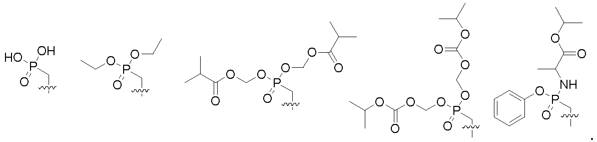

### Preparation Method

Methods for preparing the compound having the structure of formula (I) of the present disclosure are described in more detail below, but these specific methods do not limit the present disclosure in any way. The compounds of the present disclosure can also be conveniently prepared by optionally combining various synthetic methods described herein or known in the art, and such combinations can be easily determined by those skilled in the art to which the present disclosure pertains.

Typically, the process for preparing the compound of the present disclosure is as follows, in which the materials and reagents used are commercially available unless otherwise specified. Preferably, the compound of the present disclosure is prepared by the following method: 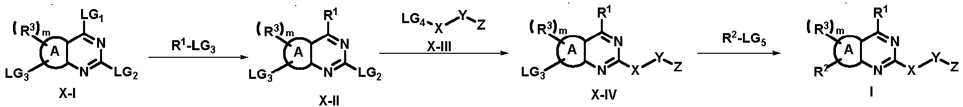
(i) reacting a compound of formula X-I with a compound R¹-LG₃ in an inert solvent, in the presence of a base, with or without a Pd catalyst, and with or without a condensing agent, to give a compound of formula X-II;
(ii) reacting the compound of formula X-II with a compound of formula X-III in an inert solvent, in the presence of a base and with or without a Pd catalyst, to give a compound of formula X-IV; and
(iii) reacting the compound of formula X-IV with a compound of formula V-6 (R²-LG₅) in an inert solvent, in the presence of a base and with or without a Pd catalyst, to give the compound of formula I;

LG₁, LG₂, LG₃, LG₄, and LG₅ are leaving groups, each independently selected from: H, OH, halogen, OTf, OTs, OMs, -B(OH)₂, -B(KBF₃), -Sn(ⁿBu)₃, 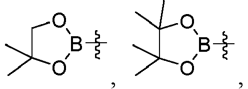 and the like;
the Pd catalyst is selected from: Pd(OAc)2, Pd(dba)2, Pd2(dba)3, XPhos Pd G2, RuPhos Pd G2, XantPhos-Pd-G2, cataCXium A-Pd-G2, XPhos Pd G3, RuPhos Pd G3, XantPhos-Pd-G3, cataCXium A-Pd-G3, BrettPhos Pd G3, SPhos Pd G3, tBuXPhos-Pd-G3, XantPhos-Pd-G4, BrettPhos Pd G4, SPhos Pd G4, cataCXium A-Pd-G4, Rockphos Pd G4, and the like;
R¹, R², R³, ring A, X, Y, Z, and m are as defined above.

### Pharmaceutical Composition and Administration Route

The pharmaceutical composition disclosed herein is used for preventing and/or treating the following diseases: inflammation, cancer, cardiovascular diseases, infections, immune diseases, and metabolic diseases.

The compound of general formula (I) may be administered in combination with other drugs known to treat or ameliorate similar conditions. During administration in combination, the administration route and dose of the original drug may remain unchanged, and the compound of formula I may be administered simultaneously or subsequently. When the compound of formula I is administered simultaneously with one or more other drugs, a pharmaceutical composition containing one or more known drugs and the compound of formula I may be preferably used. The administration of the drugs in combination also includes the administration of the compound of formula I and one or more other known drugs in overlapping time periods. When the compound of formula I is administered in combination with one or more other drugs, the doses of the compound of formula I or the known drugs may be lower than their respective doses when administered alone.

Drugs or active ingredients that may be administered in combination with the compound of general formula (I) include, but are not limited to: PD-1 inhibitors (e.g., nivolumab, pembrolizumab, pidilizumab, cemiplimab, JS-001, SHR-120, BGB-A317, IBI-308, GLS-010, GB-226, STW204, HX008, HLX10, BAT 1306, AK105, LZM 009, or biosimilars thereof), PD-L1 inhibitors (e.g., durvalumab, atezolizumab, avelumab, CS1001, KN035, HLX20, SHR-1316, BGB-A333, JS003, CS1003, KL-A167, F520, GR1405, MSB2311, or biosimilars thereof), CD20 antibodies (e.g., rituximab, obinutuzumab, ofatumumab, veltuzumab, tositumomab, 131I-tositumomab, ibritumomab, 90Y-ibritumomab, 90In-ibritumomab, and ibritumomab tiuxetan), CD47 antibodies (e.g., Hu5F9-G4, CC-90002, TTI-621, TTI-622, OSE-172, SRF-231, ALX-148, NI-1701, SHR-1603, IBI188, and IMM01), ALK inhibitors (e.g., ceritinib, alectinib, brigatinib, lorlatinib, and ocatinib), PI3K inhibitors (e.g., idelalisib, duvelisib, dactolisib, taselisib, bimiralisib, omipalisib, and buparlisib), BTK inhibitors (e.g., ibrutinib, tirabrutinib, acalabrutinib, zanubrutinib, and vecabrutinib), EGFR inhibitors (e.g., afatinib, gefitinib, erlotinib, lapatinib, dacomitinib, icotinib, canertinib, sapitinib, naquotinib, pyrotinib, rociletinib, and osimertinib), VEGFR inhibitors (e.g., sorafenib, pazopanib, regorafenib, sitravatinib, ningetinib, cabozantinib, sunitinib, and donafenib), HDAC inhibitors (e.g., givinostat, tucidinostat, vorinostat, fimepinostat, droxinostat, entinostat, dacinostat, quisinostat, and tacedinaline), CDK inhibitors (e.g., palbociclib, ribociclib, abemaciclib, milciclib, trilaciclib, and lerociclib), MEK inhibitors (e.g., selumetinib (AZD6244), trametinib (GSK1120212), PD0325901, U0126, pimasertib (AS-703026), and PD184352 (CI-1040)), mTOR inhibitors (e.g., vistusertib), SHP2 inhibitors (e.g., RMC-4630, JAB-3068, and TNO155), or combinations thereof. Dosage forms of the pharmaceutical composition disclosed herein include (but are not limited to): injections, tablets, capsules, aerosols, suppositories, films, dripping pills, topical liniments, controlled-release or sustained-release or nanometer formulations.

The pharmaceutical composition of the present disclosure comprises a safe and effective amount of the compound or the pharmacologically acceptable salt thereof of the present disclosure, as well as a pharmacologically acceptable excipient or carrier, wherein the "safe and effective amount" refers to an amount of the compound sufficient to significantly improve the condition without causing severe side effects. Generally, the pharmaceutical composition comprises 1-2000 mg of the compound of the present disclosure per dose, more preferably 10-1000 mg of the compound of the present disclosure per dose. Preferably, the "dose" is a single capsule or tablet. "Pharmaceutically acceptable carrier" refers to one or more compatible solid or liquid fillers or gel substances that are suitable for human use and must be of sufficient purity and sufficiently low toxicity. "Compatible" herein means that the components of the composition are capable of intermixing with the compound of the present disclosure and with each other, without significantly diminishing the pharmaceutical efficacy of the compound. Examples of the pharmaceutically acceptable carriers include cellulose and derivatives thereof (e.g., sodium carboxymethylcellulose, sodium ethylcellulose, and cellulose acetate), gelatin, talc, solid lubricants (e.g., stearic acid and magnesium stearate), calcium sulfate, vegetable oils (e.g., soybean oil, sesame oil, peanut oil, and olive oil), polyols (e.g., propylene glycol, glycerol, mannitol, and sorbitol), emulsifiers (e.g., Tween^{®}), wetting agents (e.g., sodium dodecyl sulfate), colorants, flavoring agents, stabilizers, antioxidants, preservatives, pyrogen-free water, and the like.

The administration routes of the compound or the pharmaceutical composition of the present disclosure are not specifically limited, typically including (but not limited to): oral administration, intratumoral administration, rectal administration, parenteral (intravenous, intramuscular or subcutaneous) administration, and topical administration.

Solid dosage forms for oral administration include capsules, tablets, pills, pulvises, and granules. In these solid dosage forms, the active compound is mixed with at least one conventional inert excipient (or carrier), such as sodium citrate or calcium phosphate, or mixed with the following ingredients: (a) fillers or extenders, such as starch, lactose, sucrose, glucose, mannitol, and silicic acid; (b) binders, such as hydroxymethyl cellulose, alginate, gelatin, polyvinylpyrrolidone, sucrose, and acacia; (c) humectants, such as glycerol; (d) disintegrants, such as agar, calcium carbonate, potato starch or tapioca starch, alginic acid, certain complex silicates, and sodium carbonate; (e) solution retarders, such as paraffin; (f) absorption accelerators, such as quaternary amine compounds; (g) wetting agents, such as cetyl alcohol and glycerol monostearate; (h) adsorbents, such as kaolin; (i) lubricants, such as talc, calcium stearate, magnesium stearate, solid polyethylene glycol, and sodium dodecyl sulfate, or mixtures thereof. In the case of capsules, tablets, and pills, the dosage forms may also include buffers.

Solid dosage forms, such as tablets, dragees, capsules, pills, and granules, can be prepared using coatings and shell materials, such as enteric coatings and other materials well known in the art. They may comprise opacifying agents, and the active compound or compound in such a composition may be released in a certain part of the digestive tract in a delayed manner. Examples of embedding components that can be used are polymeric substances and wax-based substances. If necessary, the active compound may also be formulated into a microcapsule form with one or more of the aforementioned excipients.

Liquid dosage forms for oral administration include pharmaceutically acceptable emulsions, solutions, suspensions, syrups, or tinctures. In addition to the active compound, the liquid dosage forms may comprise inert diluents commonly used in the art, such as water or other solvents, solubilizers and emulsifiers, for example, ethanol, isopropanol, ethyl carbonate, ethyl acetate, propylene glycol, 1,3-butanediol, dimethylformamide, and oils, especially cottonseed oil, peanut oil, corn germ oil, olive oil, castor oil and sesame oil, or mixtures of these substances.

Besides such inert diluents, the composition may further include adjuvants, such as wetting agents, emulsifiers, suspending agents, sweeteners, corrigents, and perfuming agents.

In addition to the active compound, the suspensions may comprise suspending agents, such as ethoxylated isostearyl alcohol, polyoxyethylene sorbitol and sorbitan esters, microcrystalline cellulose, aluminium methoxide and agar, or mixtures of these substances.

Compositions for parenteral injection may include physiologically acceptable sterile aqueous or anhydrous solutions, dispersions, suspensions or emulsions, and sterile powders for redissolving into sterile injectable solutions or dispersions. Suitable aqueous and non-aqueous carriers, diluents, solvents or excipients include water, ethanol, polyols, and suitable mixtures thereof.

Dosage forms of the compound of the present disclosure for topical administration include ointments, pulvises, patches, sprays, and inhalants. The active ingredient is mixed under sterile conditions with a physiologically acceptable carrier and any preservatives, buffers or propellants that may be required if necessary.

The treatment method of the present disclosure may be applied alone or in combination with other therapeutic means or therapeutic drugs.

When the pharmaceutical composition is used, a safe and effective amount of the compound of the present disclosure is administered to a mammal (e.g., a human) in need of treatment, wherein the administration dose is a pharmaceutically effective administration dose. For a human weighing 60 kg, the daily administration dose is usually 1-2000 mg, preferably 50-1000 mg. Of course, the specific dose should also take into account factors such as the administration route and the patient's health condition, all of which are within the skill set of a skilled physician. The present disclosure further provides a method for preparing the pharmaceutical composition, which comprises the step of: mixing a pharmaceutically acceptable carrier with the compound of general formula (I) or the crystalline form, the pharmaceutically acceptable salt, the hydrate or the solvate thereof disclosed herein to form the pharmaceutical composition.

The present disclosure further provides a treatment method, which comprises the step of: administering to a subject in need of treatment the compound of general formula (I), or the crystalline form, the pharmaceutically acceptable salt, the hydrate or the solvate thereof disclosed herein, or the pharmaceutical composition disclosed herein to selectively inhibit a KRAS mutation (e.g., a KRAS G12 mutation).

**Compared with the prior art, the present disclosure has the following main advantages:**
(1) the compound has a good selective inhibitory effect on KRAS G12 mutations; and
(2) the compound has better pharmacodynamic and pharmacokinetic properties, and lower toxic and side effects.

The present disclosure will be further illustrated with reference to the following specific examples. It should be understood that these examples are merely intended to illustrate the present disclosure rather than limit the scope of the present disclosure. In the following examples, experimental methods without specifically stated conditions are generally conducted under conventional conditions such as those described in Sambrook et al., Molecular Cloning: A Laboratory Manual (New York: Cold Spring Harbor Laboratory Press, 1989), or conditions recommended by the manufacturers. Unless otherwise specified, percentages and parts are calculated by weight.

Unless otherwise defined, all technical and scientific terms used herein have the same meaning as commonly understood by those skilled in the art. In addition, any methods and materials similar or equivalent to those described herein can be applied in the methods of the present disclosure. The preferred embodiments and materials described herein are for illustrative purposes only.

The compound structures of the present disclosure were determined by nuclear magnetic resonance (NMR) and liquid chromatography-mass spectrometry (LC-MS).

The NMR analysis was performed using a Bruker AVANCE-400 nuclear magnetic resonance instrument, with deuterated dimethyl sulfoxide (DMSO-d₆), deuterated acetone (CD₃COCD₃), deuterated chloroform (CDCl₃), deuterated methanol (CD₃OD) and the like as determination solvents and tetramethylsilane (TMS) as an internal standard. The chemical shifts are reported in parts per million (ppm).

Liquid chromatography-mass spectrometry (LC-MS) was performed using a Waters SQD2 mass spectrometer. HPLC determination was performed using an Agilent 1100 high-pressure chromatograph (Microsorb 5 micron C18 100 × 3.0 mm column).

Thin-layer chromatography (TLC) was performed using Qingdao GF254 silica gel plates. The specification adopted for TLC was 0.15-0.20 mm, and the specification adopted for preparative thin-layer chromatography was 0.4-0.5 mm. Qingdao silica gel with a particle size of 200-300 mesh was generally adopted as a carrier in column chromatography.

Starting materials in the examples of the present disclosure are all known and commercially available, or can be synthesized by adopting or according to the literature reported in the art. Unless otherwise specified, all the reactions in the present disclosure are carried out under the protection of a dry inert gas (e.g., nitrogen or argon) with continuous magnetic stirring, and all the reaction temperatures are in degrees Celsius.

### Examples

### Preparation of intermediate 1-1: (3-(methoxymethyl)tetrahydro-1H-pyrrolizin-7a(5H)-yl)methanol

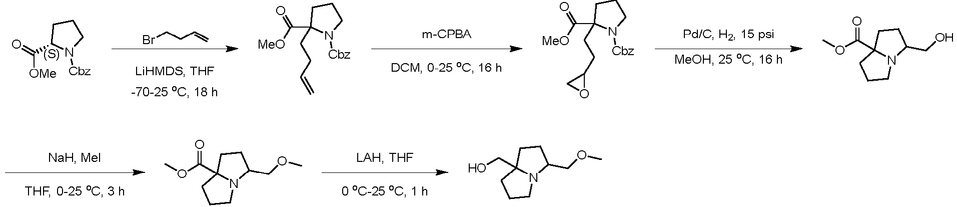

### Step 1: Preparation of 1-benzyl 2-methyl 2-(but-3-en-1-yl)pyrrolidine-1,2-dicarboxylate

A solution of 1-benzyl 2-methyl (S)-pyrrolidine-1,2-dicarboxylate (50.0 g, 190 mmol, 1.00 eq) in THF (100 mL) was added dropwise to LiHMDS (1.00 M, 285 mL, 1.50 *eq)* at -70 °C under nitrogen atmosphere. The resulting reaction solution was reacted at -70 °C for 2 h, followed by dropwise addition of 4-bromobut-1-ene (51.2 g, 380 mmol, 38.6 mL, 2.00 *eq).* The resulting reaction solution was warmed to 25 °C and reacted for 16 h, then quenched with a saturated aqueous NH₄Cl solution (200 mL), and extracted with EtOAc (100 mL × 3). The organic phases were combined, washed with saturated brine (200 mL), then dried over anhydrous sodium sulfate, and filtered. The filtrate was concentrated under reduced pressure to give the target product (38.0 g, 120 mmol, yield: 63.1%).

¹H NMR (400 MHz, CDCl₃) δ 7.33 (m, 5 H), 5.77 (m, 1 H), 5.07 (m, 4 H), 3.72 (m, 3 H), 3.48 (m, 2 H), 2.01 (m, 8 H).

### Step 2: Preparation of 1-benzyl 2-methyl 2-(2-(oxiran-2-yl)ethyl)pyrrolidine-1,2-dicarboxylate

To a solution of 1-benzyl 2-methyl 2-(but-3-en-1-yl)pyrrolidine-1,2-dicarboxylate (37.5 g, 118 mmol, 1.00 *eq)* in DCM (650 mL) was added m-CPBA (60.0 g, 295 mmol, purity: 85.0%, 2.50 *eq)* in portions at 0 °C. The resulting reaction solution was reacted at 25 °C for 16 h and then washed with saturated Na₂SO₃ (300 mL × 3). The organic phase was dried over anhydrous sodium sulfate and then filtered. The filtrate was concentrated under reduced pressure, and the residue was separated by silica gel column chromatography to give the target product (29.0 g, 87.0 mmol, yield: 73.6%).

¹H NMR (400 MHz, CDCl₃) δ 7.33 (br d, J=7.88 Hz, 5 H), 5.11 (m, 2 H), 3.70 (m, 3 H), 3.48 (m, 2 H), 2.73 (m, 2 H), 2.36 (m, 2 H), 1.98 (m, 5 H), 1.50 (m, 2 H).

### Step 3: Preparation of methyl 3-(hydroxymethyl)tetrahydro-1H-pyrrolizine-7a(5H)-carboxylate

To a solution of 1-benzyl 2-methyl 2-(2-(oxiran-2-yl)ethyl)pyrrolidine-1,2-dicarboxylate (28.0 g, 84.0 mmol, 1.00 *eq)* in MeOH (600 mL) was added Pd/C (6.00 g, 10.0% wt). The reaction mixture was reacted at 25 °C for 16 h under hydrogen atmosphere and then filtered. The filtrate was concentrated under reduced pressure to give the target product (16.8 g), which was used directly in the next step without purification.

### Step 4: Preparation of methyl 3-(methoxymethyl)tetrahydro-1H-pyrrolizine-7a(5H)-carboxylate

To a solution of 3-(hydroxymethyl)tetrahydro-1H-pyrrolizine-7a(5H)-carboxylate (1.00 g, 5.02 mmol, 1.00 *eq)* in THF (20 mL) was added NaH (602 mg, 15.1 mmol, 60.0% wt, 3.00 *eq)* at 0 °C under nitrogen atmosphere. The reaction solution was reacted at 25 °C for 0.5 h, followed by addition of MeI (1.42 g, 10.0 mmol, 625 µL, 2.00 *eq).* The resulting reaction solution was reacted at 25 °C for 3 h, then quenched with H₂O (10 mL) at 0 °C, and extracted with EtOAc (30 mL × 2). The organic phases were combined, dried over anhydrous magnesium sulfate, and then filtered. The filtrate was concentrated under reduced pressure, and the residue was separated by silica gel column chromatography to give the target product (1.0 g, 1.88 mmol, yield: 37.5%).

¹H NMR (400 MHz, CDCl₃) δ 3.66 - 3.72 (m, 3H), 3.30 - 3.33 (m, 3 H), 2.22 - 2.43 (m, 1 H), 1.83 - 2.12 (m, 6 H), 1.56 - 1.72 (m, 1 H).

### Step 5: Preparation of (3-(methoxymethyl)tetrahydro-1H-pyrrolizin-7a(5H)-yl)methanol

To a solution of 3-(methoxymethyl)tetrahydro-1H-pyrrolizine-7a(5H)-carboxylate (1.00 g, 4.69 mmol, 1.00 *eq)* in THF (10 mL) was added LiAlH₄ (356 mg, 9.38 mmol, 2.00 *eq)* at 0 °C under nitrogen atmosphere. The reaction solution was reacted at 25 °C for 1 h, then quenched with sequential addition of H₂O (10 mL), 15% NaOH (10 mL) and H₂O (30 mL) at 0 °C, and extracted with EtOAc (50 mL × 2). The organic phases were combined, dried over anhydrous magnesium sulfate, and then filtered. The filtrate was concentrated under reduced pressure, and the residue was separated by silica gel column chromatography to give the target product (1.1 g).

LC-MS: m/z 186 (M+H)⁺. ¹H NMR (400 MHz, CDCl₃) δ 4.39 (dd, *J*=10.54, 9.03 Hz, 1 H), 4.26 (br d, *J*=6.53 Hz, 1 H), 3.97 - 4.06 (m, 1 H), 3.80 - 3.96 (m, 2 H), 3.68 - 3.76 (m, 2 H), 3.50 (s, 3 H), 2.04 - 2.40 (m, 6 H), 1.78 - 1.93 (m, 2 H).

### SFC separation was carried out to give isomer A intermediate 1-1A and isomer B intermediate 1-1B:

### Intermediate 1-1A: cis-(3-(methoxymethyl)tetrahydro-1H-pyrrolizin-7a(5H)-yl)methanol

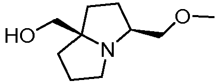

LC-MS: m/z 186 (M+H)⁺.

### Intermediate 1-1B: trans-(3-(methoxymethyl)tetrahydro-1H-pyrrolizin-7a(5H)-yl)methanol

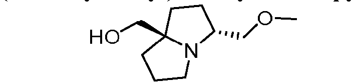

LC-MS: m/z 186 (M+H)⁺.

The following compounds were synthesized using the same synthetic method as intermediate 1-1 but with different starting materials:

### Intermediate 1-2: (3-((ethoxy)methyl)tetrahydro-1H-pyrrolizin-7a(5H)-yl)methanol

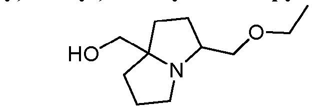

LC-MS: m/z 200 (M+H)⁺.

### Intermediate 1-3: (3-((isopropoxy)methyl)tetrahydro-1H-pyrrolizin-7a(5H)-yl)methanol

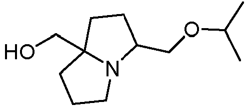

LC-MS: m/z 214 (M+H)⁺.

### Intermediate 1-4: (3-((cyclopropylmethoxy)methyl)tetrahydro-1H-pyrrolizin-7a(5H)-yl)methanol

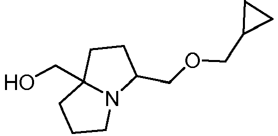

LC-MS: m/z 226 (M+H)⁺.

### Intermediate 1-5: (3-(cyclopropoxymethyl)tetrahydro-1H-pyrrolizin-7a(5H)-yl)methanol

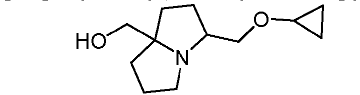

LC-MS: m/z 212 (M+H)⁺.

### Intermediate 1-6: ((2R)-2-fluoro-5-(methoxymethyl)tetrahydro-1H-pyrrolizin-7a(5H)-yl)methanol

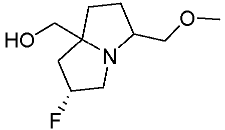

LC-MS: m/z 204 (M+H)⁺.

### Intermediate 1-7: ((2R)-2-fluoro-5-(cyclopropoxymethyl)tetrahydro-1H-pyrrolizin-7a(5H)-yl)methanol

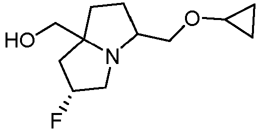

LC-MS: m/z 230 (M+H)⁺.

### Preparation of intermediate 2-1: ((2-fluoro-6-(methoxymethoxy)-8-(4,4,5,5-tetramethyl-1,3,2-dioxaborolan-2-yl)naphthalen-1-yl)ethynyl)triisopropylsilane

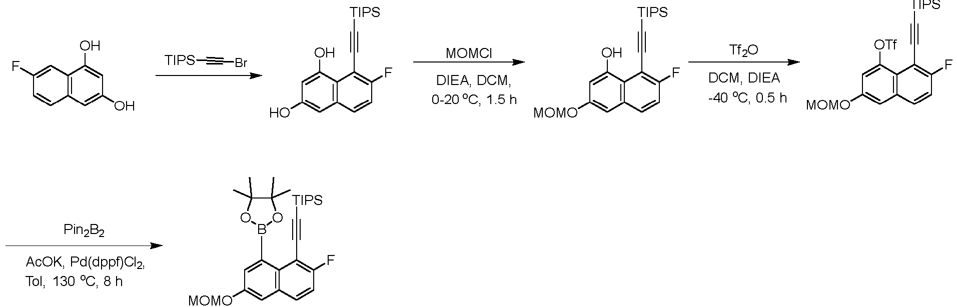

### Step 1: Preparation of 7-fluoro-8-((triisopropylsilyl)ethynyl)naphthalene-1,3-diol

To a solution of 7-fluoronaphthalene-1,3-diol (5 g, 28.1 mmol, 1 eq), (bromoethynyl)triisopropylsilane (7.33 g, 28.1 mmol, 1 eq), and potassium acetate (5.51 g, 56.1 mmol, 2 eq) in dioxane (120 mL) was added ruthenium dichloride 1-isopropyl-4-methyl-benzene (1.20 g, 1.96 mmol, 0.07 eq) at room temperature under nitrogen atmosphere. The reaction solution was reacted at 110 °C for 16 h and then filtered. The filter cake was washed with EtOAc (200 mL × 2). The organic phases were combined and concentrated under reduced pressure. The residue was separated by silica gel column chromatography to give the target product (8 g, 22.3 mmol, yield: 79.5%).

¹H NMR (400 MHz, CDCl₃) δ 9.19 (m, 1 H) 7.60 (dd, *J*=9.17, 5.62 Hz, 1 H), 7.18 (m, 1 H), 6.73 (m, 2 H), 5.29 (br s, 1 H), 1.22 (m, 21 H).

### Step 2: Preparation of 7-fluoro-3-(methoxymethoxy)-8-((triisopropylsilyl)ethynyl) naphthalen-1-ol

To a solution of 7-fluoro-8-((triisopropylsilyl)ethynyl)naphthalene-1,3-diol (7 g, 19.5 mmol, 1 eq) and DIEA (5.05 g, 39.0 mmol, 6.80 mL, 2 eq) in DCM (100 mL) was added MOMCl (1.66 g, 20.6 mmol, 1.57 mL, 1.06 eq) at 0 °C. The resulting reaction solution was reacted at 25 °C for 1.5 h, then quenched with H₂O (200 mL), adjusted to pH 6-7 with 1 N HCl, and extracted with DCM (80.0 mL × 2). The organic phases were combined, washed with saturated brine (200 mL), then dried over anhydrous sodium sulfate, and filtered. The filtrate was concentrated under reduced pressure, and the residue was separated by silica gel column chromatography to give the target product (4.2 g, 10.4 mmol, yield: 53.4%).

¹H NMR (400 MHz, DMSO-*d*₆) δ 8.94 - 9.20 (m, 1 H), 8.93 - 9.13 (m, 1 H), 7.58 (dd, *J*=9.07, 5.69 Hz, 1 H), 7.10 (t, *J*=8.82 Hz, 1 H), 6.89 (d, J=2.50 Hz, 1 H), 6.73 (d, *J*=1.88 Hz, 1 H), 5.12 - 5.24 (m, 2 H), 3.43 (s, 3 H), 1.09 - 1.13 (m, 21 H).

### Step 3: Preparation of 7-fluoro-3-(methoxymethoxy)-8-((triisopropylsilyl)ethynyl) naphthalen-1-yl trifluoromethanesulfonate

To a solution of 7-fluoro-3-(methoxymethoxy)-8-((triisopropylsilyl)ethynyl)naphthalen-1-ol (4 g, 9.94 mmol, 1 *eq)* in DCM (40 mL) were added DIEA (3.85 g, 29.8 mmol, 5.19 mL, 3 *eq)* and Tf₂O (4.21 g, 14.9 mmol, 2.46 mL, 1.5 *eq)* at -40 °C. The resulting reaction solution was reacted at -40 °C for 0.5 h, then diluted with H₂O (20 mL), and extracted with DCM (50 mL × 2). The organic phases were combined, washed with saturated brine (100 mL), then dried over anhydrous sodium sulfate, and filtered. The filtrate was concentrated under reduced pressure, and the residue was separated by silica gel column chromatography to give the target product as a yellow oil (5 g, 8.88 mmol, yield: 89.4%, purity: 95%).

¹H NMR (400 MHz, CDCl₃) δ 7.72 (dd, J=9.03, 5.27 Hz, 1 H), 7.44 (d, J=2.26 Hz, 1 H), 7.36 - 7.39 (m, 1 H), 7.31 - 7.35 (m, 1 H), 5.20 - 5.37 (m, 2 H), 3.46 - 3.63 (m, 3 H), 1.17 - 1.33 (m, 17 H).

### Step 4: Preparation of ((2-fluoro-6-(methoxymethoxy)-8-(4,4,5,5-tetramethyl-1,3,2-dioxaborolan-2-yl)naphthalen-1-yl)ethynyl)triisopropylsilane

To a solution of 7-fluoro-3-(methoxymethoxy)-8-((triisopropylsilyl)ethynyl)naphthalen-1-yl trifluoromethanesulfonate (5 g, 9.35 mmol, 1 eq), 4,4,5,5-tetramethyl-2-(4,4,5,5-tetramethyl-1,3,2-dioxaborolan-2-yl)-1,3,2-dioxaborolane (4.75 g, 18.70 mmol, 2 eq), and AcOK (2.75 g, 28.06 mmol, 3 eq) in toluene (100 mL) was added Pd(dppf)Cl₂ (684 mg, 935 µmol, 0.1 eq) under nitrogen atmosphere. The resulting reaction solution was reacted at 130 °C for 8 h and then filtered. The filtrate was concentrated under reduced pressure, and the residue was separated by silica gel column chromatography to give the target product (3.5 g, 6.49 mmol, yield: 69.3%). LC-MS: m/z 513 (M+H)⁺. ¹H NMR (400 MHz, CDCl₃) δ 7.68 (dd, J=8.93, 5.62 Hz, 1 H), 7.52 (d, J=2.45 Hz, 1 H), 7.39 (d, J=2.57 Hz, 1 H), 7.24 (t, J=8.80 Hz, 1 H), 5.18 - 5.39 (m, 2 H), 3.43 - 3.58 (m, 3 H), 1.45 (s, 12 H), 1.15 - 1.21 (m, 20 H).

The following compounds were synthesized using the same synthetic method as intermediate 2-2 but with different starting materials:

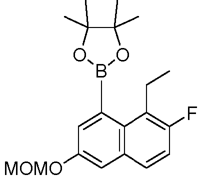

LC-MS: m/z 361 (M+H)⁺.

### Preparation of intermediate 3-1: 2,4,7-trichloro-8-fluoropyrido[4,3-d]pyrimidine

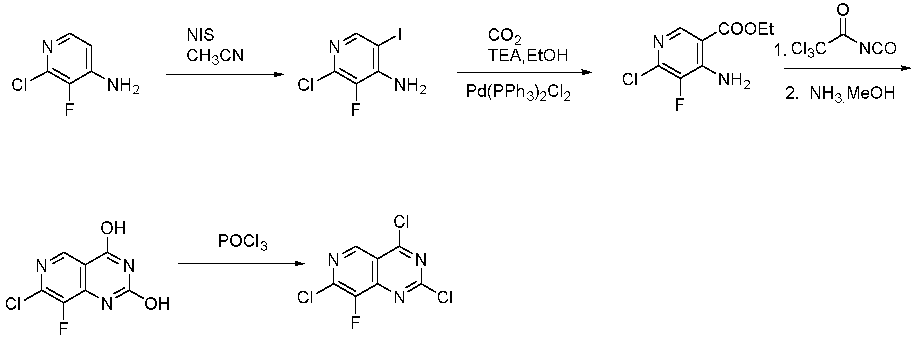

### Step 1: Preparation of 2-chloro-3-fluoro-5-iodopyridin-4-amine

To a solution of 2-chloro-3-fluoropyridin-4-amine (10 g, 68.5 mmol) and NIS (18.5 g, 82.2 mmol, 1.2 eq) in acetonitrile (50 mL) was added *p*-toluenesulfonic acid monohydrate (0.65 g, 3.43 mmol, 0.05 eq). The reaction solution was stirred at 70 °C for 16 h and then diluted with water (30 mL) and EtOAc (200 mL). The separated organic phase was washed sequentially with S. aq. Na₂CO₃, S. aq. Na₂SO₃, and saturated brine, dried over anhydrous sodium sulfate, and then filtered. The filtrate was concentrated under reduced pressure to give the target product (16.6 g, 61 mmol, yield: 89%), which was used directly in the next step without purification.

LC-MS: m/z 273 (M+H)⁺.

### Step 2: Preparation of ethyl 4-amino-6-chloro-5-fluoronicotinate

To a solution of 2-chloro-3-fluoro-5-iodopyridin-4-amine (8.2 g, 30 mmol, 1.0 eq) in EtOH (150 mL) were added Pd(PPh₃)₂Cl₂ (2.1 g, 3 mmol, 0.1 eq) and TEA (11.1 g, 0.11 mmol, 3.6 eq) under nitrogen atmosphere. The resulting reaction solution was reacted at 80 °C for 15 h under CO₂ atmosphere and then filtered. The filtrate was concentrated under reduced pressure to 70%-80% of the original volume and then filtered again. The filter cakes were collected, combined, and dried under vacuum to give the target product (quantitative yield), which was used directly in the next step without purification.

LC-MS: m/z 219 (M+H)⁺.

### Step 3: Preparation of 7-chloro-8-fluoropyrido[4,3-d]pyrimidine-2,4-diol

To a solution of the previously obtained ethyl 4-amino-6-chloro-5-fluoronicotinate (657 mg, 3 mmol) in THF (7 mL) was added trichloroacetyl isocyanate (673 mg, 3.6 mmol, 1.2 eq) at 0 °C. The reaction solution was reacted at rt for 30 min and then concentrated under reduced pressure. MeOH (15 mL) was added to the residue, and the mixture was cooled to 0 °C, followed by addition of a solution of NH₃ in methanol (7 M in MeOH, 15 mL, 105 mmol). The resulting reaction solution was reacted at rt for 16 h and then filtered. The filter cake was washed with methanol and then dried under vacuum to give the target product (quantitative yield), which was used directly in the next step without purification.

LC-MS: m/z 216 (M+H)⁺.

### Step 4: Preparation of 2,4,7-trichloro-8-fluoropyrido[4,3-d]pyrimidine

A solution of 7-chloro-8-fluoropyrido[4,3-*d*]pyrimidine-2,4-diol (500 mg, 2.4 mmol) and DIPEA (1.55 g, 12 mmol, 5.0 eq) in POCl₃ (5 mL) was reacted at 100 °C for 1 h and then concentrated under reduced pressure to give the target compound (quantitative yield), which was used directly in the next step without purification.

LC-MS: m/z 252 (M+H)⁺.

### Example A-1: Preparation of 1-(7-(8-ethyl-7-fluoro-3-hydroxynaphthalen-1-yl)-8-fluoro-2-(((cis)-3-(methoxymethyl)tetrahydro-1H-pyrrolizin-7a(5H)-yl)methoxy)pyrido[4,3-d]pyrimidin-4-yl)-3-methylpiperidin-3-ol

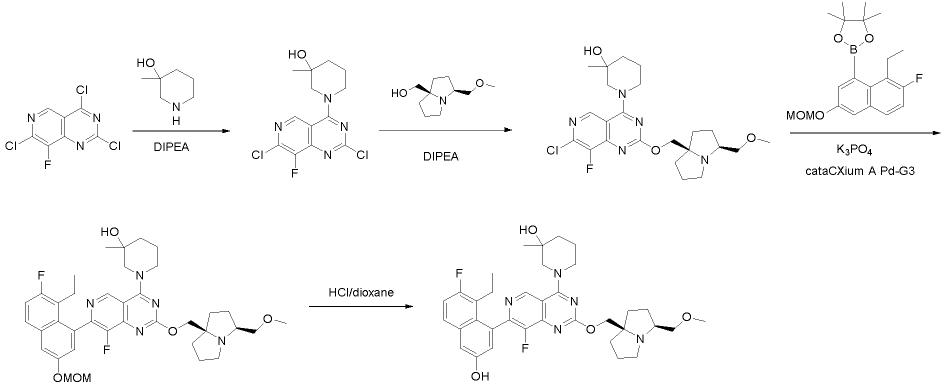

### Step 1: Preparation of 1-(2,7-dichloro-8-fluoropyrido[4,3-d]pyrimidin-4-yl)-3-methylpiperidin-3-ol

To a solution of 2,4,7-trichloro-8-fluoropyrido[4,3-*d*]pyrimidine (1 g, 4 mmol) and DIPEA (2.06 g, 16 mmol) in DCM (10 mL) was added 3-methylpiperidin-3-ol (0.46 g, 4 mmol) at -40 °C. The reaction solution was reacted at -40 °C for 0.5 h, then quenched with water (10 mL), and extracted with DCM (20 mL). The organic phase was dried over anhydrous sodium sulfate and then filtered. The filtrate was concentrated under reduced pressure, and the residue was separated using a medium-pressure column chromatograph to give the target product (0.53 g, yield: 40%).

LC-MS: m/z 331 (M+H)⁺.

### Step 2: Preparation of 1-(7-chloro-8-fluoro-2-(((cis)-3-(methoxymethyl)tetrahydro-1H-pyrrolizin-7a(5H)-yl)methoxy)pyrido[4,3-d)pyrimidin-4-yl)-3-methylpiperidin-3-ol

To a solution of 1-(2,7-dichloro-8-fluoropyrido[4,3-*d*]pyrimidin-4-yl)-3-methylpiperidin-3-ol (0.53 g, 1.6 mmol), ((*cis*)-3-(methoxymethyl)tetrahydro-1*H*-pyrrolizin-7a(5*H*)-yl)methanol (0.33 g, 1.78 mmol), and 4A molecular sieves (0.5 g) in dioxane (4 mL) was added DIPEA (0.62 g, 4.8 mmol). The reaction solution was reacted at 90 °C for 10 h and then filtered. Ethyl acetate (10 mL) and water (5 mL) were added to the filtrate, and the mixture was separated into layers. The aqueous phase was separated out and extracted with ethyl acetate (10 mL). The organic phases were combined, washed with saturated brine (5 mL), then dried over anhydrous sodium sulfate, and filtered. The filtrate was concentrated under reduced pressure, and the residue was separated by preparative liquid chromatography to give the target product (0.42 g, yield: 54%). LC-MS: m/z 480 (M+H)⁺.

### Step 3: Preparation of 1-(7-(8-ethyl-7-fluoro-3-(methoxymethoxy)naphthalen-1-yl)-8-fluoro-2-(((cis)-3-(methoxymethyl)tetrahydro-1H-pyrrolizin-7a(5H)-yl)methoxy)pyrido [4,3-d]pyrimidin-4-yl)-3-methylpiperidin-3-ol

To a solution of 1-(7-chloro-8-fluoro-2-(((*cis*)-3-(methoxymethyl)tetrahydro-1*H*-pyrrolizin-7a(5*H*)-yl)methoxy)pyrido[4,3-*d*]pyrimidin-4-yl)-3-methylpiperidin-3-ol (0.4 g, 0.83 mmol), 2-(8-ethyl-7-fluoro-3-(methoxymethoxy)naphthalen-1-yl)-4,4,5,5-tetramethyl-1,3,2-dioxaborolane (0.4 g, 1.12 mmol), and K₃PO₄ (1.5 M aqueous solution, 1.1 mL) in THF (2 mL) was added cataCXium A Pd-G3 (60 mg, 0.083 mmol) under nitrogen atmosphere. The reaction solution was reacted at 65 °C for 8 h. Ethyl acetate (3 mL) and water (2 mL) were then added, and the mixture was separated into layers. The aqueous phase was separated out and extracted with ethyl acetate (3 mL). The organic phases were combined, washed with saturated brine (2 mL), then dried over anhydrous sodium sulfate, and filtered. The filtrate was concentrated under reduced pressure, and the residue was separated by preparative liquid chromatography to give the target product (0.31 g, yield: 55%).

LC-MS: m/z 678 (M+H)⁺.

### Step 4: Preparation of 1-(7-(8-ethyl-7-fluoro-3-hydroxynaphthalen-1-yl)-8-fluoro-2-(((cis)-3-(methoxymethyl)tetrahydro-1H-pyrrolizin-7a(5H)-yl)methoxy)pyrido[4,3-d]pyrimidin-4-yl)-3-methylpiperidin-3-ol

To a solution of 1-(7-(8-ethyl-7-fluoro-3-(methoxymethoxy)naphthalen-1-yl)-8-fluoro-2-(((*cis*)-3-(methoxymethyl)tetrahydro-1*H*-pyrrolizin-7a(5*H*)-yl)methoxy)pyrido[4,3-*d*]pyrimidin-4-yl)-3-methylpiperidin-3-ol (0.3 g, 0.44 mmol) in acetonitrile (1.5 mL) was added HCl•dioxane (4 M, 1.5 mL) at 0 °C. The reaction solution was reacted at 0 °C for 0.5 h and then concentrated under reduced pressure. The residue was separated by preparative liquid chromatography to give the target product (86.5 g, yield: 31%).

LC-MS: m/z 634 (M+H)⁺.

**According to the method of Example A-1, the following examples were synthesized with different starting materials:**

### Example A-2: Preparation of 1-(7-(8-ethyl-7-fluoro-3-hydroxynaphthalen-1-yl)-8-fluoro-2-(((trans)-3-(methoxymethyl)tetrahydro-1H-pyrrolizin-7a(5H)-yl)methoxy)pyrido[4,3-d]pyrimidin-4-yl)-3-methylpiperidin-3-ol

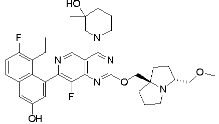

LC-MS: m/z 634 (M+H)⁺.

### Example A-3: Preparation of 1-(7-(8-ethyl-7-fluoro-3-hydroxynaphthalen-1-yl)-8-fluoro-2-(((2R)-2-fluoro-5-(methoxymethyl)tetrahydro-1H-pyrrolizin-7a(5H)-yl)methoxy)pyrido [4,3-d]pyrimidin-4-yl)-3-methylpiperidin-3-ol

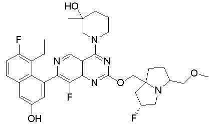

LCMS: m/z 652 (M+H)⁺.

### Example A-4: 7-(7-(8-Ethyl-7-fluoro-3-hydroxynaphthalen-1-yl)-8-fluoro-2-(((cis)-3-(methoxymethyl)tetrahydro-1H-pyrrolizin-7a(5H)-yl)methoxy)pyrido[4,3-d]pyrimidin-4-yl)-1,3,7-triazaspiro[4.5]decane-2,4-dione

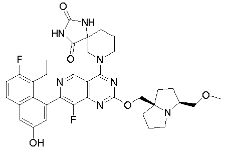

LCMS: m/z 688 (M+H)⁺.

### Example A-5: 7-(7-(8-Ethyl-7-fluoro-3-hydroxynaphthalen-1-yl)-8-fluoro-2-(((trans)-3-(methoxymethyl)tetrahydro-1H-pyrrolizin-7a(5H)-yl)methoxy)pyrido[4,3-d]pyrimidin-4-yl)-1,3,7-triazaspiro[4.5]decane-2,4-dione

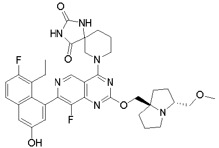

LCMS: m/z 688 (M+H)⁺.

### Example A-6: 7(7-(8-Ethyl-7-fluoro-3-hydroxynaphthalen-1-yl)-8-fluoro-2-(((2R)-2-fluoro-5-(methoxymethyl)tetrahydro-1H-pyrrolizin-7a(5H)-yl)methoxy)pyrido[4,3-d]pyrimidin-4-yl)-1,3,7-triazaspiro[4.5]decane-2,4-dione

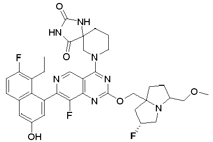

LCMS: m/z 706 (M+H)⁺.

### Example A-7: 5-(7-(8-Ethyl-7-fluoro-3-hydroxynaphthalen-1-yl)-8-fluoro-2-(((cis)-3-(methoxymethyl)tetrahydro-1H-pyrrolizin-7a(5H)-yl)methoxy)pyrido[4,3-d]pyrimidin-4-yl)-N,N-dimethyl-5,6,7,8-tetrahydro-4H-pyrazolo[1,5-a][1,4]diazepine-2-carboxamide

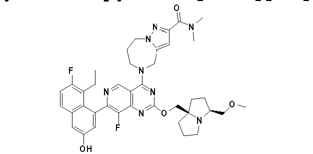

LCMS: m/z 727 (M+H)⁺.

### Example A-8: 5-(7-(8-Ethyl-7-fluoro-3-hydroxynaphthalen-1-yl)-8-fluoro-2-(((trans)-3-(methoxymethyl)tetrahydro-1H-pyrrolizin-7a(5H)-yl)methoxy)pyrido[4,3-d]pyrimidin-4-yl)-N,N-dimethyl-5,6,7,8-tetrahydro-4H-pyrazolo[1,5-a][1,4]diazepine-2-carboxamide

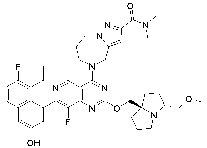

LCMS: m/z 727 (M+H)⁺.

### Example A-9: 5-(7-(8-Ethyl-7-fluoro-3-hydroxynaphthalen-1-yl)-8-fluoro-2-(((2R)-2-fluoro-5-(methoxymethyl)tetrahydro-1H-pyrrolizin-7a(5H)-yl)methoxy)pyrido[4,3-d]pyrimidin-4-yl)-N,N-dimethyl-5,6,7,8-tetrahydro-4H-pyrazolo[1,5-a][1,4]diazepine-2-carboxamide

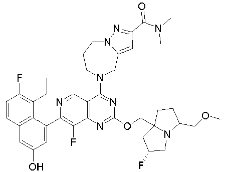

LCMS: m/z 745 (M+H)⁺.

### Example A-10: 1-(7-(8-Ethyl-7-fluoro-3-hydroxynaphthalen-1-yl)-8-fluoro-2-((1-(morpholinomethyl)cyclopropyl)methoxy)pyrido[4,3-d]pyrimidin-4-yl)-3-methylpiperidin-3-ol

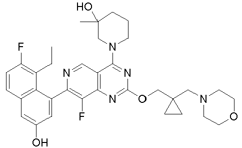

LCMS: m/z 620 (M+H)⁺.

### Example A-11: 7-(7-(8-Ethyl-7-fluoro-3-hydroxynaphthalen-1-yl)-8-fluoro-2-((1-(morpholinomethyl)cyclopropyl)methoxy)pyrido[4,3-d]pyrimidin-4-yl)-1,3,7-triazaspiro [4.5] decane-2,4-dione

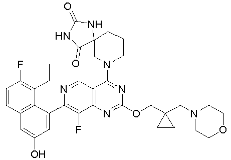

LCMS: m/z 674 (M+H)⁺.

### Example A-12: 5-(7-(8-Ethyl-7-fluoro-3-hydroxynaphthalen-1-yl)-8-fluoro-2-((1-(morpholinomethyl)cyclopropyl)methoxy)pyrido[4,3-d]pyrimidin-4-yl)-N,N-dimethyl-5,6,7,8-tetrahydro-4H-pyrazolo[1,5-a][1,4]diazepine-2-carboxamide

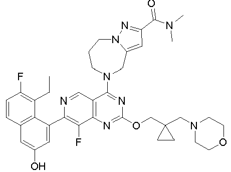

LCMS: m/z 713 (M+H)⁺.

### Example A-13: 1-(2-(((trans)-2,2-Difluorodihydro-1'H,3'H-spiro[cyclopropane-1,2'-pyrrolizin]-7a'(5'H)-yl)methoxy)-7-(8-ethyl-7-fluoro-3-hydroxynaphthalen-1-yl)-8-fluoropyrido[4,3-d]pyrimidin-4-yl)-3-methylpiperidin-3-ol

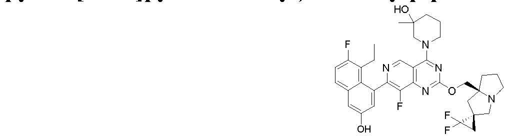

LCMS: m/z 652 (M+H)⁺. ¹H NMR (400MHz, DMSO-*d*₆) *δ* 9.93 (d, *J*=2.88Hz, 1 H), 9.23 (d, *J*=2.12Hz, 1 H), 7.62(dd, *J*=6.04Hz, *J*=9.12Hz,1 H), 7.33(m, 2 H) , 7.03 (d, *J*=2.60Hz, 1 H), 4.74(d, *J*=6.88Hz, 1 H), 4.26(m, 3 H), 4.05(m, 1 H), 3.62 (d, *J*=13.16Hz, 0.5 H), 3.52 (d, *J*=13.24Hz, 0.5 H), 3.40 (m, 1 H), 3.04(m,1 H), 2.74 (m, 1 H), 2.53 (m, 1 H), 2.33 (m,1 H), 1.77 (m, 13 H).

### Example A-13 was subjected to chiral resolution to give isomers A-13A and A-13B: (S)-1-(2-(((trans)-2,2-difluorodihydro-1'H,3'H-spiro[cyclopropane-1,2'-pyrrolizin]-7a'(5'H)-yl)methoxy)-7-(8-ethyl-7-fluoro-3-hydroxynaphthalen-1-yl)-8-fluoropyrido[4,3-d]pyrimidin-4-yl)-3-methylpiperidin-3-ol and (R)-1-(2-(((trans)-2,2-difluorodihydro-1'H,3'H-spiro[cyclopropane-1,2'-pyrrolizin]-7a'(5'H)-yl)methoxy)-7-(8-ethyl-7-fluoro-3-hydroxynaphthalen-1-yl)-8-fluoropyrido[4,3-d]pyrimidin-4-yl)-3-methylpiperidin-3-ol

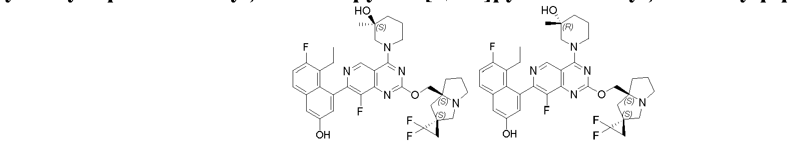

### Example A-13A

LCMS: m/z 652 (M+H)⁺. ¹H NMR (400MHz, DMSO-*d*₆) *δ* 11.46(br, 1 H), 10.09 (d,*J*=8.0Hz,1 H), 9.27 (s, 1 H), 7.76 (m, 1 H), 7.35(m, 2 H), 7.06(m, 1H), 4.78(m, 1 H), 4.49(m, 5 H), 4.08(m, 1 H), 3.60(m, 5 H), 2.02 (m, 13 H), 1.17 (d,*J*=8.0Hz, 3 H), 0.73(m,3 H).

### Example A-13B

LCMS: m/z 652 (M+H)⁺. ¹H NMR (400MHz, DMSO-*d*₆) *δ* 11.41(br, 1 H), 10.08 (d,*J*=8.0Hz,1 H), 9.29 (s, 1 H), 7.76 (m, 1 H), 7.34(m, 2 H), 7.08(m, 1H), 4.81(m, 1 H), 4.64(m,2 H), 4.43(m, 2H), 4.12(m, 1 H), 3.60(m, 5 H), 3.12(m, 1 H), 2.02 (m, 13 H), 1.15 (d,*J*=8.0Hz, 3 H), 0.73(m,3 H).

### Example A-14: 3-Chloro-5-(2-(((1S,7a'S)-2,2-difluorodihydro-1'H,3'H-spiro[cyclopropane-1,2'-pyrrolizin]-7a'(5'H)-yl)methoxy)-8-fluoro-4-(1-oxa-6-azaspiro[3.5]nonan-6-yl)pyrido[4,3-d]pyrimidin-7-yl)-4-(trifluoromethyl)aniline

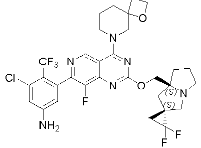

LCMS: m/z 669 (M+H)⁺. ¹H NMR (400MHz, DMSO-*d*₆) *δ* 9.19 (s, 1 H), 6.90 (d, *J*=2.24Hz, 1 H), 6.51(d, *J*=2.24Hz, 1 H), 6.34(s, 2 H), 4.36(m, 3 H), 4.22 (m, 3 H), 3.83 (m, 1 H), 3.44 (m, 1 H), 3.11(m, 1 H), 3.02 (m, 1 H), 2.73(m, 1 H), 2.54 (m, 1 H), 2.45 (m,1 H), 2.36(m,1 H), 1.77 (m, 14 H).

### Example A-15: 4-(2-(((1S,7a'S)-2,2-difluorodihydro-1'H,3'H-spiro[cyclopropane-1,2'-pyrrolizin]-7a'(5'H)-yl)methoxy)-8-fluoro-4-(1-oxa-6-azaspiro[3.5]nonan-6-yl)pyrido[4,3-d]pyrimidin-7-yl)-5-ethynyl-6-fluoronaphthalen-2-ol

LCMS: m/z 660 (M+H)⁺. ¹H NMR (400MHz, DMSO-*d*₆) *δ* 10.18(m,1H), 9.19-9.13(d, *J* = 24 Hz, 1H), 8.00-7.97(m, 1H),7.47 (t, *J=* 8 Hz, 1H), 7.40 (s, 1H), 7.23-7.21(m, 1H), 4.45-4.37 (m, 3H), 4.29-4.13(m, 3H), 3.91-3.87(m, 1H), 3.81-3.78(m, 1H), 3.51-3.38(m, 1H), 3.11-3.03(m, 2H), 2.74-2.68(m, 1H), 2.45-2.33(m, 2H), 2.18-1.47(m, 13H).

### Example A-15 was subjected to chiral resolution to give isomers A15A and A15B: 4-(2-(((1S,7a'S)-2,2-difluorodihydro-1'H,3'H-spiro[cyclopropane-1,2'-pyrrolizin]-7a'(5'H)-yl)methoxy)-8-fluoro-4-((R)-1-oxa-6-azaspiro[3.5]nonan-6-yl)pyrido[4,3-d]pyrimidin-7-yl)-5-ethynyl-6-fluoronaphthalen-2-ol and 4-(2-(((1S,7a'S)-2,2-difluorodihydro-1'H,3'H-spiro[cyclopropane-1,2'-pyrrolizin]-7a'(5'H)-yl)methoxy)-8-fluoro-4-((S)-1-oxa-6-azaspiro[3.5]nonan-6-yl)pyrido[4,3-d]pyrimidin-7-yl)-5-ethynyl-6-fluoronaphthalen-2-ol

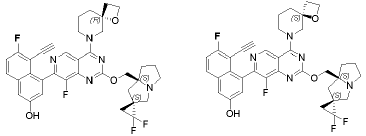

### Isomer A-15A

LCMS: m/z 660 (M+H)⁺.

### Isomer A-15B

LCMS: m/z 660 (M+H)⁺.

### Example A-16: 4-(2-(((1S,7a'S)-2,2-Difluorodihydro-1'H,3'H-spiro[cyclopropane-1,2'-pyrrolizin]-7a'(5'H)-yl)methoxy)-8-fluoro-4-(1-oxa-6-azaspiro[3.5]nonan-6-yl)pyrido[4,3-d]pyrimidin-7-yl)-5-ethyl-6-fluoronaphthalen-2-ol

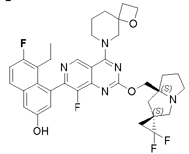

LCMS: m/z 664 (M+H)⁺.

**Example A-16** was subjected to chiral resolution (SFC (instrument: SFC-150 (Waters); column: (R,R)-WHELK-01 25 × 250 mm, 10 µm (Daicel); column temperature: 35 °C; mobile phase: CO₂/EtOH [0.5% NH₃ (7 M in MeOH)] = 55/45; flow rate: 100 mL/min; back pressure: 100 bar; detection wavelength: 214 nm; cycle time: 5 min)) and chiral HPLC (instrument: Gilson-281; column: RRW 25 × 250 mm, 10 µm (Daicel); mobile phase: HEX:EtOH (0.2% DEA) = 70:30; flow rate: 30 mL/min; detection wavelength: 214 nm; cycle time: 32 min) to give isomers A16A and A16B: 4-(2-(((1*S*,7a'*S*)-2,2-difluorodihydro-1'*H*,3'*H*-spiro[cyclopropane-1,2'-pyrrolizin]-7a'(5'*H*)-yl)methoxy)-8-fluoro-4-((*R*)-1-oxa-6-azaspiro[3.5]nonan-6-yl)pyrido[4,3-*d*]pyrimidin-7-yl)-5-ethyl-6-fluoronaphthalen-2-ol and 4-(2-(((1*S*,7a'*S*)-2,2-difluorodihydro-1'*H*,3'*H-*spiro[cyclopropane-1,2'-pyrrolizin]-7a'(5'*H*)-yl)methoxy)-8-fluoro-4-((*S*)-1-oxa-6-azaspiro[3.5] nonan-6-yl)pyrido[4,3-*d*]pyrimidin-7-yl)-5-ethyl-6-fluoronaphthalen-2-ol.

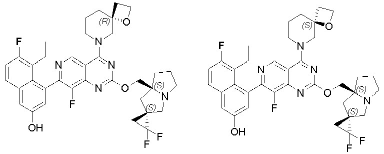

### Isomer A-16A

RT: 14.092 min. LCMS: m/z 664 (M+H)⁺. ¹H NMR (400MHz, DMSO-*d*₆) *δ* 9.23(d, *J* =4 Hz, 1H), 7.77-7.74(m, 1H),7.36-7.32 (m, 2H), 7.06-7.04 (m, 1H), 4.52-4.48(m, 1H), 4.42-4.15(m, 5H), 3.95-3.91 (m, 1H), 3.84-3.80 (m, 1H), 3.55-3.50(m, 1H), 3.42-3.38(m, 1H), 3.12-3.07(m, 1H), 3.04-2.99(m, 1H),2.73-2.70(m, 1H), 2.56-2.54(m, 1H), 2.44-2.33 (m, 3H), 2.16-2.06(m, 3H), 2.00-1.97(m, 1H), 1.92-1.70(m, 6H), 1.64-1.44(m, 3H) ,0.76-0.71(m, 3H).

### Isomer A-16B

RT: 16.978 min. LCMS: m/z 664 (M+H)⁺. ¹H NMR (400MHz, DMSO-*d*₆) *δ* 9.23(d, *J* =4 Hz, 1H), 7.77-7.74(m, 1H),7.36-7.32 (m, 2H), 7.06-7.04 (m, 1H), 4.51-4.19(m, 6H), 3.95-3.92 (m, 1H), 3.84-3.81 (m, 1H), 3.54-3.50(m, 1H), 3.43-3.41(m, 1H), 3.12-3.07(m, 1H), 3.03-2.99(m, 1H), 2.73-2.70(m, 1H), 2.56-2.54(m, 1H), 2.44-2.33 (m, 3H), 2.16-2.06(m, 3H), 2.00-1.97(m, 1H), 1.92-1.70(m, 6H), 1.64-1.44(m, 3H) ,0.76-0.71(m, 3H).

### Example A-17: 2-Amino-4-(2-(((1S,7a'S)-2,2-Difluorodihydro-1'H,3'H-spiro[cyclopropane-1,2'-pyrrolizin]-7a'(5'H)-yl)methoxy)-8-fluoro-4-(1-oxa-6-azaspiro[3.5]nonan-6-yl)pyrido [4,3-d]pyrimidin-7-yl)-7-fluorobenzo[b]thiophene-3-carbonitrile

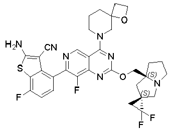

LCMS: m/z 666 (M+H)⁺. ¹H NMR (400MHz, DMSO-*d*₆) d9.26 (d, *J =* 6.1 Hz, 1H), 8.13 (s, 2H), 7.44 (dd, *J=* 8.5, 5.3 Hz, 1H), 7.16 (t, *J =* 8.9 Hz, 1H), 4.50 - 4.34 (m, 5H), 4.24 - 4.15 (m, 1H), 3.91 (d, *J=* 13.4 Hz, 1H), 3.11 - 2.99 (m, 1H), 2.88 - 2.75 (m, 1H), 2.41 - 2.33 (m, 2H), 2.31 - 2.22 (m, 2H), 2.17 - 1.96 (m, 6H), 1.93 - 1.84 (m, 4H), 1.77 - 1.61 (m, 3H).

### Example A-18: 6-(7-(8-Chloro-7-fluoronaphthalen-1-yl)-2-(((1S,7a'S)-2,2-difluorodihydro-1'H,3'H-spiro[cyclopropane-1,2'-pyrrolizin]-7a'(5'H)-yl)methoxy)-8-fluoropyrido[4,3-d]pyrimidin-4-yl)-1-oxa-6-azaspiro[3.5]nonane

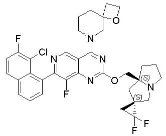

LCMS: m/z 654 (M+H)⁺. ¹H NMR (400MHz, DMSO-*d*₆) d 9.24 (s, 1H), 8.30 - 8.19 (m, 2H), 7.78 - 7.69 (m, 3H), 4.50 - 4.34 (m, 3H), 4.31 - 4.16 (m, 3H), 3.94 - 3.81 (m, 1H), 3.20 - 3.03 (m, 2H), 2.78 (d, *J=* 11.9 Hz, 1H), 2.63 - 2.54 (m, 1H), 2.49 - 2.34 (m, 2H), 2.16 - 2.07 (m, 2H), 2.06 - 1.99 (m, 1H), 1.97 - 1.80 (m, 6H), 1.80 - 1.69 (m, 2H), 1.69 - 1.55 (m, 2H), 1.54 - 1.45 (m, 1H).

### Example A-19: 3-Chloro-4-cyclopropyl-5-(2-(((1S,7a'S)-2,2-difluorodihydro-1'H,3'H-spiro[cyclopropane-1,2'-pyrrolizin]-7a'(5'H)-yl)methoxy)-8-fluoro-4-(1-oxa-6-azaspiro[3.5]nonan-6-yl)pyrido[4,3-d]pyrimidin-7-yl)phenol

LCMS: m/z 642 (M+H)⁺. ¹H NMR (400MHz, DMSO-*d*₆) d 10.35 (s, 1H), 9.26 (d, *J =* 8.7 Hz, 1H), 7.00 (d, *J* = 2.5 Hz, 1H), 6.86 - 6.79 (m, 1H), 4.52 - 4.34 (m, 5H), 4.26 - 4.17 (m, 1H), 3.88 (d, *J* = 13.4 Hz, 1H), 3.13 - 3.03 (m, 1H), 2.91 - 2.77 (m, 1H), 2.50 - 2.25 (m, 4H), 2.22 - 1.96 (m, 6H), 1.94 - 1.86 (m, 3H), 1.84 - 1.64 (m, 5H), 0.68 - 0.54 (m, 2H), 0.09 - -0.11 (m, 2H).

### Example A-20: 5-Chloro-4-(2-(((1S,7a'S)-2,2-Difluorodihydro-1'H,3'H-spiro[cyclopropane-1,2'-pyrrolizin]-7a'(5'H)-yl)methoxy)-8-fluoro-4-(1-oxa-6-azaspiro[3.5]nonan-6-yl)pyrido[4,3-d]pyrimidin-7-yl)-6-fluoronaphthalen-2-ol

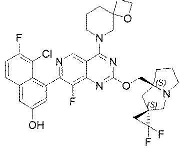

LCMS: m/z 670 (M+H)⁺.

### Example A-21: 4-(2-(((1S,7a'S)-2,2-Difluorodihydro-1'H,3'H-spiro[cyclopropane-1,2'-pyrrolizin]-7a'(5'H)-yl)methoxy)-8-fluoro-4-(2-oxa-6-azaspiro[3.4]octan-6-yl)pyrido[4,3-d]pyrimidin-7-yl)-5-ethyl-6-fluoronaphthalen-2-ol

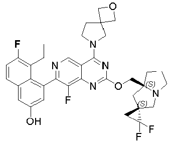

LCMS: m/z 650 (M+H)⁺. ¹H NMR (400MHz, DMSO-*d*₆) δ 9.95(s,1H), 9.28 (s, 1H), 7.79-7.75(m, 1H),7.38-7.33 (m, 2H), 7.01 (m, 1H), 4.71(d, *J*=4Hz, 2H), 4.55(d, *J*=4Hz, 2H), 4.27-3.98 (m, 6H), 3.13-3.08(m, 1H), 3.04-2.99(m, 1H), 2.73-2.70(m, 1H), 2.56-2.54 (m, 1H), 2.38-2.33(m, 3H), 2.18-1.88(m, 4H), 1.80-1.71(m, 2H) ,1.64-1.44(m, 3H), 0.73(t, *J*=8Hz, 3H).

### Example A-22: 4-(2-(((1S,7a'S)-2,2-Difluorodihydro-1'H,3'H-spiro[cyclopropane-1,2'-pyrrolizin]-7a'(5'H)-yl)methoxy)-8-fluoro-4-(1-oxa-6-azaspiro[3.4]octan-6-yl)pyrido[4,3-d]pyrimidin-7-yl)-5-ethyl-6-fluoronaphthalen-2-ol

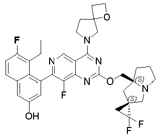

LCMS: m/z 650 (M+H)⁺. ¹H NMR (400MHz, DMSO-*d*₆) *δ* 9.93(s,1H), 9.30 (d, *J*=4Hz,1H), 7.79-7.75(m, 1H),7.38-7.33 (m, 2H), 7.03-7.01 (m, 1H), 4.47(t, J=8Hz, 2H), 4.28-4.19 (m, 6H), 3.13-3.08(m, 1H), 3.04-2.99(m, 1H), 2.92-2.85(m, 1H), 2.73-2.67 (m, 2H), 2.56-2.54(m, 1H), 2.39-2.32(m, 2H), 2.15-2.05(m, 3H), 2.01-1.97(m, 1H), 1.91-1.88(m, 1H) ,1.81-1.71(m, 2H), 1.64-1.44(m, 3H), 0.76-0.70(m, 3H).

### Example A-23: 4-(2-(((1S,7a'S)-2,2-Difluorodihydro-1'H,3'H-spiro[cyclopropane-1,2'-pyrrolizin]-7a'(5'H)-yl)methoxy)-8-fluoro-4-(1-oxa-6-azaspiro[3.4]heptan-6-yl)pyrido[4,3-d]pyrimidin-7-yl)-5-ethyl-6-fluoronaphthalen-2-ol

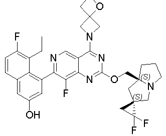

LCMS: m/z 636 (M+H)⁺. ¹H NMR (400MHz, DMSO-*d*₆) *δ* 10.86 (s, 1H), 9.94 (s, 1H), 9.01 (s, 1H), 7.78 (dd, *J=* 9.1, 6.0 Hz, 1H), 7.36 (dd, *J=* 11.8, 6.1 Hz, 2H), 7.00 (d, *J* = 2.3 Hz, 1H), 5.05 (s, 2H), 4.65 (t, *J=* 15.7 Hz, 3H), 4.49 (t, *J =* 7.4 Hz, 3H), 3.77 (s, 1H), 3.65 (s, 1H), 3.44 (dd, *J* = 13.9, 7.0 Hz, 1H), 3.18 - 3.08 (m, 1H), 2.96 (t, *J =* 7.5 Hz, 2H), 2.40 - 1.82 (m, 10H), 0.71 (t, *J=* 7.4 Hz, 3H).

### Example A-24: 5-(2-(((1S,7a'S)-2,2-Difluorodihydro-1'H,3'H-spiro[cyclopropane-1,2'-pyrrolizin]-7a'(5'H)-yl)methoxy)-7-(8-ethyl-7-fluoro-3-hydroxynaphthalen-1-yl)-8-fluoropyrido[4,3-d]pyrimidin-4-yl)-N,N-dimethyl-5,6,7,8-tetrahydro-4H-pyrazolo[1,5-a][1,4]diazepine-2-carboxamide

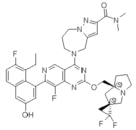

LCMS: m/z 745 (M+H)⁺. ¹H NMR (400MHz, DMSO-*d*₆) *δ* 9.94(s,1 H), 9.21(s,1 H), 7.80-7.76 (m, 1H), 7.39-7.34 (m, 2H),7.02(m,1H), 6.62(s,1H), 5.33-5.17(m,2H), 4.54-4.52 (m,2H), 4.39-4.19(m,4H), 3.27(s,3H), 3.15-3.09(m, 1H), 3.05-3.00(m, 1H), 2.95(s, 3H), 2.74(d, *J* = 12 Hz, 1H), 2.57-2.55(m,1H), 2.42-2.34(m,3H), 2.15-2.05(m, 2H), 2.02-1.95 (m,1H), 1.92-1.89(m, 1H),1.81-1.45(m, 5H), 0.73(t, *J =* 4 Hz, 3H).

### Example A-25: 5-(7-(5-Amino-3-chloro-2-(trifluoromethyl)phenyl)-2-(((1S,7a'S)-2,2-difluorodihydro-1'H,3'H-spiro[cyclopropane-1,2'-pyrrolizin]-7a'(5'H)-yl)methoxy)-8-fluoropyrido[4,3-d]pyrimidin-4-yl)-N,N-dimethyl-5,6,7,8-tetrahydro-4H-pyrazolo[1,5-a][1,4]diazepine-2-carboxamide

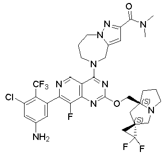

LCMS: m/z 750 (M+H)⁺. ¹H NMR (400MHz, DMSO-*d*₆) *δ* 9.14(s,1 H), 6.88(m,1H), 6.62(s,1H), 6.47 (m,1H), 6.34 (s,2H), 5.21(d, *J =* 8Hz, 2H),4.51-4.48(m,2H), 4.30-4.28 (m, 2H), 4.24-4.18 (m, 2H), 3.25(s,3H), 3.12(m, 1H), 3.02(m, 1H), 2.94(s, 3H), 2.74(d, *J* = 12 Hz, 1H), 2.57-2.53(m,1H), 2.32(m,2H), 2.08-2.05(m, 1H), 1.99-1.95 (m,1H), 1.91-1.88(m, 1H),1.81-1.45(m, 5H).

### Example A-26: (S)-7-(2-(((1S,7a'S)-2,2-Difluorodihydro-1'H,3'H-spiro[cyclopropane-1,2'-pyrrolizin]-7a'(5'H)-yl)methoxy)-7-(8-ethyl-7-fluoro-3-hydroxynaphthalen-1-yl)-8-fluoropyrido[4,3-d]pyrimidin-4-yl)-1,3,7-triazaspiro[4.5]decane-2,4-dione

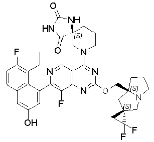

LCMS: m/z 706 (M+H)⁺. ¹H NMR (400MHz, DMSO-*d*₆) *δ* 11.19(br, 1 H), 10.90 (m, 1 H), 10.09 (br, 1 H), 9.14 (s, 1 H), 8.72 (d, *J*=7.64Hz, 1 H), 7.73 (m, 1 H), 7.35(m, 2 H), 7.07(m, 1H), 4.59(m, 3 H), 3.61(m, 2 H), 3.49(m, 3H), 3.09(m, 1 H), 2.10 (m, 14 H), 0.72(m,3 H)

### Example A-27: (R)-7-(2-(((1S,7a'S)-2,2-Difluorodihydro-1'H,3'H-spiro[cyclopropane-1,2'-pyrrolizin]-7a'(5'H)-yl)methoxy)-7-(8-ethyl-7-fluoro-3-hydroxynaphthalen-1-yl)-8-fluoropyrido[4,3-d]pyrimidin-4-yl)-1,3,7-triazaspiro[4.5]decane-2,4-dione

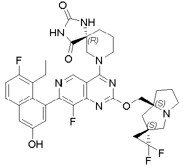

LCMS: m/z 706 (M+H)⁺. ¹H NMR (400MHz, DMSO-*d*₆) *δ* 11.29(br, 1 H), 10.88 (m, 1 H), 10.08 (br, 1 H), 9.14 (s, 1 H), 8.71 (d, *J*=5.20Hz, 1 H), 7.75 (m, 1 H), 7.35(m, 2 H), 7.07(m, 1H), 4.51(m, 5 H), 3.79(m, 1 H), 3.69(m, 2H), 3.60(m, 2H), 3.10(m, 1 H), 2.36 (m, 3 H), 2.14 (m, 6 H), 1.96 (m, 2 H), 1.88 (m, 3 H), 0.72(m,3 H).

### Example A-28: 4-(4-(2-Oxa-6-azabicyclo[5.1.0]octan-6-yl)-2-(((1S,7a'S)-2,2-difluorodihydro-1'H,3'H-spiro[cyclopropane-1,2'-pyrrolizin]-7a'(5'H)-yl)methoxy)-8-fluoropyrido[4,3-d]pyrimidin-7-yl)-5-ethyl-6-fluoronaphthalen-2-ol

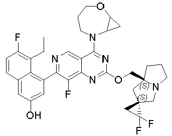

LCMS: m/z 650 (M+H)⁺. ¹H NMR (400MHz, DMSO-*d*₆) *δ* 9.94(m,1H), 9.40(m, 1H), 7.80-7.76 (m, 1H),7.38-7.29 (m, 2H), 7.07 (s, 1H),4.55-4.52(m, 1H), 4.27-4.18 (m, 2H), 3.92-3.89(m, 1H), 3.80-3.74(m, 2H), 3.66-3.59(m, 1H), 3.50-3.44(m, 2H), 3.13-3.02(m, 2H), 2.75-2.72(m, 1H), 2.58-2.55(m, 1H), 2.35-2.33(m, 1H),2.26-2.07(m, 2H), 2.02-1.73(m, 6H), 1.67-1.45(m, 3H),1.36-1.30(m, 1H), 0.78-0.70(m, 3H).

### Example A-29: 4-(4-((cis)-2-Oxa-6-azabicyclo[5.1.0]octan-6-yl)-2-(((1S,7a'S)-2,2-difluorodihydro-1'H,3'H-spiro[cyclopropane-1,2'-pyrrolizin]-7a'(5'H)-yl)methoxy)-8-fluoropyrido[4,3-d]pyrimidin-7-yl)-5-ethynyl-6-fluoronaphthalen-2-ol

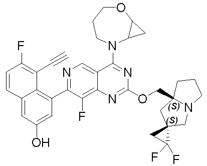

LCMS: m/z 646 (M+H)⁺. ¹H NMR (400MHz, DMSO-*d*₆) *δ* 10.14(m,1H), 9.39(m, 1H), 8.00-7.97(m, 1H),7.50-7.46 (m, 1H), 7.40 (s, 1H), 7.20-7.18 (m, 1H),4.51-4.49(m, 1H), 4.26-4.16(m, 3H), 3.93-3.89(m, 1H), 3.81-3.72(m, 2H), 3.65-3.59(m, 1H), 3.48-3.42(m, 1H), 3.14-3.09(m, 1H), 3.03-3.00(m, 1H), 2.75-2.72(m, 1H), 2.58-2.54(m, 1H), 2.25-2.17(m, 1H), 2.12-2.07(m, 1H), 2.02-1.72(m, 6H), 1.66-1.45(m, 3H),1.34-1.27(m, 1H), 0.97-0.93(m, 1H).

### Example A-29 was subjected to chiral resolution to give two isomers, Example A-29A and Example A-29B: 4-(4-((1R,7S)-2-oxa-6-azabicyclo[5.1.0]octan-6-yl)-2-(((1S,7a'S)-2,2-difluorodihydro-1'H,3'H-spiro[cyclopropane-1,2'-pyrrolizin]-7a'(5'H)-yl)methoxy)-8-fluoropyrido[4,3-d]pyrimidin-7-yl)-5-ethynyl-6-fluoronaphthalen-2-ol and 4-(4-((1S,7R)-2-oxa-6-azabicyclo[5.1.0]octan-6-yl)-2-(((1S,7a'S)-2,2-difluorodihydro-1'H,3'H-spiro[cyclopropane-1,2'-pyrrolizin]-7a'(5'H)-yl)methoxy)-8-fluoropyrido[4,3-d]pyrimidin-7-yl)-5-ethynyl-6-fluoronaphthalen-2-ol

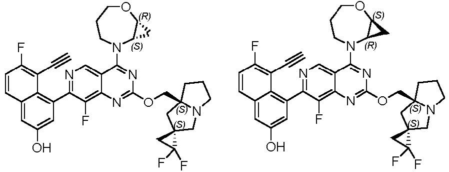

### Example A-29A

LCMS: m/z 646 (M+H)⁺.

### Example A-29B

LCMS: m/z 646 (M+H)⁺.

### Example A-30: 3-(4-(2-Oxa-6-azabicyclo[5.1.0]octan-6-yl)-2-(((1S,7a'S)-2,2-difluorodihydro-1'H,3'H-spiro[cyclopropane-1,2'-pyrrolizin]-7a'(5'H)-yl)methoxy)-8-fluoropyrido[4,3-d]pyrimidin-7-yl)-5-chloro-4-(trifluoromethyl)aniline

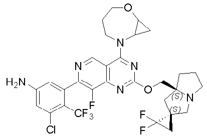

LCMS: m/z 655 (M+H)⁺. ¹H NMR (400MHz, DMSO-*d*₆) δ 9.46(s,1H), 6.88(s, 1H), 6.52 (s, 1H), 6.31 (s, 1H), 4.51 (d, *J*=16Hz, 1H),4.27-4.18(m, 2H), 3.89-3.85(m, 1H), 3.77-3.71(m, 2H), 3.62-3.56(m, 1H), 3.45-3.41(m, 1H), 3.13-3.04(m, 2H), 2.77-2.74 (m, 1H), 2.59-2.50(m, 1H), 2.19-2.08(m, 2H),1.99-1.75(m, 6H), 1.64-1.48(m, 3H),1.35-1.27(m, 1H), 0.65-0.49(m, 1H).

### Example A-31: 4-(4-(9-Oxa-3-azabicyclo[4.2.1]nonan-3-yl)-2-(((1S,7a'S)-2,2-difluorodihydro-1'H,3'H-spiro[cyclopropane-1,2'-pyrrolizin]-7a'(5'H)-yl)methoxy)-8-fluoropyrido[4,3-d]pyrimidin-7-yl)-5-ethynyl-6-fluoronaphthalen-2-ol

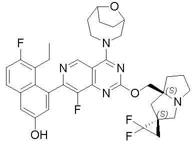

LCMS: m/z 664 (M+H)⁺. ¹H NMR (400MHz, DMSO-*d*₆) *δ* 9.95(d, *J*=8Hz,1H), 9.15 (s,1H), 7.79-7.75(m, 1H),7.38-7.33 (m, 2H), 7.07-7.02 (m, 1H), 4.72-4.57(m, 3H), 4.29-4.11 (m, 3H), 3.89-3.82(m, 1H), 3.66-3.54(m, 1H), 3.13-3.04(m, 2H), 2.77-2.74 (m, 1H), 2.57-2.55(m, 1H), 2.39-2.24(m, 2H), 2.12-1.90(m, 7H), 1.82-1.48(m, 7H), 0.76-0.71(m, 3H).

### Example A-32: 3-(4-(6-Oxa-3-azabicyclo[4.2.1]nonan-3-yl)-2-(((1S,7a'S)-2,2-difluorodihydro-1'H,3'H-spiro[cyclopropane-1,2'-pyrrolizin]-7a'(5'H)-yl)methoxy)-8-fluoropyrido[4,3-d]pyrimidin-7-yl)-5-chloro-4-(trifluoromethyl)aniline

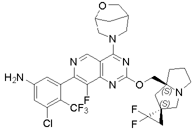

LCMS: m/z 655 (M+H)⁺. ¹H NMR (400MHz, DMSO-*d*₆) *δ* 9.13 (s, 1 H), 6.88(d, *J*=2.20Hz, 1 H), 6.47(d, *J*=2.24Hz, 1 H), 6.33(s, 2 H), 4.67(m, 1 H), 4.39(m, 2 H), 4.20 (m, 2 H), 3.77 (m, 1 H), 3.65 (m, 3 H), 3.09(m, 1 H), 3.01 (m, 1 H), 2.70(m, 2 H), 2.54 (m, 1 H), 1.77 (m, 10 H).

### Example A-33: 4-(4-(6-Oxa-3-azabicyclo[4.2.1]nonan-3-yl)-2-(((1S,7a'S)-2,2-difluorodihydro-1'H,3'H-spiro[cyclopropane-1,2'-pyrrolizin]-7a'(5'H)-yl)methoxy)-8-fluoropyrido[4,3-d]pyrimidin-7-yl)-5-ethyl-6-fluoronaphthalen-2-ol

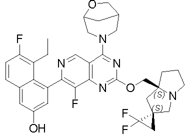

LCMS: m/z 650 (M+H)⁺. ¹H NMR (400MHz, DMSO-*d*₆) δ 9.94(s, 1 H), 9.19(s, 1 H), 7.77(m, 1 H), 7.33(m, 2 H), 7.02(m, 1 H), 4.73(m, 1 H), 4.38(m, 1 H), 4.22(m, 2 H), 3.81(m, 1 H), 3.69(m, 3 H), 3.11(m, 1H), 3.01(m, 1H), 2.71(m, 2 H), 2.54 (m, 1 H), 2.37 (m, 1 H), 2.03 (m, 11 H), 0.73 (m, 3 H).

### Example A-34: 4-(2-(((1S,7a'S)-2,2-Difluorodihydro-1'H,3'H-spiro[cyclopropane-1,2'-pyrrolizin]-7a'(5'H)-yl)methoxy)-8-fluoro-4-(6-fluoro-1,4-oxazepan-4-yl)pyrido[4,3-d]pyrimidin-7-yl)-5-ethyl-6-fluoronaphthalen-2-ol

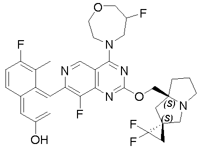

LCMS: m/z 655 (M+H)⁺. ¹H NMR (400MHz, DMSO-*d*₆) δ 9.94(s, 1 H), 9.24(d, *J*=3.00Hz, 1 H), 7.77(m, 1 H), 7.35(m, 2 H), 7.02(d, *J*=2.60Hz, 1 H), 5.12(m,1 H), 4.62(m, 2 H), 4.11(m, 8 H), 3.10(m, 2 H), 2.74(m, 1 H), 2.54 (m, 1 H), 2.36 (m, 1 H), 1.82 (m, 9 H), 0.73 (m, 3 H).

### Example A-35: 4-(4-(6,6-Difluoro-1,4-oxazepan-4-yl)-2-(((1S,7a'S)-2,2-difluorodihydro-1'H,3'H-spiro[cyclopropane-1,2'-pyrrolizin]-7a'(5'H)-yl)methoxy)-8-fluoropyrido[4,3-d]pyrimidin-7-yl)-5-ethyl-6-fluoronaphthalen-2-ol

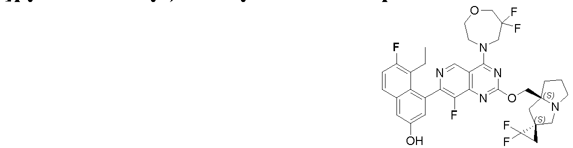

LCMS: m/z 674 (M+H)⁺. ¹H NMR (400MHz, DMSO-*d*₆) δ 9.96 (s, 1H), 9.20 (s, 1H), 7.77 (dd, *J=* 9.1, 6.0 Hz, 1H), 7.35 (dd, *J=* 11.2, 6.0 Hz, 2H), 7.04 (d, *J =* 2.5 Hz, 1H), 4.79 (s, 1H), 4.63 (s, 1H), 4.33 - 4.15 (m, 6H), 4.15 - 3.99 (m, 2H), 3.02 (m,2H), 2.71 (d, *J* = 29.5 Hz, 1H), 2.43 - 2.30 (m, 1H), 2.18 - 1.71 (m, 7H), 1.57 (dd, *J* = 39.7, 24.2 Hz, 3H), 0.73 (t, *J =* 7.3 Hz, 3H).

### Example A-36: 4-(2-(((1S,7a'S)-2,2-Difluorodihydro-1'H,3'H-spiro[cyclopropane-1,2'-pyrrolizin]-7a'(5'H)-yl)methoxy)-8-fluoro-4-(3-fluoroazepan-1-yl)pyrido[4,3-d]pyrimidin-7-yl)-5-ethyl-6-fluoronaphthalen-2-ol

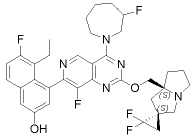

LCMS: m/z 654 (M+H)⁺. ¹H NMR (400MHz, DMSO-*d*₆) *δ* 9.95(s, 1H), 9.22 (s, 1H), 7.79-7.75(m, 1H),7.38-7.33 (m, 2H), 7.03-7.01 (m, 1H), 5.16-5.05(m, 1H), 4.42-4.11 (m, 6H), 4.00-3.90(m, 1H), 3.12-3.07(m, 1H),3.04-2.99(m, 1H), 2.74-2.71 (m, 1H), 2.56-2.54(m, 1H), 2.40-2.33(m, 1H), 2.15-1.73(m, 12H), 1.65-1.43(m, 4H), 0.75-0.71(m, 3H).

### Example A-37: 4-(2-(((1S,7a'S)-2,2-Difluorodihydro-1'H,3'H-spiro[cyclopropane-1,2'-pyrrolizin]-7a'(5'H)-yl)methoxy)-8-fluoro-4-(5-methyl-1,4-oxazepan-4-yl)pyrido[4,3-d]pyrimidin-7-yl)-5-ethyl-6-fluoronaphthalen-2-ol

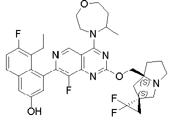

LCMS: m/z 652 (M+H)⁺. ¹H NMR (400MHz, DMSO-*d*₆) δ 9.96 (s, 1H), 8.99 (d, *J* = 6.4 Hz, 1H), 7.78 (dd, *J* = 9.0, 6.0 Hz, 1H), 7.36 (dd, *J =* 14.0, 6.0 Hz, 2H), 7.02 (dd, *J =* 19.3, 2.3 Hz, 1H), 4.80 (s, 1H), 4.38 (d, *J* = 5.1 Hz, 1H), 4.31 - 4.06 (m, 3H), 3.87 (dd, *J =* 12.9, 5.9 Hz, 2H), 3.69 (d, *J=* 10.5 Hz, 1H), 3.10 (dd, *J =* 11.6, 6.9 Hz, 1H), 3.02 (s, 1H), 2.73 (d, *J =* 11.7 Hz, 1H), 2.33 (d, *J* = 6.3 Hz, 2H), 2.13 (d, *J* = 42.5 Hz, 2H), 1.96 (dd, *J =* 40.9, 10.8 Hz, 3H), 1.79 (d, *J* = 22.8 Hz, 2H), 1.61 (dd, *J* = 22.8, 10.5 Hz, 2H), 1.45 (s, 4H), 1.25 (s, 2H), 0.75 (q, *J =* 7.1 Hz, 3H).

### Example A-38: 4-(2-(((1S,7a'S)-2,2-Difluorodihydro-1'H,3'H-spiro[cyclopropane-1,2'-pyrrolizin]-7a'(5'H)-yl)methoxy)-7-(8-ethyl-7-fluoro-3-hydroxynaphthalen-1-yl)-8-fluoropyrido[4,3-d]pyrimidin-4-yl)-6-methyl-1,4-oxazepan-6-ol

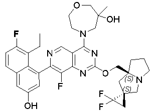

LCMS: m/z 668 (M+H)⁺. ¹H NMR (400MHz, DMSO-*d*₆) *δ* 10.04 (s, 1H), 9.56 (s, 1H), 9.07 (s, 1H), 7.82 - 7.74 (m, 1H), 7.42 - 7.34 (m, 2H), 7.04 (d, *J =* 12.4 Hz, 1H), 5.18 (d, *J =* 8.4 Hz, 1H), 4.66 (s, 2H), 4.38 (m, 2H), 4.10 - 3.87 (m, 3H), 3.59 (m, 1H), 3.53 - 3.47 (m, 2H), 3.47 - 3.42 (m, 4H), 2.34 (m, 2H), 2.14 (m, 3H), 1.88 (m, 2H), 1.32 - 1.14 (m, 4H), 1.07 (t, *J =* 7.0 Hz, 3H), 0.75 (t, *J=* 7.3 Hz, 3H).

### Example A-39: 4-(7-(5-Amino-3-chloro-2-(trifluoromethyl)phenyl)-2-(((1S,7a'S)-2,2-difluorodihydro-1'H,3'H-spiro[cyclopropane-1,2'-pyrrolizin]-7a'(5'H)-yl)methoxy)-8-fluoropyrido[4,3-d]pyrimidin-4-yl)-6-methyl-1,4-oxazepan-6-ol

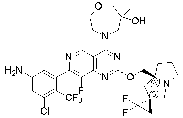

LCMS: m/z 673 (M+H)⁺. ¹H NMR (400MHz, DMSO-*d*₆) *δ* 13.13 (s,1H), 10.96 (s,1H), 9.02 (s, 1H), 6.90 (s, 1H), 6.48 (d, *J* = 2.1 Hz, 3H), 4.80 - 4.61 (m, 2H), 4.43 - 3.36 (m, 15H), 3.07 (m, 1H), 2.42 - 2.24 (m, 2H), 2.21 - 2.03 (m, 3H), 1.98 - 1.83 (m, 3H).

### Example A-40: 4-(4-(Cyclopropyl(methyl)amino)-2-(((1S,7a'S)-2,2-difluorodihydro-1'H,3'H-spiro[cyclopropane-1,2'-pyrrolizin]-7a'(5'H)-yl)methoxy)8-fluoropyrido[4,3-d]pyrimidin-7-yl)-5-ethyl-6-fluoronaphthalen-2-ol

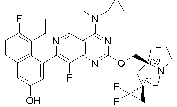

LCMS: m/z 608 (M+H)⁺. ¹H NMR (400MHz, DMSO-*d*₆) *δ* 10.99 (s, 1H), 9.98 (s, 1H), 9.68 (s, 1H), 7.79 (dd, *J=* 9.1, 6.0 Hz, 1H), 7.43 - 7.28 (m, 2H), 7.07 (d, *J* = 2.4 Hz, 1H), 4.70 - 4.61 (m, 2H), 3.81 (dd, *J =* 13.5, 10.4 Hz, 1H), 3.69 (dd, *J =* 12.5, 5.8 Hz, 2H), 3.52 - 3.45 (m, 1H), 3.43 (d, *J=* 6.0 Hz, 1H), 3.39 (d, *J* = 4.0 Hz, 3H), 3.12 (s, 1H), 2.31 (ddd, *J=* 22.1, 15.5, 8.4 Hz, 3H), 2.22 - 2.13 (m, 2H), 2.06 (dd, *J =* 13.1, 6.4 Hz, 1H), 2.02 - 1.94 (m, 1H), 1.94 - 1.84 (m, 2H), 1.10 - 1.02 (m, 2H), 0.85 (d, *J=* 7.5 Hz, 1H), 0.73 (dd, *J=* 17.0, 9.5 Hz, 4H).

### Example A-41: 7-(5-Amino-3-chloro-2-(trifluoromethyl)phenyl)-N-cyclopropyl-2-(((1S,7a'S)-2,2-difluorodihydro-1'H,3'H-spiro[cyclopropane-1,2'-pyrrolizin]-7a'(5'H)-yl)methoxy)-8-fluoro-N-methylpyrido[4,3-d]pyrimidin-4-amine

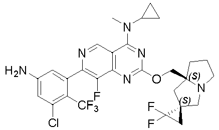

LCMS: m/z 613 (M+H)⁺. ¹H NMR (400MHz, DMSO-*d*₆) *δ* 9.59 (s, 1H), 6.89 (d, *J* = 2.0 Hz, 1H), 6.52 (d, *J =* 2.1 Hz, 1H), 6.34 (s, 2H), 4.21 (d, *J =* 10.6 Hz, 2H), 3.63 (s, 1H), 3.48 - 3.42 (m, 1H), 3.10 (dd, *J=* 11.6, 6.8 Hz, 1H), 3.06 - 2.94 (m, 1H), 2.73 (d, *J =* 11.8 Hz, 1H), 2.56 (d, *J =* 9.2 Hz, 1H), 2.07 (dd, *J =* 13.3, 5.7 Hz, 1H), 1.98 (dd, *J =* 10.3, 5.7 Hz, 1H), 1.90 (d, *J =* 13.4 Hz, 1H), 1.87 - 1.67 (m, 3H), 1.66 - 1.44 (m,4H), 1.02 (d, *J=* 6.4 Hz, 2H), 0.76 (s, 2H).

### Example A-42: 4-(4-((1-Cyclopropylethyl)(methyl)amino)-2-(((1S,7a'S)-2,2-difluorodihydro-1'H,3'H-spiro[cyclopropane-1,2'-pyrrolizin]-7a'(5'H)-yl)methoxy)-8-fluoropyrido[4,3-d]pyrimidin-7-yl)-5-ethyl-6-fluoronaphthalen-2-ol trifluoroacetate

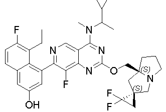

LCMS: m/z 636 (M+H)⁺. ¹H NMR (400MHz, DMSO-*d*₆) *δ* 11.01 (s, 1H), 10.06 (s, 1H), 9.30 (s, 1H), 7.77 (dd, *J =* 9.1, 6.0 Hz, 1H), 7.36 (dd, *J =* 10.4, 6.0 Hz, 2H), 7.03 (dd, *J =* 8.6, 2.5 Hz, 1H), 4.67 - 4.56 (m, 3H), 4.31 (d, *J=* 6.3 Hz, 2H), 3.85 - 3.73 (m, 1H), 3.64 (s, 1H), 3.44 (ddd, *J=* 18.1, 9.7, 4.2 Hz, 2H), 3.21 - 3.07 (m, 1H), 2.46 - 2.32 (m, 2H), 2.29 - 2.12 (m, 4H), 2.07 - 1.95 (m, 2H), 1.87 (dd, *J=* 11.5, 6.8 Hz, 2H), 1.40 (dd, *J =* 17.1, 10.6 Hz, 3H), 1.26 (dd, *J =* 8.1, 4.1 Hz, 1H), 0.79 - 0.70 (m, 3H), 0.66 (dt, *J=* 11.2, 8.5 Hz, 1H), 0.49 (tdd, *J=* 14.2, 9.4, 5.0 Hz, 2H), 0.36 - 0.17 (m, 1H).

### Example A-43: 7-(5-Amino-3-chloro-2-(trifluoromethyl)phenyl)-N-(1-cyclopropylethyl)-2-(((1S,7a'S)-2,2-difluorodihydro-1'H,3'H-spiro[cyclopropane-1,2'-pyrrolizin]-7a'(5'H)-yl)methoxy)-8-fluoro-N-methylpyrido[4,3-d]pyrimidin-4-amine trifluoroacetate

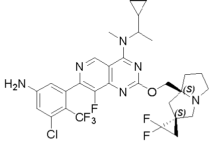

LCMS: m/z 641 (M+H)⁺. ¹H NMR (400MHz, DMSO-*d*₆) *δ* 10.90 (s, 1H), 9.26 (s, 1H), 6.89 (s, 1H), 6.47 (s, 2H), 4.69 - 4.52 (m, 2H), 4.39 - 4.20 (m, 1H), 3.76 (s, 1H), 3.64 (d, *J =* 6.8 Hz, 1H), 3.57 - 3.36 (m, 5H), 3.15 (s, 1H), 2.48 - 2.40 (m, 1H), 2.32 - 2.10 (m, 3H), 2.10 - 1.79 (m, 4H), 1.36 (dd, *J=* 19.6, 6.3 Hz, 3H), 1.31 - 1.16 (m, 1H), 0.63 (s, 1H), 0.56 - 0.36 (m, 2H), 0.26 (s, 1H).

### Example A-44: 4-(2-(((1S,7a'S)-2,2-Difluorodihydro-1'H,3'H-spiro[cyclopropane-1,2'-pyrrolizin]-7a'(5'H)-yl)methoxy)-8-fluoro-4-(((trans)-2-fluorocyclopropyl)(methyl)amino) pyrido[4,3-d]pyrimidin-7-yl)-5-ethynyl-6-fluoronaphthalen-2-ol

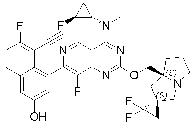

LCMS: m/z 622 (M+H)⁺. ¹H NMR (400MHz, DMSO-*d*₆) *δ* 10.13(s, 1H), 9.50(d, *J* =16 Hz, 1 H), 8.0-7.96(m, 1H), 7.47(t, *J* =8 Hz, 1H), 7.40(d, *J* =4 Hz, 1H), 7.20(s, 1H), 5.09-4.83 (m, 1H), 4.24-4.05(m, 3H), 4.01(d, *J* =20 Hz, 1H), 3.34-3.28(m,3H), 3.13-3.07 (m, 1H), 3.03-2.99 (m, 1H), 2.73(d, *J* =12 Hz, 1H), 2.56-2.52 (m,1H), 2.09-2.03 (m, 1H) ,2.00-1.95 (m, 1H) , 1.92(d, *J* =16 Hz, 1H), 1.84-1.43 (m, 6H), 1.33-1.24 (m, 1H).

### Example A-44 was subjected to chiral resolution to give two isomers, Example A-44 A and Example A-44 B: 4-(2-(((1S,7a'S)-2,2-difluorodihydro-1'H,3'H-spiro[cyclopropane-1,2'-pyrrolizin]-7a'(5'H)-yl)methoxy)-8-fluoro-4-(((1S,2S)-2-fluorocyclopropyl)(methyl)amino)pyrido[4,3-d]pyrimidin-7-yl)-5-ethynyl-6-fluoronaphthalen-2-ol and 4-(2-(((1S,7a'S)-2,2-difluorodihydro-1'H,3'H-spiro[cyclopropane-1,2'-pyrrolizin]-7a'(5'H)-yl)methoxy)-8-fluoro-4-(((1R,2R)-2-fluorocyclopropyl)(methyl)amino)pyrido[4,3-d]pyrimidin-7-yl)-5-ethynyl-6-fluoronaphthalen-2-ol

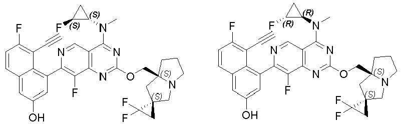

**Example A-44 A:** LCMS: m/z 622 (M+H)⁺.
**Example A-44 B:** LCMS: m/z 622 (M+H)⁺.

### Example A-45: 4-(2-(((1S,7a'S)-2,2-Difluorodihydro-1'H,3'H-spiro[cyclopropane-1,2'-pyrrolizin]-7a'(5'H)-yl)methoxy)-8-fluoro-4-(((trans)-2-fluorocyclopropyl)(methyl)amino) pyrido[4,3-d]pyrimidin-7-yl)-5-ethyl-6-fluoronaphthalen-2-ol

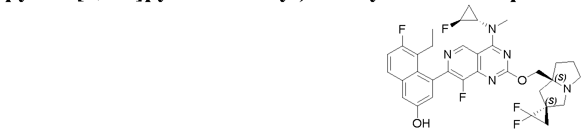

LCMS: m/z 626 (M+H)⁺. ¹H NMR (400MHz, DMSO-*d*₆) *δ* 9.91(s, 1H), 9.53(s, 1 H), 7.79-7.75(m, 1H), 7.37-7.33(m, 2H), 7.05(s, 1H), 5.06-4.78 (m, 1H), 4.26-4.18(m, 2H), 4.15-4.08(m, 1H), 3.32-3.30(m,3H), 3.11-3.07 (m, 1H), 3.03-2.98 (m, 1H), 2.73(d, *J* =12 Hz, 1H), 2.56-2.54 (m,1H), 2.33(s, 1H), 2.19-1.97 (m,3H), 1.91(d, *J* =12Hz, 1H), 1.84-1.43 (m, 6H), 1.29-1.15 (m, 1H) 0.75-0.71 (m, 3H) .

### Example A-46: 7-(5-Amino-3-chloro-2-(trifluoromethyl)phenyl)-2-(((1S,7a'S)-2,2-difluorodihydro-1'H,3'H-spiro[cyclopropane-1,2'-pyrrolizin]-7a'(5'H)-yl)methoxy)-8-fluoro-N-((trans)-2-fluorocyclopropyl)-N-methylpyrido[4,3-d]pyrimidin-4-amine

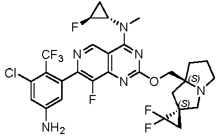

LCMS: m/z 631 (M+H)⁺. ¹H NMR (400MHz, DMSO-*d*₆) δ 9.50(s, 1 H), 6.90(m, 1H), 6.51(m, 1H), 6.33(s, 2H), 5.01-4.82 (m, 1H), 4.25-4.17(m, 2H), 4.15-4.06(m, 1H), 3.29(s,3H), 3.14-3.02 (m, 2H), 2.76(d, *J* =12 Hz, 1H), 2.58-2.51 (m,1H), 2.10-1.96 (m,2H) , 1.93(d, *J* =16Hz, 1H), 1.85-1.46 (m, 6H), 1.21-1.18(m, 1H).

### Example A-47: 4-(2-(((1S,7a'S)-2,2-Difluorodihydro-1'H,3'H-spiro[cyclopropane-1,2'-pyrrolizin]-7a'(5'H)-yl)methoxy)-8-fluoro-4-(((trans)-4-fluorotetrahydrofuran-3-yl)amino)pyrido[4,3-d]pyrimidin-7-yl)-5-ethynyl-6-fluoronaphthalen-2-ol

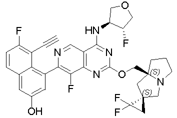

LCMS: m/z 638 (M+H)⁺. ¹H NMR (400MHz, DMSO-*d*₆) *δ* 10.24(s,1H), 9.36(d, *J =* 8 Hz, 1H), 8.92(s, 1H), 7.99-7.95(m, 1H), 7.46 (t, *J =* 8 Hz, 1H), 7.39 (s, 1H), 7.17-7.14(m, 1H),5.49-5.30 (m, 1H), 4.94-4.86(m, 1H), 4.31-4.20(m, 3H), 4.07-4.05(m, 1H), 4.00-3.92(m, 2H), 3.88-3.84(m, 1H), 3.13-3.00(m, 2H), 2.74-2.67(m, 1H), 2.57-2.53(m, 1H), 2.12-2.05(m, 1H), 2.02-1.97(m, 1H), 1.93-1.90(m, 1H), 1.85-1.72(m, 2H), 1.66-1.42(m, 3H).

### Example A-48: 4-(2-(((1S,7a'S)-2,2-Difluorodihydro-1'H,3'H-spiro[cyclopropane-1,2'-pyrrolizin]-7a'(5'H)-yl)methoxy)-8-fluoro-4-(((cis)-4-fluorotetrahydrofuran-3-yl)amino) pyrido[4,3-d]pyrimidin-7-yl)-5-ethynyl-6-fluoronaphthalen-2-ol

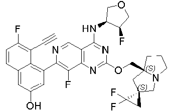

LCMS: m/z 638 (M+H)⁺. ¹H NMR (400MHz, DMSO-*d*₆) *δ* 10.13(s,1H), 9.46(s,1H), 8.88(t, *J =* 8 Hz, 1H), 7.99 (t, *J=* 8 Hz, 1H), 7.50-7.45(m, 1H),7.41(s,1H), 7.19-7.16(m, 1H), 5.50-5.36 (m, 1H), 5.05-4.97(m, 1H), 4.29-3.93(m, 7H), 3.15-3.01(m, 2H), 2.75-2.72(m, 1H), 2.59-2.56(m, 1H), 2.12-1.99(m, 2H), 1.94-1.91(m, 1H), 1.83-1.73(m, 2H), 1.67-1.49(m, 3H).

### Example A-49: 4-(4-(3,3-Difluoroazetidin-1-yl)-2-(((1S,7a'S)-2,2-difluorodihydro-1'H,3'H-spiro[cyclopropane-1,2'-pyrrolizin]-7a'(5'H)-yl)methoxy)-8-fluoropyrido[4,3-d]pyrimidin-7-yl)-5-ethynyl-6-fluoronaphthalen-2-ol

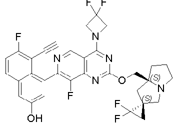

LCMS: m/z 626 (M+H)⁺. ¹H NMR (400MHz, DMSO-*d*₆) *δ* 10.18 (s, 1H), 8.96 (s, 1H), 7.99 (dd, *J=* 9.2, 5.9 Hz, 1H), 7.53 - 7.36 (m, 2H), 7.19 (d, *J* = 2.5 Hz, 1H), 5.07 (m, 4H), 4.31 - 4.16 (m, 2H), 3.98 (s, 1H), 3.16 - 2.97 (m, 2H), 2.73 (d, *J =* 11.8 Hz, 1H), 2.56 (m, 1H), 2.01 (m, 3H), 1.78 (m, 2H), 1.68 - 1.44 (m, 3H).

### Example A-50: 4-(2-(((1S,7a'S)-2,2-difluorodihydro-1'H,3'H-spiro[cyclopropane-1,2'-pyrrolizin]-7a'(5'H)-yl)methoxy)-4-(3,3-difluoropyrrolidin-1-yl)-8-fluoropyrido[4,3-d]pyrimidin-7-yl)-5-ethynyl-6-fluoronaphthalen-2-ol

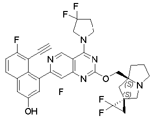

LCMS: m/z 640 (M+H)⁺. ¹H NMR (400MHz, DMSO-*d*₆) *δ* 10.19 (s, 1H), 9.24 (s, 1H), 7.99 (dd, *J =* 9.2, 5.9 Hz, 1H), 7.47 (t, *J =* 9.0 Hz, 1H), 7.41 (d, *J =* 2.5 Hz, 1H), 7.20 (d, *J =* 2.4 Hz, 1H), 4.42 (m,, 2H), 4.34 - 4.16 (m, 4H), 3.97 (s, 1H), 3.18 - 3.08 (m, 1H), 3.03 (m, 1H), 2.78 - 2.53 (m, 4H), 2.14 - 2.05 (m, 1H), 2.05 - 1.97 (m, 1H), 1.96 - 1.88 (m, 1H), 1.88 - 1.71 (m, 2H), 1.67 - 1.43 (m, 3H).

### Example A-51: 4-(4-(1,1-Difluoro-5-azaspiro[2.4]heptan-5-yl)-2-(((1S,7a'S)-2,2-difluorodihydro-1'H,3'H-spiro[cyclopropane-1,2'-pyrrolizin]-7a'(5'H)-yl)methoxy)-8-fluoropyrido[4,3-d]pyrimidin-7-yl)-5-ethynyl-6-fluoronaphthalen-2-ol

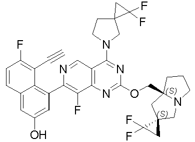

LCMS: m/z 666 (M+H)⁺. ¹H NMR (400MHz, DMSO-*d*₆) *δ* 10.15 (s, 1H), 9.22 (d, *J =* 4.6 Hz, 1H), 7.97 (dd, *J =* 9.2, 5.9 Hz, 1H), 7.46 (t, *J =* 9.0 Hz, 1H), 7.39 (d, *J =* 2.5 Hz, 1H), 7.18 (s, 1H), 4.26 - 4.12 (m, 4H), 3.96 (d, *J* = 2.5 Hz, 1H), 3.15 - 3.06 (m, 1H), 3.06 - 2.97 (m, 1H), 2.72 (m, 1H), 2.55 (m, 1H), 2.34 - 2.18 (m, 2H), 2.11 - 2.04 (m, 1H), 1.98 (m, 1H), 1.94 - 1.68 (m, 6H), 1.67 - 1.41 (m, 4H).

### Example B-1: Preparation of 1-(7-(8-ethyl-7-fluoro-3-hydroxynaphthalen-1-yl)-2-(((cis)-3-(methoxymethyl)tetrahydro-1H-pyrrolizin-7a(5H)-yl)methoxy)-5,6,7,8-tetrahydropyrido [3,4-d]pyrimidin-4-yl)-3-methylpiperidin-3-ol

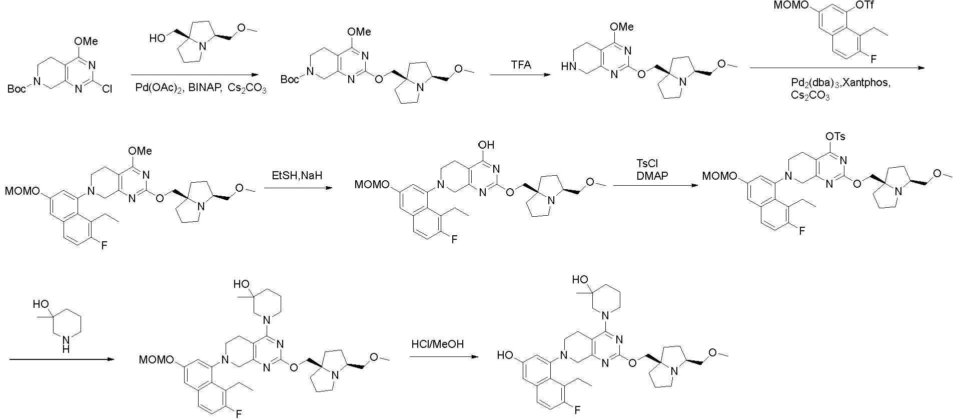

### Step 1: Preparation of tert-butyl 4-methoxy-2-(((cis)-3-(methoxymethyl)tetrahydro-1H-pyrrolizin-7a(5H)-yl)methoxy)-5,6,7,8-tetrahydropyrido[3,4-d]pyrimidine-7(6H)-carboxylate

To a solution of *tert*-butyl 2-chloro-4-methoxy-5,6-dihydropyrido[3,4-*d*]pyrimidine-7(8*H*)-carboxylate (3 g, 10 mmol) and ((*cis*)-3-(methoxymethyl)tetrahydro-1*H*-pyrrolizin-7a(5*H*)-yl)methanol (2.22 g, 12 mmol) in toluene (30 mL) was sequentially added cesium carbonate (9.8 g, 30 mmol), BINAP (1.25 g), and palladium acetate (0.25 g) under nitrogen atmosphere. The reaction solution was reacted at 110 °C for 12 h, then quenched by addition of water (60 mL), and extracted with ethyl acetate (30 mL × 3). The organic phases were combined, dried over anhydrous sodium sulfate, and then filtered, and the filtrate was concentrated under reduced pressure. The residue was separated by silica gel column chromatography to give the target product (3.1 g, yield: 69%).

LCMS: m/z 449 (M+H)⁺.

### Step 2: Preparation of 4-methoxy-2-(((cis)-3-(methoxymethyl)tetrahydro-1H-pyrrolizin-7a(5H)-yl)methoxy)-5,6,7,8-tetrahydropyrido[3,4-d]pyrimidine

To a solution of *tert*-butyl 4-methoxy-2-(((*cis*)-3-(methoxymethyl)tetrahydro-1*H*-pyrrolizin-7a(5*H*)-yl)methoxy)-5,6,7,8-tetrahydropyrido[3,4-*d*]pyrimidine-7(6*H*)-carboxylate (3 g, 6.7 mmol) in DCM (12 mL) was slowly added dropwise TFA (12 mL) at 0 °C. The reaction solution was reacted at room temperature for 2 h and then concentrated under reduced pressure. The residue was diluted with ice water (10 mL), then adjusted to pH 7 with a saturated aqueous sodium carbonate solution, and concentrated under reduced pressure and high vacuum to remove water. The residue was diluted with a saturated aqueous sodium carbonate solution (30 mL) and then extracted with ethyl acetate (15 mL × 6). The organic phases were combined, dried over anhydrous sodium sulfate, and then filtered, and the filtrate was concentrated under reduced pressure to give the target product (2.1 g), which was used directly in the next step without further purification.

LCMS: m/z 349 (M+H)⁺.

### Step 3: Preparation of 7-(8-ethyl-7-fluoro-3-(methoxymethoxy)naphthalen-1-yl)-4-methoxy-2-(((cis)-3-(methoxymethyl)tetrahydro-1H-pyrrolizin-7a(5H)-yl)methoxy)-5,6,7,8-tetrahydropyrido[3,4-d] pyrimidine

To a solution of 4-methoxy-2-(((*cis*)-3-(methoxymethyl)tetrahydro-1*H*-pyrrolizin-7a(5*H*)-yl)methoxy)-5,6,7,8-tetrahydropyrido[3,4-*d*]pyrimidine obtained in the previous step (2 g, 5.73 mmol) and 8-ethyl-7-fluoro-3-(methoxymethoxy)naphthalen-1-yl trifluoromethanesulfonate (2.85 g, 7.45 mmol) in toluene (20 mL) was sequentially added cesium carbonate (5.6 g), Xantphos (0.7 g), and Pd₂(dba)₃ (0.53 g) under nitrogen atmosphere. The reaction solution was reacted at 110 °C for 6 h and then filtered through celite, and the filter cake was washed with ethyl acetate (5 mL × 3). The organic phases were combined and concentrated under reduced pressure, and the residue was separated by preparative liquid chromatography to give the target product (2.53 g, yield (over two steps): 65%).

LCMS: m/z 581 (M+H)⁺.

### Step 4: Preparation of 7-(8-ethyl-7-fluoro-3-(methoxymethoxy)naphthalen-1-yl)-2-(((cis)-3-(methoxymethyl)tetrahydro-1H-pyrrolizin-7a(5H)-yl)methoxy)-5,6,7,8-tetrahydropyrido [3,4-d]pyrimidin-4-ol

To a solution of EtSH (0.64 g, 10.3 mmol) in DMAc (20 mL) was added NaH (0.41 g, 60% wt, 10.3 mmol) at 0 °C. The reaction solution was reacted at 0 °C for 0.5 h, followed by addition of 7-(8-ethyl-7-fluoro-3-(methoxymethoxy)naphthalen-1-yl)-4-methoxy-2-(((*cis*)-3-(methoxymethyl)tetrahydro-1*H*-pyrrolizin-7a(5*H*)-yl)methoxy)-5,6,7,8-tetrahydropyrido[3,4-*d*]pyrimidine (2 g, 3.44 mmol). The reaction solution was reacted at 20 °C for 1 h and then quenched by addition of water (400 mL). The resulting mixture was adjusted to pH 6 with 2 N HCl and then extracted with ethyl acetate (20 mL × 3). The organic phases were combined, washed with saturated brine (20 mL × 2), then dried over anhydrous sodium sulfate, and filtered. The filtrate was concentrated under reduced pressure to give the target product (2 g), which was used directly in the next step without further purification.

LCMS: m/z 567 (M+H)⁺.

### Step 5: Preparation of 7-(8-ethyl-7-fluoro-3-(methoxymethoxy)naphthalen-1-yl)-2-(((cis)-3-(methoxymethyl)tetrahydro-1H-pyrrolizin-7a(5H)-yl)methoxy)-5,6,7,8-tetrahydropyrido[3,4-d]pyrimidin-4-yl 4-methylbenzenesulfonate

To a solution of 7-(8-ethyl-7-fluoro-3-(methoxymethoxy)naphthalen-1-yl)-2-(((*cis*)-3-(methoxymethyl)tetrahydro-1*H*-pyrrolizin-7a(5*H*)-yl)methoxy)-5,6,7,8-tetrahydropyrido[3,4-*d*]pyrimidin-4-ol obtained in the previous step (2 g), DIPEA (1.33 g, 10.3 mmol), and DMAP (50 mg, 0.41 mmol) in DCM (20 mL) was added *p*-toluenesulfonyl chloride (1 g) at 0 °C. The reaction solution was reacted at 25 °C for 0.5 h and then concentrated under reduced pressure. The residue was separated by preparative liquid chromatography to give the target product (1.74 g, yield (over two steps): 70%).

LCMS: m/z 721 (M+H)⁺.

### Step 6: Preparation of 1-(7-(8-ethyl-7-fluoro-3-(methoxymethoxy)naphthalen-1-yl)-2-(((cis)-3-(methoxymethyl)tetrahydro-1H-pyrrolizin-7a(5H)-yl)methoxy)-5,6,7,8-tetrahydropyrido[3,4-d]pyrimidin-4-yl)-3-methylpiperidin-3-ol

To a solution of 7-(8-ethyl-7-fluoro-3-(methoxymethoxy)naphthalen-1-yl)-2-(((*cis*)-3-(methoxymethyl)tetrahydro-1*H*-pyrrolizin-7a(5*H*)-yl)methoxy)-5,6,7,8-tetrahydropyrido[3,4-*d*]pyrimidin-4-yl 4-methylbenzenesulfonate (100 mg, 0.14 mmol), 3-methylpiperidin-3-ol (33 mg, 0.28 mmol), and 4A molecular sieves (20 mg) in DMF (1 mL) was added DIPEA (75 mg). The resulting reaction solution was reacted at 60 °C until complete conversion. The mixture was filtered, and the filter cake was washed with DMF (1 mL). The filtrate was separated by preparative liquid chromatography to give the target product (75 mg, yield: 80.7%).

LCMS: m/z 664 (M+H)⁺.

### Step 7: Preparation of 1-(7-(8-ethyl-7-fluoro-3-hydroxynaphthalen-1-yl)-2-(((cis)-3-(methoxymethyl)tetrahydro-1H-pyrrolizin-7a(5H)-yl)methoxy)-5,6,7,8-tetrahydropyrido[3,4-d]pyrimidin-4-yl)-3-methylpiperidin-3-ol

To a solution of 1-(7-(8-ethyl-7-fluoro-3-(methoxymethoxy)naphthalen-1-yl)-2-(((*cis*)-3-(methoxymethyl)tetrahydro-1*H*-pyrrolizin-7a(5*H*)-yl)methoxy)-5,6,7,8-tetrahydropyrido[3,4-*d*]pyrimidin-4-yl)-3-methylpiperidin-3-ol (70 mg, 0.105 mmol) in methanol (1.5 mL) was added a solution of HCl in methanol (4 M, 1.5 mL) at 0 °C. The reaction solution was reacted at 0 °C for 0.5 h and then adjusted to pH 8 with a saturated aqueous sodium bicarbonate solution. The resulting mixture was extracted with ethyl acetate (3 × 5 mL). The organic phases were combined, dried over anhydrous sodium sulfate, and then filtered. The filtrate was concentrated under reduced pressure, and the residue was separated by preparative liquid chromatography to give the target product (25 mg, yield: 38.4%).

LCMS: m/z 620 (M+H)⁺.

**According to the method of Example B-1, the following examples were synthesized with different starting materials:**

### Example B-2: 1-(7-(8-Ethyl-7-fluoro-3-hydroxynaphthalen-1-yl)-2-(((trans)-3-(methoxymethyl) tetrahydro-1H-pyrrolizin-7a(5H)-yl)methoxy)-5,6,7,8-tetrahydropyrido[3,4-d]pyrimidin-4-yl)-3-methylpiperidin-3-ol

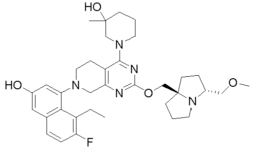

LCMS: m/z 620 (M+H)⁺.

### Example B-3: 1-(7-(8-Ethyl-7-fluoro-3-hydroxynaphthalen-1-yl)-2-(((2R)-2-fluoro-5-(methoxymethyl)tetrahydro-1H-pyrrolizin-7a(5H)-yl)methoxy)-5,6,7,8-tetrahydropyrido[3,4-d]pyrimidin-4-yl)-3-methylpiperidin-3-ol

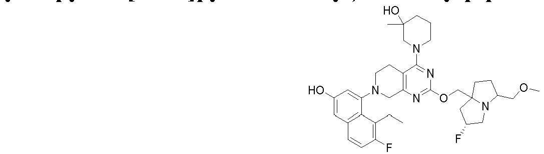

LCMS: m/z 638 (M+H)⁺.

### Example B-4: 7-(7-(8-Ethyl-7-fluoro-3-hydroxynaphthalen-1-yl)-2-(((cis)-3-(methoxymethyl) tetrahydro-1H-pyrrolizin-7a(5H)-yl)methoxy)-5,6,7,8-tetrahydropyrido[3,4-d]pyrimidin-4-yl)-1,3,7-triazaspiro[4.5]decane-2,4-dione

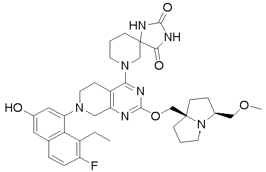

LCMS: m/z 674 (M+H)⁺.

### Example B-5: 7-(7-(8-Ethyl-7-fluoro-3-hydroxynaphthalen-1-yl)-2-(((trans)-3-(methoxymethyl) tetrahydro-1H-pyrrolizin-7a(5H)-yl)methoxy)-5,6,7,8-tetrahydropyrido[3,4-d]pyrimidin-4-yl)-1,3,7-triazaspiro[4.5]decane-2,4-dione

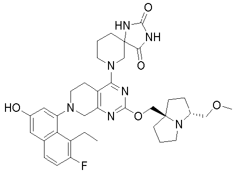

LCMS: m/z 674 (M+H)⁺.

### Example B-6: 7-(7-(8-Ethyl-7-fluoro-3-hydroxynaphthalen-1-yl)-2-(((2R)-2-fluoro-5-(methoxymethyl)tetrahydro-1H-pyrrolizin-7a(5H)-yl)methoxy)-5,6,7,8-tetrahydropyrido[3,4-d]pyrimidin-4-yl)-1,3,7-triazaspiro[4.5]decane-2,4-dione

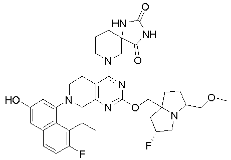

LCMS: m/z 692 (M+H)⁺.

### Example B-7: 5-(7-(8-Ethyl-7-fluoro-3-hydroxynaphthalen-1-yl)-2-(((cis)-3-(methoxymethyl) tetrahydro-1H-pyrrolizin-7a(5H)-yl)methoxy)-5,6,7,8-tetrahydropyrido[3,4-d]pyrimidin-4-yl)-N,N-dimethyl-5,6,7,8-tetrahydro-4H-pyrazolo[1,5-a][1,4]diazepine-2-carboxamide

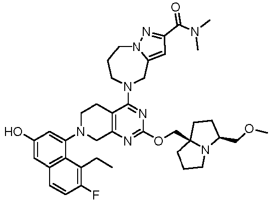

LCMS: m/z 713 (M+H)⁺.

### Example B-8: 5-(7-(8-Ethyl-7-fluoro-3-hydroxynaphthalen-1-yl)-2-(((trans)-3-(methoxymethyl) tetrahydro-1H-pyrrolizin-7a(5H)-yl)methoxy)-5,6,7,8-tetrahydropyrido[3,4-d]pyrimidin-4-yl)-N,N-dimethyl-5,6,7,8-tetrahydro-4H-pyrazolo[1,5-a][1,4]diazepine-2-carboxamide

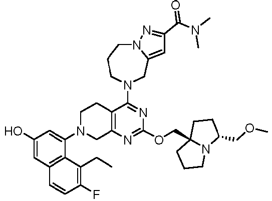

LCMS: m/z 713 (M+H)⁺.

### Example B-9: 5-(7-(8-Ethyl-7-fluoro-3-hydroxynaphthalen-1-yl)-2-(((2R)-2-fluoro-5-(methoxymethyl)tetrahydro-1H-pyrrolizin-7a(5H)-yl)methoxy)-5,6,7,8-tetrahydropyrido[3,4-d]pyrimidin-4-yl)-N,N-dimethyl-5,6,7,8-tetrahydro-4H-pyrazolo[1,5-a][1,4]diazepine-2-carboxamide

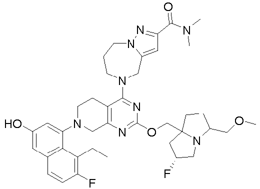

LCMS: m/z 731 (M+H)⁺.

### Example B-10: 1-(7-(8-Ethyl-7-fluoro-3-hydroxynaphthalen-1-yl)-2-((1-(morpholinomethyl) cyclopropyl)methoxy)-5,6,7,8-tetrahydropyrido[3,4-d]pyrimidin-4-yl)-3-methylpiperidin-3-ol

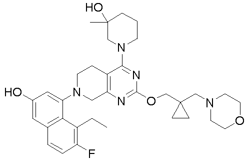

LCMS: m/z 606 (M+H)⁺.

### Example B-11: 7-(7-(8-Ethyl-7-fluoro-3-hydroxynaphthalen-1-yl)-2-((1-(morpholinomethyl) cyclopropyl)methoxy)-5,6,7,8-tetrahydropyrido[3,4-d]pyrimidin-4-yl)-1,3,7-triazaspiro[4.5] decane-2,4-dione

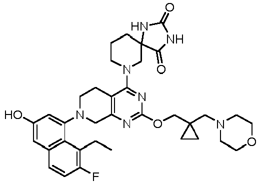

LCMS: m/z 660 (M+H)⁺.

### Example B-12: 5-(7-(8-Ethyl-7-fluoro-3-hydroxynaphthalen-1-yl)-2-((1-(morpholinomethyl) cyclopropyl)methoxy)-5,6,7,8-tetrahydropyrido[3,4-d]pyrimidin-4-yl)-N,N-dimethyl-5,6,7,8-tetrahydro-4H-pyrazolo[1,5-a][1,4]diazepine-2-carboxamide

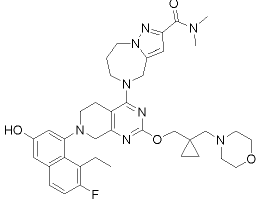

LCMS: m/z 699 (M+H)⁺.

### Example B-13: 5-(2-(((1S,7a'S)-2,2-Difluorodihydro-1'H,3'H-spiro[cyclopropane-1,2'-pyrrolizin]-7a'(5'H)-yl)methoxy)-7-(8-ethyl-7-fluoro-3-hydroxynaphthalen-1-yl)-5,6,7,8-tetrahydropyrido[3,4-d]pyrimidin-4-yl)-N,N-dimethyl-5,6,7,8-tetrahydro-4H-pyrazolo[1,5-a][1,4]diazepine-2-carboxamide

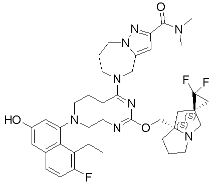

LCMS: m/z 731 (M+H)⁺. ¹H NMR (400MHz, DMSO-*d*₆) *δ* 7.60 (dd, *J*=5.92Hz, *J*=8.92Hz, 1 H), 7.26(t, *J*=9.52Hz, 1 H), 7.01(s, 2 H), 6.52(s, 1H), 6.07(s, 1H), 5.05 (dd, *J*=4.44Hz, *J*=16.16Hz, 1 H), 4.75(dd, *J*=4.44Hz, *J*=16.16Hz, 1 H), 4.51 (m, 2 H), 4.15(m, 1 H), 3.97(m, 4 H), 3.60(m, 1 H), 3.44(m, 3 H), 3.04 (m, 9 H), 2.68(m, 2 H), 2.20 (m, 1 H), 1.73 (m,11H), 1.08(t, *J*=7.12Hz,3 H).

### Example B-14: 5-(2-(((1R,7a'R)-2,2-Difluorodihydro-1'H,3'H-spiro[cyclopropane-1,2'-pyrrolizin]-7a'(5'H)-yl)methoxy)-7-(8-ethyl-7-fluoro-3-hydroxynaphthalen-1-yl)-5,6,7,8-tetrahydropyrido[3,4-d]pyrimidin-4-yl)-N,N-dimethyl-5,6,7,8-tetrahydro-4H-pyrazolo[1,5-a][1,4]diazepine-2-carboxamide hydrochloride

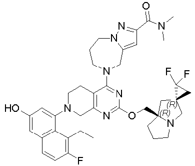

LCMS: m/z 731 (M+H)⁺. ¹H NMR (400MHz, DMSO-*d*₆) *δ* 9.72 (s, 1 H), 9.27 (s, 0.5 H), 7.61(m, 1 H), 7.26(m, 1 H), 7.01(s, 2 H), 6.53(s, 1H), 5.84(m, 0.5H), 5.01 (m, 1 H), 4.75(m, 1 H), 4.51 (m, 2 H), 4.17(m, 1 H), 3.91(m, 4 H), 3.60(m, 1 H), 3.26(m, 3 H), 2.95 (m,6 H), 2.69(m,3 H), 1.88 (m,12H), 1.08(m, 3 H).

### Example B-15: 5-(7-(2-Amino-3-cyano-7-fluorobenzo[b]thiophen-4-yl)-2-(((trans)-2,2-fluorodihydro-1'H,3'H-spiro[cyclopropane-1,2'-pyrrolizin]-7a'(5'H)-yl)methoxy)-5,6,7,8-tetrahydropyrido[3,4-d]pyrimidin-4-yl)-N,N-dimethyl-5,6,7,8-tetrahydro-4H-pyrazolo[1,5-a][1,4]diazepine-2-carboxamide

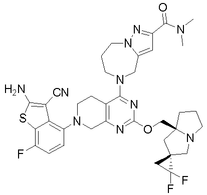

LCMS: m/z 733 (M+H)⁺. ¹H NMR (400 MHz, DMSO-*d*₆) *δ* 7.97 (s, 2 H), 7.08-7.04(m, 1 H), 6.98(t, *J =* 8Hz, 1H), 6.54 (s, 1H), 4.81(s, 2 H), 4.48 (t, *J* =4 Hz, 2H), 4.13(s, 2 H), 3.97 (s,3H), 3.22(s, 4H), 2.93 (s, 4H), 2.67 (s, 1H), 2.18-1.44 (m, 11H), 1.24(s,4H), 0.85 (t, *J* =8 Hz, 1H).

### Example B-16: 5-(7-(8-Chloro-7-fluoronaphthalen-1-yl)-2-(((trans)-2,2-2,2-fluorodihydro-1'H,3'H-spiro[cyclopropane-1,2'-pyrrolizin]-7a'(5'H)-yl)methoxy)-5,6,7,8-tetrahydropyrido [3,4-d]pyrimidin-4-yl)-N,N-dimethyl-5,6,7,8-tetrahydro-4H-pyrazolo[1,5-a][1,4]diazepine-2-carboxamide

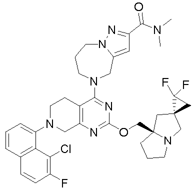

LCMS: m/z 721 (M+H)⁺. ¹H NMR (400 MHz, DMSO- *d₆*) *δ* 8.02 (dd, *J* =12Hz, *J* =8Hz, 1 H), 7.79(d, *J =* 8Hz, 1 H), 7.59(t, *J =* 8Hz, 1H), 7.54(t, *J =* 8Hz, 1H), 7.37(d, *J =* 8Hz, 1H), 6.46(s, 1H), 4.93(d, *J =* 16Hz, 1 H), 4.79 (d, *J =* 16Hz, 1 H), 4.49 (t, *J* =4 Hz, 2H), 4.12-3.91(m, 5 H), 3.76 (d, *J =* 16Hz, 1H), 3.49 (d, *J =* 8Hz, 1H),3.24-2.94(m, 10H), 2.71 (d, *J =* 12Hz, 1H), 2.59 (d, *J=* 16Hz, 1H), 2.15 (s, 1H), 2.05-1.65(m,7H ), 1.60-1.41(m, 3H).

### Example P-1: Synthesis of 4-(2-(((1S,7a'S)-2,2-Difluorodihydro-1'H,3'H-spiro[cyclopropane-1,2'-pyrrolizin]-7a'(5'H)-yl)methoxy)-8-fluoro-4-(1-oxa-6-azaspiro[3.5]nonan-6-yl)pyrido[4,3-d]pyrimidin-7-yl)-5-ethyl-6-fluoronaphthalen-2-yl methylcarbamate

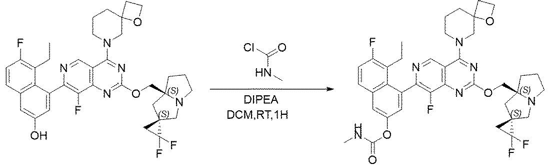

To a solution of 4-(2-(((1*S*,7a'*S*)-2,2-difluorodihydro-1'*H*,3'*H*-spiro[cyclopropane-1,2'-pyrrolizin]-7a'(5'*H*)-yl)methoxy)-8-fluoro-4-(1-oxa-6-azaspiro[3.5]nonan-6-yl)pyrido[4,3-*d*]pyrimidin-7-yl)-5-ethyl-6-fluoronaphthalen-2-ol (30 mg, 0.045 mmol), DIPEA (12 mg, 0.090 mmol), and dichloromethane (1 mL) was added methylcarbamoyl chloride (9 mg, 0.090 mmol) under argon atmosphere. The mixture was reacted at room temperature for 2 h and then separated by preparative liquid chromatography to give the target product (15 mg, yield: 46.3%).

LCMS: m/z 721 (M+H)⁺. ¹H NMR (400 MHz, DMSO- *d₆*) *δ* 9.25 (m, 1 H), 7.99(m, 1 H), 7.87(d, *J*=2.52Hz, 1 H), 7.75 (m, 1 H), 7.50(m, 1 H), 7.31(m, 1H), 4.23(m, 7 H), 3.54(m, 1 H), 3.11(m, 1H), 3.01(m, 1 H), 2.69(d, *J*=4.64Hz, 3 H), 2.55 (m, 1 H), 2.42 (m, 2 H), 2.01 (m, 15 H), 0.76(m,3 H).

**According to the method of Example P-1, the following examples were synthesized with different starting materials:**

### Example P-2: 4-(4-((cis)-2-Oxa-6-azabicyclo[5.1.0]octan-6-yl)-2-(((1S,7a'S)-2,2-difluorodihydro-1'H,3'H-spiro[cyclopropane-1,2'-pyrrolizin]-7a'(5'H)-yl)methoxy)-8-fluoropyrido[4,3-d]pyrimidin-7-yl)-5-ethynyl-6-fluoronaphthalen-2-yl methylcarbamate

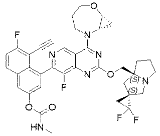

LCMS: m/z 703 (M+H)⁺. ¹H NMR (400 MHz, DMSO- *d₆*) *δ* 9.43 (s, 0.33 H), 9.38 (s, 0.67 H), 8.18(m, 1 H), 7.95(m, 1 H), 7.80(m, 1 H), 7.61(m, 1 H), 7.43(m, 1 H), 4.48(m, 1 H), 4.26 (s, 0.67 H), 4.20(m, 2 H), 3.97 (s, 0.33 H), 3.89(m, 1 H), 3.73(m, 2H), 3.62(m, 1 H), 3.44(m, 1 H), 3.11(m, 1 H), 3.02(m, 1 H), 2.70(m, 3 H), 2.55 (m, 1 H), 1.81 (m, 12 H), 0.93(m, 0.67 H), 0.66(m,0.33H).

### Example P-3: 4-(2-(((1S,7a'S)-2,2-Difluorodihydro-1'H,3'H-spiro[cyclopropane-1,2'-pyrrolizin]-7a'(5'H)-yl)methoxy)-8-fluoro-4-(((trans)-2-fluorocyclopropyl)(methyl)amino) pyrido[4,3-d]pyrimidin-7-yl)-5-ethynyl-6-fluoronaphthalen-2-yl methylcarbamate

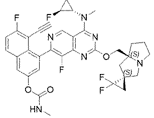

LCMS: m/z 679 (M+H)⁺. ¹H NMR (400 MHz, DMSO- *d₆*) *δ* 9.50 (m, 1 H), 8.19(m, 1 H), 7.97(m, 1 H), 7.81 (m, 1 H), 7.62(m, 1 H), 7.46(m, 1H), 5.00(m, 1 H), 4.19(m, 4 H), 3.35(m, 1 H), 3.28(m, 1 H), 3.11(m, 1H), 3.01(m, 1 H), 2.70(m, 3 H), 2.54 (m, 1 H), 1.75(m,12 H).

### Example: Biological test examples

### Biological test evaluation

The present disclosure is further described and explained with reference to the following biological test examples, but these examples are not intended to limit the scope of the present disclosure.

### ERK phosphorylation inhibition assay

### Procedure

KRAS G12 mutant cells (e.g., AsPC-1 (G12D, human metastatic pancreatic adenocarcinoma cells), A549 (G12S, human non-small cell lung cancer cells), HCT116 (G13D, human colon cancer cells), NCI-H358 (G12C, human non-small cell lung cancer cells), NCI-H460 (Q61H, large cell lung cancer cells), NCI-H727 (G12V, human lung bronchial tumor cells), or MKN1 (WTdep, human gastric cancer cells)) were seeded into a 384-well plate and incubated in an incubator with 5% CO₂ at 37 °C overnight.

200 nL of diluted compound was added using Echo 500, with a final DMSO concentration of 0.5%, and incubated in an incubator with 5% CO₂ at 37 °C for 1 h. hEGF was then added and incubated for 10 min.

The medium was removed, and a cell fixation solution was added to fix the cells.

The cells were washed once with PBS and incubated with cold 100% methanol.

The methanol was then removed, and the plate was washed once with PBS.

The PBS was then removed, and a Li-Cor blocking buffer was added to each well for blocking at room temperature for 1 h.

The blocking buffer was then removed, a primary antibody mixture was added to each well, and the plate was incubated at room temperature overnight.

The primary antibody mixture was then removed, and the plate was washed 3 times with PBST. A secondary antibody mixture was added, and the plate was incubated at room temperature in the dark for 45 min.

The secondary antibody mixture was then removed and washed 3 times with PBST. Finally, the PBST was aspirated, and the plate was inverted and centrifuged at 1000 rpm for 1 min. Readings were taken using Odyssey CLx.

The results indicate that the compounds in the examples of the present disclosure showed good inhibitory activity against KRAS mutations.

### Assay for inhibition of KRAS mutant cell proliferation by compounds

| **Reagent** | **Supplier** | **Catalog No.** |
|---|---|---|
| AsPC-1 (G12D) | ATCC | CRL-1682 |
| H727 (G12V) | ATCC | CRL-5815 |
| SW480 (G12V) | ATCC | CCL-228 |
| PSN-1 (G12R) | ATCC | CRL-3211 |
| MNK1 (WT) | JCRB | JCRB0252 |
| RPMI 1640 medium | Gibco | A10491-01 |
| CellTiter-Glo^{®} 3D cell activity assay kit | Promega | G9683 |

### Positive control: MRTX1133

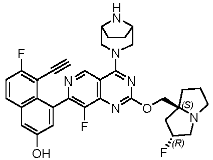

### Procedure

### 1. Cell culture

(a) The cells were thawed in T75 cell culture flasks:
(b) When 80%-90% cell confluence was achieved, the cells were passaged.

### 2. Cell proliferation assay

### Procedure

The diluted test compound was added to a 384-well cell culture plate using a nanoliter pipetting system, and replicate wells were set. An equal volume of medium was added to the positive control group; an equal volume of DMSO was added to the negative control group. The plate was centrifuged at 1000 rpm at room temperature for 1 min.

The cells were seeded into a) 384-well culture plate. An equal volume of cells was added to the negative control group, and only an equal volume of medium was added to the positive control group. The plate was centrifuged at 1000 rpm at room temperature for 1 min. The final DMSO concentration in the final compound was 0.5%. The plate was incubated in a thermostatic incubator at 37 °C with 5% CO₂ for 7 days.

CellTiter-Glo^{®} 3D was added to b) 384-well cell culture plate at 20 µL/well. The plate was shaken at 320 rpm in the dark for 20 min, and incubated at room temperature in the dark for 2 h. The luminescence values were read using an Envision multi-mode microplate reader.

### 3. Data analysis

The inhibition rates (IRs) of the test compounds were calculated according to the following formula: IR (%) = (1 - (RLU compound - RLU blank control) / (RLU vehicle control - RLU blank control)) × 100%. The inhibition rates of the compounds at different concentrations were calculated in Excel, and then inhibition curves were plotted and relevant parameters including minimum inhibition rate, maximum inhibition rate and IC₅₀ were calculated using GraphPad Prism software.

**Table 1. Anti-cell proliferation activity of compounds**

| **Example** | **AsPC-1 IC₅₀ (nM)** | **H727 IC₅₀ (nM)** | **SW480 IC₅₀ (nM)** | **PSN1IC₅₀ (nM)** | **MNK1 IC₅₀ (nM)** |
|---|---|---|---|---|---|
| **MRTX1133** | 10.09 | 2266.56 | 432.02 | >10000 | 319.27 |
| **Example A-13A** | >1000 | 1991 | / | >10000 | 633.8 |
| **Example A-13B** | 18.78 | 51.66 | / | 506 | 12.89 |
| **Example A-14** | 61.09 | 242.15 | / | >10000 | 132.87 |
| **Example A-15** | 163.56 | 449.97 | / | >10000 | 171.2 |
| **Example A-16** | 11.42 | 45.08 | 68.20 | 1000 | 22.71 |
| **Example A-16A** | 5.7 | / | 11.07 | / | / |
| **Example A-16B** | 387 | / | 660 | / | / |
| **Example A-21** | >1000 | / | / | / | / |
| **Example A-22** | >1000 | / | / | / | / |
| **Example A-23** | >1000 | / | / | / | / |
| **Example A-24** | 11.13 | 101.30 | / | >10000 | 19.67 |
| **Example A-25** | 33.63 | 140.81 | / | >10000 | 66.32 |
| **Example A-26** | >1000 | 7373 | / | >10000 | 1624 |
| **Example A-27** | 23.16 | 35.90 | / | 79.74 | 5.35 |
| **Example A-28** | 27.59 | / | 37.30 | / | / |
| **Example A-29** | 4.85 | / | 2.10 | / | / |
| **Example A-30** | / | / | / | / | / |
| **Example A-31** | 736 | / | 651 | / | / |
| **Example A-32** | / | / | 335.98 | / | / |
| **Example A-33** | / | / | 168.14 | / | / |
| **Example A-34** | / | / | 66.68 | / | / |
| **Example A-35** | / | / | 158.83 | | |
| **Example A-36** | / | / | 102.32 | / | / |
| **Example A-37** | 572 | / | 307 | / | / |
| **Example A-38** | 31.58 | 118.67 | / | >10000 | 21.40 |
| **Example A-39** | 292.72 | 603.71 | / | >10000 | 272.95 |
| **Example A-40** | >1000 | / | / | / | / |
| **Example A-41** | >1000 | / | / | / | / |
| **Example A-42** | >1000 | / | / | / | / |
| **Example A-43** | 986 | / | / | / | / |
| **Example A-44** | 25.13 | / | 7.13 | / | / |
| **Example A-45** | 74.40 | / | 40.99 | / | / |
| **Example A-46** | 732 | / | 482 | / | / |
| **Example B-13** | 8.98 | 47.34 | / | 2349 | 24.31 |
| **Example B-14** | 26.9 | 126.71 | / | 7556 | 92.29 |
| **Example B-15** | >1000 | / | / | / | / |
| **Example B-16** | >1000 | / | / | / | / |

In the table, "/" denotes that no assay was performed.

The results indicate that the compounds in the examples of the present disclosure showed good inhibitory activity against KRAS mutations.

**Pharmacokinetic test evaluation**

Male SD rats weighing approximately 220 g were fasted overnight and then given a solution of the compound of the present disclosure or the control compound [with 10% cortisol (captisol) and 50 mM sodium citrate, pH 5 as the vehicle] via intraperitoneal injection at 30 mg/kg. Blood samples were collected at 0.083, 0.25, 0.5, 1.0, 2.0, 4.0, 6.0, 8.0, 12.0, and 24 h after administration of the compound of the present disclosure. The concentration of the compound of the present disclosure or the control compound in plasma was determined by LC/MS/MS.

As can be seen from the test results, the compounds of the present disclosure had good pharmacokinetic properties.

### Pharmacodynamic test evaluation of antitumor activity (NCI-H727 CDX tumor model)

100 µL of a suspension containing 5 × 10⁶ NCI-H727 tumor cells was subcutaneously inoculated into the right posterior abdomen of nude mice. The mice were monitored daily for health condition, and the measurement was started when tumors grew to be palpable. The tumor volume was calculated as follows: 0.5 × L × W², where L and W represent the length and width of the tumor, respectively. When the tumors grew to about 200 mm³, the mice were randomized into groups. The mice were intragastrically given the corresponding dose of the compound daily, while their general condition was monitored. The tumors were measured 3 times a week, and the body weight was measured twice a week.

As can be seen from the test results, the compounds of the present disclosure had good antitumor effects.

All documents mentioned in the present disclosure are incorporated herein by reference, just as each document is individually cited as a reference. In addition, it should be understood that various modifications or changes may be made by those skilled in the art after reading the above teachings of the present disclosure, and these equivalent forms also fall within the scope defined by the appended claims of the present application.

## Claims

1. A compound of formula (I), or a stereoisomer, a tautomer, a crystalline form, a pharmaceutically acceptable salt, a hydrate, a solvate or a prodrug thereof: 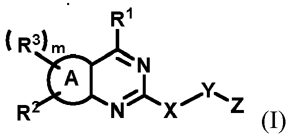 wherein in the formula, 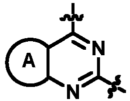 is selected from the group consisting of the following groups: 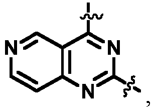 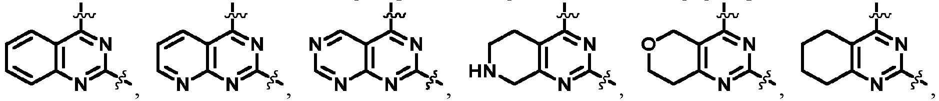 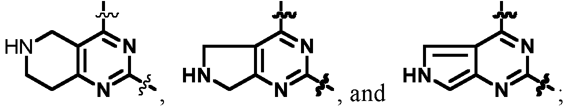 R¹ is selected from the group consisting of the following substituted or unsubstituted groups: C₁-C₁₀ alkyl, C₃-C₂₀ cycloalkyl, saturated or unsaturated 4- to 20-membered heterocyclyl, -NR^{a}R^{b}, and -OR^{a}, wherein the substitution refers to substitution with one or more R; with the proviso that R¹ is not substituted or unsubstituted 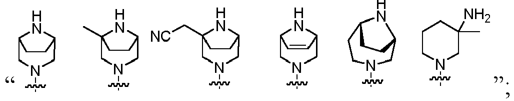 R^{a} and R^{b} are each independently selected from the group consisting of the following substituted or unsubstituted groups: hydrogen, deuterium, C₁-C₁₀ alkyl, C₃-C₂₀ cycloalkyl, and saturated or unsaturated 4- to 20-membered heterocyclyl, wherein the substitution refers to substitution with one or more R;
preferably, R¹ is selected from the group consisting of the following substituted or unsubstituted groups: 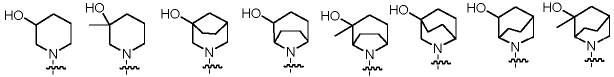 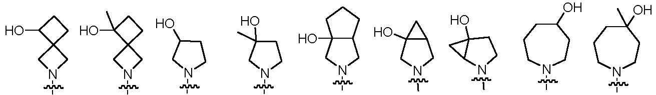 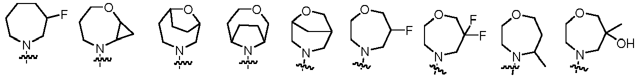 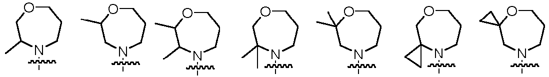 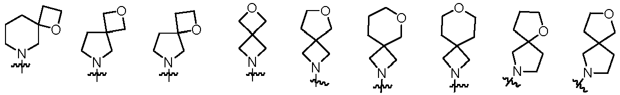 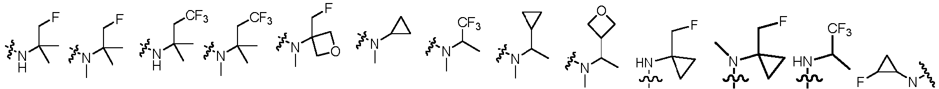 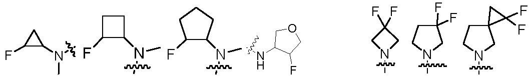 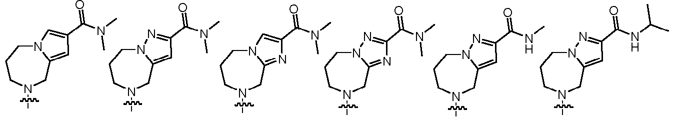 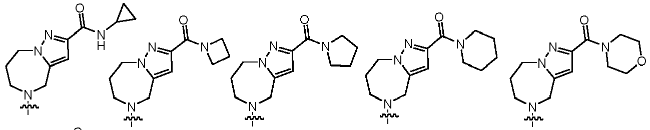 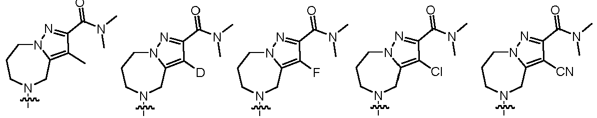 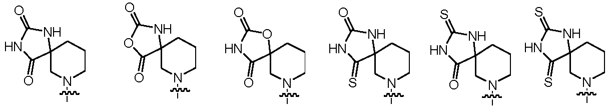 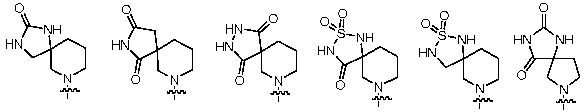 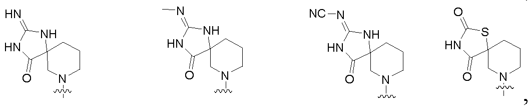
wherein the substitution refers to substitution with one or more R;
R² is selected from the group consisting of the following substituted or unsubstituted groups: C₆-C₁₄ aryl and 5- to 14-membered heteroaryl, wherein the substitution refers to substitution with one or more R;
each R³ is independently selected from the group consisting of the following substituted or unsubstituted groups: H, deuterium, halogen, cyano, ester group, amino, amido, sulfonyl, ureido, C₁-C₆ alkyl, C₁-C₆ deuterated alkyl, C₁-C₆ haloalkyl, C₃-C₆ cycloalkyl, 4- to 6-membered heterocyclyl, C₁-C₆ alkyloxy, C₃-C₆ cycloalkyloxy, and 4- to 6-membered heterocyclyloxy; m is an integer of 0, 1, 2, 3, 4, 5, or 6;
X is selected from: a bond, O, NH, and N(C₁-C₃ alkyl);
Y is selected from: a bond and substituted or unsubstituted C₁-C₆ alkylene, wherein the substitution refers to substitution with one or more R;
1) Z is selected from: 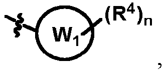
wherein ring W₁ is selected from the group consisting of the following substituted or unsubstituted groups: C₃-C₂₀ cycloalkylene and saturated or unsaturated 4- to 20-membered heterocyclylene, wherein the substitution refers to substitution with one or more R;
R⁴ is selected from: H and -L₁-Q₁-L₂-L₃, with the proviso that when W₁ is 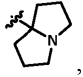 R⁴ is not H,
wherein
i) Q₁ is selected from: O, S, SO₂, NH, NR⁵, CONH, CONR⁵, SO₂NH, and SO₂NR⁵, wherein R⁵ is selected from the group consisting of the following substituted or unsubstituted groups: -C₁-C₆ alkyl, -C₃-C₆ cycloalkyl, -4- to 6-membered heterocyclyl, -C₆-C₁₀ aryl, and -5- to 10-membered heteroaryl, wherein the substitution refers to substitution with one or more R; and
L₁ is selected from: none and substituted or unsubstituted C₁-C₆ alkylene, wherein the substitution refers to substitution with one or more R; and
L₂ is selected from: none and substituted or unsubstituted C₁-C₆ alkylene, wherein the substitution refers to substitution with one or more R; and
L₃ is selected from the group consisting of the following substituted or unsubstituted groups: - C₁-C₆ alkyl, C₁-C₆ alkoxy, -C₃-C₆ cycloalkyl, -4- to 10-membered heterocyclyl, -O-C₃-C₆ cycloalkyl, -O-4- to 6-membered heterocyclyl, -C₁-C₆ alkylene C₃-C₆ cycloalkyl, -C₁-C₆ alkylene 4- to 6-membered heterocyclyl, -C₁-C₆ alkylene-O-C₁-C₆ alkyl, -C₁-C₆ alkylene-O-C₃-C₆ cycloalkyl, -C₁-C₆ alkylene-O-4- to 6-membered heterocyclyl, NHR^{9'}, and NR^{9'}R^{10'}; R^{9'} and R^{10'} are each independently selected from the group consisting of the following substituted or unsubstituted groups: C₁-C₆ alkyl, C₃-C₁₀ cycloalkyl, 4- to 10-membered heterocyclyl, (C₃-C₁₀ cycloalkyl)C₁-C₆ alkyl, and (4- to 10-membered heterocyclyl)C₁-C₆ alkyl, or R^{9'} and R^{10'}, together with the N atom to which they are attached, form substituted or unsubstituted 4- to 10-membered heterocyclyl, wherein the substitution refers to substitution with one or more R;
or
ii) -L₁-Q₁ is selected from the group consisting of the following substituted or unsubstituted groups: -C₄-C₈ alkyl, -C₁-C₆ haloalkyl, -C₃-C₁₀ cycloalkyl, -4- to 10-membered heterocyclyl, - C₆-C₁₀ aryl, -5- to 10-membered heteroaryl, -C₁-C₃ alkylene-(C₃-C₁₀ cycloalkyl), -C₁-C₃ alkylene-(4- to 10-membered heterocyclyl), -C₁-C₃ alkylene-(C₆-C₁₀ aryl), and -C₁-C₃ alkylene-(5- to 10-membered heteroaryl), wherein the substitution refers to substitution with one or more R; and
L₂ and L₃ are both absent;
when X is a bond, n is selected from: integers of 0, 1, 2, 3, 4, 5, and 6; when X is not a bond, n is selected from: integers of 1, 2, 3, 4, 5, and 6;
or
2) Z is selected from: 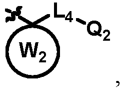 with the proviso that when 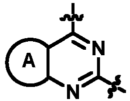 is 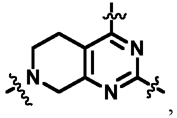 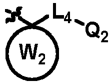 is not 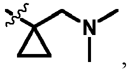
wherein ring W₂ is selected from the group consisting of the following substituted or unsubstituted groups: C₃-C₆ cycloalkylene and saturated or unsaturated 4- to 6-membered heterocyclylene, wherein the substitution refers to substitution with one or more R;
L₄ is selected from the group consisting of the following substituted or unsubstituted groups: a bond, C₁-C₆ alkylene, and deuterated C₁-C₆ alkylene, wherein the substitution refers to substitution with one or more R;
Q₂ is selected from the group consisting of the following substituted or unsubstituted groups: C₁-C₆ alkoxy, C₃-C₁₀ cycloalkyloxy, 4- to 10-membered heterocyclyloxy, C₃-C₁₀ cycloalkyl C₁-C₆ alkyleneoxy, 4- to 10-membered heterocyclyl C₁-C₆ alkyleneoxy, C₃-C₁₀ cycloalkyl, 4- to 10-membered heterocyclyl, NHR⁹, and NR⁹R^{10'}; R⁹ and R¹⁰ are each independently selected from the group consisting of the following substituted or unsubstituted groups: C₁-C₆ alkyl, C₃-C₁₀ cycloalkyl, 4- to 10-membered heterocyclyl, (C₃-C₁₀ cycloalkyl)C₁-C₆ alkyl, and (4- to 10-membered heterocyclyl)C₁-C₆ alkyl, or R⁹ and R¹⁰, together with the N atom to which they are attached, form substituted or unsubstituted 4- to 10-membered heterocyclyl, wherein the substitution refers to substitution with one or more R;
each R is identical or different and is independently selected from: H, deuterium, vinyl, ethynyl, C₁-C₆ alkyl, deuterated C₁-C₆ alkyl, halogenated C₁-C₆ alkyl, (C₃-C₆ cycloalkyl)C₁-C₆ alkyl, (4-to 6-membered heterocyclyl)C₁-C₆ alkyl, (C₁-C₆ alkoxy)C₁-C₆ alkyl, (C₃-C₆ cycloalkyloxy)C₁-C₆ alkyl, (4- to 6-membered heterocyclyloxy)C₁-C₆ alkyl, (C₁-C₆ alkyl)vinyl, deuterated (C₁-C₆ alkyl)vinyl, halogenated (C₁-C₆ alkyl)vinyl, (C₁-C₆ alkyl)ethynyl, deuterated (C₁-C₆ alkyl)ethynyl, halogenated (C₁-C₆ alkyl)ethynyl, (C₃-C₆ cycloalkyl)ethynyl, (4- to 6-membered heterocyclyl)ethynyl, C₁-C₆ alkoxy, deuterated C₁-C₆ alkoxy, halogenated C₁-C₆ alkoxy, C₃-C₆ cycloalkyloxy, 4- to 6-membered heterocyclyloxy, C₃-C₆ cycloalkyl, 4- to 6-membered heterocyclyl, C₆-C₁₀ aryl, 5- to 10-membered heteroaryl, halogen, nitro, hydroxy, oxo (=O), cyano, ester group, amino, amido, sulfonyl, and ureido.

2. The compound, or the stereoisomer, the tautomer, the crystalline form, the pharmaceutically acceptable salt, the hydrate, the solvate or the prodrug thereof according to claim 1, having a structure represented by formula (II-A) or formula (II-B): 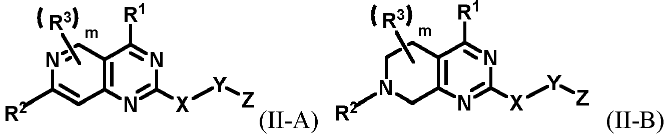 wherein in the formula,
R¹, R², R³, X, Y, Z, and m are as defined in claim 1.

3. The compound, or the stereoisomer, the tautomer, the crystalline form, the pharmaceutically acceptable salt, the hydrate, the solvate or the prodrug thereof according to claim 1, having a structure represented by formula (III-A1), formula (III-A2), formula (III-B1), or formula (III-B2): 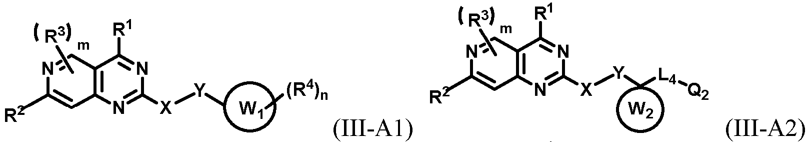 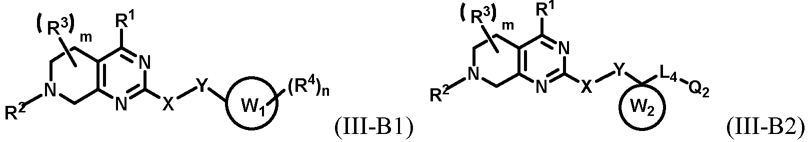 wherein in the formula,
R¹, R², R³, R⁴, X, Y, W₁, W₂, L₄, Q₂, m, and n are as defined in claim 1.

4. The compound, or the stereoisomer, the tautomer, the crystalline form, the pharmaceutically acceptable salt, the hydrate, the solvate or the prodrug thereof according to claim 1, having a structure represented by formula (IV-A1), formula (IV-A2), formula (IV-B1), or formula (IV-B2): 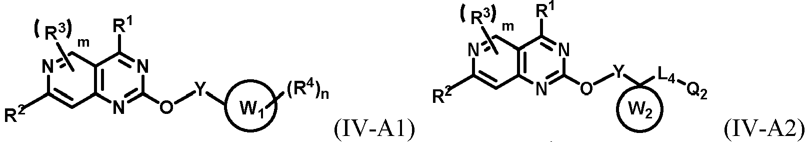 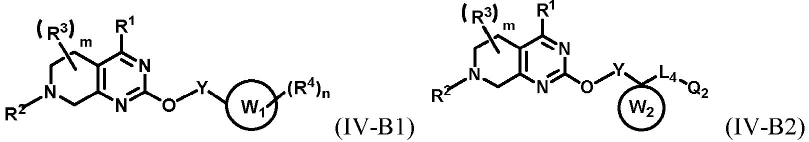 wherein in the formula,
R¹, R², R³, Y, W₁, W₂, L₄, Q₂, m, and n are as defined in claim 1.

5. The compound, or the stereoisomer, the tautomer, the crystalline form, the pharmaceutically acceptable salt, the hydrate, the solvate or the prodrug thereof according to any one of claims 1-4, wherein W₁ is substituted or unsubstituted 7- to 10-membered bicyclic heterocyclyl, substituted or unsubstituted C₇-C₁₀ bicyclic cycloalkyl, substituted or unsubstituted 7- to 12-membered tricyclic heterocyclyl, or substituted or unsubstituted C₇-C₁₂ tricyclic cycloalkyl, and preferably, W₁ is substituted or unsubstituted 8- to 12-membered N-containing heterocyclyl, wherein the substitution refers to substitution with one or more R, and R is as defined above; preferably, 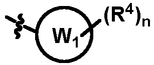 is selected from: 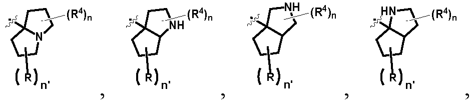 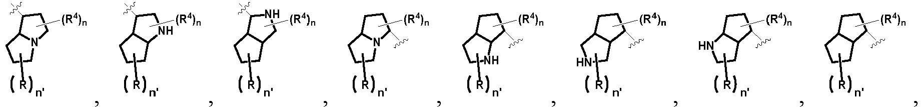 and 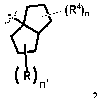 or is selected from: 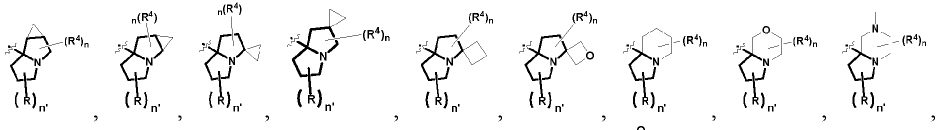 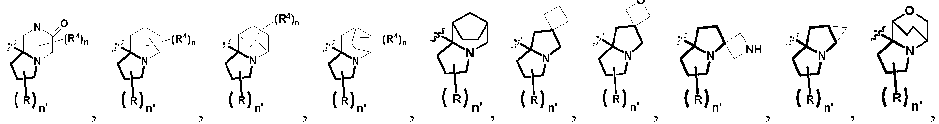 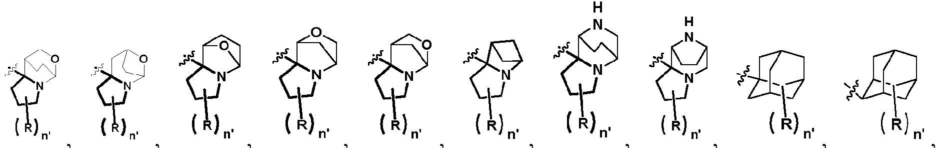 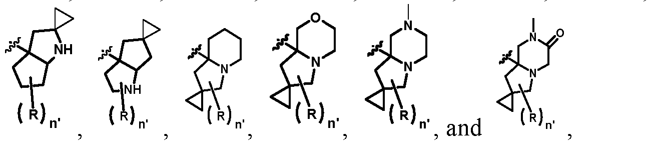
wherein n' is an integer of 0, 1, 2, 3, 4, 5, or 6; R, R⁴, and n are as defined in claim 1, and substitution with R or R⁴ can occur on any one of the rings of the polycyclic group (e.g., bridged or spiro cyclic group);
more preferably, 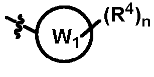
is selected from: 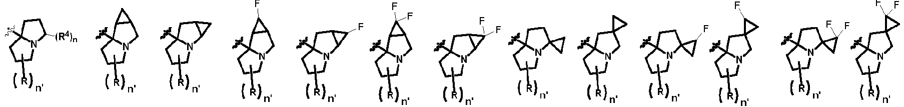 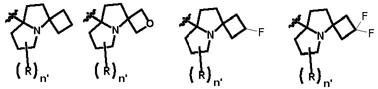 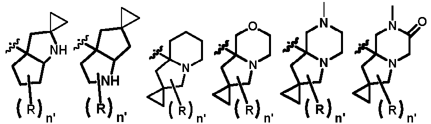
wherein n' is an integer of 0, 1, 2, 3, 4, 5, or 6; R⁴ and R are as defined in claim 1, and substitution with R can occur on any one of the rings of the polycyclic group (e.g., bridged or spiro cyclic group).

6. The compound, or the stereoisomer, the tautomer, the crystalline form, the pharmaceutically acceptable salt, the hydrate, the solvate or the prodrug thereof according to any one of claims 1-4, wherein 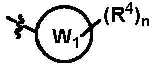 is selected from 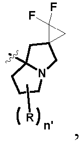 preferably 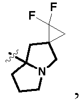 more preferably

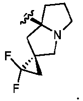

7. The compound, or the stereoisomer, the tautomer, the crystalline form, the pharmaceutically acceptable salt, the hydrate, the solvate or the prodrug thereof according to claim 4, wherein 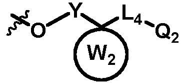 is selected from:

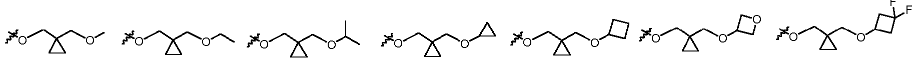

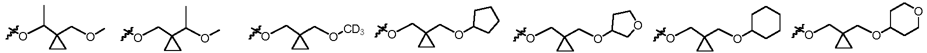

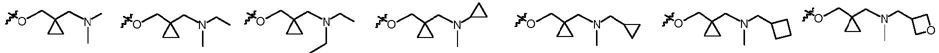

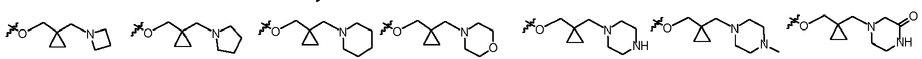

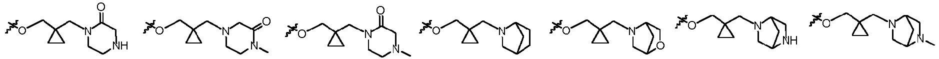

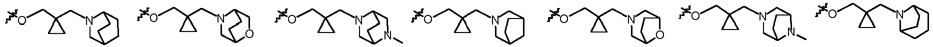

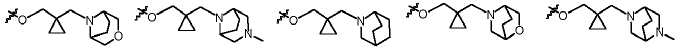

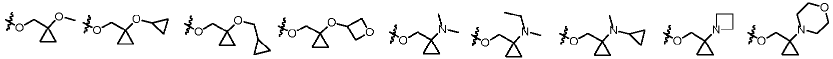

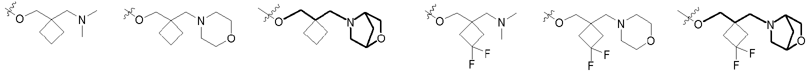

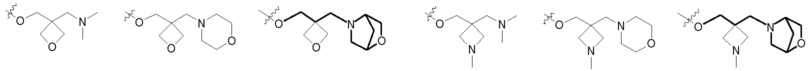

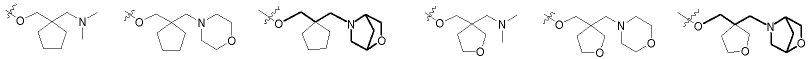

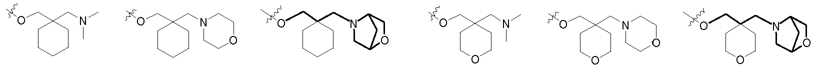

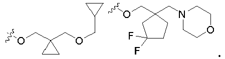

8. The compound, or the stereoisomer, the tautomer, the crystalline form, the pharmaceutically acceptable salt, the hydrate, the solvate or the prodrug thereof according to claim 1, having a structure represented by formula (V-A1) or formula (V-B1): 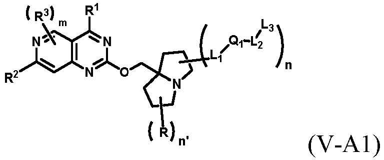 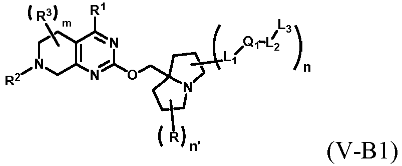 wherein in the formula,
n' is an integer of 0, 1, 2, 3, 4, 5, or 6;
R¹, R², R³, R, L₁, L₂, L₃, Q₁, m, n, and n' are as defined in claim 1;
preferably, 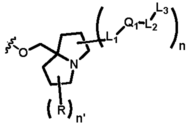 is selected from 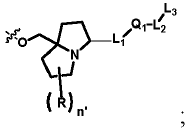
more preferably, 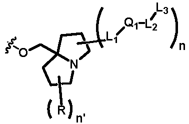 is selected from: 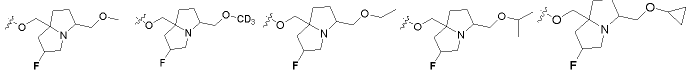 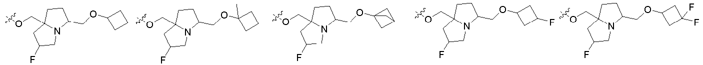 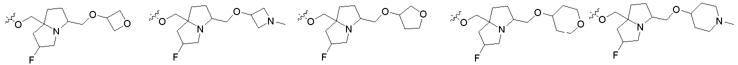 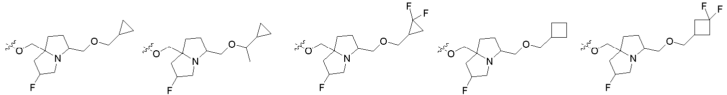 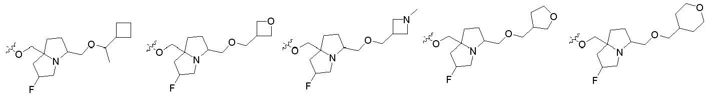 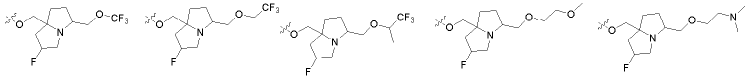 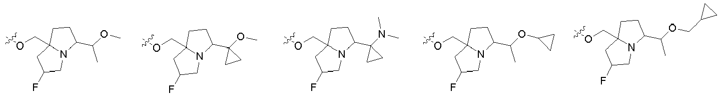 or is selected from: 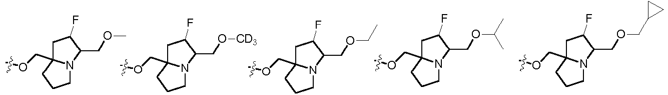 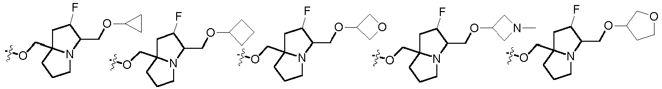 or is selected from: 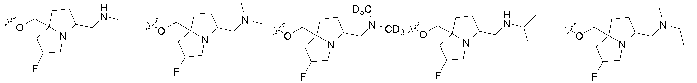 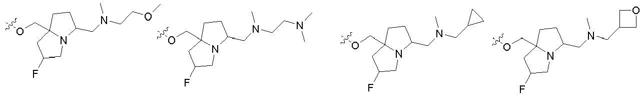 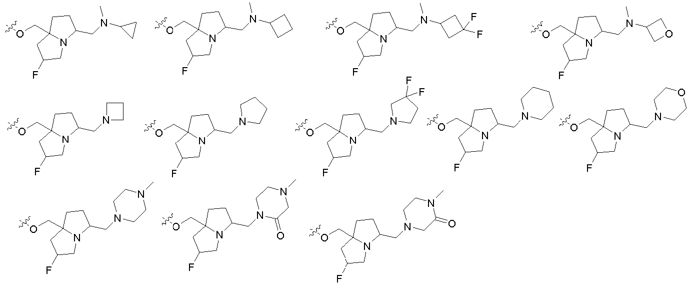 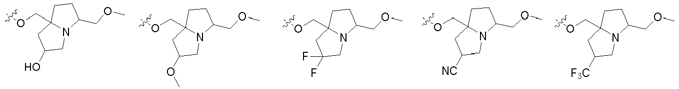 or is selected from: 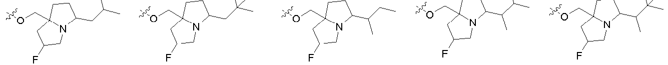 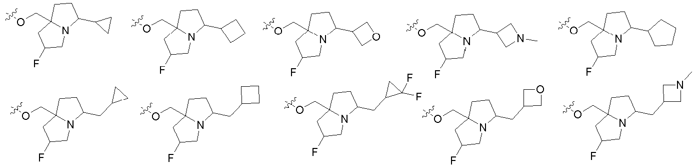 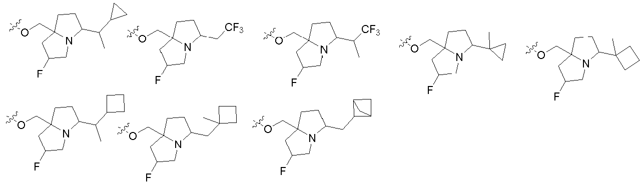 or is selected from:

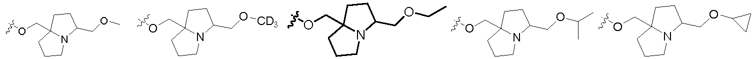

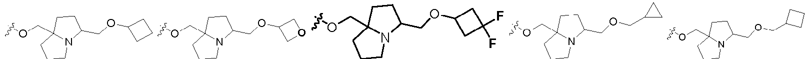

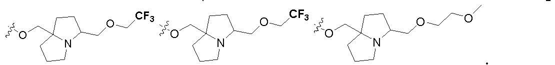

9. The compound, or the stereoisomer, the tautomer, the crystalline form, the pharmaceutically acceptable salt, the hydrate, the solvate or the prodrug thereof according to any one of claims 1-8, wherein R² is selected from the group consisting of the following substituted or unsubstituted groups: phenyl, naphthyl, 5- to 6-membered monocyclic heteroaryl (e.g., pyridyl), 9- to 10-membered bicyclic heteroaryl (e.g., indazolyl, benzothiazole, benzothiophene, and benzofuran), wherein the substitution is substitution with one or more groups selected from the group consisting of: halogen, hydroxy, cyano, NH₂, C₁-C₆ alkyl, halogenated C₁-C₆ alkyl, C₁-C₆ alkoxy, halogenated C₁-C₆ alkoxy, C₃-C₆ cycloalkyl, 4- to 6-membered heterocyclyl, C₂-C₆ alkenyl, C₂-C₆ alkynyl, ester group, amino, amido, sulfonyl, ureido, sulfonamido, C₃-C₆ cycloalkyl O-, 4- to 6-membered heterocyclyl O-, C₃-C₆ cycloalkyl OH, and 4- to 6-membered heterocyclyl OH; preferably, R² is selected from:

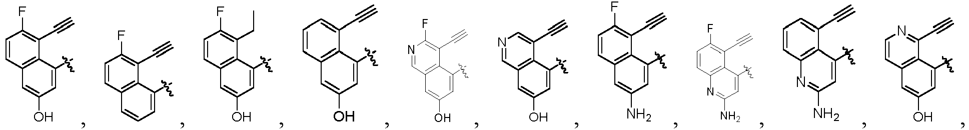

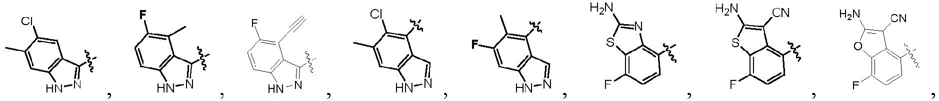

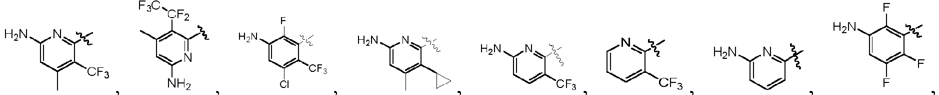

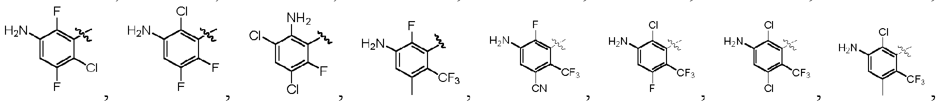

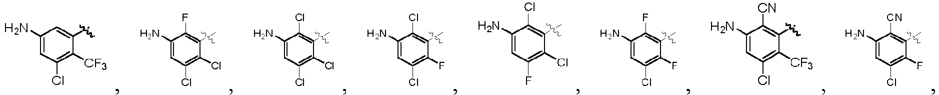

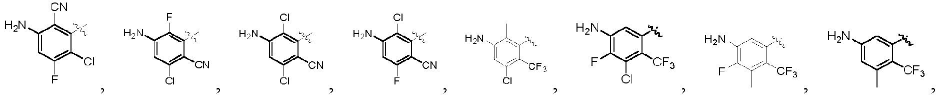

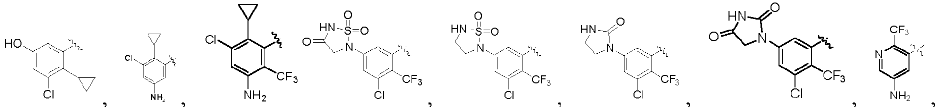

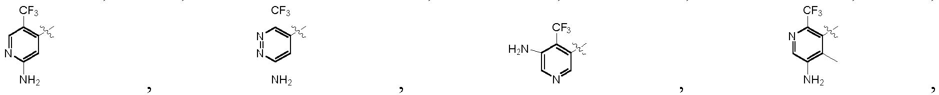

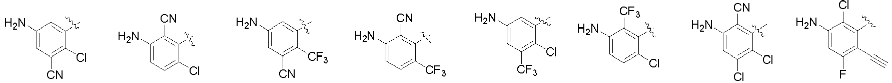

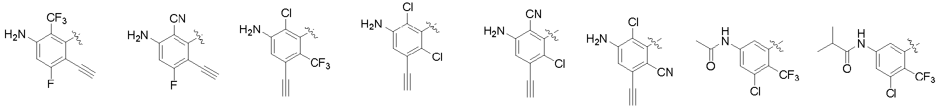

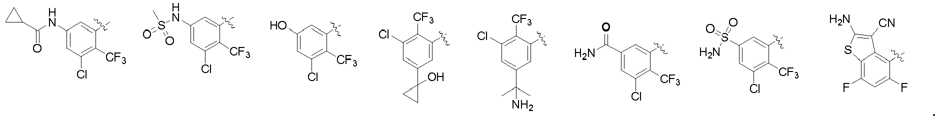

10. The compound, or the stereoisomer, the tautomer, the crystalline form, the pharmaceutically acceptable salt, the hydrate, the solvate or the prodrug thereof according to any one of claims 1-8, wherein the compound is selected from the group consisting of: 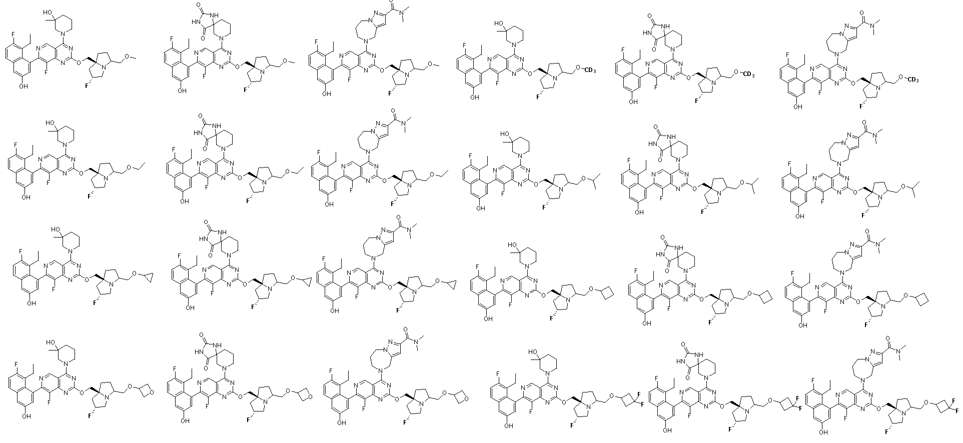 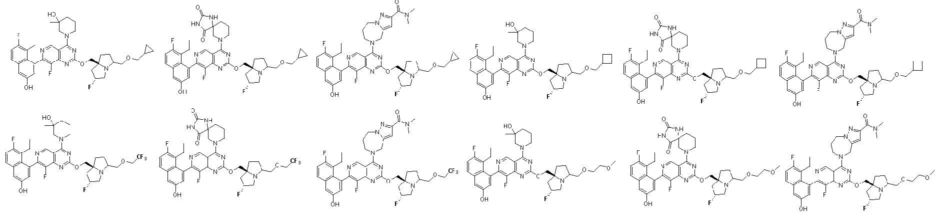 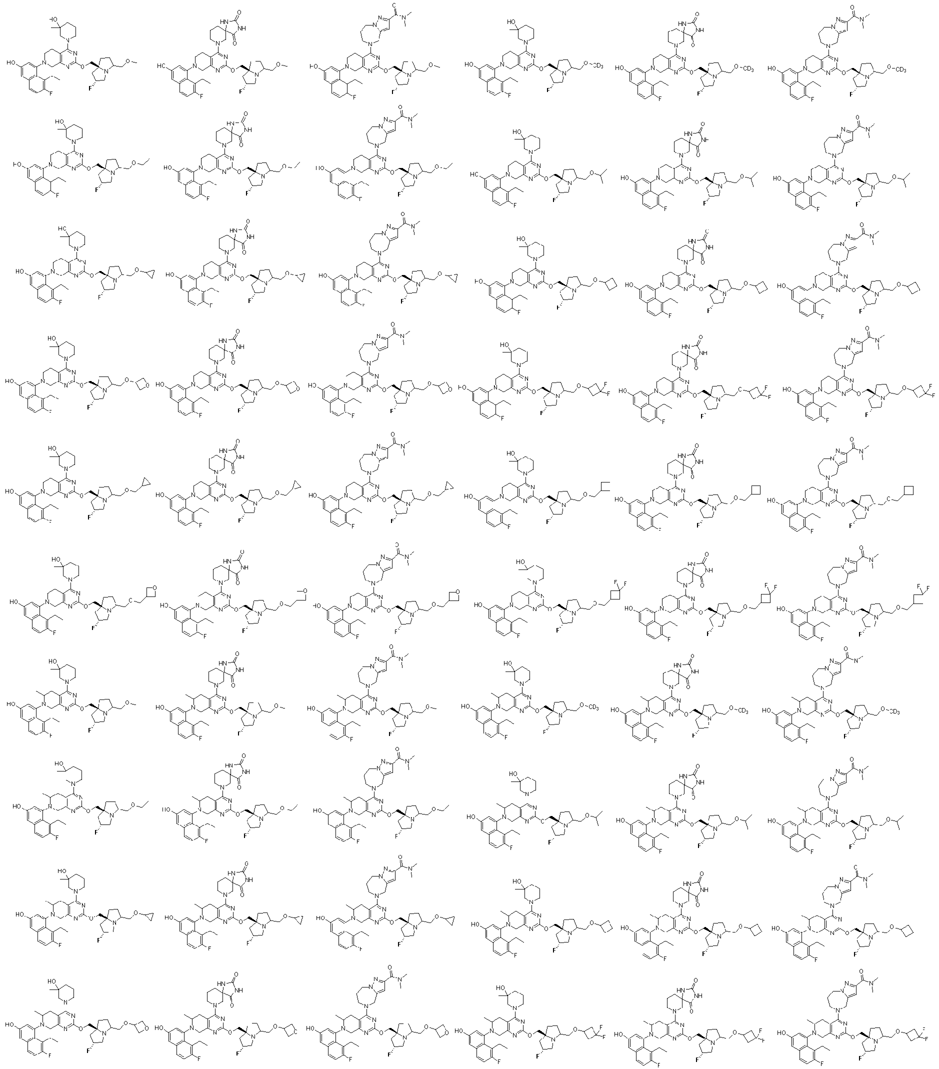 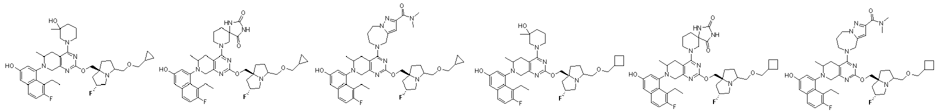 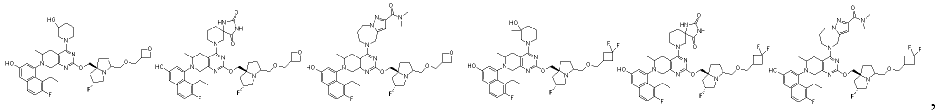 or is selected from: 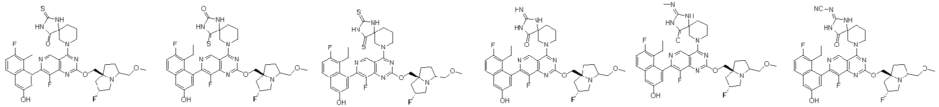 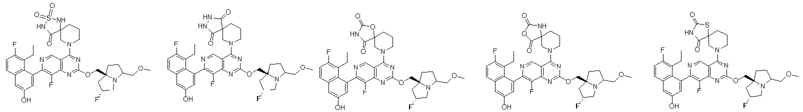 or is selected from: 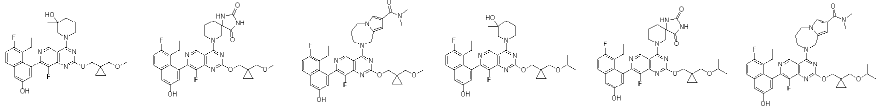 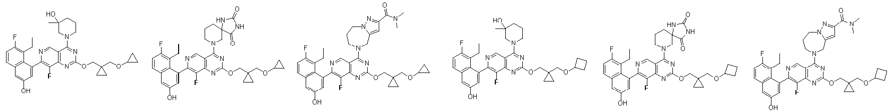 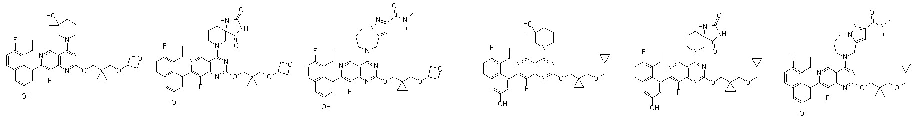 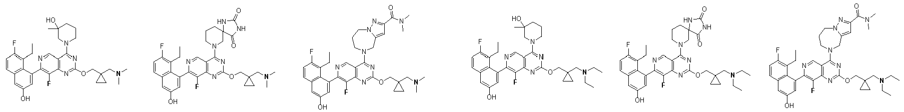 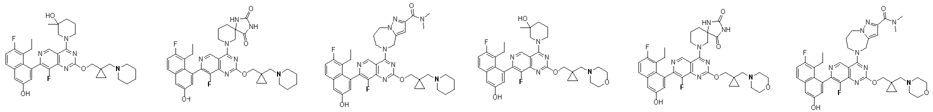 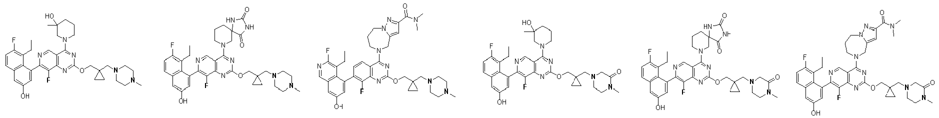 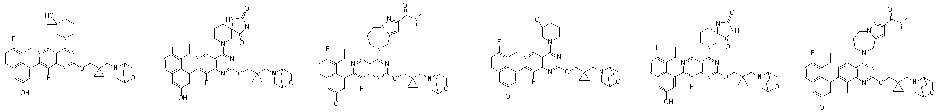 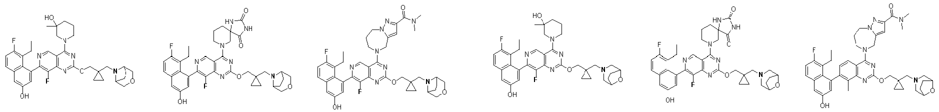 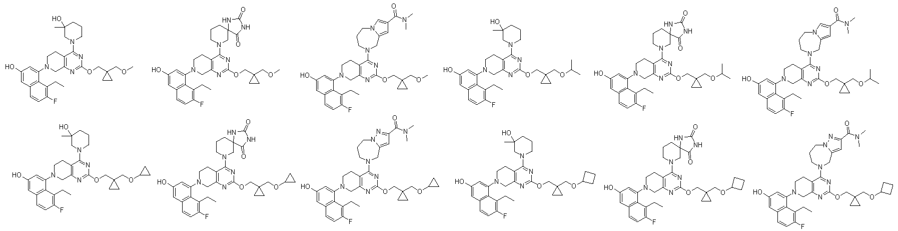 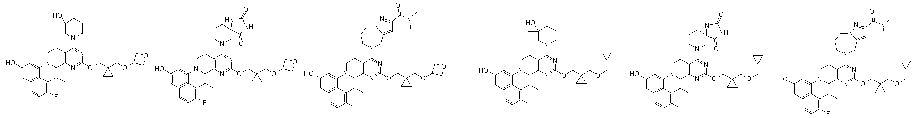 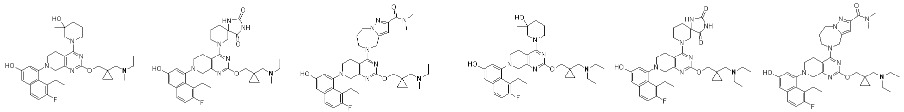 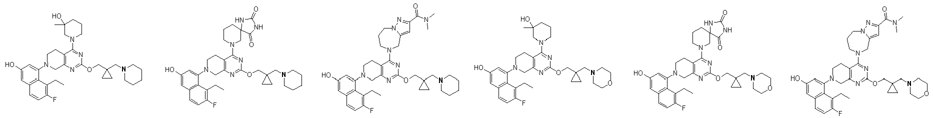 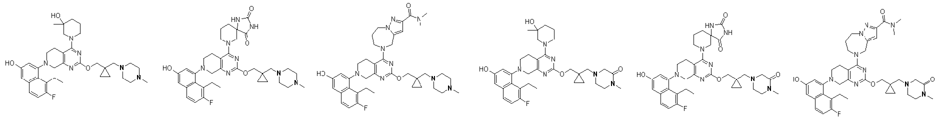 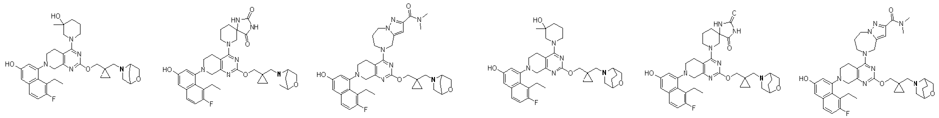 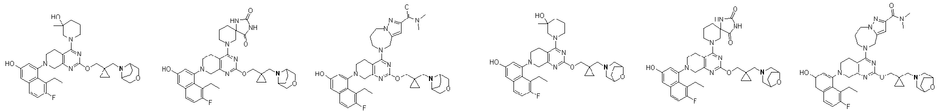 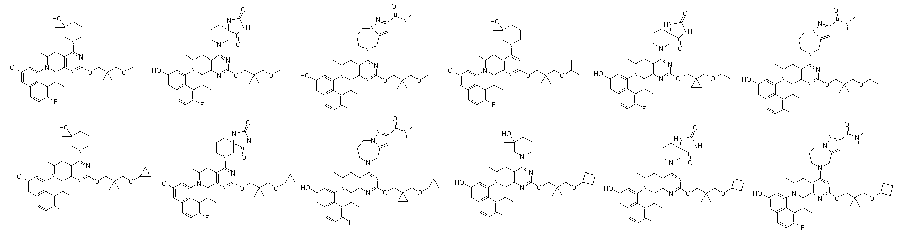 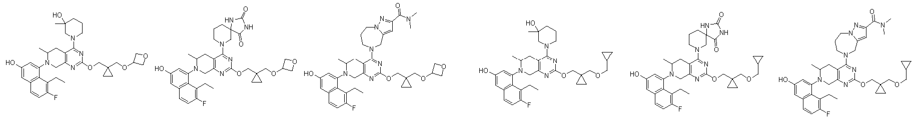 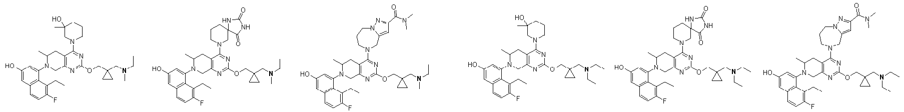 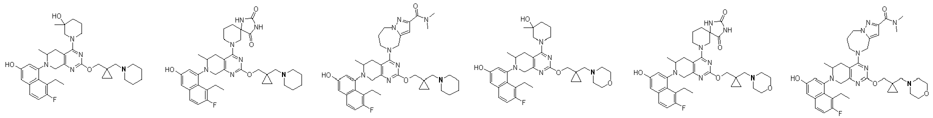 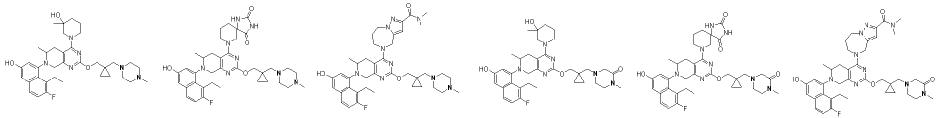 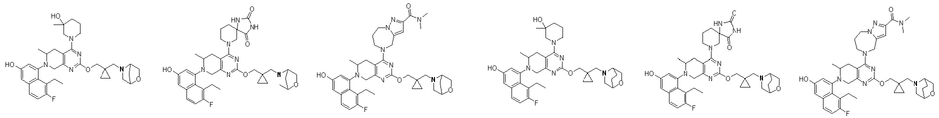 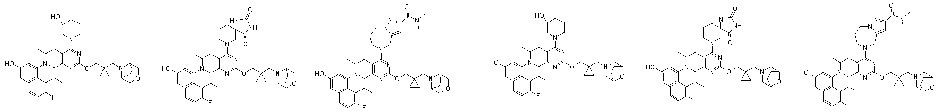 or is selected from: 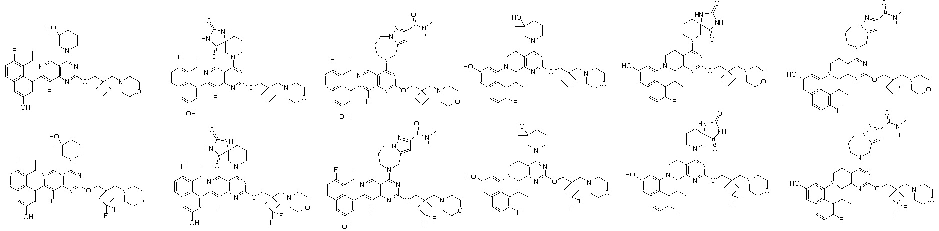 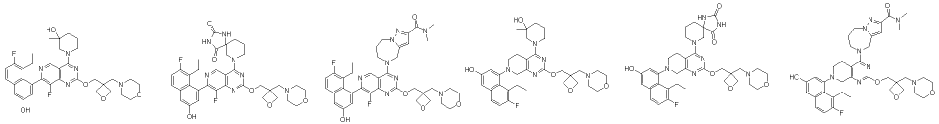 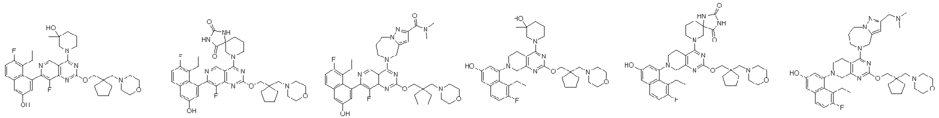 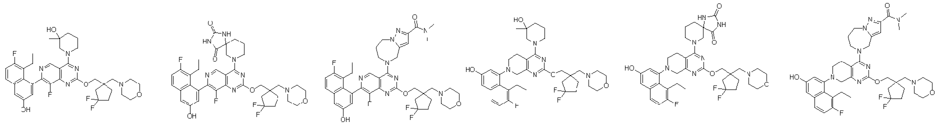 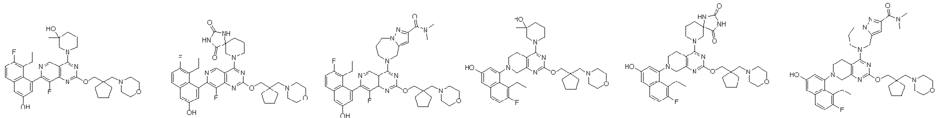 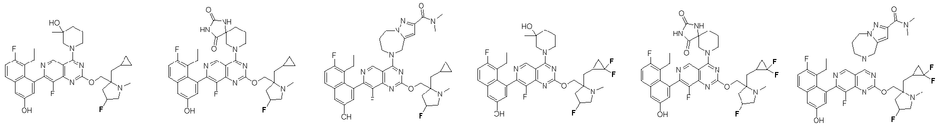 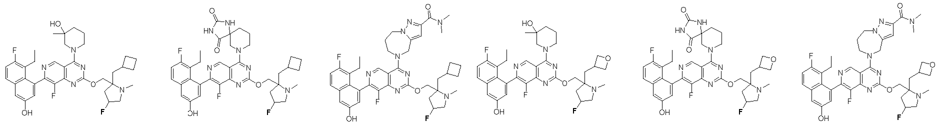 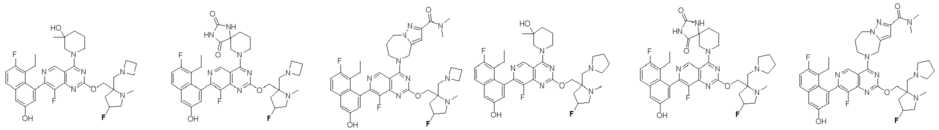 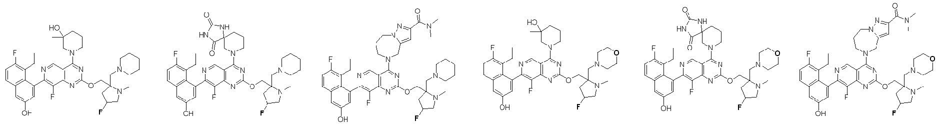 or is selected from: 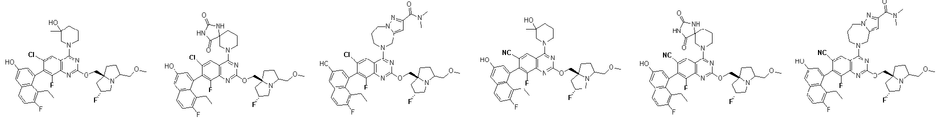 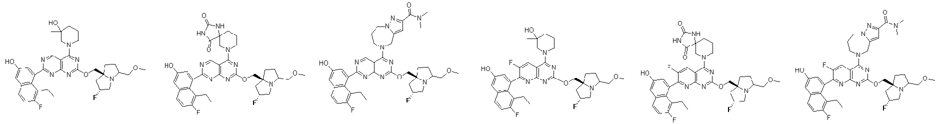 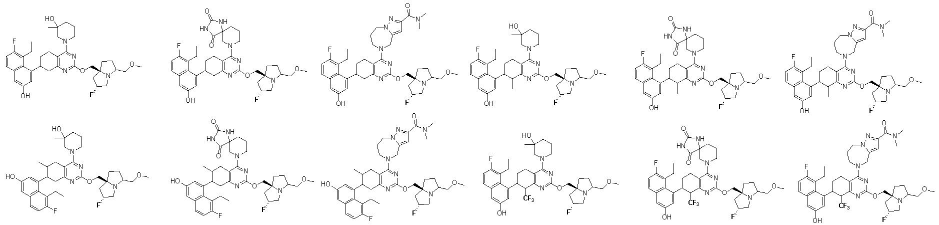 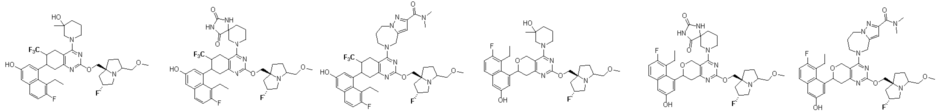 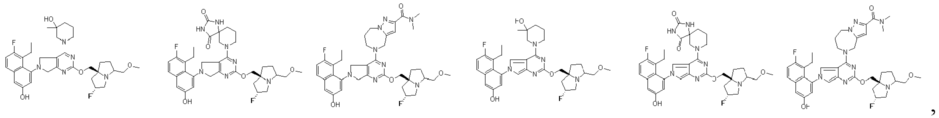 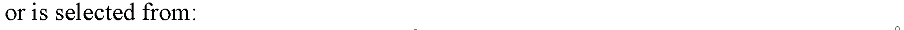 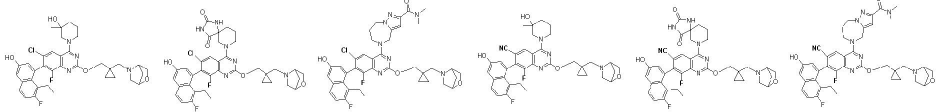 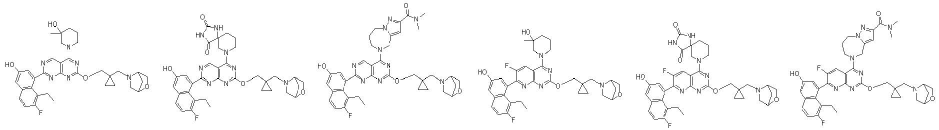 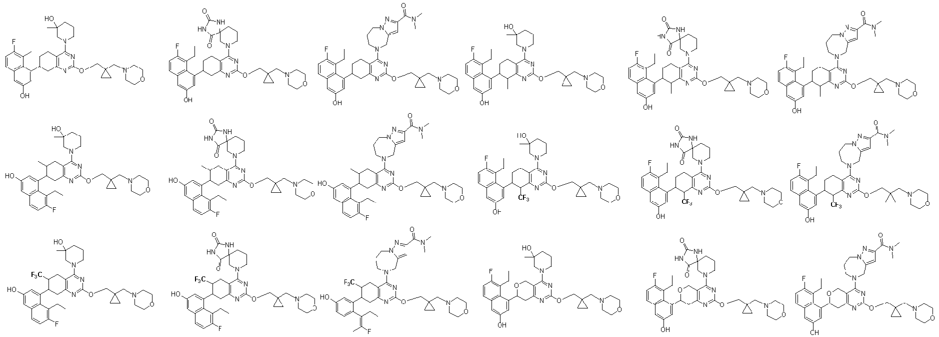 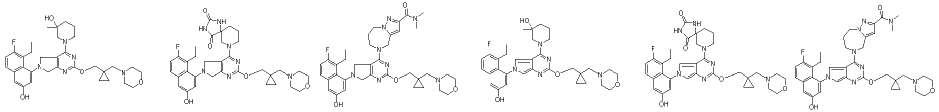 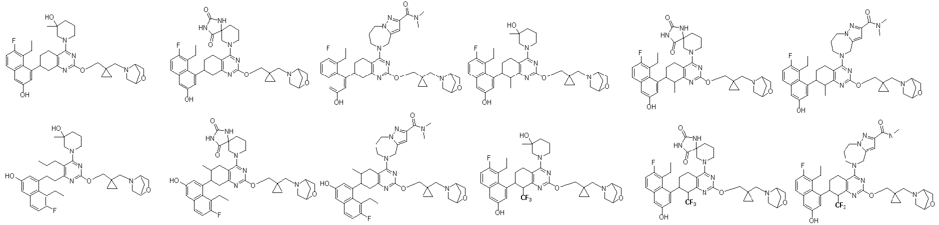 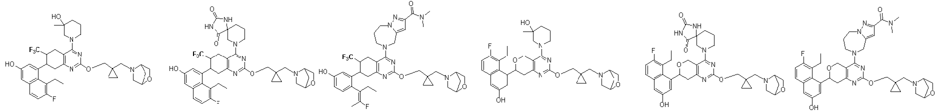 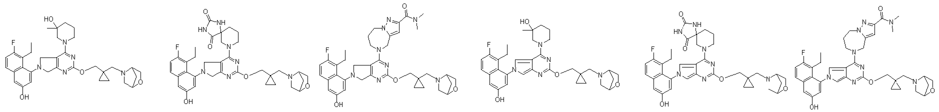 or is selected from: 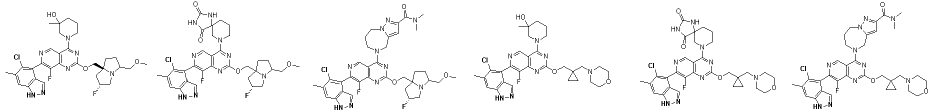 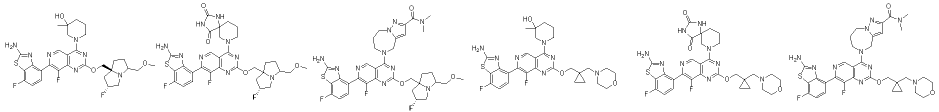 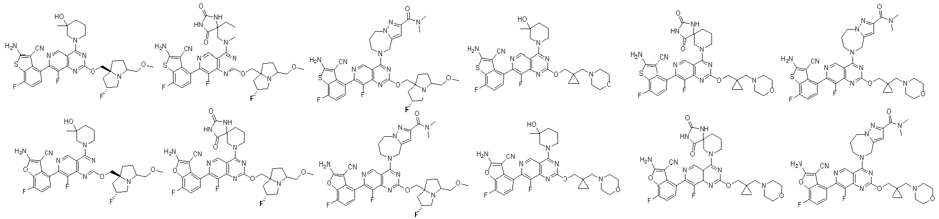 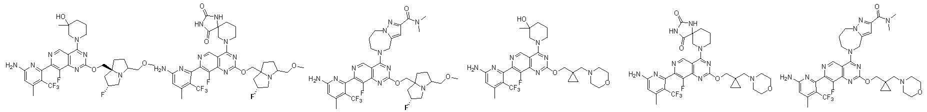 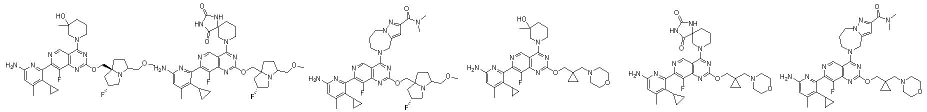 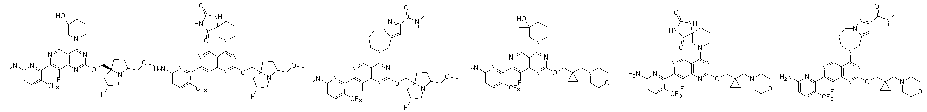 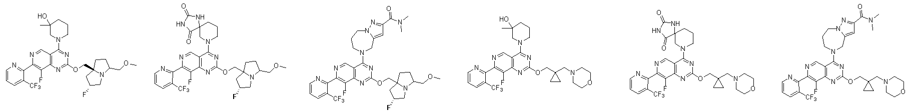 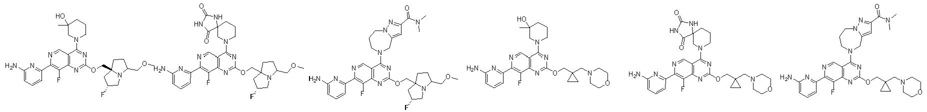 or is selected from: 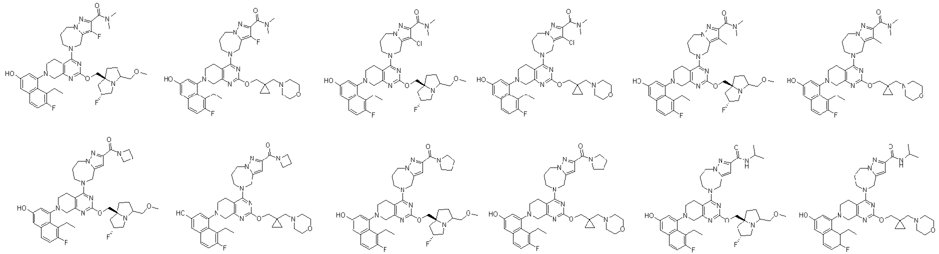 or is selected from: 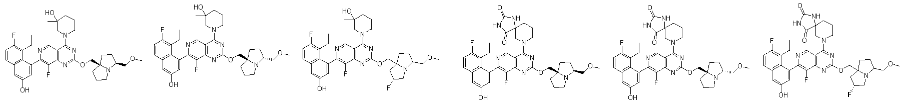 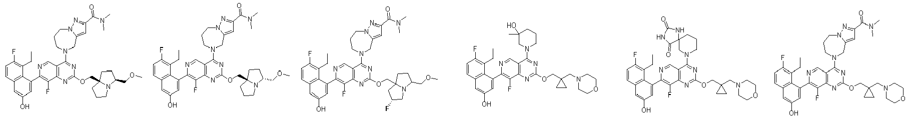 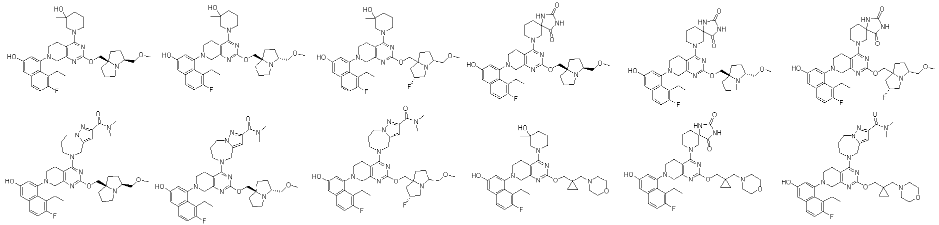 or is selected from:

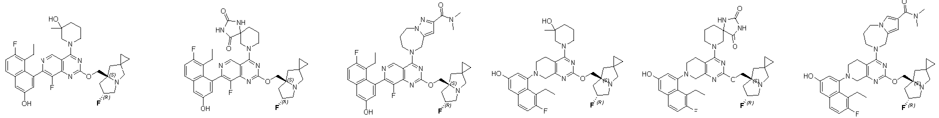

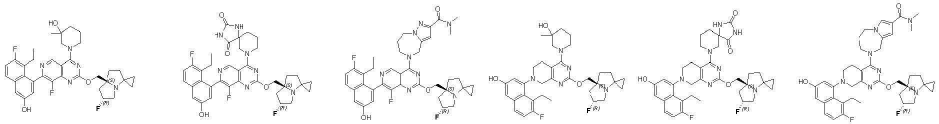

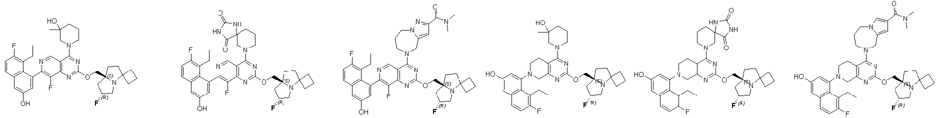

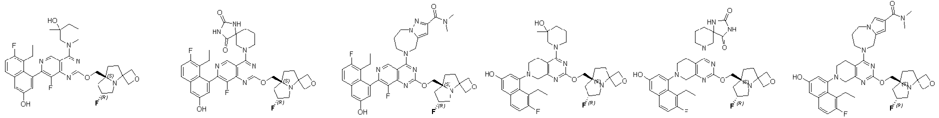

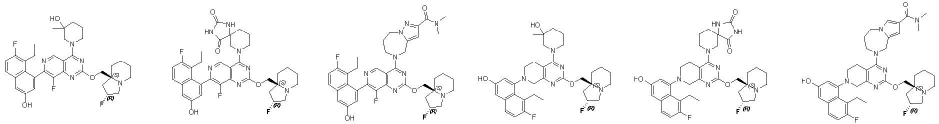

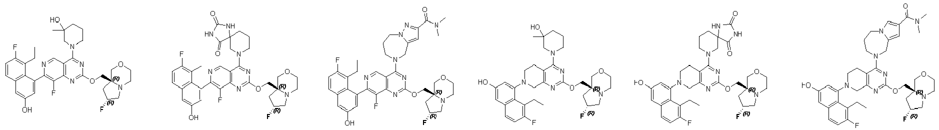

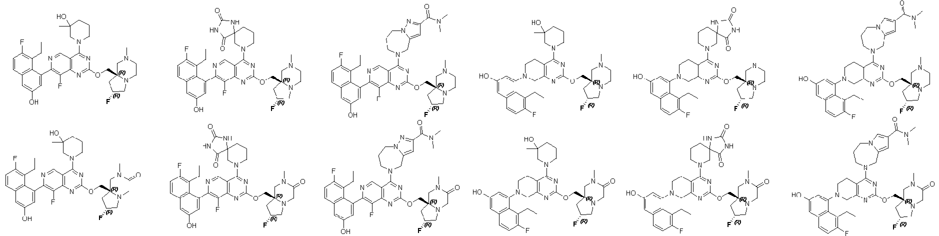

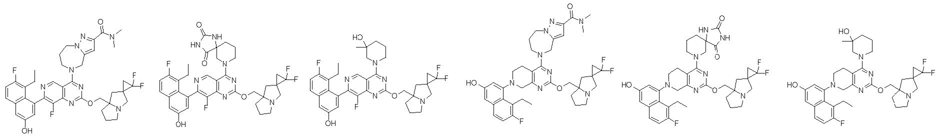

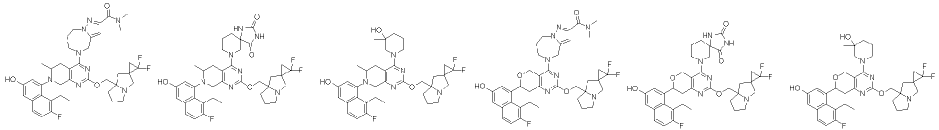

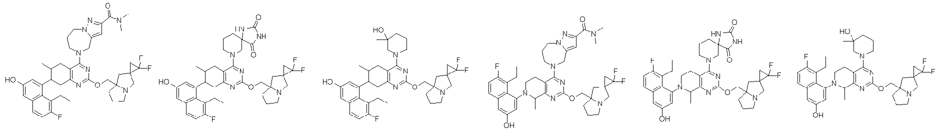

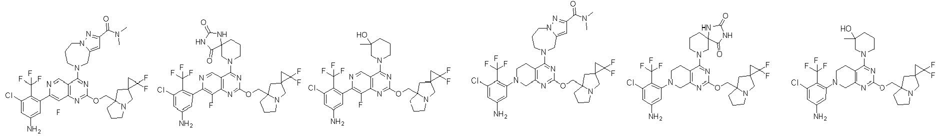

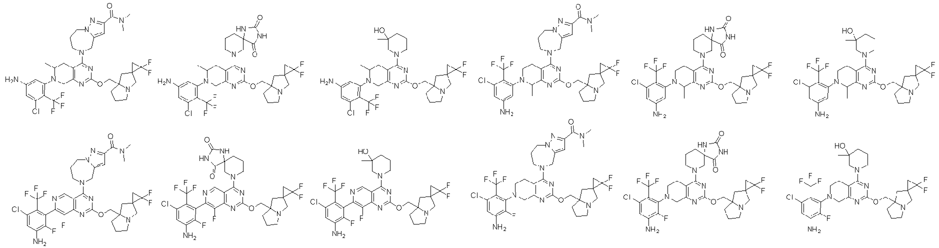

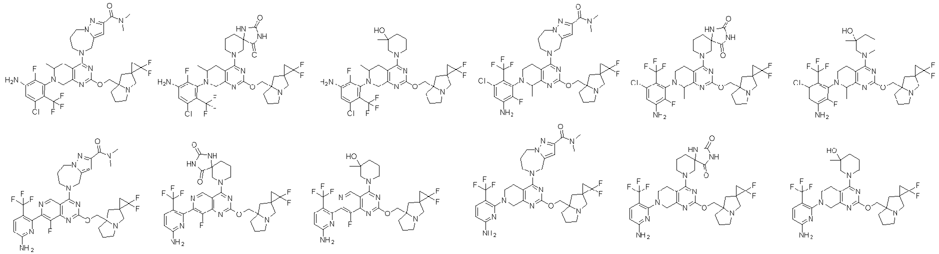

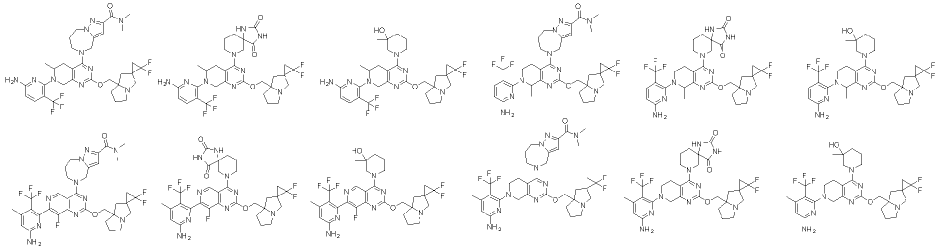

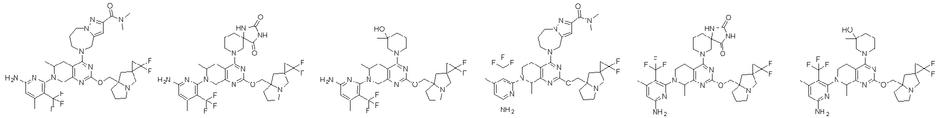

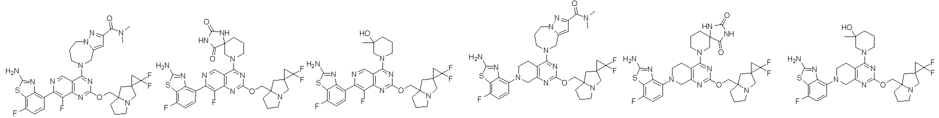

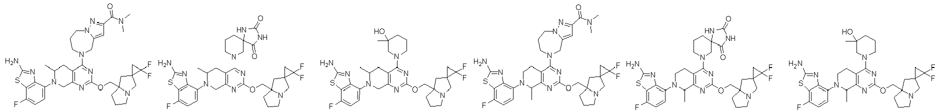

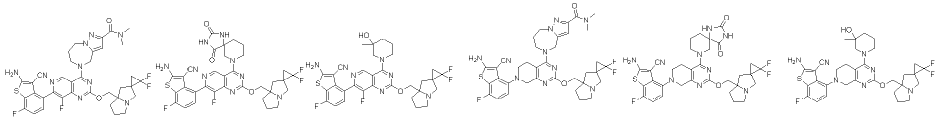

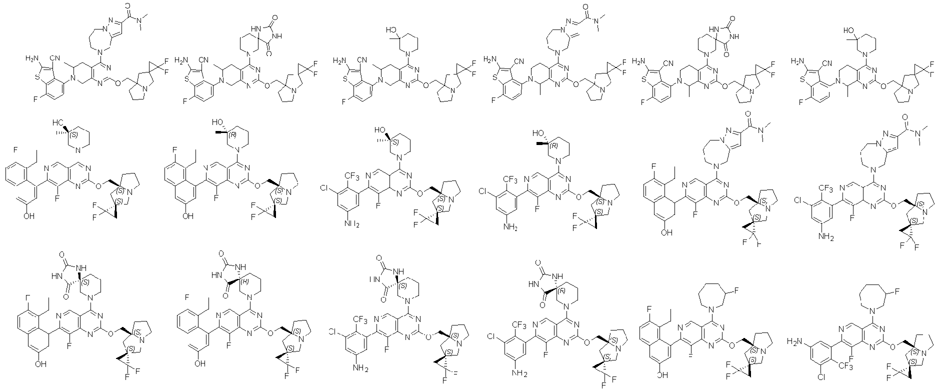

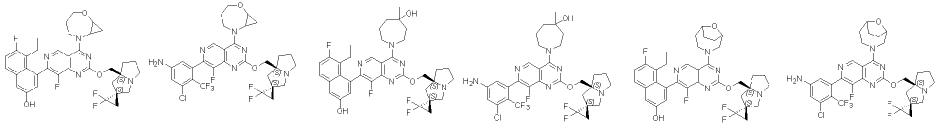

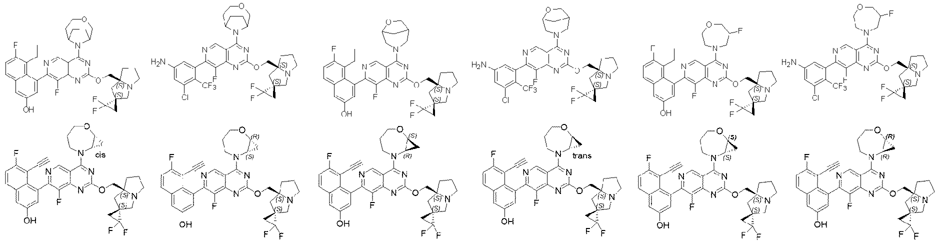

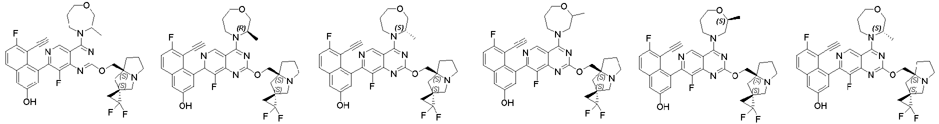

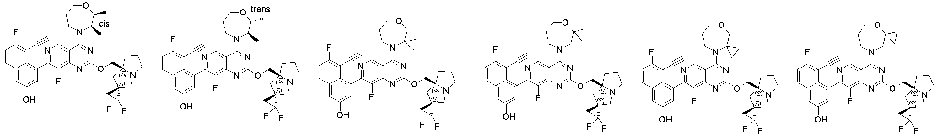

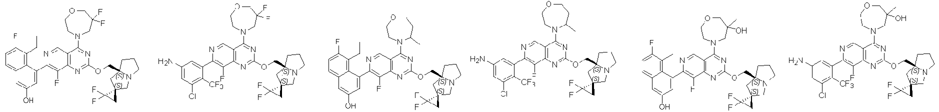

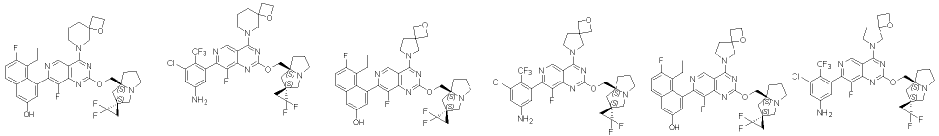

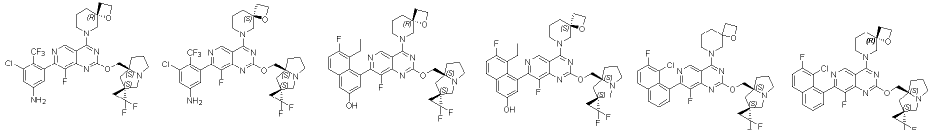

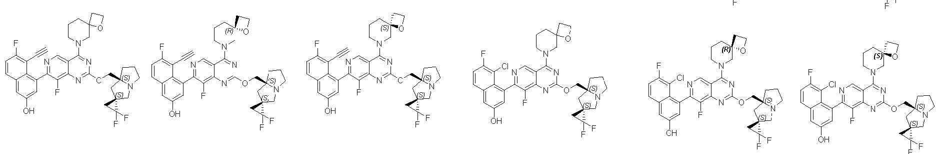

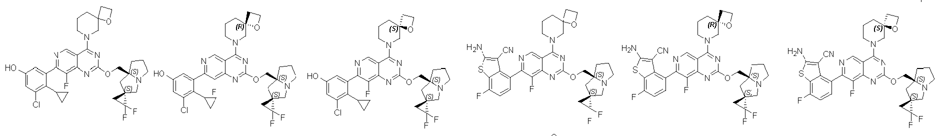

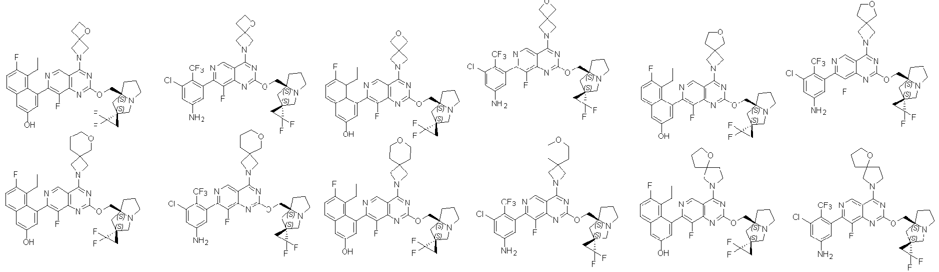

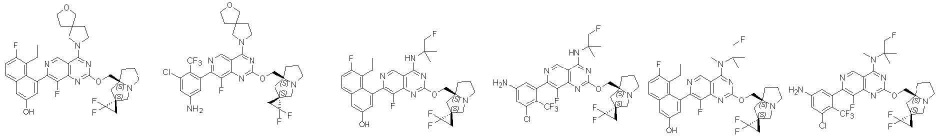

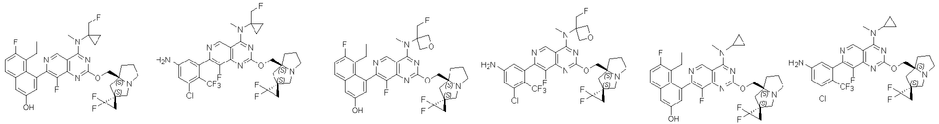

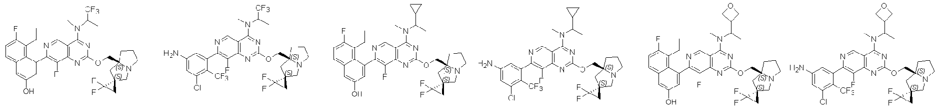

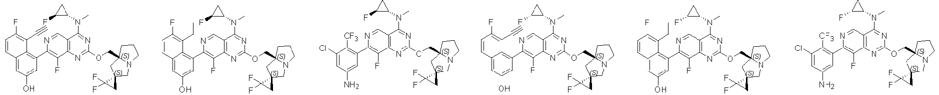

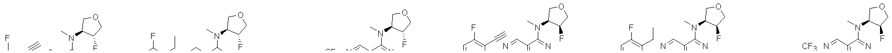

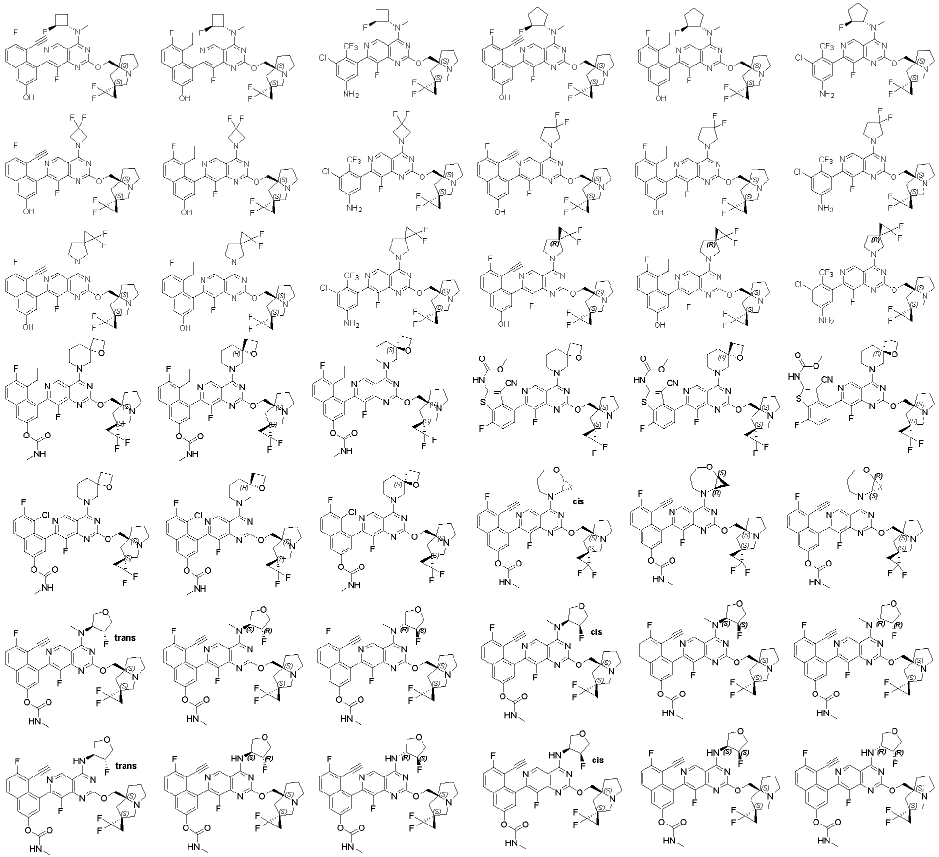

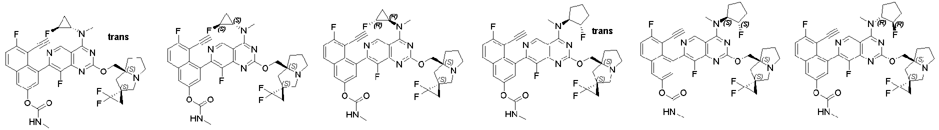

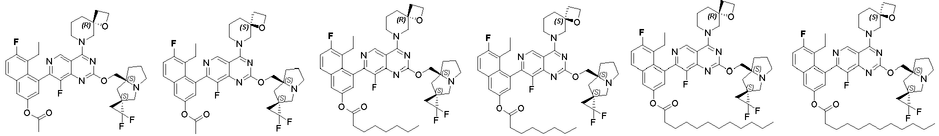

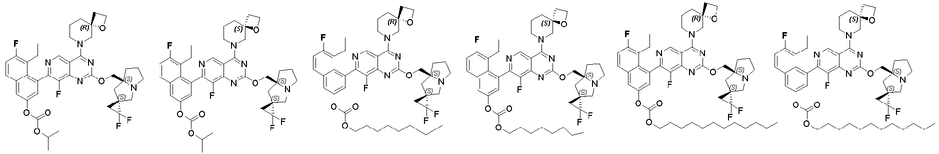

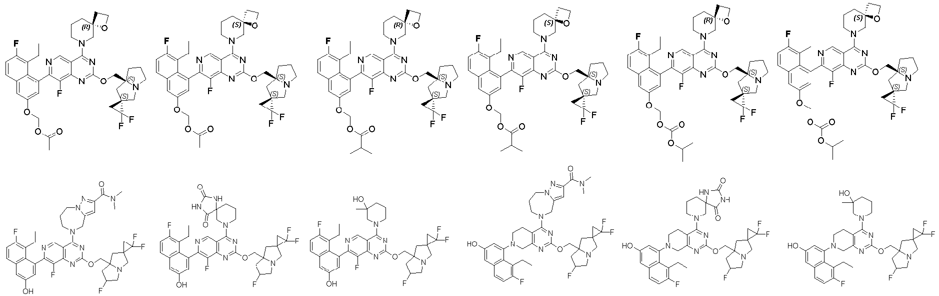

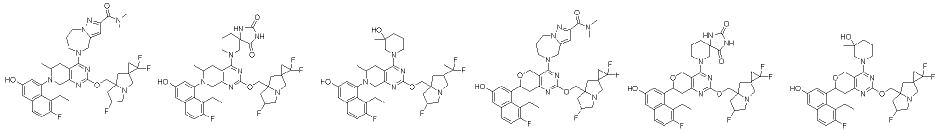

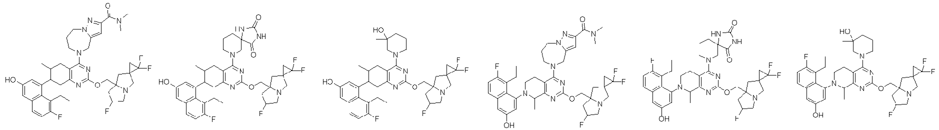

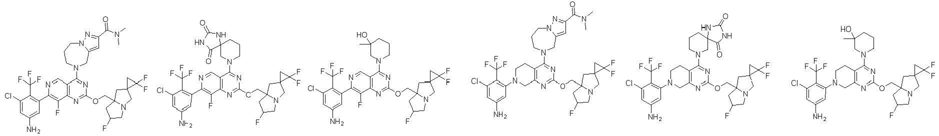

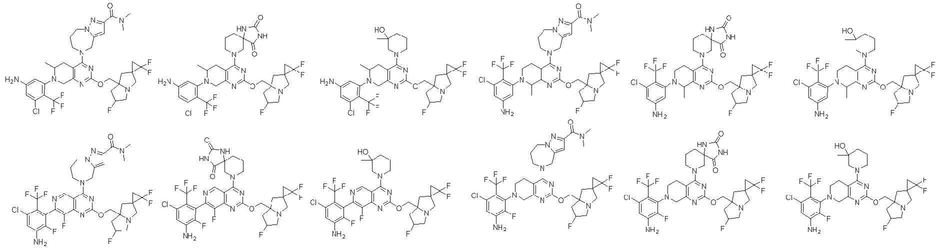

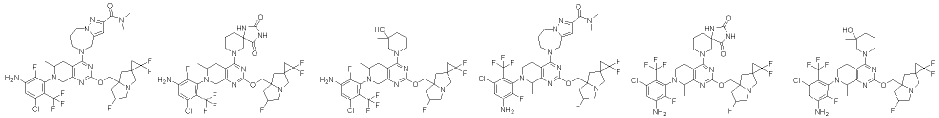

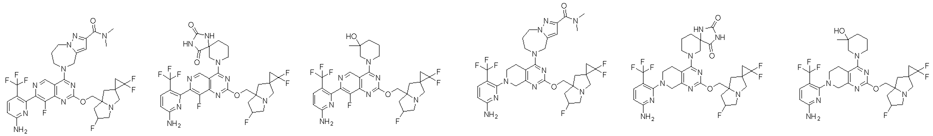

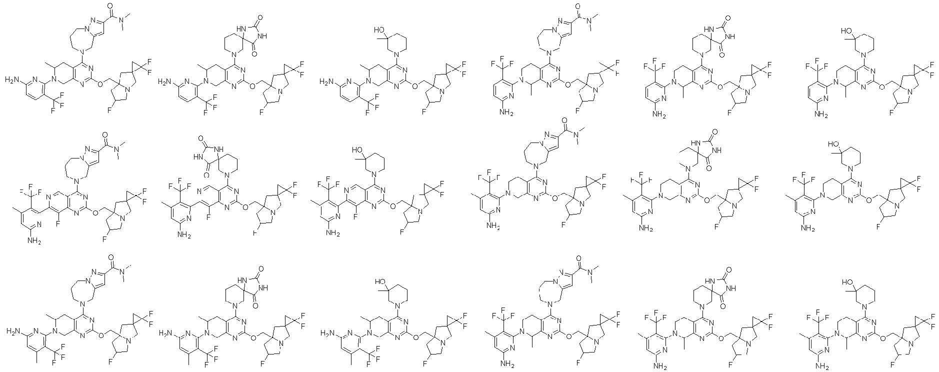

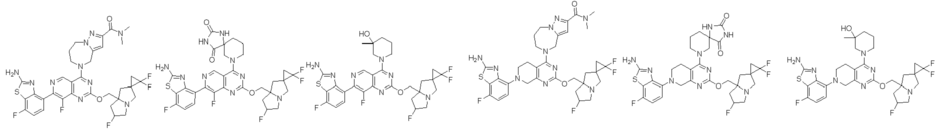

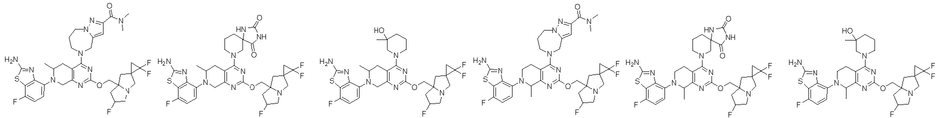

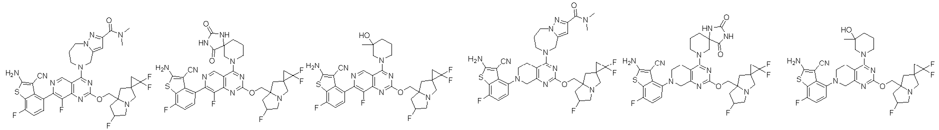

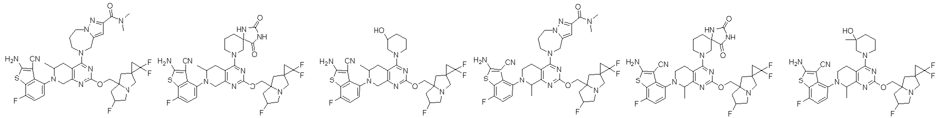

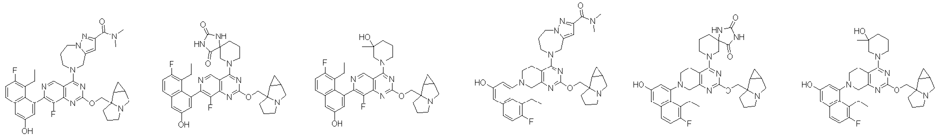

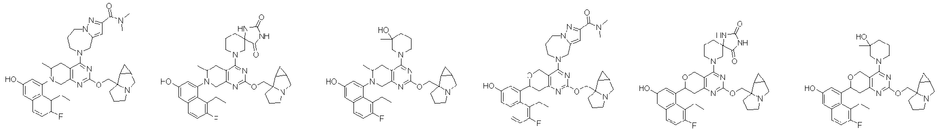

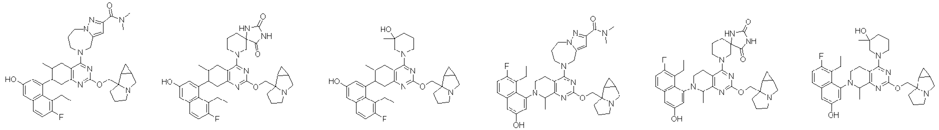

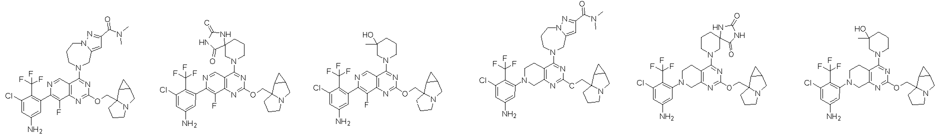

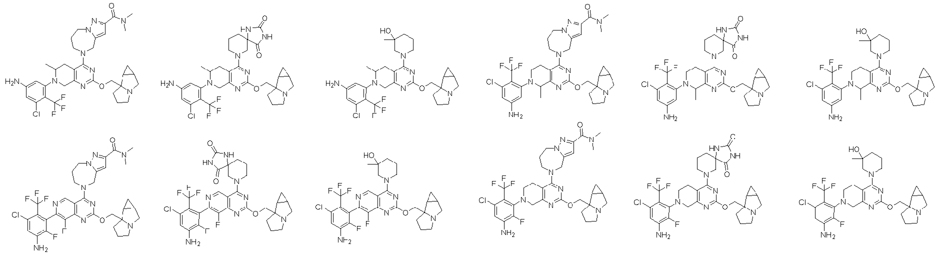

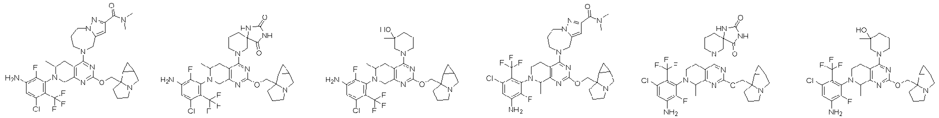

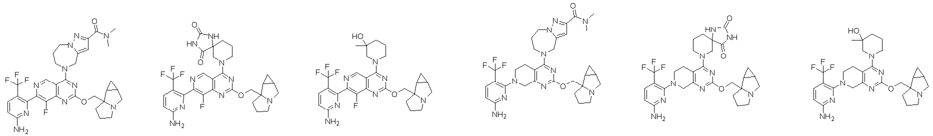

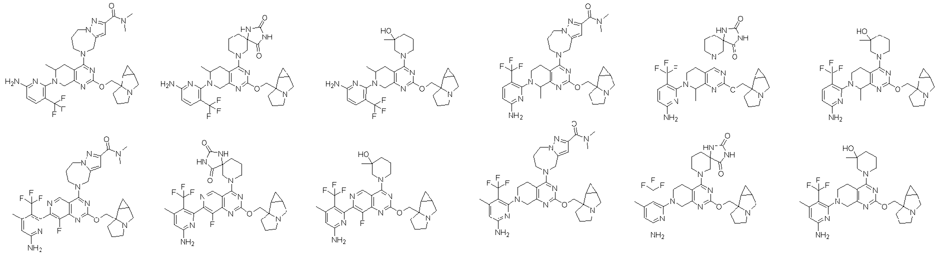

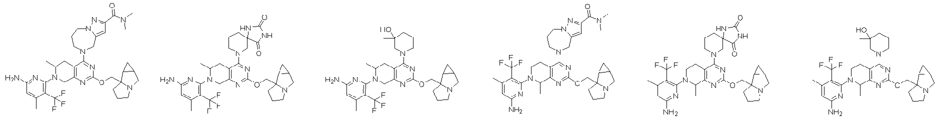

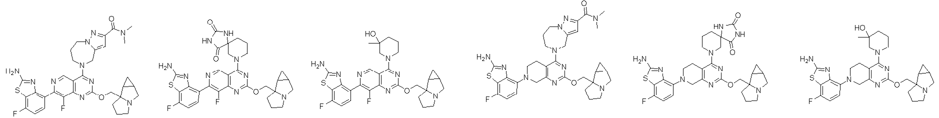

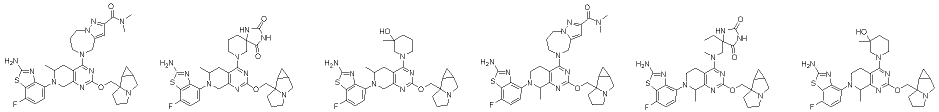

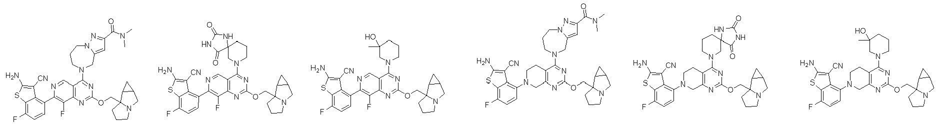

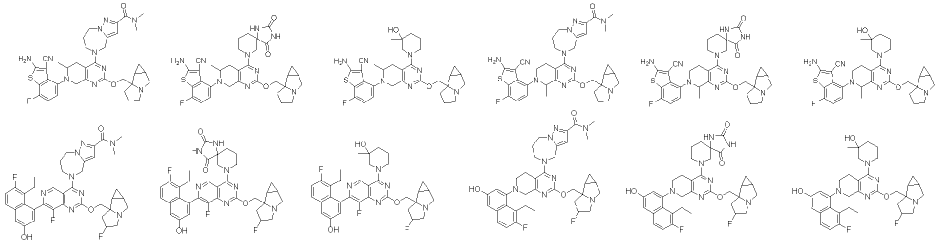

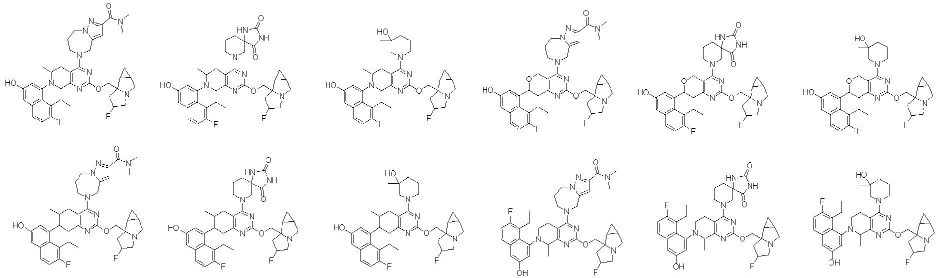

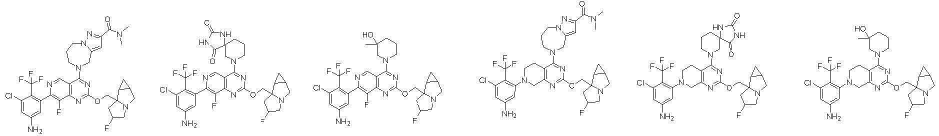

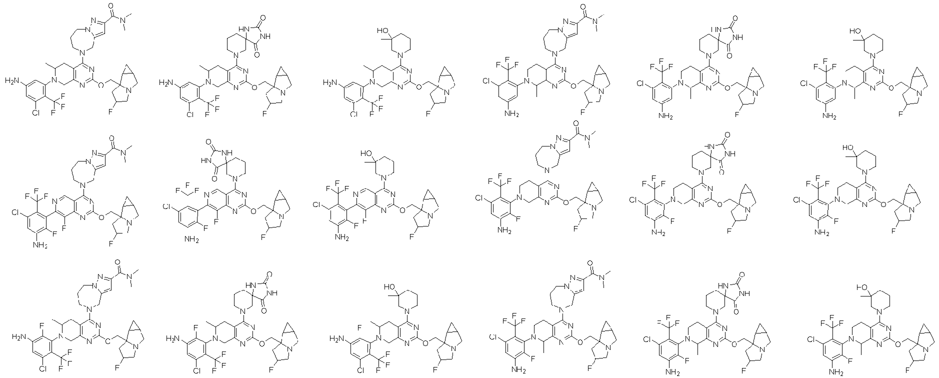

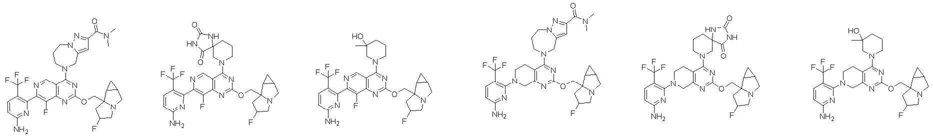

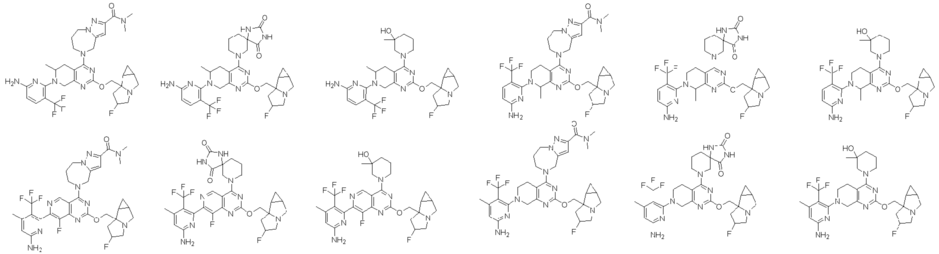

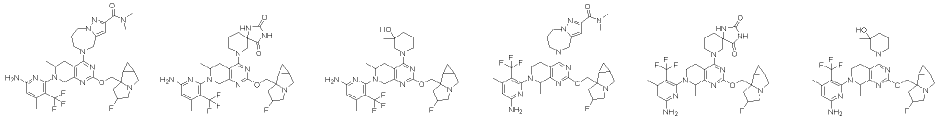

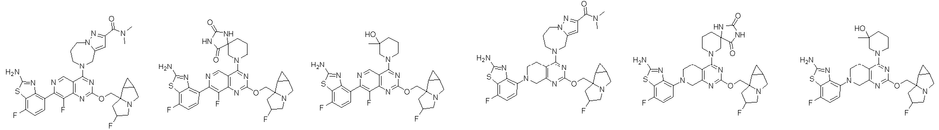

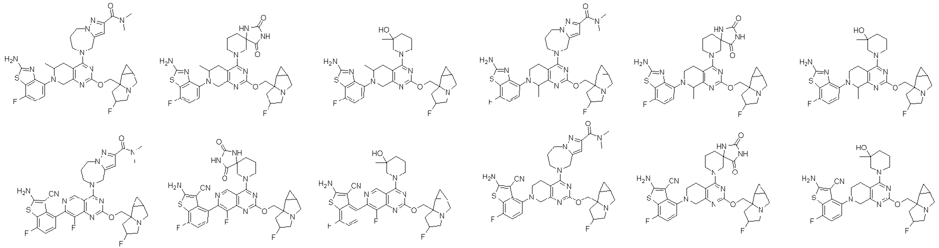

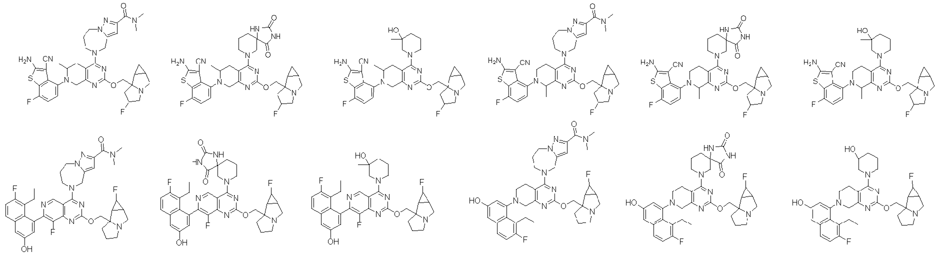

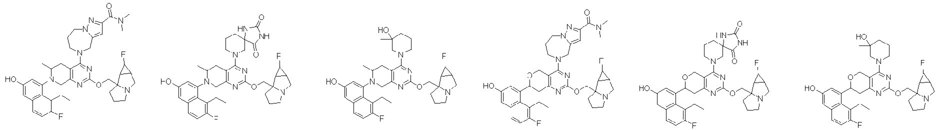

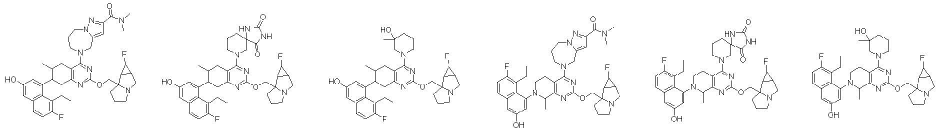

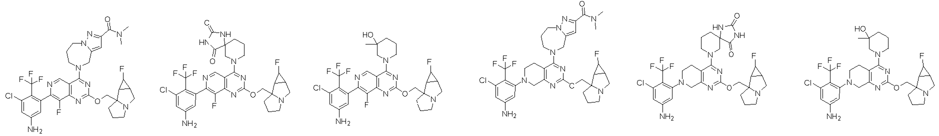

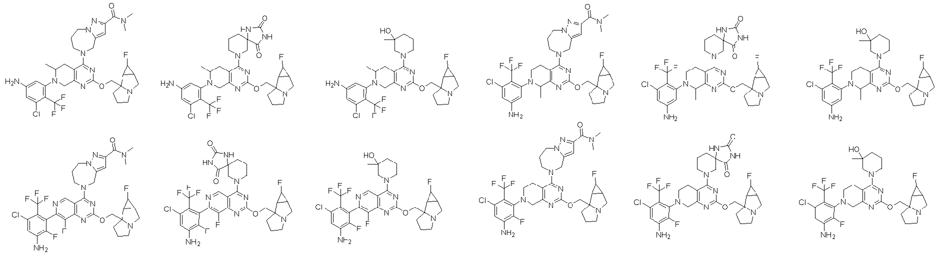

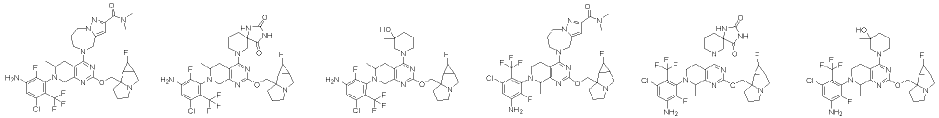

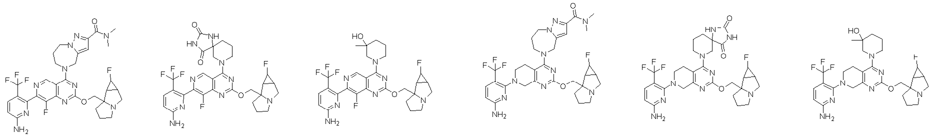

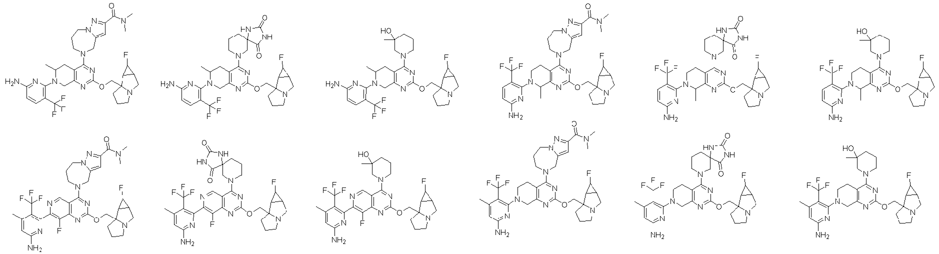

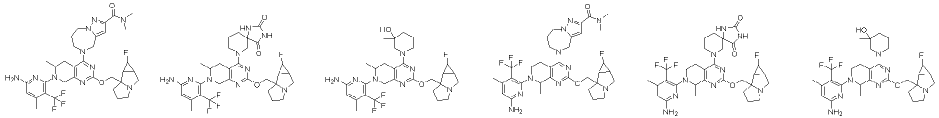

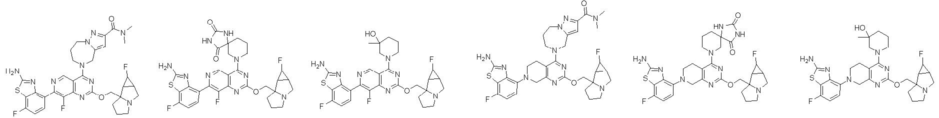

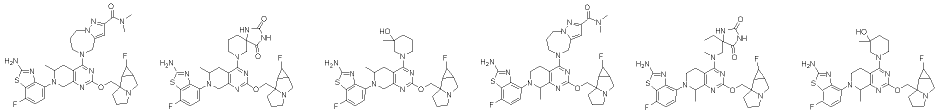

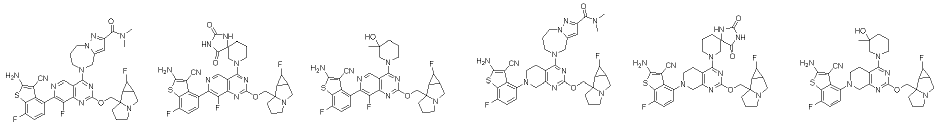

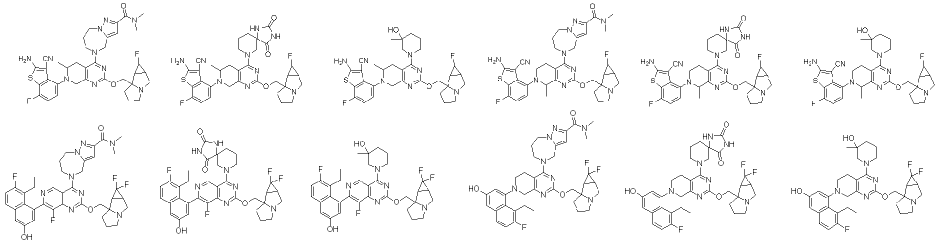

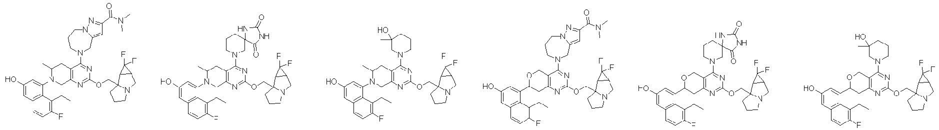

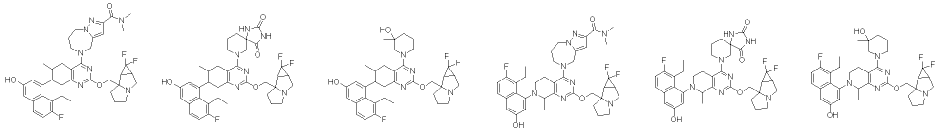

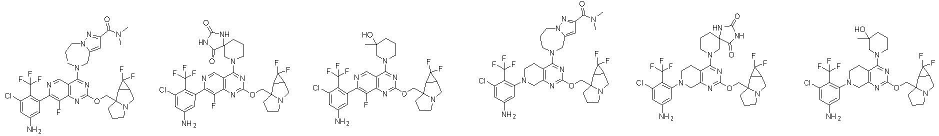

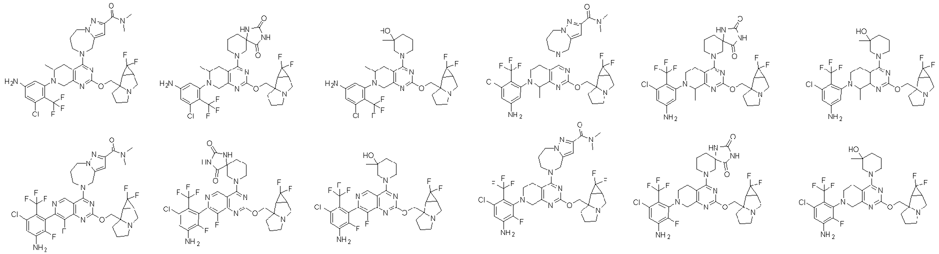

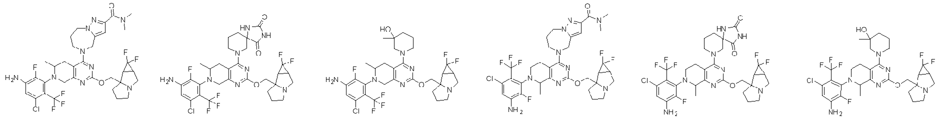

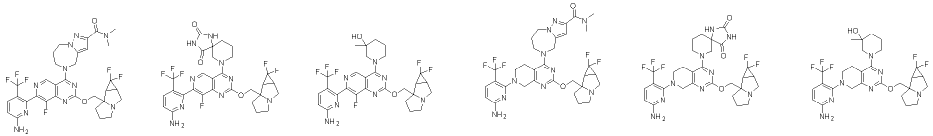

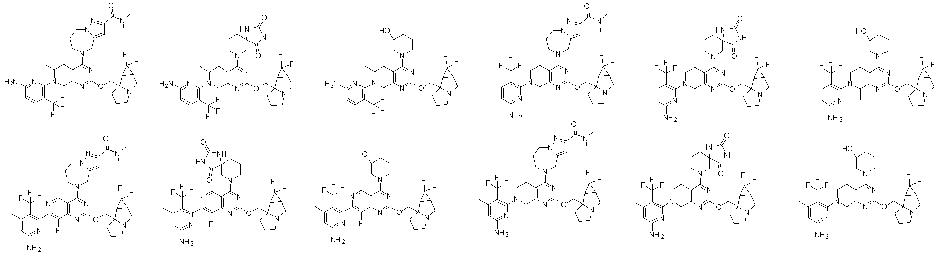

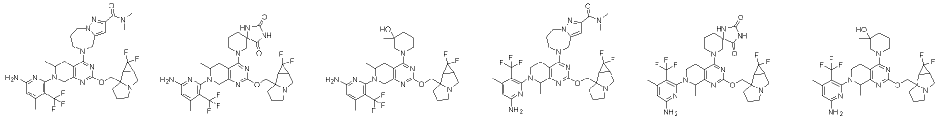

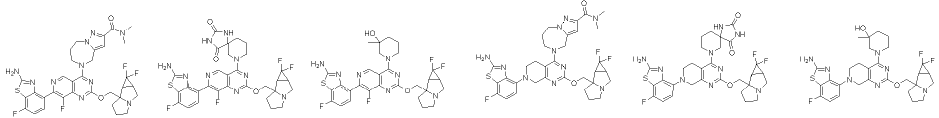

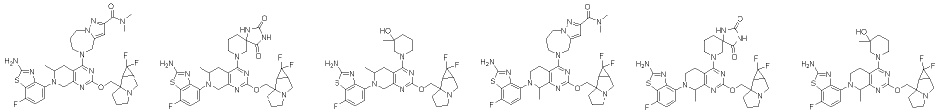

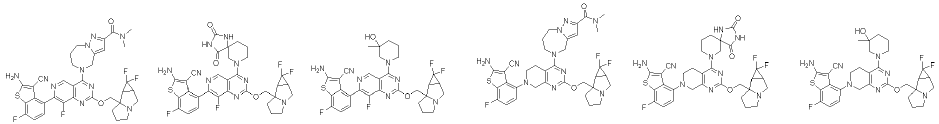

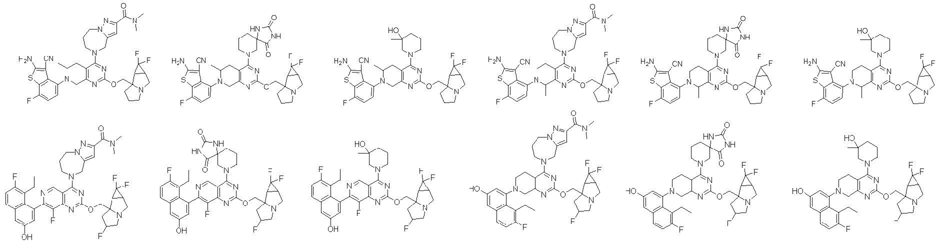

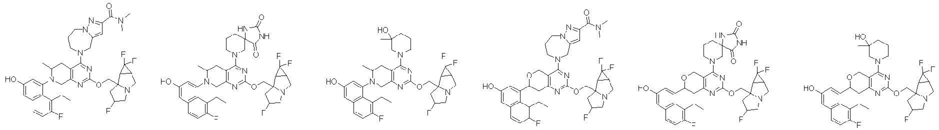

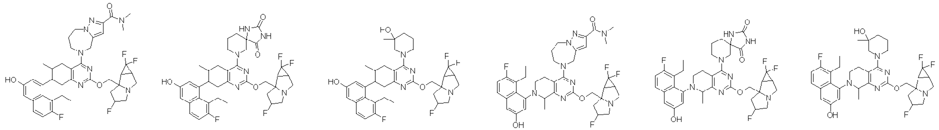

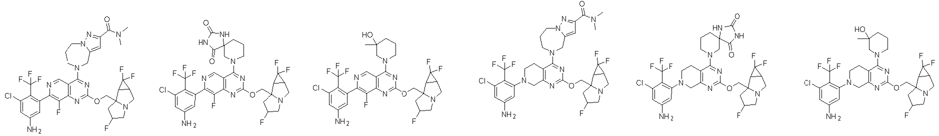

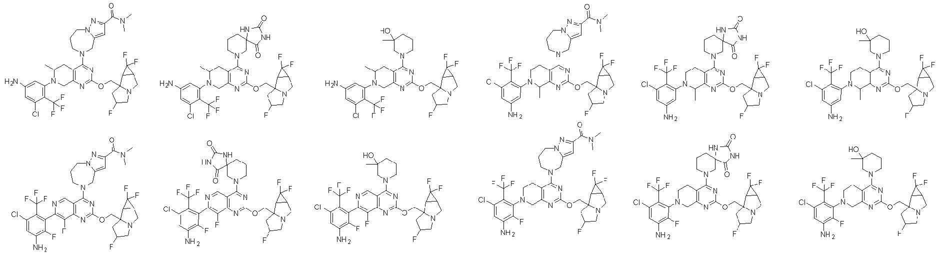

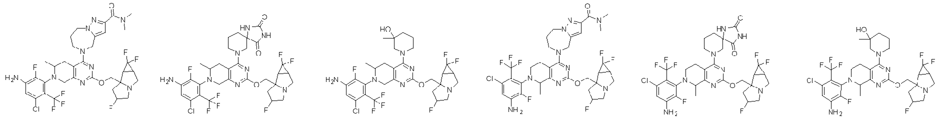

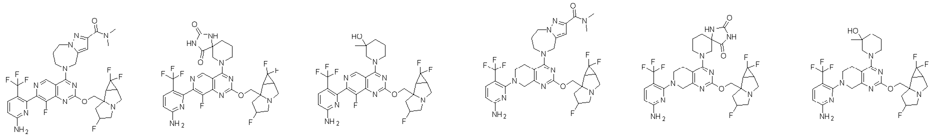

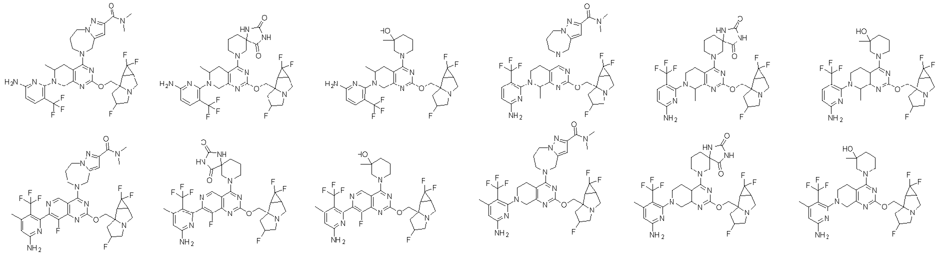

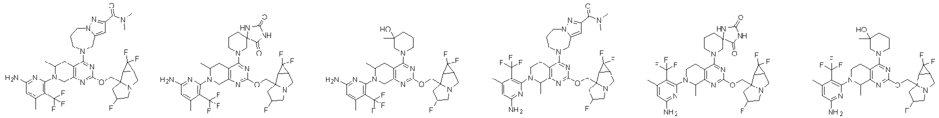

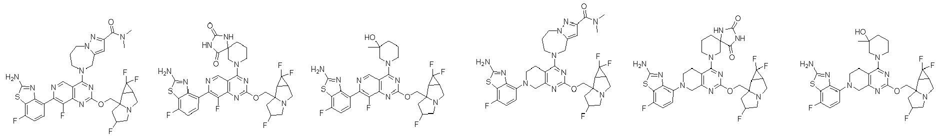

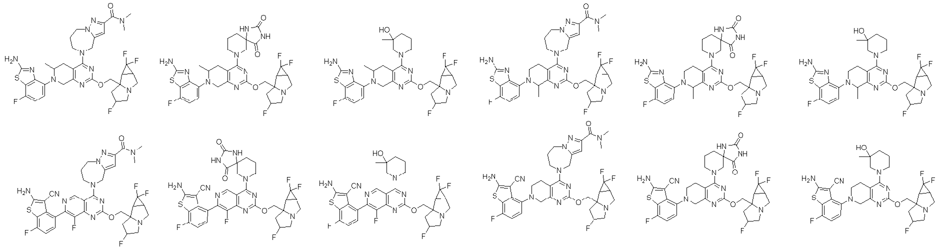

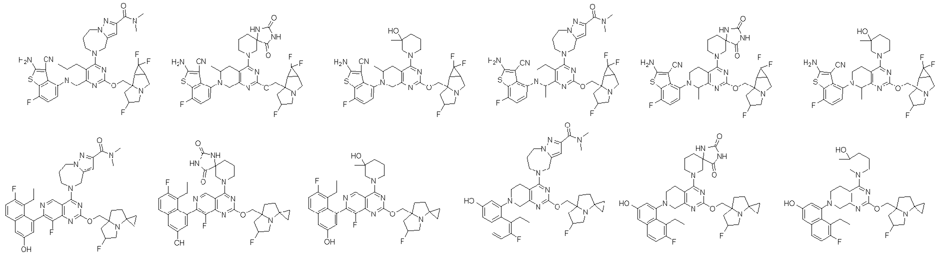

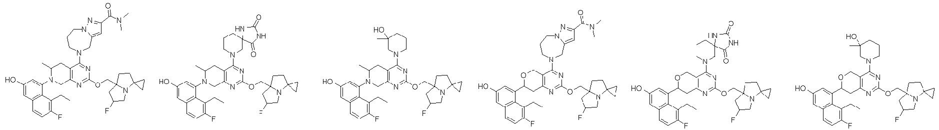

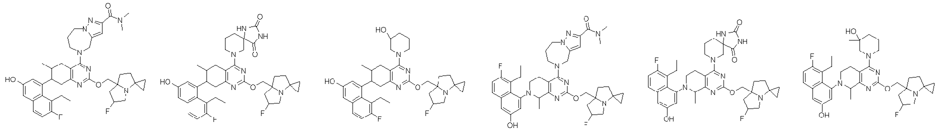

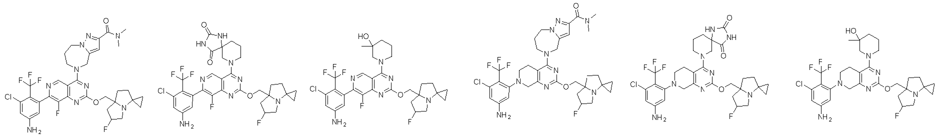

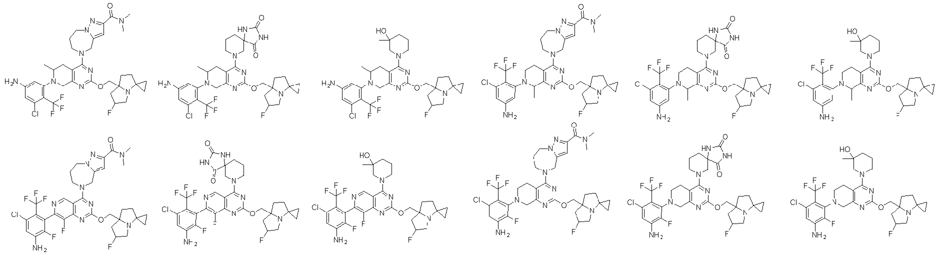

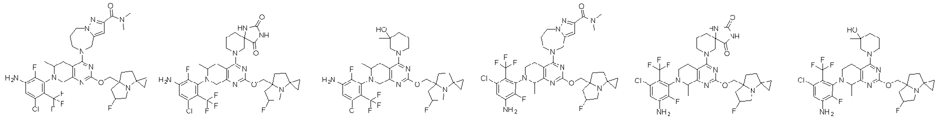

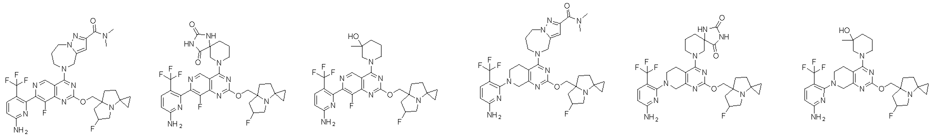

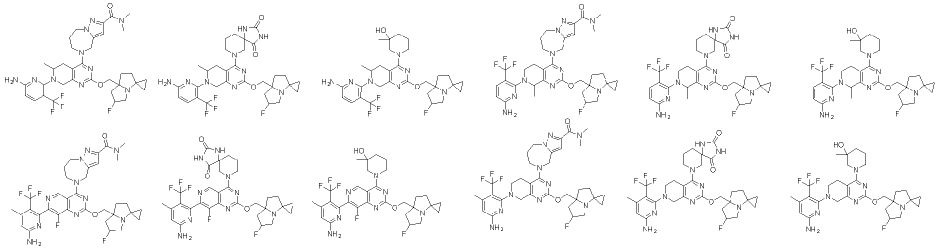

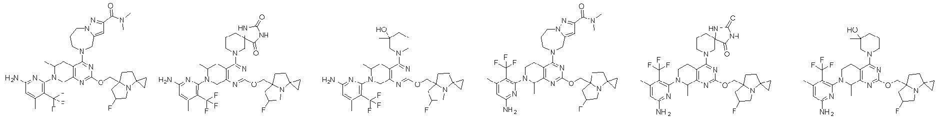

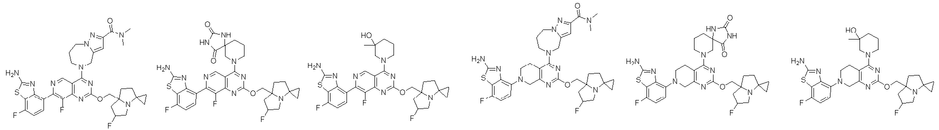

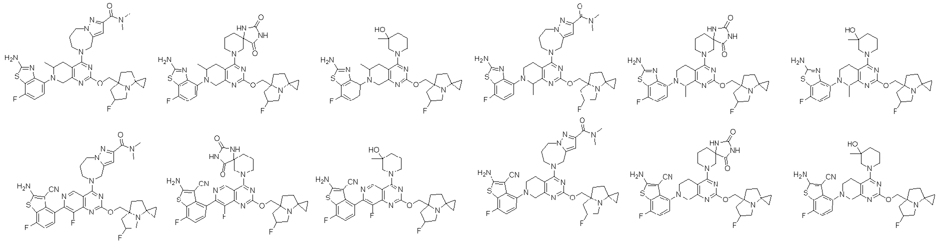

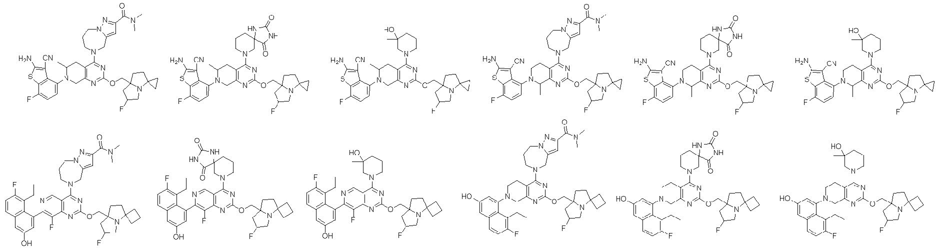

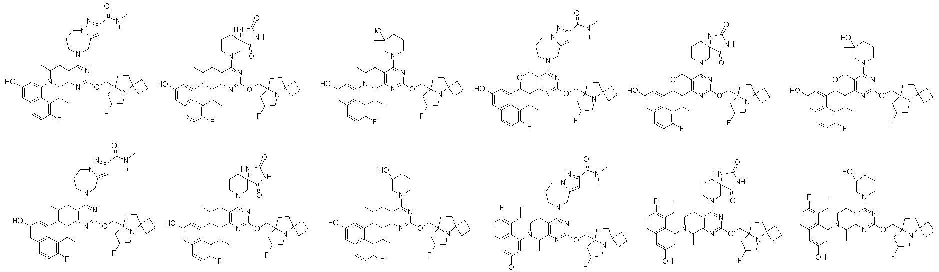

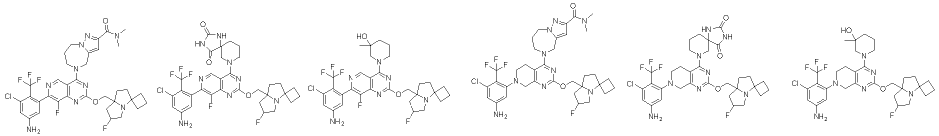

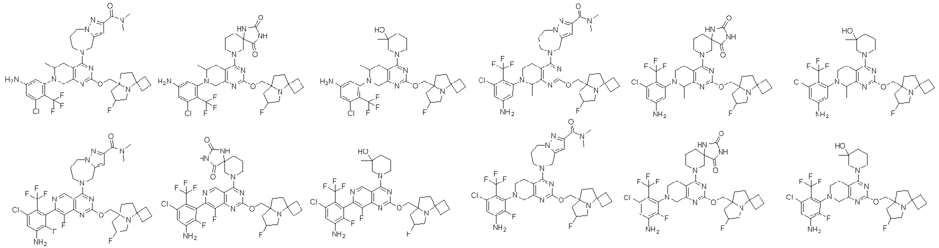

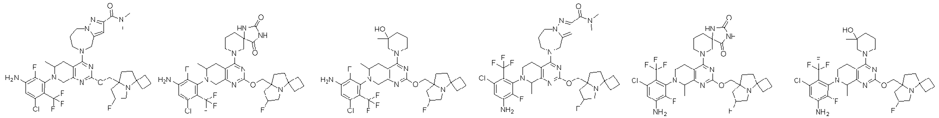

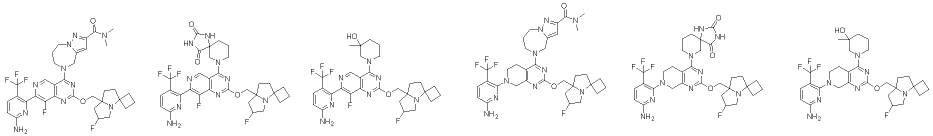

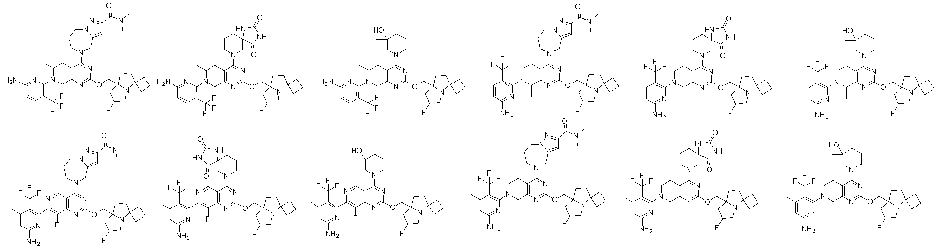

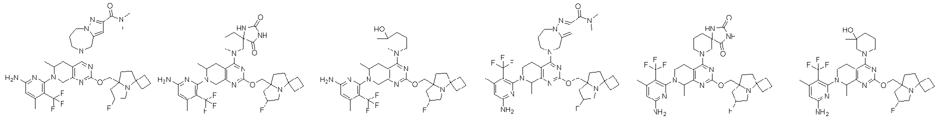

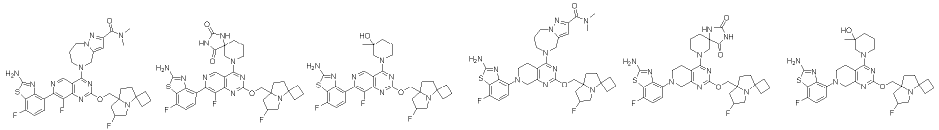

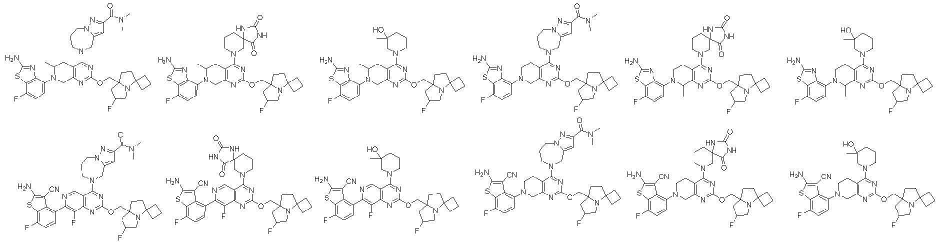

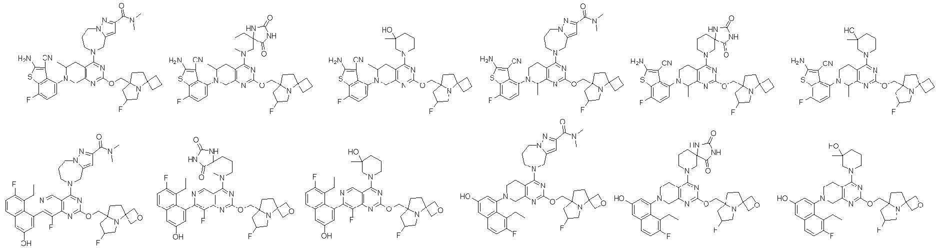

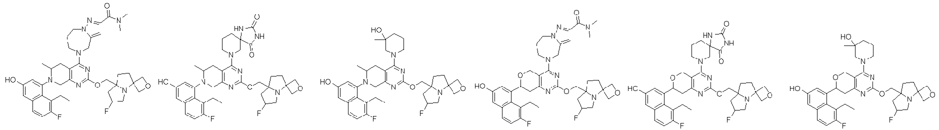

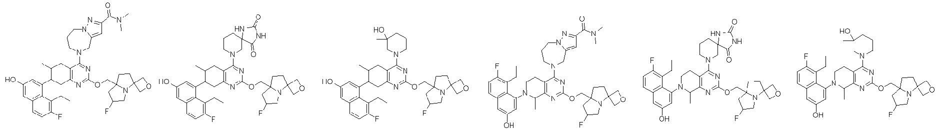

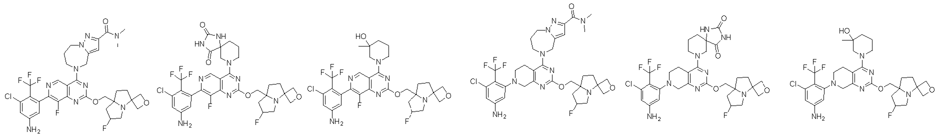

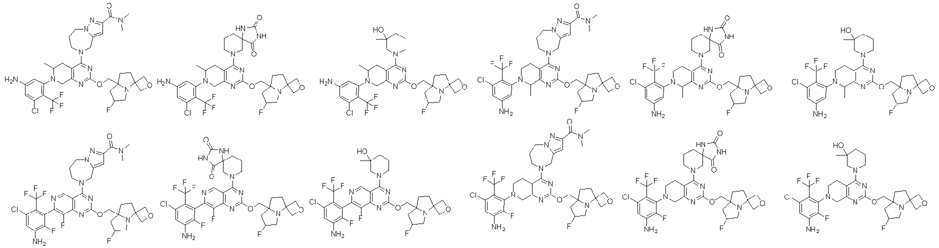

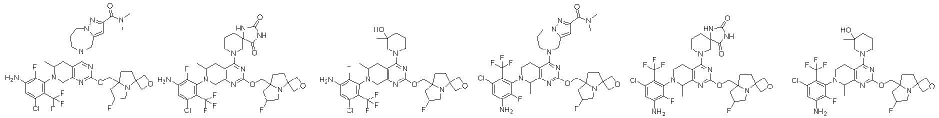

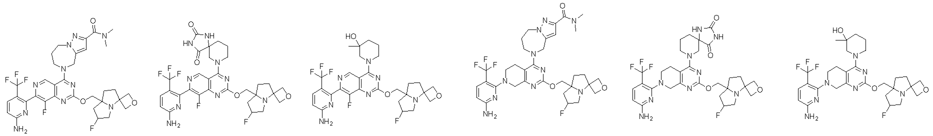

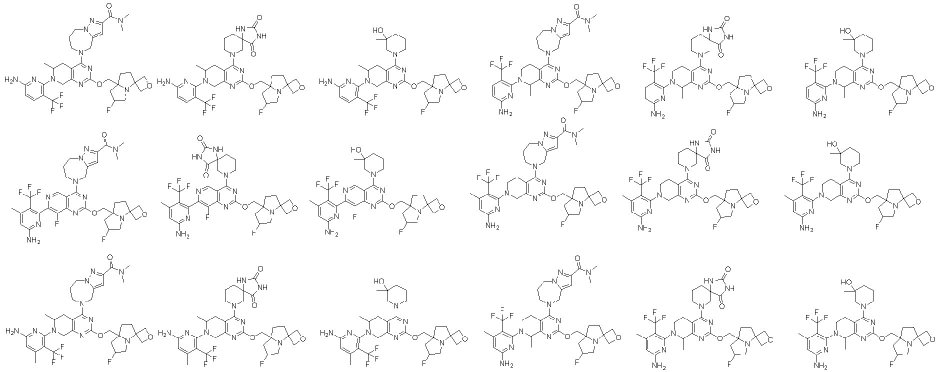

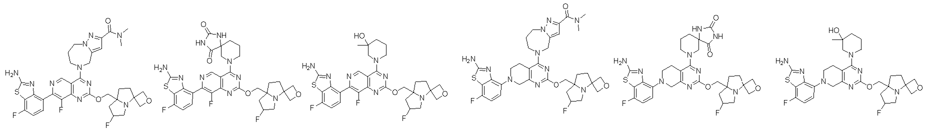

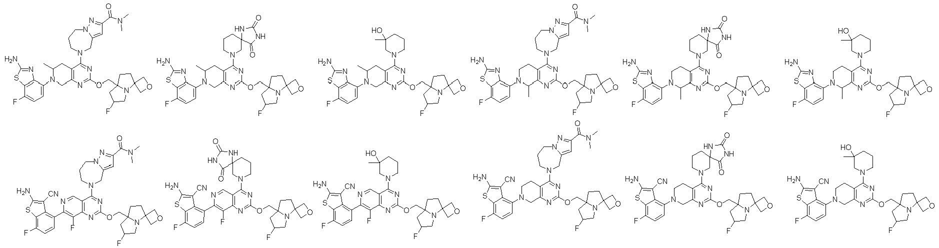

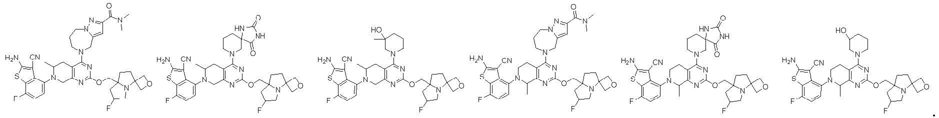

11. A pharmaceutical composition, comprising one or more compounds, or stereoisomers, tautomers, crystalline forms, pharmaceutically acceptable salts, hydrates, solvates or prodrugs thereof according to any one of claims 1-10; and a pharmaceutically acceptable carrier.

12. Use of the compound, or the stereoisomer, the tautomer, the crystalline form, the pharmaceutically acceptable salt, the hydrate, the solvate or the prodrug thereof according to any one of claims 1-10, or the pharmaceutical composition according to claim 11 in the preparation of a medicament for preventing and/or treating a disease associated with the activity or expression level of a KRAS mutation, wherein preferably, the disease is a tumor or a disorder.

13. The use according to claim 12, wherein the disease is selected from the group consisting of: lung cancer, breast cancer, prostate cancer, esophageal cancer, colorectal cancer, bone cancer, kidney cancer, gastric cancer, liver cancer, colon cancer, melanoma, lymphoma, blood cancer, brain tumor, myeloma, soft tissue sarcoma, pancreatic cancer, and skin cancer.
